# EUROPEAN PATENT SPECIFICATION

(11) **EP 4 518 970 B1**
(45) Date of publication and mention of the grant of the patent: **25.03.2026**
(21) Application number: 23729539.9
(22) Date of filing: 03.05.2023
(51) Int. Cl.: A61P 21/00, C07K 16/22, C12Q 1/34, A61K 31/7125, A61K 39/395

(54) **USE OF MYOSTATIN INHIBITOR FOR TREATING SPINAL MUSCULAR ATROPHY**
VERWENDUNG VON MYOSTATIN-HEMMER ZUR BEHANDLUNG VON SPINALER MUSKELATROPHIE
UTILISATION D'UN INHIBITEUR DE LA MYOSTATINE POUR LE TRAITEMENT DE L'AMYOTROPHIE SPINALE

(30) Priority: 04.05.2022 US 202263364188 P; 15.06.2022 US 202263366447 P; 20.06.2022 US 202263366662 P; 10.10.2022 US 202263378987 P
(43) Date of publication of application: 12.03.2025
(73) Proprietor: Scholar Rock, Inc., Cambridge, MA 02142 (US)
(72) Inventor: NOMIKOS, George, Cambridge, Massachusetts 02142 (US); SONG, Guochen, Cambridge, Massachusetts 02142 (US); CHYUNG, Yung, Cambridge, Massachusetts 02142 (US); WEBSTER, Micah T., Cambridge, Massachusetts 02142 (US); KERTESZ, Nathalie, Cambridge, Massachusetts 02142 (US)
(74) Representative: Carpmaels & Ransford LLP
(86) International application number: PCT/US2023/020843
(87) International publication number: WO 2023/215384

(56) References cited:
- WO-A1-2017/218592
- WO-A1-2022/093724
- CRAWFORD THOMAS ET AL: "Apitegromab in Spinal Muscular Atrophy (SMA): An Analysis of Multiple Efficacy Endpoints in the TOPAZ Trial (P15-5.005)", NEUROLOGY, vol. 98, no. 18 Supp., 3 May 2022 (2022-05-03), XP093070187, Retrieved from the Internet <URL:https://n.neurology.org/content/98/18_Supplement/1859> [retrieved on 20230803]
- ZHOU HAIYAN ET AL: "Myostatin inhibition in combination with antisense oligonucleotide therapy improves outcomes in spinal muscular atrophy", JOURNAL OF CACHEXIA, SARCOPENIA AND MUSCLE DEC 2013, vol. 11, no. 3, 7 February 2020 (2020-02-07), pages 768 - 782, XP093070192, ISSN: 2190-5991, Retrieved from the Internet <URL:https://onlinelibrary.wiley.com/doi/full-xml/10.1002/jcsm.12542> [retrieved on 20230803], DOI: 10.1002/jcsm.12542
- ANONYMOUS: "NCT05115110: A Study to Investigate the Safety and Efficacy of RO7204239 in Combination With Risdiplam (RO7034067) in Ambulat Spinal Muscular Atrophy", 1 November 2021 (2021-11-01), XP055903138, Retrieved from the Internet <URL:https://clinicaltrials.gov/ct2/history/NCT05115110?V_1=View#StudyPageTop> [retrieved on 20220318]
- FERRILL F. ROSE ET AL: "Delivery of recombinant follistatin lessens disease severity in a mouse model of spinal muscular atrophy", HUMAN MOLECULAR GENETICS, vol. 18, no. 6, 15 March 2009 (2009-03-15), GB, pages 997 - 1005, XP055403670, ISSN: 0964-6906, DOI: 10.1093/hmg/ddn426
- ANONYMOUS: "Scholar Rock Announces Positive Proof-of-Concept Data from TOPAZ Phase 2 Trial Interim Analysis of SRK-015 in Patients with Type 2 and Type 3 Spinal Muscular Atrophy | Business Wire", 27 October 2020 (2020-10-27), XP055874803, Retrieved from the Internet <URL:https://www.businesswire.com/news/home/20201027005388/en/Scholar-Rock-Announces-Positive-Proof-of-Concept-Data-from-TOPAZ-Phase-2-Trial-Interim-Analysis-of-SRK-015-in-Patients-with-Type-2-and-Type-3-Spinal-Muscular-Atrophy> [retrieved on 20211220]
- PLACE AMY ET AL: "SRK-015: A fully human antibody that blocks cleavage of the Myostatin prodomain A Phase 2 Study to Evaluate the Efficacy and Safety of SRK-015 in Patients with Later-Onset Spinal Muscular Atrophy (TOPAZ): An Introduction Study Design (Continued) Demographics* Disease History* Functional Motor Skills", 26 May 2020 (2020-05-26), XP055874801, Retrieved from the Internet <URL:https://scholarrock.com/wp-content/uploads/2020/09/MDA-2020-TOPAZ-SMA-Introduction.pdf> [retrieved on 20211220]

## Description

### FIELD

The present disclosure relates to therapeutic methods, uses, and compositions comprising an anti-pro/latent myostatin antibody to treat spinal muscular atrophy (SMA) in human patients.

### BACKGROUND

Myostatin, also known as growth differentiation factor 8, abbreviated GDF-8 or GDF8, is a regulator of muscle homeostasis. Mutations that cause loss of myostatin, as well as pharmacological inhibition of myostatin activities, have been shown to increase muscle growth in a number of species, including human. Over the past 15 years, at least 15 different myostatin inhibitor drug candidates, including small molecules and biologics, have been evaluated in human patients aimed to treat various muscle disorders but none to date have succeeded in the clinic (Hanna et al. (2019) Lancet Neurol. 18(9):834-844; Rooks et al. (2020) JAMA Network Open. 3(10):e2020836). Furthermore, most of these inhibitors lacked selectivity and therefore antagonized other related growth factors such as Activin A, raising toxicity concerns. Most of these programs are now discontinued. Thus, in many cases, satisfactory preclinical results have not successfully translated into a safe and effective drug.

In most cases of SMA, a deletion mutation on chromosome 5q13.2 is responsible for causing SMA, however, in a minority of cases, SMA is not linked to a 5q13.2 mutation (Peeters et al. (2014) Brain 137:2879-2896). Non-5q13.2 mutations can result in early onset or late onset forms of SMA with a range of clinical phenotypes. Sequencing methods can detect a variety of genes associated with spinal muscular atrophy, which may present as conditions such as, but not limited to, early onset scapuloperoneal spinal muscular atrophy and Farber disease (Teoh et al. (2017) Neural Plasticity 2017;2017:6509493. doi: 10.1155/2017/6509493. Epub 2017 May 28; Axente et al. (2021) J. Medicine and Life 14(3):424-427).

The SMN1 gene, which is deleted or mutated in SMA, is responsible for the majority of SMN protein production. A second gene (SMN2) that is located near SMN1, is responsible for a small amount of SMN protein production. As SMN protein is critical to the function and survival of motor neurons that control muscle function, the deficiency of SMN protein caused by the deletion or mutation of the SMN1 gene in SMA leads to significant but incomplete loss of the motor neurons in the anterior horn of the spinal cord, ensuring at least some intact innervation. This partial denervation causes substantial atrophy of fast-twitch muscle fibers that in turn leads to motor function impairment and subsequent debilitating muscular atrophy and weakness. Patients' muscles can become so weak that moving, breathing, and eating become difficult.

In some countries, SPINRAZA^{®} (nusinersen) is approved in the treatment of pediatric and adult SMA patients and ZOLGENSMA^{®} (onasemnogene abeparvovec-xioi) is approved for the treatment of pediatric patients less than two years of age with SMA with bi-allelic mutations in the SMN1 gene. EVRYSDI^{™} (risdiplam), a small molecule SMN therapy, is also approved in the United States and in Europe. Nusinersen is an SMN2-directed antisense oligonucleotide (ASO) designed to treat SMA caused by mutations that lead to SMN protein deficiency. Risdiplam works in a similar way and is a pyridazine derivative that modifies the splicing of SMN2 messenger RNA. Onasemnogene abeparvovec-xioi is a recombinant adeno-associated virus vector 9-based gene therapy designed to deliver a copy of the gene encoding the human SMN protein.

SMN therapies (e.g., SMN-directed therapies or SMN-targeted therapies), such as nusinersen, risdiplam, onasemnogene abeparvovec-xioi, and other products in development act primarily upon motor neurons to prevent further loss. Consistent with this notion, clinical data reported from an extended SMN-targeted therapy indicate that after improvement in the first 15 months of treatment with nusinersen, as measured by mean change in HFMSE scores from baseline, the effects appear to level off to a near steady state. A limited further enhancement is observed in motor function over the next three or more years (Darras et al. (2019) Neurology 92(21):e2492-e2506). Similarly, long term evaluation of risdiplam showed only stabilization or minor/variable improvement after 12 months in the primary and secondary endpoints (Oskoui et al. "SUNFISH Part 2: 24-month efficacy and safety of risdiplam in patients with type 2 or non-ambulant type 3 spinal muscular atrophy (SMA)." Presented at MDA Clinical and Scientific Conference 2021; March 15-18. Poster 80). SMN therapies may therefore help maintain motor function over time but may provide limited long-term enhancement in motor function. Although SMN therapies approved for the treatment of SMA have been shown to significantly improve clinical outcomes by providing incremental improvements in motor function and developmental milestones and preventing the worsening of symptoms in SMA, patients may continue to have substantial motor impairment because SMN therapies focus on the motor neuron within the motor unit but do not target the muscle atrophy that has already occurred. Irrespective of when SMN therapy is started, patients who receive these disease-stabilizing therapies after symptom onset continue to have significant unmet medical needs and may not see continued progression of benefit, e.g., over multiple years of treatment. Such patients may benefit from therapies, such as the myostatin inhibition methods disclosed herein, which surprisingly provide durable improvement over longer periods of time.

There are currently no approved muscle-targeted therapies (i.e., muscle-directed therapies or muscle-enhancing therapies) for the treatment of SMA. Consequently, there remains an unmet need for an effective and enduring muscle-targeted therapy that can address muscle atrophy and motor functional impairment in patients with SMA (Day et al. (2022) BMC Pediatrics 22:632).

### SUMMARY

The invention, as defined in the claims, provides apitegromab for use in the treatment of fatigue, impaired bulbar function and/or impaired emptying in a subject having spinal muscular atrophy (SMA) and who receives an SMN therapy, wherein the subject suffers from fatigue, impaired bulbar function and/or impaired emptying as measured by subject or caregiver reported outcomes.

Herein, the terminologies "embodiment", "embodiments", "disclosure", "disclosures" or "disclosed" are to be construed as embodiment(s) or disclosure(s) of the invention only in as far as they comprise all the technical features of the appended claims. The references to methods of treatment of the human or animal body by surgery or therapy herein are to be interpreted as references to apitegromab for use in those methods.

The present disclosure includes, inter alia, therapeutic methods, uses, and compositions for treating SMA patients using a muscle-enhancing agent (i.e., muscle-directed agent or muscle-targeted agent), such as apitegromab, also known as SRK-015. Apitegromab or a composition comprising apitegromab may be used in the treatment of later-onset SMA in a human subject, either as monotherapy or as an adjunct to a motor neuron-directed therapy, such as an SMN upregulator/corrector therapy (i.e., SMN therapy). The data provided herein represent evidence of an enduring effect of a muscle-enhancing agent administered for a period of 24 months to patients with type 2 and type 3 SMA. Apitegromab therapy for treating subjects with SMA is provided herein.

Furthermore, selection of certain SMA patient subpopulations who are particularly likely to benefit from a muscle-enhancing agent, such as a myostatin inhibitor, is disclosed herein. According to the invention, the myostatin inhibitor is a myostatin-selective antibody that binds latent myostatin thereby inhibiting its activation, known as apitegromab. In some embodiments, the myostatin inhibitor binds myostatin and GDF11 but does not bind Activin A.

According to the present disclosure, the certain SMA patient subpopulations who are likely to benefit from a muscle-enhancing agent include: patients who suffer from muscle weakness and/or tightness. According to the invention, the patient suffers from fatigue; difficulty with or impaired bulbar function (e.g., difficulty with coughing, swallowing, and/or feeding); and/or patients who suffer from difficulty with or impaired emptying (e.g., urgency and frequency or urination, bowel changes, etc.).

In some embodiments, the patient has type 2 or type 2-like SMA, wherein, optionally, the patient is 2 years or older. In some embodiments, the patient has non-ambulatory type 3 or type 3-like SMA, wherein, optionally, the patient is 2 years or older. In some embodiments, the patient has ambulatory SMA. In some embodiments, the patient has non-ambulatory SMA. In some embodiments, the patient is less than 2 years old. In some embodiments, the patient is 13-21 years old, wherein, optionally, the patient has non-ambulatory type 2 or type 3 SMA.

According to the present disclosure and the data presented herein, in some embodiments, the therapeutic dose is greater than 2 mg/kg and up to 20 mg/kg of apitegromab, when dosed every 4 weeks (i.e., Q4W) or monthly. In some embodiments, therapeutic doses of less than 20 mg/kg may be used, such as 2 mg/kg, 5 mg/kg, 7.5 mg/kg, 10 mg/kg, 12 mg/kg and 15 mg/kg. In some embodiments, the therapeutic dose is 10 mg/kg. In some embodiments, the therapeutic dose is 20 mg/kg. In some embodiments, the therapeutic dose is 10 mg/kg administered once every four weeks (i.e., Q4W) or once monthly. In some embodiments, the therapeutic dose is 20 mg/kg administered once every four weeks (i.e., Q4W) or once monthly. In various embodiments, apitegromab therapy may improve motor function in patients having later-onset SMA.

Pharmacokinetic (PK) analyses provide the relationship between dosage (e.g., therapeutic dose) and bioavailability (serum exposure) of therapeutics. In some embodiments, the therapeutic dose is a dose that achieves or produces between about 25 and 250 µg/mL of serum exposure to apitegromab, when the serum concentration of apitegromab is measured at trough (C_{trough}) at steady state. The dose achieving this result may be administered by any suitable route, e.g., intravenous or subcutaneous. In some embodiments, the doses described herein are achieved by intravenous administration.

In some embodiments, the therapeutic dose is a dose that achieves or produces up to about 1100 µg/mL of serum exposure, for example, between about 25 and 1100 µg/mL, when the serum concentration of apitegromab is measured at peak (Cₘₐₓ) within about two hours of administration (dosing). The dose achieving this result may be administered by any suitable route, e.g., intravenous or subcutaneous.

In some embodiments, pharmacodynamics (PD) analyses allow the assessment of target engagement, i.e., the assessment of the therapeutic antibody binding to pro/latent myostatin, as measured by serum concentrations of latent myostatin (LM). In some embodiments, the therapeutic dose is a dose that achieves or produces in a subject at least about 100 or preferably at least about 250 ng/mL of serum concentrations of latent myostatin, preferably measured at steady state, e.g., 14 days after administration of apitegromab or later. The dose achieving this result may be administered by any suitable route, e.g., intravenous or subcutaneous. For example, the serum concentration of latent myostatin may be about 250 ng/mL or above, 400 ng/mL or above 550 ng/ml or above 700 ng/ml or above 950 ng/ml or above 1100 ng/ml, etc.

In some embodiments, a PD analysis that allows the assessment of target engagement comprises measuring a serum concentration of creatinine. In some embodiments, a therapeutic dose of a treatment comprising apitegromab is a dose that achieves or produces in a subject at least 10% (e.g., at least 20%, at least 25%, at least 30%, or more) increase in a serum concentration of creatinine as compared to a concentration before the start of treatment. In some embodiments, serum concentrations of creatinine are measured at steady state, e.g., at least two weeks (e.g., at least three weeks, at least four weeks, or at least one month) after starting treatment. In some embodiments, treatment with apitegromab comprises administering an amount and/or dosing regimen sufficient to obtain a 10% or greater (e.g., at least 20%, at least 25%, at least 30%, or more) increase in serum creatinine, when measured before and after treatment, e.g., before treatment and at steady state after starting treatment.

Apitegromab or another selective myostatin inhibitor may be used to treat SMA either alone (e.g., monotherapy) or in conjunction with another therapy, such as an SMN therapy or SMN-targeted therapy, including SMN upregulator or corrector therapy (e.g., add-on/adjunct therapy or combination therapy). In some embodiments, the subject is treated with an SMN upregulator therapy such as nusinersen (SPINRAZA^{®}) or risdiplam (EVRYSDI^{®}). In some embodiments, the subject is treated with an SMN corrector therapy such as an SMN gene therapy, e.g., onasemnogene abeparvovec-xioi (ZOLGENSMA^{®}). In some embodiments, the subject initiated the SMN therapy at or after the age of five. In some embodiments, the neuronal directed therapy increases progranulin, maintaining neuronal viability.

In some embodiments, an SMN therapy (e.g., an SMN-targeted therapy, e.g., an SMN upregulator/corrector therapy) and apitegromab therapy (e.g., muscle-targeted therapy) are used as a combination, or add-on, or adjunct therapy. Thus, an SMN therapy and apitegromab may be used in the treatment of SMA in a patient, wherein the treatment comprises administration of the SMN therapy and the apitegromab in amounts sufficient to treat SMA, wherein the apitegromab therapy is intravenously administered to the patient at a dose of greater than 2 mg/kg and up to 20 mg/kg every four weeks or monthly. In some embodiments, the SMN therapy is an SMN1-directed gene therapy. In some embodiments, the SMN therapy is an SMN upregulator therapy such as an SMN2-directed therapy, wherein optionally the SMN2-directed therapy is a splice modifier. In some embodiments, an SMN corrector may be administered orally, intrathecally or intravenously. In some embodiments, the patient has later-onset SMA.

In some embodiments, the present disclosure provides a therapy comprising apitegromab and an SMN therapy (e.g., an SMN upregulator or corrector therapy, e.g., nusinersen, risdiplam, and or onasemnogene abeparvovec-xioi) for use in the treatment of SMA in a patient 2 years or older with type 2 or type 3 SMA, wherein the treatment comprises administration of the SMN therapy and the apitegromab in amounts sufficient to treat SMA, wherein the apitegromab therapy is intravenously administered to the patient at a dose of greater than 2 mg/kg and up to 20 mg/kg every four weeks or monthly.

In some embodiments, the present disclosure provides a therapy comprising apitegromab and an SMN therapy (e.g., an SMN upregulator or corrector therapy, e.g., nusinersen, risdiplam, and or onasemnogene abeparvovec-xioi) for use in the treatment of SMA in a patient with ambulatory SMA, wherein the treatment comprises administration of the SMN therapy and the apitegromab in amounts sufficient to treat SMA, wherein the apitegromab therapy is intravenously administered to the patient at a dose of greater than 2 mg/kg and up to 20 mg/kg every four weeks or monthly.

In some embodiments, the present disclosure provides a therapy comprising apitegromab and an SMN therapy (e.g., an SMN upregulator or corrector therapy, e.g., nusinersen, risdiplam, and or onasemnogene abeparvovec-xioi) for use in the treatment of SMA in a patient aged 13-21 years with non-ambulatory type 2 or type 3 SMA, wherein the treatment comprises administration of the SMN therapy and the apitegromab in amounts sufficient to treat SMA, wherein the apitegromab therapy is intravenously administered to the patient at a dose of greater than 2 mg/kg and up to 20 mg/kg every four weeks or monthly.

In some embodiments, the present disclosure provides a therapy comprising apitegromab and an SMN therapy (e.g., an SMN upregulator or corrector therapy, e.g., nusinersen, risdiplam, and or onasemnogene abeparvovec-xioi) for use in the treatment of SMA in a patient under the age of 2 years, wherein the treatment comprises administration of the SMN therapy and the apitegromab in amounts sufficient to treat SMA, wherein the apitegromab therapy is intravenously administered to the patient at a dose of greater than 2 mg/kg and up to 20 mg/kg every four weeks or monthly.

In some embodiments, the patient has two copies of the SMN2 gene. In some embodiments, the patient has three copies of the SMN2 gene. In some embodiments, the patient has four copies of the SMN2 gene. In some embodiments, the patient has five copies of the SMN2 gene. In some embodiments, the patient has six copies of the SMN2 gene.

In some embodiments, the patient commences the combination therapy at age five or older. In some embodiments, the patient commences the combination therapy before the age of five. In some embodiments, the patient commences the combination therapy before the age of two. In some embodiments, the patient commences the combination therapy before the age of six weeks. In some embodiments, the patient is diagnosed with SMA (e.g., identified as a carrier of an SMN1 mutation) by genetic screening, wherein optionally the genetic screening is a newborn screening, or in utero screening, e.g., for SMN1 mutation(s).

In some embodiments, apitegromab therapy is used as an add-on or adjunct therapy to treat SMA. Thus, a composition comprising apitegromab may be used in the treatment of later-onset SMA in a patient, wherein the treatment comprises intravenous administration of the composition comprising a therapeutic dose of apitegromab, wherein the therapeutic dose is greater than 2 mg/kg and up to 20 mg/kg every four weeks or monthly, and wherein the patient is treated with an SMN therapy. In some embodiments, the SMN therapy is an SMN1-directed therapy, wherein optionally the SMN1-directed therapy is a gene therapy. In some embodiments, the SMN therapy is an SMN2-directed therapy, wherein optionally the SMN2-directed therapy is an SMN upregulator therapy, e.g., a splice modifier. In some embodiments, any of the SMN therapies may be administered orally, intrathecally, or intravenously. In some embodiments, the patient has type 2 or type 2-like SMA. In some embodiments, the patient has non-ambulatory type 3 or type 3-like SMA. In some embodiments, the patient has ambulatory type 3 or type 3-like SMA. In some embodiments, the patient has two copies of the SMN2 gene. In some embodiments, the patient has three copies of the SMN2 gene. In some embodiments, the patient has four copies of the SMN2 gene. In some embodiments, the patient has five copies of the SMN2 gene. In some embodiments, the patient has six copies of the SMN2 gene. In some embodiments, the patient commences the SMN corrector therapy before the age of five. In some embodiments, the patient commences the SMN corrector therapy at age five or older. In some embodiments, the patient is diagnosed with SMA (e.g., identified as a carrier of an SMN1 mutation) by genetic screening, wherein optionally the genetic screening is a newborn screening or an in utero screening. In some embodiments, the patient is treated with the SMN corrector prior to apitegromab therapy. In some embodiments, the patient is treated with apitegromab prior to receiving the SMN corrector therapy.

SMA patients who may benefit from an apitegromab therapy include those who meet one or more of the following criteria: has a documented diagnosis of 5q SMA and later-onset (and/or type 2, type 2-like, type 3 or type 3-like) SMA prior to receiving a therapy for SMA; has SMA that is not caused by a 5q mutation; non-ambulatory subjects who are able to sit independently per WHO motor milestones definition; ambulatory subjects who are able to independently ambulate without aids over 10 meters in 30 seconds or less; subjects having a Revised Hammersmith Scale (RHS) score of less than or equal to 63 and/or a Hammersmith Functional Motor Scale Expanded (HFSME) score of 10 or greater; subjects who do not use tracheostomy with positive pressure or chronic daytime non-invasive ventilatory support for greater than 16 hours daily within two weeks prior to treatment; subjects who do not have any acute or comorbid condition interfering with the well-being of the subject within two weeks prior to treatment; subjects who do not have severe scoliosis or contractures; and/or subjects who do not use systemic corticosteroids, valproic acid, or therapies with potential muscular or neuromuscular effects within 60 days except approved SMN- targeted therapy, e.g., SMN upregulator (also known as SMN corrector) therapy. Therapies with potential muscular or neuromuscular effects include androgens, insulin-like growth factor, growth hormone, systemic beta-agonist, botulinum toxin, muscle relaxants, muscle enhancing supplements or acetylcholinesterase inhibitors. In some embodiments, the patient has a documented diagnosis of 5q SMA and later-onset (and/or type 2, type 2-like, type 3, or type 3-like) SMA prior to receiving a therapy for SMA and meets one or more of the additional criteria listed above. In various embodiments, the methods disclosed herein include the selection of such a patient or patient population(s) for treatment with apitegromab, e.g., according to a dosage or regimen disclosed herein.

SMA patients who may benefit from an apitegromab therapy include those who meet one or more of the following criteria: has 1, 2, 3 or 4 copies of the SMN2 genes and achieves one or more of the gross motor milestones according to the World Health Organization (WHO) motor development scale: 1) sitting up without support (e.g., head erect for at least 10 seconds; no use of arms or hands to balance); 2) crawling on hands and knees (e.g., at least three movements in a row and stomach does not touch supporting surface); 3) standing with assistance (e.g., upright on both feet for at least 10 seconds without leaning on any object); 4) walking with assistance (e.g., takes at least five steps holding a stable object); 5) standing without support (e.g., at least 10 seconds with no contact with person or object); and, 6) walking without support (e.g., takes at least five steps independently).

SMA patients who may benefit from an apitegromab therapy include those who meet one or more of the following criteria: has 1, 2, 3 or 4 copies of the SMN2 genes and achieves one or more of the gross motor milestones according to HFMSE: 1) neck holding (e.g., while laying on their stomach able to raise or hold head); 2) rolls over; 3) sits in tripod position (e.g., uses hands to support self while sitting); 4) sits without support; 5) stands with support; 6) creeps/crawls; 7) pulls to standing position (e.g., pulls to stand and cruises along furniture); 8) stands without support; 9) takes several independent steps but falls; 10) walks alone (e.g., walks independently, walks without support); 11) squats to pick up an object (e.g., a toy); 12) walks/creeps up and down the stairs; 13) jumps; 14) alternates feet going upstairs; 15) hops on one foot; 16) alternates feet going downstairs.

In some embodiments, a patient treated with apitegromab as disclosed herein has received or is treated with an SMN-targeted therapy (also referred to as an SMN upregulator or corrector), such as nusinersen, risdiplam, or onasemnogene abeparvovec-xioi. In some embodiments, a patient initiates the SMN upregulator (corrector) therapy before the age of five. In some embodiments, the patient initiates the SMN upregulator (corrector) therapy at or after the age of five. In some embodiments, an SMN corrector therapy is an SMN2 upregulator therapy. In some embodiments, an SMN corrector therapy is an SMN1 gene therapy.

In some embodiments, a patient receives apitegromab therapy for at least six months (e.g., 6 months, 12 months, 24 months, or longer) at a therapeutic dose that is sufficient to achieve a clinical benefit characterized by motor function improvement, disease stabilization, and/or delay in disease progression.

In some embodiments, a patient who receives apitegromab therapy may attain improvement in motor function. Motor function improvement may correspond to an increase in an HFMSE score or RHS score. For example, the patient may achieve an increase of at least 1 point, 2 points, 3 points, 4 points, 5 points or more in the HFMSE score over a baseline after 6 months, 12 months, or 24 months of treatment with apitegromab (i.e., apitegromab therapy). In some embodiments, 12 months of apitegromab therapy may produce 3 points or greater increase (e.g., at least 3 points, at least 5 points, at least 10 points, up to about 20 points) in the HFMSE score over baseline in patients who had initiated a background SMN-targeted therapy at an early age. In some embodiments, improvements in the HFMSE score are additive and may be synergistic with background therapy, e.g., background SMN upregulator/corrector therapy. In some embodiments, 24 months of apitegromab therapy may produce 3 points or greater increase (e.g., at least 3 points, at least 5 points, at least 10 points, up to about 20 points) in the HFMSE score over baseline in patients who had initiated a background SMN therapy at an early age (e.g., less than 2 years of age). In some embodiments, improvements in the HFMSE score are additive and may be synergistic with background therapy, e.g., background SMN upregulator/corrector therapy.

In some embodiments, a patient who receives apitegromab therapy may have disease stabilization. Disease stabilization may correspond to a net zero (e.g., at least no change or an increase) or near-zero change in an HFMSE score or RHS score over a baseline, e.g., for at least 24 months. In some embodiments, this is a clinically meaningful outcome over an untreated patient population (e.g., natural history), or one treated with prior art methods (e.g., background therapy), in which a gradual decline in motor function is expected.

In some embodiments, a patient who receives apitegromab therapy may have a delay in disease progression. Delay in disease progression may include, for example, a slower rate of decrease in an HFMSE score over time, e.g., after at least 12 or 24 months of treatment, as compared to a suitable control (e.g., an untreated patient exhibiting the natural history of the particular patient population). In some embodiments, delay may include a later transition from ambulatory to non-ambulatory SMA.

In some embodiments, apitegromab is able to increase a treatment response rate in a patient population, relative to control that does not receive apitegromab.

In any of the embodiments, a therapeutically effective amount of apitegromab does not cause serious adverse events in patients following twelve months of treatment or following 24 months of treatment.

The present disclosure is based, at least in part, on the finding that an anti-pro/latent myostatin antibody capable of selectively inhibiting the activation of latent myostatin can improve muscle function in human patients with SMA, including SMA patients who may or may not be on a background SMN upregulator therapy (e.g., nusinersen, risdiplam, or onasemnogene abeparvovec-xioi), and do so in some cases to a surprising degree that is typically not expected or observed in the particular patient population, while avoiding adverse events (compare, for example, to Mercuri et al. (2018) New Engl J Med 378(7):625-635). Accordingly, the present disclosure provides various embodiments of methods, uses, and compositions comprising an anti-pro/latent myostatin antibody for treating SMA in a human subject. Moreover, based on the data presented herein demonstrating clinical benefit of selective myostatin inhibition for treating a neuromuscular disease, the present disclosure also encompasses the notion that other selective myostatin inhibitors may also be used in a similar fashion. These may include, for example, neutralizing antibodies capable of selectively inhibiting myostatin, but that spare other related growth factors such as Activin A, and ligand traps engineered to preferentially bind myostatin.

In some embodiments, a therapeutically effective amount of the apitegromab of greater than 2 and up to 20 mg/kg achieves one or more of the following in the subject: retaining motor function as compared to a decline in a control, delaying disease progression, delaying or preventing an ambulatory type 3 SMA patient from becoming non-ambulatory, delaying or preventing need for respiratory aid or intervention, reducing the rate of a decline in one or more motor function scores as compared to a control, and/or maintaining at least net zero change in one or more motor function scores as compared to a baseline. In some embodiments, the amount is a dose of greater than 2 and up to 20 mg/kg apitegromab intravenously administered every four weeks or monthly. In some embodiments, a mean decline from baseline may be observed in this patient population but the majority of patients show disease stabilization (no change or increase in RHS).

In some embodiments, the present disclosure provides a method of treating SMA in a human subject, comprising administering to the subject a composition comprising apitegromab and a composition comprising an SMN-targeted therapy (e.g., SMN upregulator), wherein the subject is administered apitegromab by intravenous infusion every four weeks or monthly for at least six months or 12 months in an amount greater than 2 and up to 20 mg/kg sufficient to produce an at least one point mean increase in an HFMSE score relative to a baseline score prior to treatment, e.g., in a cohort of at least 14 subjects.

In some embodiments, the present disclosure provides a composition comprising apitegromab for use in treating SMA in a human subject receiving an SMN upregulator, wherein the subject is administered the apitegromab by intravenous infusion every four weeks or monthly for at least six months in an amount greater than 2 and up to 20 mg/kg sufficient to produce at least a one point mean increase in an HFMSE score relative to a baseline score prior to treatment, e.g., in a cohort of at least 14 subjects.

In some embodiments, the composition comprising apitegromab for use in treating SMA in a human subject receiving an SMN treatment is administered intravenously at a health care facility. In some embodiments, the composition comprising apitegromab for use in treating SMA in a human subject receiving an SMN treatment is administered intravenously at a place other than a health care facility, e.g., in the subject's home. In some embodiments, the composition comprising apitegromab for use in treating SMA in a human subject receiving an SMN treatment is administered intravenously in the subject's home by a health care worker, e.g., a health care professional. In some embodiments, the composition comprising apitegromab for use in treating SMA in a human subject receiving an SMN upregulator is administered subcutaneously in the subject's home, e.g., by a health care worker, e.g., a health care professional. In some embodiments, the composition comprising apitegromab is administered subcutaneously by the patient.

In some embodiments, the SMA is later-onset SMA. In some embodiments, the SMA is type 2 SMA or type 2-like SMA. In some embodiments, the SMA is non-ambulatory type 3 SMA or type 3-like SMA. In some embodiments, the subject is 5 to 21 years old. In some embodiments, the SMN upregulator therapy is nusinersen, risdiplam, and/or onasemnogene abeparvovec-xioi. In some embodiments, the SMN upregulator therapy is nusinersen. In some embodiments, the SMN upregulator therapy is risdiplam. In some embodiments, the sufficient amount is an intravenous dose of greater than 2 and up to 20 mg/kg, optionally about 5, 7.5, 10, 15, or 20 mg/kg. In some embodiments, the therapeutic dose of apitegromab is sufficient to increase one or more points in HFMSE in a patient, e.g., in a majority of a patient population (e.g., over 50%, such as over 60%), and/or, increase three or more points in HFMSE in a patient, e.g., in at least 20% of a patient population (e.g., at least 25%).

In some embodiments, the SMA is later-onset SMA, wherein the later-onset SMA is type 2 SMA or type 2-like SMA, in which the patient-initiated background SMN corrector therapy at an early age (e.g., before the age of five). Apitegromab therapy may achieve significant motor function improvements (e.g., a five point or greater increase in HFMSE scores) in this patient population. In some embodiments, the patient attains a 3+ point increase, 5+ point increase, or 10+ point increase at 12 months of apitegromab therapy in HFMSE over baseline, wherein the baseline is measured prior to or at the time of the first administration of apitegromab. In some embodiments, the patient attains a 3+ point increase, 5+ point increase, or 10+ point increase at 24 months of apitegromab therapy in HFMSE over baseline, wherein the baseline is measured prior to or at the time of the first administration of apitegromab. In some embodiments, the patient attains greater HFMSE increase over baseline at 24 months of apitegromab treatment as compared to at 12 months of apitegromab treatment.

In some embodiments, the subject is a patient who has not gained the ability to sit without help at age 4-9 months, based on the WHO Motor Developmental Milestones classification. In some embodiments, the subject is a patient who has the ability to sit without help at age 4-9 months. In some embodiments, the subject is a patient who has not gained the ability to stand with help at age 5-11 months. In some embodiments, the subject is a patient who has the ability to stand with help at age 5-11 months. In some embodiments, the subject is a patient who has not gained the ability to crawl on hands and knees at age 5-13 months. In some embodiments, the subject is a patient who has the ability to crawl on hands and knees at age 5-13 months. In some embodiments, the subject is a patient who has not gained the ability to walk with help at age 6-14 months. In some embodiments, the subject is a patient who has the ability to walk with help at age 6-14 months. In some embodiments, the subject is a patient who has not gained the ability to stand alone at age 7-14 months. In some embodiments, the subject is a patient who has the ability to stand alone at age 7-14 months. In some embodiments, the subject is a patient who has not gained the ability to walk alone at age 8-18 months. In some embodiments, any of the above-mentioned patients who are unable to walk can also be characterized as having type 2, type 2-like, type 3 or type 3-like SMA. In some embodiments, any of the above-mentioned patients who are able to walk (with or without assistance) can also be characterized as having type 3 or type 3-like SMA. In some embodiments, the subject initiated SMN upregulator/corrector therapy before the age of five. In some embodiments, the subject initiated SMN upregulator/corrector therapy after the age of five. In some embodiments, any of the above-mentioned patients may, after 6-12 months treatment with apitegromab, gain one or more new milestone(s) according to the WHO Motor Developmental Milestones. In some embodiments, any of the above-mentioned patients may, after 6-24 months treatment with apitegromab, gain one, two, or three new milestones according to the WHO Motor Developmental Milestones, e.g., one or more of ability to walk independently, ability to stand independently, standing with assistance, hands and knees crawling, and/or walking with assistance.

In some embodiments, the disclosure provides a method of treating SMA in a patient, the method comprising administering to the patient a therapeutically effective amount of apitegromab, wherein the therapeutically effective amount is an amount sufficient to achieve one or more of the WHO Motor Developmental Milestones after 12, 24, or 36 months of treatment, wherein the patient is unable to perform the one or more WHO Motor Developmental Milestones at baseline.

In some embodiments, a target patient population to be treated with apitegromab includes those who are 12 years old or younger at the time of starting apitegromab therapy. In some embodiments, a target patient population includes those who are 2 years or older. In some embodiments, a target patient population includes those who are between 2-12 years of age. In some embodiments, a target patient population includes those who are between 2-5 years of age.

The present disclosure provides use of apitegromab in the manufacture of a pharmaceutical composition (e.g., a medicament) for the treatment of later-onset SMA in a human subject. The medicament is intended for administration to the subject at a dose of more than 2 mg/kg and up to 20 mg/kg of apitegromab every four weeks or monthly, wherein optionally the subject initiated a motor neuron-directed therapy for SMA before the age of five, and wherein the motor neuron-directed therapy increases SMN1 or SMN2 expression. The manufacture process may include providing a cell line comprising a vector or vectors with nucleic acid sequences of the heavy chain and the light chain of apitegromab immunoglobulin polypeptides, capable of producing a recombinant antibody corresponding to apitegromab, or antigen-binding fragment thereof. Such a cell line can be used to produce apitegromab in a cell culture, such as mammalian cell culture, e.g., CHO cells. A large-scale (e.g., 250L, 1000L, 2000L, 3000L, 4000L, etc.) bioreactor may be employed to produce apitegromab. The recombinant antibody molecules may then be purified from the cell culture, and the purified antibody formulated into a pharmaceutical composition comprising apitegromab and one or more excipients. The process typically includes a sterile filtration step. In some embodiments, the pharmaceutical composition is a liquid formulation containing about 50 mg/mL apitegromab, suitable for intravenous administration.

In some embodiments, apitegromab is contained in a multidose vial, such as a glass vial. In some embodiments, a container containing apitegromab therein (such as glass vial) is part of a kit. In some embodiments, apitegromab is contained in a pre-filled syringe.

### BRIEF DESCRIPTION OF THE FIGURES

**FIG. 1** shows HFMSE score following 15 months of nusinersen treatment.
**FIG. 2A** shows the mean change over baseline in HFSME (95% CI) over 24 months.
**FIG. 2B** shows the mean change in RULM (95% CI) over 24 months.
**FIG. 3A** shows the correlation between HFSME and RULM at 12 months of apitegromab treatment. **FIG. 3B** shows the correlation between HFSME and RULM at 24 months of apitegromab treatment.
**FIG. 4** shows mean change of HFMSE scores (left panel) and RULM scores (right panel) over 24 months of apitegromab treatment.
**FIG. 5** shows the relationship between the change in HFMSE score from baseline and baseline age in Type 2 and non-ambulatory Type 3 SMA patients.
**FIG. 6A** shows a correlation of change in HFMSE from baseline and the ratio of latent myostatin concentration and age.
**FIG. 6B** shows a correlation of change in HFMSE from baseline and the ratio of latent myostatin fold-change and age.
**FIG. 7** shows PEDI-CAT scores of Type 2 and non-ambulatory Type 3 SMA patients.
**FIG. 8** shows PROMIS scores in Type 2 and non-ambulatory Type 3 SMA patients.
**FIG 9A** shows muscle function severity of Type 2 and Type 3 SMA patients.
**FIG 9B** shows fatigue severity of Type 2 and Type 3 SMA patients.
**FIG 9C** shows bulbar function severity of Type 2 and Type 3 SMA patients.
**FIG 9D** shows emptying severity of Type 2 and Type 3 SMA patients.
**FIG 9E** shows daily living severity of Type 2 and Type 3 SMA patients.
**FIG 9F** shows social impact severity of Type 2 and Type 3 SMA patients.
**FIG. 10A** shows the relationship between change in muscle volume and change in serum creatinine in non-human primates treated with apitegromab.
**FIG. 10B** shows percent change of serum creatinine in healthy human volunteers treated with apitegromab.

### DETAILED DESCRIPTION OF CERTAIN EMBODIMENTS

The invention is as defined in the claims.

### Definitions

In order that the disclosure may be more readily understood, certain terms are first defined. These definitions should be read in light of the remainder of the disclosure and as understood by a person of ordinary skill in the art. Unless defined otherwise, all technical and scientific terms used herein have the same meaning as commonly understood by a person of ordinary skill in the art. Additional definitions are set forth throughout the detailed description.

*Administer*: The terms "administer," "administering," and "administration" include any method of delivery of a therapeutic agent, (e.g., an anti-pro/latent myostatin antibody, e.g., apitegromab), into a subject's system or to a particular region in or on a subject (systemic and local administration, respectively). According to the invention, the therapeutic agent is apitegromab. In some embodiments, the apitegromab is formulated for administration as a composition, e.g., a pharmaceutical composition. In some embodiments, apitegromab is formulated for intravenous administration. For example, in some embodiments, the apitegromab is administered by intravenous injection/infusion, e.g., by intravenous infusion. In some embodiments, the apitegromab is administered by intravenous infusion, e.g., over approximately one to two hours. In some embodiments, the apitegromab is administered by intravenous infusion over approximately two hours. In some embodiments, the infusion duration is less than two hours but no shorter than one hour.

*Antibody:* As used herein, the term "antibody" encompasses any naturally occurring, recombinant, modified or engineered immunoglobulin or immunoglobulin-like structure or antigen-binding fragment or portion thereof, or derivative thereof. Thus, the term refers to an immunoglobulin molecule that specifically binds to a target antigen, and includes, for example, chimeric, humanized, fully human, and multispecific antibodies (including bispecific antibodies). An intact antibody will generally comprise at least two full-length heavy chains and two full-length light chains, but in some instances can include fewer chains such as antibodies naturally occurring in camelids which can comprise only heavy chains. Antibodies can be derived solely from a single source, or can be "chimeric," that is, different portions of the antibody can be derived from two different antibodies. Antibodies, or antigen binding portions thereof, can be produced in hybridomas, by recombinant DNA techniques, or by enzymatic or chemical cleavage of intact antibodies. The term antibodies, as used herein, includes monoclonal antibodies, multispecific antibodies such as bispecific antibodies, minibodies, domain antibodies, synthetic antibodies (sometimes referred to herein as "antibody mimetics"), chimeric antibodies, humanized antibodies, human antibodies, antibody fusions (sometimes referred to herein as "antibody conjugates"). The term "antigen binding fragment" encompasses an adnectin. In various embodiments of the therapeutic methods, uses, and compositions disclosed herein, an antibody is a pro/latent myostatin antibody (e.g., apitegromab).

*Apitegromab:* Apitegromab (also referred to as "SRK-015") is an investigational, fully human, monoclonal antibody that specifically binds to the proforms, i.e., the inactive precursor forms, of myostatin-namely, promyostatin and latent myostatin-with high affinity, thereby inhibiting myostatin activation. See, e.g., CAS Reg No. 2278276-46-1; International Nonproprietary Names for Pharmaceutical Substances (INN) (2020) WHO Drug Information, Vol. 34, No. 2 at pages 272-273.; *see also* GtoPdb Ligand ID: 11180; GtoPdb PubChem SID: 434122240; IMGT/mAb-DB Database ID: 829; NIH ChemlDplus Database: RN: 2278276-46-1; UNII: UZ54216N0Y. Apitegromab is of the human immunoglobulin G4 (IgG4)/lambda isotype. Apitegromab binds to latent myostatin in such a way as to inhibit the tolloid-mediated cleavage of latent myostatin to its active form, thereby preventing activation and release of the mature myostatin growth factor that inhibits muscle growth. Apitegromab is a selective myostatin inhibitor (a myostatin-selective activation inhibitor). *See, e.g.,* International Pub. No. WO 2017/049011. The term "selective myostatin inhibitor" (or "myostatin-selective inhibitor") refers to an agent capable of blocking, inhibiting, or otherwise antagonizing the activation or activities of myostatin/GDF-8 without affecting structurally related growth factors, such as Activin A. Apitegromab comprises CDR regions of SEQ ID NO: 1 (HCDR1), SEQ ID NO: 2 (HCDR2), SEQ ID NO: 3 (HCDR3), SEQ ID NO: 4 (LCDR1), SEQ ID NO: 5 (LCDR2), and SEQ ID NO: 6 (LCDR3), as defined by the Kabat numbering system; or SEQ ID NO: 7 (HCDR1), SEQ ID NO: 8 (HCDR2), SEQ ID NO: 9 (HCDR3); SEQ ID NO: 10 (LCDR1), SEQ ID NO: 11 (LCDR2), and SEQ ID NO: 12 (LCDR3), as defined by the IMGT numbering system; a heavy chain variable region of SEQ ID NO: 13 and a light chain variable region of SEQ ID NO: 14; and a heavy chain amino acid sequence of SEQ ID NO: 15 and a light chain amino acid sequence of SEQ ID NO: 16. Apitegromab is being developed for the treatment of SMA in pediatric and adult patients.

*Baseline:* As used herein, the term "baseline" or "baseline score" in the context of an SMA-related parameter refers to the numerical value of the SMA-related parameter of a patient at the start of or prior to treatment (e.g., within 6 months prior to starting treating) with an SMA therapy of the present disclosure (e.g., at the time of initial administration of apitegromab, alone or as an adjunct to a background therapy comprising an SMN therapy). Examples of SMA-related parameters include a score on a motor functional scale, e.g., a Revised Upper Limb Module (RULM) score, a Hammersmith Functional Motor Scale Expanded (HFMSE) score, and/or a Revised Hammersmith Scale (RHS) score.

In other embodiments, a baseline RULM, RHS, or HFMSE score is measured in a patient to be treated with apitegromab, e.g., apitegromab as an adjunct to a background therapy of an SMN therapy (e.g., in a patient with later-onset SMA, e.g., a patient who has a non-ambulatory form of SMA, such as type 2 or non-ambulatory type 3 SMA). In some embodiments, the baseline score is measured at or prior to the start of treatment with the apitegromab (i.e., apitegromab therapy). In some embodiments, the patient is on the SMN therapy at the time of baseline measurement. In some embodiments, the patient has been on the SMN therapy for at least six months prior to baseline measurement. In some embodiments, the patient has been on the SMN therapy for at least two years at the time of initiating apitegromab therapy, at which time the baseline measurement is made. In some embodiments, the baseline scores used to assess therapeutic effects of the apitegromab therapy therefore are on top of therapeutic effects, if any, as a result of the background therapy. In some embodiments, the patient had responded to the background therapy alone by an increase in the motor function scores of, for example, 1-7 points measured against pre-treatment, demonstrating the therapeutic ability of apitegromab to improve upon the effects of SMN background therapy alone.

Both the RHS (Ramsey et al. (2022) PLOS ONE https://doi.org/10.1371/journal.pone.0278996) and HFMSE (Main et al. (2003). Eur J Paediatr Neurol. 7(4):155-9) have been validated for assessing the physical abilities of patients with type 2 and type 3 SMA. RULM has also been used to investigate the upper limb function of ambulatory and nonambulatory patients with SMA (Mazzone et al. (2017) Muscle Nerve. 55(6):869-874).

In some embodiments, the HFMSE is used to assess the physical abilities of patients with type 2 and type 3 SMA (O'Hagen et al. (2007) Neuromuscul Disord. 17(9-10):693-697; Glanzman et al. (2011) J Child Neurol. 26(12):1499-1507). In some embodiments, the HFMSE is used to evaluate non-ambulatory SMA patients. The HFMSE consists of 33 items to assess an individual's ability to perform various activities. Quality and execution of each movement for the parameters listed above are graded on a scale of 0, 1, 2, where 0 denotes unable, 1 denotes performed with modification or adaption, and 2 denotes without modification or adaptation. The maximum achievable score is 66.

In other embodiments, a baseline Hammersmith Infant Neurological Exam (HINE) score is measured in a patient to be treated with apitegromab, e.g., apitegromab as an adjunct to a background therapy of an SMN therapy. The HINE evaluates seven different areas of motor milestone development, e.g., kicking, head control, rolling, sitting, crawling, standing or walking, with a maximum score of 2-4 points for each developmental motor milestone (Day et al. (2022) BMC Pediatrics 22:632). The HINE can be used in its entirety or the HINE-2, which is the development module, can be used independently to assess the motor milestones during development. In other embodiments, Hammersmith Neonatal Neurological Examination (HNNE) is measured in an infant patient to be treated with apitegromab, e.g., apitegromab as an adjunct to a background therapy of an SMN therapy. The HNNE evaluates 34 items grouped in the categories of posture and tone, tone patterns, reflexes, movements, abnormal signs/patterns, and orientation/behavior (Pane et al. (2022) Eur. J Pediatr 181:2821-29).

The RULM is based on 19 scorable items that reflect different functional domains and are graded on a 3- point system with a score of 0 (unable), 1 (able, with modification), and a maximum of 2 (able, no difficulty), with one additional item that is scored as a can/cannot score (a score of 1 is the highest score). The maximum total RULM score is 37 (Mazzone et al. (2017) Muscle Nerve. 55(6):869-874).

RHS is a clinician rated SMA specific outcome measure that contains 36 items assessing physical motor performance. The assessed motor functional activities relate to sitting, supine, rolling, prone, ability to move and get up from the floor, balance, standing, run/walk, stairs, ascending and descending a step and the ability to jump. Thirty-three items are graded according to an ordinal 0, 1, 2 scale where 0 represents the least physical ability or function achieved, and 2 the highest. Three items are graded 0 and 1 where 0 represents an inability to complete the item, and 1 represents achieving the item. Two timed tests are included within the scale, and WHO motor milestones can also be completed concurrently. Ramsey et al. (2022) PLOS ONE https://doi.org/10.1371/journal.pone.0278996).

*Cohort:* As used herein, the term the "cohort" or "cohort population" refers to a group or population of human subjects with shared factors or influences, such as age, SMA disease severity (e.g., type 2 and/or type 3 SMA), concurrent treatments (e.g., an SMN therapy), etc. In some embodiments, as used herein, a "cohort" refers to a group of human subjects with shared age, SMA disease severity, and/or concurrent treatments.

*Control:* The term "control" or "control sample," as used herein, refers to any clinically or scientifically relevant comparative sample, population, or counterpart, including, for example, a sample from a healthy subject, a sample from a subject having a deficiency that can cause or make the subject susceptible to a certain disease or condition, a subject with a disease or condition of interest, a sample from a subject treated with a placebo, a sample from a subject prior to treatment, a sham or buffer treated subject or sample, an untreated subject or sample, and the like.

*Inhibit or inhibition of:* The term "inhibit" or "inhibition of," as used herein, means to reduce by a measurable amount, and can include but does not require complete prevention or inhibition.

*Effective amount:* The terms "effective amount" and "therapeutically effective amount" refer to the ability or an amount to fulfill its intended purpose(s), i.e., a desired biological or medicinal response in a subject, and/or to achieve a statistically significant clinical benefit (e.g., efficacy) in a patient population. An "effective amount" may refer to a therapeutically effective amount or therapeutic dose, which is a dosage or dosing regimen that is sufficient to produce a detectable change in a parameter of a disease, e.g., a slowing, pausing, reversing, diminution, or amelioration in a parameter, symptom, or downstream effect of the disease. The term encompasses but does not require the use of an amount that completely cures a disease. References herein to a dose of an anti-pro/latent myostatin antibody (e.g., a dose of apitegromab) may be a therapeutically effective dose, as described herein. In some embodiments, efficacy may be measured by well-known motor function assessments and/or by one or more surrogate biomarkers, e.g., serum concentration of latent TGFβ and/or serum concentration of creatinine. For example, in certain embodiments of the present disclosure, the intended purpose may be to inhibit activation of myostatin *in vivo,* to achieve clinically meaningful outcome associated with the myostatin inhibition.

Measure of the relevant intended purpose may be objective (i.e., measurable by some assay or marker) or subjective (i.e., subject gives an indication of or feels an effect). In some embodiments, a therapeutically effective amount is an amount that, when administered to a patient population that meets certain clinical criteria for SMA (for example, as determined by symptoms manifested, disease progression/stage, genetic profile, etc.), a statistically significant therapeutic response is obtained among the population.

An effective amount may also refer to an amount that, when administered according to a particular regimen, produces a positive clinical outcome with a reasonably acceptable level of adverse effects (e.g., toxicity), such that the adverse effects, if present, are tolerable enough for a patient to continue with the therapeutic regimen, and the benefit of the therapy overweighs risk of toxicity. Those of ordinary skill in the art will appreciate that in some embodiments of the disclosure, a dosage may be considered to contain an effective amount if it contains an amount appropriate for administration in the context of a dosage regimen correlated with a positive outcome.

*Latent myostatin:* As used herein, the term "latent myostatin" refers to an inactive precursor of mature myostatin which comprises a disulfide-linked homodimer, each molecule of the homodimer comprising the amino terminal prodomain non-covalently bound to the carboxyl terminal mature myostatin domain. In some embodiments, latent myostatin is generated from a pro myostatin that has been cleaved by a proprotein convertase, but which has not been cleaved by a protease from the BMP/tolloid family. In some embodiments, latent myostatin can be generated by combining the prodomain and the carboxy terminal mature myostatin domain in vitro and allowing them to fold properly. See, for example, Sengle et al. (2011) J. Biol. Chem., 286(7):5087-5099.

*Later-onset SMA:* As used herein, unless explicitly defined otherwise, the term "later-onset SMA" refers to a patient who does not exhibit onset of SMA symptoms until after six months of age (e.g., symptoms are not detected at a 6-month medical screening) irrespective of the genotype, irrespective of whether and when an SMN therapy is administered. A later-onset SMA patient may also be a patient characterized as having type 2, type 3 or type 4 SMA based on the traditional disease classifications known in the art. Patients with later-onset SMA typically have at least two copies of the SMN2 gene (e.g., 2 copies, 3 copies, 4 copies, or more, e.g., 2-4 copies). In some embodiments, a patient with later-onset SMA is treated with or has received a motor neuron-directed therapy (e.g., has received such therapy before the age of five), such as an SMN-targeted therapy, e.g., an SMN upregulator. As opposed to later-onset SMA, "early-onset SMA" or "infantile-onset SMA" refers to SMA that manifests symptoms at or prior to six months of age (including patients who can also be categorized as type 1 SMA using the traditional classification known in the art). By comparison, an early- or infantile-onset SMA patient typically has 1 or 2 copies of the SMN2 gene. In some embodiments, a patient has later-onset SMA due to having received early intervention, e.g., one or more SMN therapies, wherein the patient would be considered early-onset if the patient had not received such early intervention.

In some embodiments, a patient with later-onset SMA has 2-4 copies of the SMN2 gene. In some embodiments, a patient with later-onset SMA has 1-2 copies of the SMN2 gene. In some embodiments, patients with later-onset SMA due to early treatment intervention (i.e., patients who otherwise would have had onset of symptoms before six months of age) may also be referred to as an "emerging" or "treatment-emergent" later-onset SMA patient or patient population. In some embodiments, treatment-emergent later-onset SMA patients may have a different genotype (e.g., 1-2 copies of the SMN2 gene), phenotype (e.g., gait), and/or course of disease and response to myostatin treatment than non-treatment-emergent SMA patients. In some embodiments, a patient having later-onset SMA would not have been able to walk and/or sit unassisted (e.g., would have had type 1 and/or non-ambulatory type 2 SMA) in the absence of treatment, e.g., SMN-targeted treatment, but is able to do so because of treatment. In some embodiments, a treatment-emergent later-onset SMA patient who would have had non-ambulatory type 3 SMA such that they would have lost the ability to walk by 18 months of age in the absence of treatment, e.g., SMN-targeted treatment, retains the ability to walk at 18 months because of treatment. When considering treatment of ambulatory type 3 SMA patients, in some embodiments any ambulatory patient is treated. In some embodiments, a type 3 patient is one that would retain the ability to walk by 18 months in the absence of intervention. In some embodiments, a type 3 patient is one that would lose the ability to walk in the absence of intervention. In some embodiments, a patient is identified through newborn screening or in utero and is initiated in treatment, e.g., SMN-targeted therapy, prior to disease onset. In some embodiments, SMA symptom onset is not observed because of early treatment initiation following newborn screening. In some embodiments, the treated patient has type 4 SMA (e.g., a patient with 4 or more copies of the SMN2 gene and much later onset of symptoms, e.g., at 18 years of age or later).

*Mature* myostatin: As used herein, the term "mature myostatin" refers to a mature, biologically active form of myostatin. In some embodiments, mature myostatin is capable of myostatin receptor binding and/or activation. Activation and release of mature myostatin *in vivo* from its pro myostatin form is accomplished by several discrete protease cleavage events. To begin with, "pro myostatin" is cleaved by a proprotein convertase, resulting in "latent myostatin," in which the mature myostatin is shielded from binding to its receptors by a portion of the prodomain. Activation and release of mature myostatin is accomplished after cleavage of latent myostatin by an additional protease from the BMP/tolloid family, such as mTLL-2. As used herein, the term "mature myostatin" can refer to both full-length mature myostatin, as well as fragments of the full-length mature myostatin which retain biological activity. So-called neutralizing antibodies that bind mature myostatin thereby interfere with its ability to bind and activate its cellular receptors.

*Motor neuron therapy:* "Motor neuron therapy" refers to any therapy that can partially or completely restore motor neuron function. Examples include, but are not limited to, agents that affect neurotransmitter signaling, agents that reduce oxidative stress to neurons, and neuroprotective stem cell therapy. Motor neuron therapy incudes SMN therapy.

*Motor skills:* As used herein, "motor skill," "motor skills," or "motor function" refer to the ability of a subject to perform one or more tasks designed to measure muscle function. Motor skills can be measured by a test designed to assess certain physical activities, which yields a score indicative of the level of the patient's muscle function. For example, a subject's motor skills can be assessed using the Hammersmith Functional Motor Scale-Expanded (HFMSE) test; the Revised Upper Limb Module (RULM) test; the Revised Hammersmith Scale (RHS) test; a Motor Function Measure (MFM) test (e.g., MFM-D1, MFM-D2, MFM-32); WHO motor development milestones; or any other known test of muscle function.

*Muscle-directed therapy:* As used herein, the term "muscle-directed therapy" or "muscle-targeted therapy" refers to any therapy that restores muscle function. Examples of muscle-targeted therapies include myostatin inhibitors.

*Pro*/*latent myostatin*: As used herein, the term "pro/latent myostatin" refers to pro myostatin, latent myostatin, or both. An anti-pro/latent myostatin antibody may bind specifically to pro myostatin. An anti-pro/latent myostatin antibody may bind specifically to latent myostatin. An anti-pro/latent myostatin antibody may bind specifically to both latent myostatin and pro myostatin. Apitegromab is an anti-pro/latent myostatin antibody that binds specifically to both latent myostatin and pro myostatin. The term "pro myostatin" (or "promyostatin") refers to an inactive precursor of mature myostatin which comprises a disulfide-linked homodimer, each molecule of the homodimer comprising the amino terminal prodomain covalently bound to the carboxyl terminal mature myostatin domain. In some instances, "pro myostatin" has not been cleaved by either a proprotein convertase, or a protease from the BMP/tolloid family. Promyostatin and latent myostatin (see below) are proforms of myostatin/GDF-8. As used herein, the term "proforms of myostatin" refers to inactive (e.g., precursor or latent) forms of the myostatin growth factor that are associated with the N-terminal latency-associated peptide (LAP) domain. Proforms of myostatin are comprised of dimers. The term encompasses both "promyostatin" and "latent myostatin." The term excludes mature growth factor (GDF8) that is not associated with the LAP domain.

*Natural history*: Natural history of SMA refers to the progression of SMA in a person over time without treatment.

*Progression:* Progression of disease (e.g., SMA) is the process of worsening of symptoms or a decline of condition over a period of time. Absent pharmacological intervention (e.g., therapy), the progression is reflected by or corresponds to the natural history of the disease observed in a particular patient population. Disease progression may be assessed by the rate at which the condition worsens, and/or, the degree to which the condition becomes worse. Accordingly, efficacy may include the ability of a drug or therapy to delay or slow the progression of disease. For example, a patient may show a decline in a motor function score over time, but at a slower rate than what would be expected based on the natural history. Efficacy may include the ability of a drug or therapy to reduce the degree of decline. Efficacy may include stabilization of the disease, i.e., at least a net zero change in functional parameter(s) over time.

*Quality of life (QoL):* As used herein, QoL is defined by outcomes that are meaningful from the patient perspective and which measure the impact of therapies on dimensions of life other than assessing survival or significant changes in motor milestones, such as activities of daily living, work productivity and fatigue.

*SMN therapy*/*SMN-targeted therapy:* In the context of the present disclosure, the term "SMN therapy" or "SMN-targeted therapy" or "SMN-directed therapy" (used interchangeably herein) refers to pharmacological agents (drugs, biologics) aimed to increase the amount or availability of the functional SMN protein in patients for purposes of treating SMA.

As used herein, other synonymous terms include "SMN upregulator" or "SMN upregulator therapy" or "SMN-directed therapy" or "SMN corrector" or "SMN corrector therapy" or "SMN enhancing therapy," or "SMN enhancer". These agents encompass any therapy or compound which can be used to increase or improve SMN gene expression (e.g., SMN1 gene expression and/or SMN2 gene expression), SMN protein production, and/or functional SMN activity. For example, an SMN1-directed therapy may include gene therapies intended to supplement or replace the deleted or mutated SMN1 gene. An SMN2-directed therapy may include splice modifiers that target certain exon(s) of the SMN2 backup gene so as to increase the availability of an at least partially functional SMN protein in the body. Non-limiting examples of SMN therapies include nusinersen, onasemnogene abeparvovec-xioi, and risdiplam. An SMN upregulator may be a central corrector or a systemic corrector. A central upregulator may be administered directly to the central nervous system (CNS) via the intrathecal route. In contrast, a systemic upregulator may be administered via any route, e.g., orally administered, and affects not only the CNS, but other tissues throughout the body. Systemically delivered SMN splice modifiers may also affect SMN splicing in other (i.e., non-neuronal) tissues, where SMN is expressed. In some embodiments, a "functional SMN protein" is capable of promoting motor neuron function and/or survival or partially or fully restore motor neuron function in a cell (e.g., a cell within a subject). In some embodiments, a functional SMN protein is capable of restoring at least 10%, 15%, 20%, 25%, 30%, 35%, 40%, 45%, 50%, 55%, 60%, 65%, 70%, 75%, 80%, 85%, 90%, 95%, 99%, or more of the motor neuron function in a cell (e.g., a cell within a subject). In some embodiments, full-length SMN protein is the result of protein translation (e.g., in a cell) of a correctly spliced SMN mRNA. In some embodiments, a functional SMN protein is encoded from SMN2 mRNA that contains exon 7.

*Specific binding:* As used herein, the term "specific binding" or "specifically binds" means that an antibody or antigen binding portion thereof exhibits a particular affinity for a particular structure (e.g., an antigenic determinant or epitope) in an antigen (e.g., a KD measured by Biacore^{®}). In some embodiments, an antibody or antigen binding portion thereof specifically binds to a target, e.g., pro/latent myostatin, if the antibody has a KD for the target of at least about 10⁻⁸ M, 10⁻⁹ M, 10⁻¹⁰ M, 10⁻¹¹ M, 10⁻¹² M, or less. In the context of the present disclosure, "an antibody that specifically binds an antigen with high affinity" generally refers to a KD of 1.0 x 10⁻⁸ M or less. In some embodiments, an antibody or antigen binding portion thereof may also "selectively" (i.e., "preferentially") bind a target antigen if it binds that target with a comparatively greater strength than the strength of binding shown to other antigens, e.g., a 10-fold, 100-fold, 1000-fold, or greater comparative affinity for a target antigen (e.g., pro/latent myostatin) than for a non-target antigen (e.g., mature myostatin (GDF-8), GDF-11, and/or other members of the TGFβ superfamily of growth factors).

*Steady-state:* As used herein, the term "steady-state" refers to the situation where the overall intake of a molecule (e.g., apitegromab) is fairly in dynamic equilibrium with its elimination. In some embodiments, a steady-state serum concentration of apitegromab is measured as a maximum serum concentration (i.e., Cₘₐₓ) or a minimum (trough) serum concentration (i.e., Cₘᵢₙ or C_{trough}) of the apitegromab after administration, e.g., to a human subject or to a cohort of human subjects. For example, a steady state of apitegromab may be determined using the maximum serum concentration of apitegromab or the minimum serum concentration of apitegromab in a pharmacokinetics (PK) analysis. In some embodiments, the pharmacodynamic (PD) target is latent myostatin. In some embodiments, the PD profile of apitegromab is evaluated by measuring latent myostatin concentrations in serum. In some embodiments, the serum latent myostatin concentration is a steady-state concentration. In some embodiments, the serum latent myostatin concentration is measured as a pre-dose or trough concentration (i.e., Cₘᵢₙ or C_{trough}), e.g., in a human subject or in a cohort of human subjects.

*Subject:* The term "subject" in the context of therapeutic applications refers to an individual who receives or is in need of clinical care or intervention, such as treatment, diagnosis, etc. Suitable subjects include vertebrates, including but not limited to mammals (e.g., human and non-human mammals). Where the subject is a human subject, the term "patient" may be used interchangeably. In a clinical context, the term "a patient population" or "patient subpopulation" is used to refer to a group of individuals that falls within a set of criteria, such as clinical criteria, medical history, health status, gender, age group, genetic criteria, and/or lifestyle factors. As used herein, a human subject, patient, or patient population refer to an SMA patient or patient population unless the context indicates otherwise.

*Target engagement:* As used herein, the term "target engagement" refers to the ability of a molecule (e.g., apitegromab) to bind to its intended target *in vivo* (e.g., latent myostatin), e.g., in skeletal muscle. As used herein, saturation in target engagement indicates a dosage sufficient to achieve certain therapeutic effects (e.g., efficacy), although it may be possible to obtain efficacy without saturation.

*Therapeutic dose:* The term "therapeutic dose" refers to an amount (e.g., of apitegromab) sufficient to achieve efficacy as determined by clinical endpoints that measure therapeutic effects (e.g., by one or more of the clinical endpoint measurements discussed herein) when administered to a patient in a particular way (e.g., using a particular dosing regimen). For example, a therapeutic dose of apitegromab, when administered intravenously every four weeks, may be greater than 2 mg/kg and up to 20 mg/kg. In certain embodiments, the therapeutic dose of apitegromab, when administered intravenously every four weeks, is 10 mg/kg or 20 mg/kg.

*Treatment:* As used herein, the term "treating" or "treatment" of a disease or disorder (e.g., SMA) is meant slowing, delaying, or preventing the onset of such a disease or disorder, or reversing, alleviating, ameliorating, inhibiting, slowing down, stabilizing, or stopping the progression, aggravation or deterioration, the progression or severity of a condition associated with such a disease or disorder, e.g., as compared to a baseline in the absence of the treatment (e.g., where treatment stops disease progression observed in the absence of treatment). The term includes but does not necessarily require a complete treatment or prevention of the disease or disorder. As used herein, "treating" or "treatment" refers to the administration of one or more active agents (simultaneously or sequentially), or of one or more compositions including one or more active agents, to a subject, e.g., who has SMA, with the purpose to cure, heal, alleviate, relieve, alter, remedy, ameliorate, improve, or affect the disorder, the symptom of the disease, or the predisposition toward the disease, e.g., SMA.

*Treatment emergent patient population:* The traditional classification of SMA patient populations or types (e.g., type 0, type 1, type 2, non-ambulatory type 3, ambulatory type 3, type 4 etc.) is largely based on the natural history of the disease manifestation, which shows strong correlation with the number of SMN2 gene copies. Based on the traditional classification, type 0 is the most severe form which has in utero onset with reduced or absent movements and requires mechanical ventilation at birth. With the availability of approved SMN-targeted therapies (e.g., SMN2 upregulators, SMN1 gene therapy, etc.) and newborn screening in recent years, additional classifications may be warranted to describe emerging patient populations, referred to herein as "emergent" or "treatment emergent" patient populations. In these populations, the manifestations of SMA expected based on natural history and/or genetic analysis may be altered where patients have initiated an SMN-targeted therapy at an early age. In other words, early intervention can impact the course of the disease progression expected from the natural history, irrespective of the disease genotype. For example, an infant with a confirmed SMN1 deletion and one copy of the SMN2 gene, who receives an early SMN therapy, may be able to sit independently at 12 months, who otherwise would have been expected to develop type 1 SMA. An SMA patient with two copies of the SMN2 gene may be expected to develop a severe (e.g., type 2) disease form without receiving SMN-targeted therapy, but with SMN-targeted therapy, especially with an early intervention, the clinical presentation of such a patient may resemble that of a milder disease form. A patient with non-ambulatory type 3 SMA (who lost the ability to walk) may regain the ability to walk, or a patient with type 2 SMA may walk for the first time, as a result of such therapy. Likewise, a patient expected to develop non-ambulatory type 3 SMA after 18 months of age may exhibit a retained ability to walk at a later age due to early SMN-targeted therapy yet exhibit a different phenotype (e.g., gait) and/or response to SMA treatments (Mercuri et al. (2020) Nature Reviews Neurology, 16:706-715). To accommodate changes associated with the medical advancement in SMA therapy, the field continues to evolve, including adjustments to certain nomenclature/terminology, aimed to better describe various diseases.

*Type* 1 SMA: Typically, children with type 1 SMA rapidly decline in the natural history of the disease (Cances et al. (2022) Orphanet J Rare Dis 17:300). Early symptoms include hypotonia, small or weak muscles, difficulty in breathing, trouble swallowing, weak cough or weak cry, and inability to sit. Infants with type 1 are very weak and unable to sit. At 5-6 months of age, patients typically require respiratory and/or nutritional support. Over 90% of patients die before their second birthday. Such patients typically have 1-2 copies of the SMN2 gene.

*Type 2 SMA:* Children with type 2 SMA are non-ambulant absent therapeutic intervention and often have three copies of the SMN2 gene and become symptomatic between 6-18 months of age. Muscle weakness is very common and affects the ability to stand or walk without assistance and typically require wheelchairs. Generally, they start losing abilities before the age of two. But they can sit independently, maintain head control and roll over.

*Type 3 SMA:* In type 3 SMA patients, symptoms can begin after 18 months of age, usually in early childhood, e.g., based on clinical classification and/or physical milestones such as sitting or walking. These patients often have three or four or more copies of the SMN2 gene, with the more severe form of non-ambulatory type 3 SMA typically being associated with three copies of the SMN2 gene (type 3a) and the less severe form of non-ambulatory type 3 SMA typically being associated with four copies (type 3b). Typically, type 3 SMA patients can at least initially walk and climb with assistance, eat with utensils and dress themselves, but they cannot generally run, jump or climb without help. They lose many motor functions as they grow older, including the ability to walk and climb. Among type 3 patients, those who are able to walk are referred to as ambulatory type 3 SMA, whilst those who have lost the ability to walk are referred to as non-ambulatory type 3 SMA. Type 3 patients often lose ambulation by ages 4-16, e.g., on average around age 10.

*Type 4 SMA:* Type 4 SMA is a rare, adult-onset form of SMA. These patients usually have four or more copies of the SMN2 gene and usually experience only mild muscle weakness, which may start around 18 years of age, but more often later (e.g., in the 20's or 30's).

As more patients gain access to treatments, e.g., SMA therapy, a patient diagnosed as having type 1, 2, 3, or 4 SMA may demonstrate clinical improvement such that their phenotype more closely resembles another SMA type. For example, a patient who, in the absence of treatment, would be categorized as a type 1 SMA patient may show characteristics of a type 2 patient and thus be "type 2-like"; a type 2 patient may show characteristics of a type 3 patient and thus be "type 3-like"; a type 3 patient may show characteristics of a type 4 patient and thus be "type 4-like". As used herein, the terms "type 1-like", "type 2-like", "type 3-like", and "type 4-like" with respect to SMA categorization refer to a clinical observation, measurement, or behavior of the patient irrespective of genotype or natural course of disease in the absence of intervention.

*Wearable medical device:* A wearable medical device is a device that is autonomous, noninvasive and that performs a specific medical function, e.g., monitoring or support, and is attached to the body or to clothing. Examples include, but are not limited to wearable sensor devices, electromyogram patches, etc., including Acti-Myo^{®} and AUTOMA, a wearable device that measures upper limb muscular activity.

Other than in the operating examples, or where otherwise indicated, all numbers expressing quantities of ingredients or reaction conditions used herein should be understood as modified in all instances by the term "about." The term "about" when used in connection with percentages can mean ±1%.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Other elements may optionally be present other than the elements specifically identified by the "and/or" clause, whether related or unrelated to those elements specifically identified unless clearly indicated to the contrary. Thus, as a non-limiting example, a reference to "A and/or B," when used in conjunction with open-ended language such as "comprising" can refer, in some embodiments, to A without B (optionally including elements other than B); in other embodiments, to B without A (optionally including elements other than A); in yet other embodiments, to both A and B (optionally including other elements); etc.

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified. Thus, as a non-limiting example, "at least one of A and B" (or, equivalently, "at least one of A or B," or, equivalently "at least one of A and/or B") can refer, in some embodiments, to at least one, optionally including more than one, A, with no B present (and optionally including elements other than B); in other embodiments, to at least one, optionally including more than one, B, with no A present (and optionally including elements other than A); in yet other embodiments, to at least one, optionally including more than one, A, and at least one, optionally including more than one, B (and optionally including other elements); etc.

Use of ordinal terms such as "first," "second," "third," etc., in the claims to modify a claim element does not by itself connote any priority, precedence, or order of one claim element over another or the temporal order in which acts of a method are performed, but are used merely as labels to distinguish one claim element having a certain name from another element having a same name (but for use of the ordinal term) to distinguish the claim elements.

Ranges provided herein are understood to be shorthand for all of the values within the range. For example, a range of 1 to 10 is understood to include any number, combination of numbers, or sub-range from the group consisting of 1, 2, 3, 4, 5, 6, 7, 8, 9, or 10, e.g., 2-8, 1-5, etc.

### Spinal Muscular Atrophy (SMA)

Spinal muscular atrophy (SMA) is a debilitating, frequently fatal neuromuscular disease and the most common genetic cause of infant mortality (Awano et al. (2014) Neurotherapeutics 11:786-795). SMA is an autosomal recessive genetic disorder involving a mutation or deletion in the survival motor neuron 1 (SMN1) gene. Specifically, SMA is caused by reductions in the level of SMN protein, sufficient amounts of which are necessary to promote survival of the anterior horn cells of the spinal cord. Loss of motor neurons results in profound muscle atrophy, often leading to death due to respiratory insufficiency (Monani (2005) Neuron 48:885-896). Muscle pathology in SMA is characterized by the presence of small atrophic fibers believed to represent denervated or partially denervated myofibers. The presence of atrophic fibers is a classic indication of motor neuron denervation caused by the loss or dysfunction of motor neurons. Unlike Duchenne muscular dystrophy (DMD) patients, SMA patients do not exhibit features of necrosis and inflammatory changes even in the most severe cases of the disease (Le Verche V. et al. Spinal Muscular Atrophy; Disease Mechanisms and Therapy 2017; Ch. 21:341-356).

While SMA patients typically lack a functional SMN1 gene, the paralogous gene, SMN2, produces low levels of functional SMN protein due to alternative splicing that truncates the transcript. The development of molecular medicine techniques have advanced the diagnosis of SMA patients based on genotyping of both SMN1 and SMN2 copy numbers. SMA patients are also diagnosed based on clinical presentation and categorized phenotypically based on the maximal motor milestone achieved and the age at symptom onset. Disease-modifying drugs have altered some of the traditional diagnosis and classification. Classification may, in some embodiments, be made based on one or more of age of initiation of therapy, age of symptom onset, and/or number of SMN2 copies, e.g., separately or to refine the definition of SMA phenotypes in the clinic beyond the traditional classifications defined by age of onset and severity alone (Jedrzejowska M. et al. Degener Neurol Neuromuscul Dis. 2020;10:39-47).

In some embodiments, a patient with later-onset SMA is treated with or has received a motor neuron-directed therapy (e.g., has received such therapy before the age of 5), such as an SMN therapy. In some embodiments, a patient with later-onset SMA has at least two copies of the SMN2 gene (e.g., at least three copies or at least four copies of SMN2). In some embodiments, a patient with later-onset SMA develops diagnosable symptoms of SMA after six months of age. A subject with SMA may be treated before exhibiting symptoms of the disease or after onset of diagnosable symptoms.

In some embodiments, a patient with infantile-onset SMA is treated with or has received a motor neuron-directed therapy, such as an SMN therapy. In some embodiments, a patient with infantile-onset SMA has one copy of SMN2. In some embodiments, a patient with infantile onset SMA develops diagnosable symptoms of SMA before six months of age. In some embodiments, a patient with infantile onset SMA would have developed diagnosable symptoms before six months of age but does not because of treatment, e.g., with an SMN therapy. In some embodiments, these subjects are diagnosed with infantile-onset SMA based on, e.g., SMN2 copy number, and are distinguished from later-onset SMA patients on the basis of other factors beyond age of disease onset, e.g., genotype (such as SMN2 copy number), phenotype (such as gait or other observed motor traits), and/or response to SMA therapy.

A summary of SMA categorization and disease characteristics is provided below.

| **Onset-based categorization** | **Classic categorization (by natural history)** | **SMN2 gene #copy (typically)** | **SMN-targeted therapy** | **Apitegromab** | **TOPAZ trial** | **Examples of emerging patient populations (effects of therapy; deviation from natural history)** |
|---|---|---|---|---|---|---|
| Infantile onset SMA | Type 1 (never sit independently) | 1-2 | + (initiated before age 5) | + | | - Initiated SMN-targeted therapy before 6M and remain largely asymptomatic at 6M (as if type 2) |
| (symptomatic by 6M of age) | | | | | | - Initiated SMN-targeted therapy after being symptomatic by 6M and gain ability to sit by 12M (as if type 2) |
| | | | | | | - Identified by newborn genetic screening and may remain asymptomatic after early treatment |
| | | | | | | |
| Later-onset SMA | Type 2 (never walked) | 2-3 | + (initiated before age 5) | + | Cohort 3 | - May gain ability to walk (as if ambulatory type 3) after SMN-targeted treatment |
| (becomes symptomatic after 6M of age) | | | + (initiated at age 5 or later) | + | Cohort 2 | |
| | | | - | + | | |
| | Type 3 non-ambulatory (lost ability to walk) | 2-4 (3 copies = type 3a; 4 copies = type 3b) | + (initiated before age 5) | + | | - May regain ability to walk (after being non-ambulatory) after SMN-targeted treatment but may lose ability at a later age |
| | | | + (initiated at age 5 or later) | + | Cohort 2 | |
| | | | - | + | | |
| | Type 3 ambulatory (retained ability to walk) | 3+ | + | + | | - May lose ability to walk at a later age |
| | | | - | + | Cohort 1 (monotherapy) | |
| | Type 4 (adult-onset) | 4+ | - | + | | |

### Genotype-based classifications of SMA

The clinical heterogeneity of SMA is due in part to the complicated genetics of the disease. Mutations in the SMN1 gene result in SMA (Lefebvre et al. (1995) Cell 80:155-165); however, in humans, a nearly identical gene, SMN2, is located in close proximity to SMN1 (Monani et al. (1999) Hum Mol Genet 8:1177-1183). The primary difference between these genes is a C to T transition which creates an exonic splice silencer, resulting in removal of exon 7 from the final mRNA transcript. The truncated SMN protein is unstable and quickly degraded. Nevertheless, approximately 10% of the mRNA produced from SMN2 is correctly spliced and produces full length SMN protein, but this amount is insufficient to fully compensate for loss of SMN1.

The copy number of SMN2 varies between individuals and strongly correlates with SMA disease severity. More copies (e.g., three or four copies) are generally associated with milder forms of SMA. SMA patients with a single SMN2 copy are rare but this copy number is highly predictive of a severe type 1 phenotype with a poor prognosis. The majority of patients with the type 1 form of SMA have one or two copies of SMN2; most patients with type 2 have three SMN2 copies; and most patients with type 3 have three or four SMN2 copies. To illustrate, according to Calucho et al. ((2018) Neuromuscular Disorders. 28:208-215 at Table 2), about 80% of type 1 SMA patients carry one or two SMN2 copies, about 94% of type 2 patients carry two or three copies, about 93% of type 3 patients carry three or four copies, and nearly 100% of type 4 patients carry four to six SMN2 copies.

Traditional SMA classifications include type 1, type 2, type 3 and sometimes type 4 SMA, in the order of severity. Type 1 SMA is typically diagnosed between birth and six months, and, without early intervention, patients never gain sufficient strength to sit independently. Without intervention, most type 1 patients do not survive past two years without respiratory support. People with type 2 and type 3 produce greater amounts of SMN protein and have less severe, but still life-altering forms of SMA. Type 2 patients are generally diagnosed between six and 18 months of age. While they are able to sit unassisted, they cannot walk without aid. With type 3 SMA, patients are typically diagnosed past the age of 18 months and are able to sit and walk unassisted, although they may become wheelchair-dependent later in life. Thus, type 3 SMA includes both ambulatory and non-ambulatory subpopulations. Many ambulatory type 3 SMA patients at some point transition to non-ambulatory as the disease progresses. Type 4 SMA is adult onset, mild in phenotype, and very rare.

Although SMA stratification by type is a useful clinical paradigm, the disease phenotype exists more as a continuum than as discreet classifications. For example, patients carrying an SMA genetic mutation may also be pre-symptomatic (not manifesting obvious disease phenotypes).

In some cases, a patient who carries a genetic mutation in the SMN gene (such as SMN1) may be phenotypically pre-symptomatic and identified for treatment on the basis of genetic screening. In some embodiments, the delay in disease manifestation may be at least in part due to an early intervention such as an SMN upregulator therapy (e.g., SMN2 upregulator therapy and SMN1 gene therapy). Early intervention generally means the treatment is initiated prior to age five, e.g., prior to age 4, age 3, age 2, age 1, or soon after birth. Newborn genetic screening has become more widely accessible in recent years, which allows the identification of individuals born with genetic disorders, such as SMA. In the United States, for example, many states have included SMA in a panel of routine newborn screening among other known genetic disorders, which is typically conducted during the first few days of life. Therefore, babies carrying mutations in the SMN gene (SMN1) who are likely to develop the disease can be identified early, often when asymptomatic (pre-symptomatic). Early detection and diagnosis can lead to early treatment intervention, e.g., even before the child starts to develop symptoms. This may help prevent certain clinical manifestations and/or may delay the onset or progress of SMA.

Genetic screening may be carried out in newborn/infant subjects, as well as in utero (e.g., in a fetus). In some embodiments, a subject is or has been identified as a carrier of an SMN mutation, e.g., by genetic screening either in utero or as a newborn/infant. In some embodiments, the genetic screening is carried out as a newborn/infant (e.g., within 24 hours of birth). In other embodiments, the genetic screening is carried out in utero.

### Onset-based classifications of SMA

Severe forms of SMA typically manifest early symptoms, e.g., before six months of age. This may be referred to as infantile-onset, as opposed to later-onset SMA. When SMA symptoms are present at birth or by the age of six months, the disease is also SMA type 1 (also called infantile-onset or Werdnig-Hoffmann disease). Babies typically have generalized muscle weakness, a weak cry, and breathing distress.

More recently, with the availability of approved SMN upregulator therapies, such as nusinersen, risdiplam, or onasemnogene abeparvovec-xioi, early intervention has been shown to be particularly effective in delaying the onset of and/or reducing the severity of more severe phenotypes of SMA based on the genotype of the particular patient. For example, some patients who were diagnosed with (or, due to a low copy number of SMN2 genes, were at risk of developing) type 1 SMA, early intervention with SMN upregulator therapy may alter the phenotype to one that is more consistent with later-onset SMA.

In some embodiments, symptom onset-based definition is useful to categorize certain patient populations that receive early intervention which changes the normal course or timeline of disease progression.

### Targeting Myostatin to Improve Muscle Function - Myostatin Inhibition

The present disclosure encompasses the use of an agent that inhibits myostatin activation irrespective of the mechanism of action. An inhibitor may be a small molecule. An inhibitor may be an antibody or antigen-binding fragment (e.g., an adnectin such as taldefgrobep alfa). The antibody may be a myostatin-selective antibody, e.g., a pro/latent-myostatin selective antibody.

Disclosed herein are antibodies capable of binding to pro myostatin and/or latent myostatin, thereby inhibiting myostatin activity, and uses thereof for treating diseases and disorders associated with muscle atrophy such as SMA.

The disclosure provides methods comprising the use of apitegromab or an antibody having the CDRs sequences, variable domains sequences, or full-length antibody sequences provided in Tables 1-10 below.

**Table 1. Amino acid sequences of Kabat CDRs for an anti-pro/latent myostatin antibody**

| **IgG chain** | **SEQ ID NO** | **Amino acid sequence** |
|---|---|---|
| HCDR1 | 1 | SSYGMH |
| HCDR2 | 2 | VISYDGSNKYYADSVKG |
| HCDR3 | 3 | DLLVRFLEWSHYYGMDV |
| LCDR1 | 4 | SGSSSNIGSNTVH |
| LCDR2 | 5 | SDNQRPS |
| LCDR3 | 6 | AAWDDSLNGV |

**Table 2. Amino acid sequences of IMGT CDRs for an anti-pro/latent myostatin antibody**

| **IgG chain** | **SEQ ID NO** | **Amino acid sequence** |
|---|---|---|
| HCDR1 | 7 | GFTFSSYGMH |
| HCDR2 | 8 | ISYDGSN |
| HCDR3 | 9 | DLLVRFLEWSHYYGMDV |
| LCDR1 | 10 | SSNIGSNT |
| LCDR2 | 11 | SDN |
| LCDR3 | 12 | AAWDDSLNGV |

**Table 3. Amino acid sequences of variable regions for an anti-pro/latent myostatin antibody**

| **IgG chain** | **SEQ ID NO** | **Amino acid sequence** |
|---|---|---|
| Heavy chain | 13 | QVQLVESGGGVVQPGRSLRLSCAASGFTFSSYGMHWVRQAPGKG LEWVAVISYDGSNKYYADSVKGRFTISRDNSKNTLYLQMNSLRAEDT AVYYCARDLLVRFLEWSHYYGMDVWGQGTTVTVSS |
| Light chain | 14 | QSVLTQPPSASGTPGQRVTISCSGSSSNIGSNTVHWYQQLPGTAPK LLIYSDNQRPSGVPDRFSGSKSGTSASLAISGLQSEDEADYYCAAW DDSLNGVFGGGTKLTVL |

**Table 4. Amino acid sequences of full-length Ig chains for an anti-pro/latent myostatin antibody**

| **IgG chain** | **SEQ ID NO** | **Amino acid sequence** |
|---|---|---|
| Heavy chain | 15 | QVQLVESGGGVVQPGRSLRLSCAASGFTFSSYGMHWVRQAPGKG LEWVAVISYDGSNKYYADSVKGRFTISRDNSKNTLYLQMNSLRAED TAVYYCARDLLVRFLEWSHYYGMDVWGQGTTVTVSSASTKGPSVF |
| | | PLAPCSRSTSESTAALGCLVKDYFPEPVTVSWNSGALTSGVHTFPA VLQSSGLYSLSSVVTVPSSSLGTKTYTCNVDHKPSNTKVDKRVESK YGPPCPPCPAPEFLGGPSVFLFPPKPKDTLMISRTPEVTCVVVDVS QEDPEVQFNWYVDGVEVHNAKTKPREEQFNSTYRVVSVLTVLHQD WLNGKEYKCKVSNKGLPSSIEKTISKAKGQPREPQVYTLPPSQEEM TKNQVSLTCLVKGFYPSDIAVEWESNGQPENNYKTTPPVLDSDGSF FLYSRLTVDKSRWQEGNVFSCSVMHEALHNHYTQKSLSLSLG |
| Light chain | 16 | QSVLTQPPSASGTPGQRVTISCSGSSSNIGSNTVHWYQQLPGTAPK LLIYSDNQRPSGVPDRFSGSKSGTSASLAISGLQSEDEADYYCAAW DDSLNGVFGGGTKLTVLGQPKAAPSVTLFPPSSEELQANKATLVCLI SDFYPGAVTVAWKADSSPVKAGVETTTPSKQSNNKYAASSYLSLTP EQWKSHRSYSCQVTHEGSTVEKTVAPTECS |

Inhibition of myostatin activation by apitegromab may lead to buildup of promyostatin and latent myostatin. Since promyostatin is predominantly found within the skeletal muscle and latent myostatin is predominantly found in the serum, in some embodiments, serum latent myostatin levels may serve as a target engagement marker for apitegromab, e.g., target engagement with apitegromab in skeletal muscle. In some embodiments, serum latent myostatin levels and/or serum creatinine levels may be relatively low at baseline (i.e., prior to treatment) and increase following treatment with apitegromab. In some embodiments, an increase in serum latent myostatin and/or serum creatinine levels indicates target engagement with apitegromab, e.g., in skeletal muscle. In some embodiments, low baseline serum levels of latent myostatin and/or serum creatinine as compared to high levels following treatment indicates that a major portion of the drug target resides within skeletal muscle, rather than circulating systemically. In a similar manner, serum latent myostatin levels may serve as a target engagement marker for other pro/latent myostatin antibodies such as, for example, GYM329 or an antibody or antigen-binding fragment that competes with apitegromab or GYM329 for binding in an analogous manner. In some embodiments, serum latent myostatin levels may be relatively low at baseline (i.e., prior to treatment) and increase following treatment with apitegromab. In some embodiments, an increase in serum latent myostatin levels indicates target engagement with apitegromab, e.g., target engagement with apitegromab in skeletal muscle. In some embodiments, low baseline serum levels of latent myostatin as compared to high levels following treatment indicates that a major portion of the drug target resides within skeletal muscle, rather than circulating systemically.

In some embodiments, serum creatinine levels may serve as a target engagement marker for apitegromab. In some embodiments, serum creatinine levels may be relatively low at baseline (i.e., prior to treatment) and increase following treatment with apitegromab.

In some embodiments, an increase in serum creatinine levels indicates target engagement with apitegromab, e.g., target engagement with apitegromab in skeletal muscle. Serum creatinine levels may serve as a target engagement marker for another pro/latent myostatin antibody, such as, for example, GYM329 (RO7204239) or an antibody or antigen-binding fragment that competes with apitegromab or GYM329 for antigen binding. Circulating creatine, i.e., 2-[carbamimidoyl(methyl)amino]acetic acid, is transported from the circulation into muscle where is serves as am energy storage buffer. Serum creatinine is derived from these muscle stores of creatine; creatine is converted to creatinine at a constant rate of about 1.7% per day and released into the circulation. It is known that levels of serum creatinine correlate with muscle mass and inversely correlate with disease severity in spinal muscular atrophy (Alves et al (2019) Neurology 94:e921).

In some embodiments, saturation in target engagement indicates a dosage sufficient to achieve certain therapeutic effects (e.g., efficacy), although it may be possible to obtain efficacy without saturation.

In some embodiments, a composition comprising apitegromab is used in the treatment of SMA in a patient two months of age or older, wherein optionally the treatment further comprises an SMN therapy, e.g., nusinersen, risdiplam, or onasemnogene abeparvovec-xioi. In some embodiments, apitegromab and the SMN therapy are administered to the patient as a combination therapy. In some embodiments, apitegromab is administered as an add-on or adjunct therapy to the patient who has been treated with (i.e., who has received) an SMN therapy. In some embodiments, an SMN therapy is administered as an add-on or adjunct therapy to the patient who has been treated with (i.e., who has received) apitegromab. In some embodiments, apitegromab is administered intravenously. In some embodiments, apitegromab is administered subcutaneously. In some embodiments, the patient is two to seven months of age, wherein optionally the patient has or is suspected to have type 1 SMA. In some embodiments, the patient is 2 to 25 years of age, wherein optionally the patient has type 2 or type 3 SMA. In some embodiments, the patient is 5-10 years of age. In some embodiments, the patient is 2-4 years of age. In some embodiments, the patient is under 2 years old. In some embodiments, the patient is any age (e.g., infant, child, or adult) and has type 1, type 2 or type 3 SMA. In some embodiments, the patient is any age and has ambulatory SMA. In some embodiments, the patient is any age and has non-ambulatory SMA. In some embodiments, the motor activity of the patient is monitored by a wearable device. In an embodiment the wearable device is one or more instrumented insole. In some embodiments, motor function is measured as a change from baseline in the RHS, the MFM D1, the MFM D2, the MFM32 or the SMA Independence Score (SMAIS) score. In some embodiments, muscle strength is measured by myometry. In some embodiments, muscle wasting is monitored by MRI. In some embodiments circulating myostatin levels are measured. In some embodiments, immune responses are monitored.

### Motor Neuron-Directed Therapies

In some embodiments, motor neuron-directed therapies may be considered for use in the combination therapies disclosed herein.

As used herein, the term "motor neuron-directed therapy" refers to an agent aimed to improve (e.g., enhance or restore) neuronal function. Such therapies are useful for treating conditions that involve impaired signaling between a motor neuron and its target muscle. Specifically, motor neuron-directed therapies may be particularly useful in the treatment of conditions involving partial but not complete loss of neurons that innervate muscle. In some embodiments, a motor neuron-directed therapy is a gene therapy, a small molecule, or an antisense oligonucleotide. In some embodiments, a motor neuron-directed therapy is a "SMN upregulator." In some embodiments, a motor neuron-directed therapy is an agent that is capable of fully restoring motor neuron function in a cell (e.g., a cell within a subject). In some embodiments, a motor neuron-directed therapy is an agent that is capable of partially restoring motor neuron function in a cell (e.g., a cell within a subject). In some embodiments, a motor neuron-directed therapy is an agent that is capable of restoring at least 10%, 15%, 20%, 25%, 30%, 35%, 40%, 45%, 50%, 55%, 60%, 65%, 70%, 75%, 80%, 85%, 90%, 95%, 99%, or more of the motor neuron function in a cell (e.g., a cell within a subject). A skilled artisan will understand that motor neuron function typically includes membrane excitability, axonal transport, vesicle trafficking, neurotransmitter release, mitochondrial function, and/or mitochondrial availability, and such functions are measured using assays known to those of ordinary skill in the art.

In some embodiments, motor neuron-targeted therapy comprises a therapy that slows or halts neuronal degeneration. Such therapies are known in the context of Parkinson's disease and Huntington's disease. Such therapies may improve neuronal mitochondrial function, prevent protein deregulation, or reduce apoptosis. In an embodiment, a therapy that slows or halts neuronal degeneration may comprise providing progranulin (Chitramuthu et al. (2010) Molec Neurodegen 5:41) in combination with a muscle directed therapy, e.g., by providing progranulin gene therapy, e.g., with ALPHA-0602.

In some embodiments, motor neuron-targeted therapy comprises percutaneous spinal cord stimulation. In some embodiments, the percutaneous spinal cord stimulation is combined with another motor neuron-targeted therapy, e.g., as in NCT05430113. In some embodiments, motor neuron-targeted therapy comprises a therapy that affects neurotransmitter signaling. In some embodiments, motor neuron-targeted therapy comprises a therapy that reduces oxidative stress to neurons. In some embodiments, motor neuron-targeted therapy comprises neuroprotective stem cell therapy.

Exemplary motor neuron-directed therapies suitable for use in conjunction with apitegromab according to the present disclosure include but are not limited to SMN upregulator therapies (also referred to as SMN corrector therapies). Exemplary motor neuron-directed therapies include "SMN upregulators" such as splice modifiers, SMN gene replacement or gene therapy, SMN transcription enhancers, SMN protein translation enhancers, and SMN protein stabilizers further discussed below. The terms "splice corrector," "splice modulator," and "splice modifier," as used herein, are interchangeable and refer to an agent that corrects aberrant splicing of RNA transcripts, such as those encoded by the SMN2 gene and/or modulates expression of an SMN protein. In some embodiments, SMN2 splice correctors increase the inclusion of exon 7 in the SMN2 pre-mRNA. In some embodiments, increased inclusion of exon 7 in the SMN2 pre-mRNA leads to increased expression of a functional SMN protein (e.g., from an SMN2 gene) in a cell or subject, such as an SMN protein that is capable of promoting neuron function and/or survival.

In some embodiments, an SMN upregulator is an agent, for example, a small molecule or an oligonucleotide (e.g., an antisense oligonucleotide), that increases expression of a functional SMN protein, for example, by promoting the inclusion of exon 7 in an SMN2 mRNA transcript. In some embodiments, the cell is a cell within a subject, for example, a subject that is administered an SMN upregulator. In some embodiments, an SMN upregulator increases the relative amount of SMN2 mRNA that includes exon 7 as compared to SMN2 mRNA that does not include exon 7 in a cell, for example, a cell in a subject. In some embodiments, an "effective amount" of an SMN upregulator increases the amount of correctly spliced SMN2 mRNA in a cell (e.g., a cell within a subject), such that at least 10%, 15%, 20%, 25%, 30%, 35%, 40%, 45%, 50%, 55%, 60%, 65%, 70%, 75%, 80%, 85%, 90%, 95%, 99%, or more of the SMN2 mRNA within a cell contains exon 7. In some embodiments, an "effective amount" of an SMN upregulator increases a level of SMN2 mRNA containing exon 7 in a subject by at least 5%, 10%, 20%, 30%, 40%, 50%, 60%, 70%, 80%, 90%, 100%, 150%, 200%, 400%, 500%, 600%, 700%, 800%, 900%, 1000%, or more. In some embodiments, an "effective amount" of an SMN upregulator increases a level of functional SMN protein in a subject by at least 5%, 10%, 20%, 30%, 40%, 50%, 60%, 70%, 80%, 90%, 100%, 150%, 200%, 400%, 500%, 600%, 700%, 800%, 900%, 1000%, or more.

Novartis and PTC Therapeutics/Roche have both developed small molecules which selectively enhance SMN2 exon 7 inclusion, resulting in increased full-length SMN protein levels and therapeutic efficacy in mouse models of SMA (Calder et al. (2016) J Med Chem 59:10067-10083; Naryshkin et al. (2014) Science 345:688-693; Palacino et al. (2015) Nat Chem Biol 11:511-517; Ratni et al. (2016) J Med Chem 59:6086-6100). For example, risdiplam (formerly known as RO7034067, CAS# 1825352-65-5) marketed by Roche under the brand name EVRYSDI^{®}, was the first oral medication approved to treat SMA. Branaplam (formerly known as LMI070 and NVS-SM1) is being studied in a Phase 1/2 multipart first-in-human open label clinical study for infants with type 1 SMA (see U.S. clinical trial NCT02268552, Novartis).

Oral dosing of SMN-C2 (an analog of risdiplam, CAS# 1446311-56-3), and SMN-C3 (CAS# 1449597-34-5) in mild and severe preclinical models of SMA showed that the compounds increased SMN protein levels in both brain and muscle tissues in treated mice, as compared to vehicle. The molecules also efficiently crossed the blood brain barrier (BBB). In the severe SMA mouse model, both compounds showed initial promise, yet the clinical programs were paused.

Additional small molecule based SMN2 splice correctors are described in, for example, U.S. Patent Publication No. US 2009/0031435 and U.S. Patent No. 8,399,437. It should be appreciated, however, that other small molecule splice correctors including SMN2 splice correctors known in the art, e.g., risdiplam, and would be apparent to the skilled artisan, are within the scope of this disclosure.

In some embodiments, an SMN upregulator is an oligonucleotide molecule. In some embodiments, an SMN upregulator is an antisense molecule. In some embodiments, an SMN upregulator is an antisense molecule that increases expression of an SMN2 gene. In some embodiments, an SMN upregulator is an antisense molecule that increases expression of an SMN2 mRNA that contains exon 7. In some embodiments, an SMN upregulator is an antisense molecule that increases expression of functional SMN protein, for example, SMN protein encoded by SMN2 mRNA that contains exon 7. For example, antisense oligonucleotides directed to inhibit an intronic splice silencer site (ISS) in intron 7 of the SMN2 gene can modulate pre-mRNA processing, leading to a greater probability of exon 7 inclusion within the mature mRNA transcript of SMN2, resulting in the increased production of functional SMN protein.

In some embodiments, antisense oligonucleotides (ASO) block an SMN2 intronic splicing silencer thus increasing exon 7 inclusion, again rescuing disease in mouse models of SMA (Hua et al. (2010) Genes Dev 24:1634-1644; Hua et al. (2011) Nature 478:123-126; Passini et al. (2011) Sci Transl Med 3:72ra18). Biogen/lonis have developed nusinersen, an ASO splice modifier which shows clinical efficacy and was approved by the FDA for the treatment of SMA in pediatric and adult patients and marketed as Spinraza^{®} (Chiriboga et al. (2016) Neurology 86:890-897; Finkel et al. (2016) Lancet 388:3017-3026; FDA, Nusinersen; Office of drug evaluation decisional memorandum (2016)). However, each administration of nusinersen requires intrathecal delivery under general anesthesia. Additionally, while the antisense corrector nusinersen has proven promising, clinical efficacy appears modest: 60% of patients among infantile-onset SMA (type I) are reported to be non-responders, and 43% of nusinersen-treated patients did not attain ≥ 3-point increase as measured by HFMSE, while mean increase was less than six points in the treated patient group, as compared to placebo. Thus, only a partial improvement is achieved by nusinersen treatment.

While these ASO treatments have shown efficacy preclinically, and nusinersen or clinically, none offers a complete cure for the disease. In mouse models, while both the small molecule and ASO splice modifiers significantly reduce disease severity, treated animals nevertheless have deficits in longevity, body weight, muscle mass, and muscle function compared to healthy animals (Hua et al. (2011) Nature 478:123-126; Feng et al. (2016) Hum Mol Genet 25:964-975). In a double-blinded clinical trial in infantile-onset SMA, nusinersen resulted in clinically meaningful benefits at the time of interim analysis (41% of treated versus 0% placebo had improvements in motor milestones using the Hammersmith Infant Neurological Examination). The motor function milestones reached are impressive for type I patients, with five of 81 treated patients able to sit unaided (a milestone almost never reached in these patients). Nevertheless, these patients have not achieved the full range of developmental milestones, and in a normal individual the achieved milestones would be considered disappointing. In a second placebo-controlled trial in type 2 SMA, nusinersen again showed clinically meaningful improvements, with scores on the HFMSE increasing by 5.9 points relative to the placebo group. Note that the maximum score on the HFMSE is 66 points, with most type 2 patients scoring below 20 (Glanzman et al. (2011) J Child Neurol 26:1499-1507; Kaufmann et al. (2011) Arch Neurol 68:779-786). Nevertheless, 43% of patients failed to achieve at least a 3-point improvement in motor function in this trial. These results indicate that, while SMN2 splice modulators have potential for significant effects on SMA disease course, additional functional gains are needed to further improve reduce disease burden.

In some embodiments, an SMN upregulator is a gene therapy. As used herein, the term "gene therapy" refers to any procedure that uses nucleic acids to heal, cure, or otherwise improve a condition in a subject. In gene therapy, nucleic acids are delivered into specific cells. Delivery methods include viral and non-viral means, which are known in the art. See, for example, Patil et al. (2005) AAPS J. 7(1): E61-E77; Gascon et al., Non-Viral Delivery Systems in Gene Therapy (2013); Somiari et al. (2000) Molecular Therapy, 2(3):178-187; Herweijer and Wolff (2003) Gene Therapy 10(6):453-458; Nayerossadat et al. (2012) Advanced Biomedical Research 1(2):1-11. Viral means for delivering gene therapy involve the use of viral vectors. Viral vectors are genetically modified viruses that can carry a therapeutic genetic payload and have been reprogrammed to allow for infection and subsequent transmittal of said payload into specific tissues without the side effects typically associated with wild-type viral infection. A number of viruses can be used as viral vectors, including retroviruses, adenoviruses, herpes simplex virus, lentiviruses, Poxvirus, and Epstein-Barr virus. While safer than wild-type viruses, viral vectors may induce an immune response, occasionally necessitating the use of non-viral delivery methods. In some embodiments, the viral vector is an AAV viral vector. Non-viral delivery methods include, but are not limited to, physical methods, such as injection of naked DNA, electroporation, gene gun bombardment, and ultrasound, as well as biochemical methods. Another delivery technique, magnetofection, combines physical and biochemical elements.

SMN1 gene replacement therapy using adeno-associated viral vectors (AAV) has shown benefit in treatment of SMA; ZOLGENSMA^{®} (onasemnogene abeparvovec-xioi, formerly known as AVXS-101), an AAV9-SMN1 vector from Novartis Gene Therapies (formerly AveXis), has been approved in the U.S. by the FDA for treatment of the treatment of pediatric patients less than two years of age with spinal muscular atrophy (SMA) with bi-allelic mutations in the survival motor neuron 1 (SMN1) gene. ANB-004, an AAV9-SMN1 vector from Biocad (WO 2022/164351), is in a Phase 1/2 clinical trial directed to symptomatic infants with SMA who are under six months of age.

In some embodiments, the gene therapy comprises introduction of one or more transgenes into the patient. In some embodiments, gene transfer is achieved by the use of a suitable vector, such as viral vectors and lipid-based carriers. For viral-vector-mediated gene delivery, the gene therapy may involve the use of a particular serotype for an initial treatment, followed by a different serotype for the subsequent treatment, in order to minimize adverse immune responses in the subject. In some embodiments, the gene therapy involves targeted genome editing, such as the CRISPR/Cas9 technology or variant thereof. Non-limiting examples of SMN upregulators suitable for use in conjunction with apitegromab according to the present disclosure include but are not limited to: nusinersen; risdiplam; and onasemnogene abeparvovec-xioi. Nusinersen is an SMN2-directed antisense oligonucleotide (ASO) designed to treat SMA caused by mutations that lead to SMN protein deficiency. *See, e.g.,* Darras et.al. (2019) Neurology. 92(21) e2492-e2506; Mercuri et.al. (2018) N Engl J Med. 378:625-635. Risdiplam works in a similar way and is a pyridazine derivative that modifies the splicing of SMN2 messenger RNA. *See, e.g.,* Oskoui et al. "SUNFISH Part 2: 24-month efficacy and safety of risdiplam in patients with type 2 or non-ambulant type 3 spinal muscular atrophy (SMA)." Presented at MDA Clinical and Scientific Conference 2021; March 15-18. Poster 80. Onasemnogene abeparvovec-xioi is a recombinant adeno-associated virus vector 9-based gene therapy designed to deliver a copy of the gene encoding the human SMN protein.

### Clinical Benefits of SMN Therapies

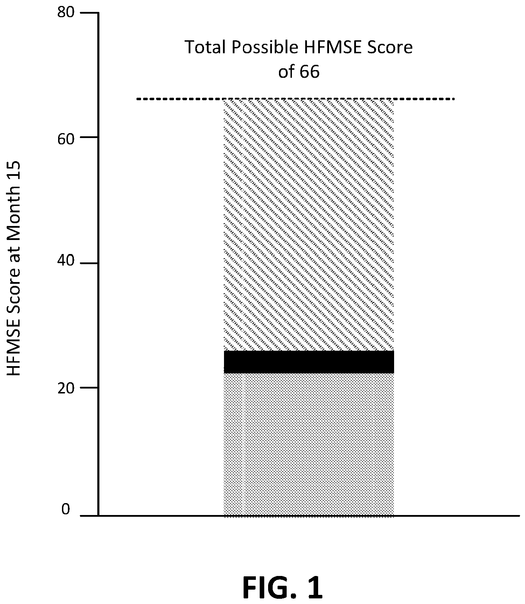

Based on the natural history (i.e., are untreated) of non-ambulatory later-onset SMA patients who are five years or older, mean HFMSE scores are expected to decline over a 12-month period, with fewer than 5% showing a 3-point or greater increase (Mercuri et al. (2016) Neuromuscul Disord. 26(2):126-131). Natural history data from a longitudinal study in patients with ambulatory type 3 SMA offer additional insights. These patients commonly experience motor function declines, which in some cases are severe, e.g., loss of ambulation (Coratti et al. (2020) American Neurology Association, 88:1109-1117, e.g., at Fig. 1). In this study of 130 subjects with a mean age of 10.05 and a mean HFMSE score of 52.81 at baseline, at 12 months the mean change in HFMSE from baseline was -0.79 points and 11 patients had lost ambulation (mean age of ambulation loss was 10.21 years (SD±6.43)). Until seven years of age the subjects were relatively stable, showing modest functional improvement prior to the steeper decline observed in subsequent years.

Among non-ambulatory later-onset SMA patients who are five years or older, based on the natural history of this patient population, mean HFMSE scores are expected to decline over a 12-month period, with few patients (e.g., less than 5%) showing a 3-point or greater increase in HFMSE score (Mercuri et al. (2016) Neuromuscul Disord. 26(2):126-131). Among type 2 and type 3 SMA patients who were not receiving a disease-modifying therapy, the natural history over 24 months showed declines in both upper limb strength and MFM32 scores (Annoussamy et al. (2020) Annals of Clinical and Translational Neurology 8:359-373).

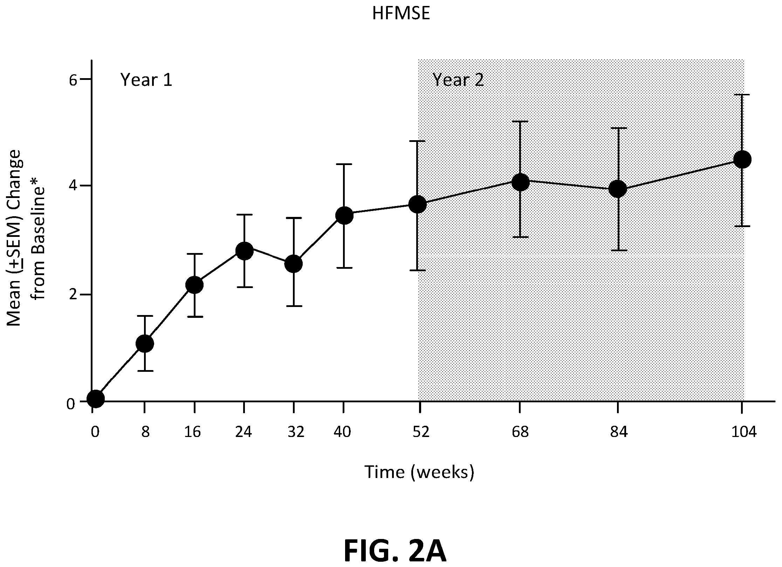

Against this background, nusinersen has been reported to provide limited clinical benefits in patients who started the therapy at age five or older. More specifically, in the CHERISH study, in patients who started on nusinersen at age five or older, there was a mean HFMSE decline of >0.5 points after 15 months of nusinersen treatment, and <15% of the patients showed a 3-point or greater increase in HFMSE score over that period (Mercuri et.al. (2018) N Engl J Med. 378:625-635, e.g., at Figure 2A). These patients were further observed beyond the 15 months in the earlier study. In the CHERISH trial, the majority of patients in this age range do not experience HFMSE improvements and rarely achieve a 3+ point increase.

Nusinersen treatment was shown to improve motor function during the first year of treatment and then plateau. In the initial CHERISH and subsequent SHINE trials, subjects achieved a 3.9-point increase in HFMSE score from baseline after the first 450 days of nusinersen treatment (mean ± SE) and a 4.6-point increase from baseline after 1650 days of treatment (mean ± SE). Thus, after approximately the first year of treatment, nusinersen provided less than one point improvement in HFSME over the long term (Mercuri et al., presented at World Muscle Society Congress 2020, p. 257).

**FIG. 1** illustrates that disease burden persists after SMN treatment. The bottom grey bar represents the baseline HFMSE score observed in the Phase 3 CHERISH trial after 15 months treatment with nusinersen. The solid black bar above it represents the mean improvement in HFMSE score in patients with type 2 and non-ambulatory type 3 SMA. The diagonally striped bar represents the opportunity for improvement for these patients to reach the total possible score of 66. Thus, limitations in mobility persist (Darras et al. (2019) Neurology 92(21):e2492-e2506). Furthermore, limitations in mobility and daily activities continue and are associated with a gradual deterioration in motor function ((Yang et al. (2022) Adv Ther 39:1915; Wan et al. (2020) Orphanet J Rare Dis 15:70).

A study reporting the efficacy and safety of risdiplam in type 2 and non-ambulant type 3 SMA patients after 24 months of treatment (SUNFISH) showed no improvement in the 32-item Motor Function Movement outcome (MFM32) between 12 and 24 months (Oskoui et al. (2023) J. Neurol. https://doi.org/10.1007/s00415-023-11560-1). The MFM32 mean change in baseline was 1.7 points at 12 months ((0.8-2.5; 95% CI) and 1.8 points at 24 months (0.7-2.9; 95% CI). Nusinersen and risdiplam data consistently show that unless initiated early in life, treatment with SMN therapy results in an initial period of improvement followed by disease stabilization, with limited or no continuous improvement (Mercuri et al. (2018) N Engl J Med 378(7):625-635; Mercuri et al. (2020) 2nd Int'l Sci Clin Cong SMA). Thus, long-term treatment with an SMN-targeted therapy appears to initially improve, then stabilize the disease, e.g., it may prevent further loss of motor function. There continues to be a need for additional therapies that improve motor function in patients treated with SMN-targeted therapies (Mercuri et al. SUNFISH Part 2, presented at Amer Acad Neurol 2020).

### Add-On/Adjunct Therapy, Combination Therapy

The present disclosure includes add-on therapy (also referred to as adjunct therapy) and combination therapy that comprise a first agent which is a motor neuron-directed therapy (e.g., SMN therapy) and a second agent which is a muscle-targeted therapy (e.g., myostatin inhibitor, e.g., apitegromab) aimed to treat SMA in patients. The two agents are used in conjunction with each other to enhance therapeutic effects. Add-on or adjunct therapy means that therapy is administered to a patient who is on a background therapy. For example, the muscle-targeted therapy such as apitegromab may be administered to SMA patients who are already receiving an SMN therapy. In comparison, a combination therapy refers to the administration of both therapies to a patient as part of a coordinated or predetermined therapeutic regimen by a physician or a team of physicians. Combinations and add-on therapy may be administered separately or in the same setting, e.g., during the same clinical visit.

In various embodiments, the present disclosure provides use of an anti-pro/latent myostatin antibody (i.e., apitegromab) for the treatment of SMA in patients who also receive a therapy to address the motor neuron defect, e.g., an SMN upregulator. The present disclosure encompasses methods for treating SMA in a subject who is treated with an SMN upregulator, comprising administration of an anti-pro/latent myostatin antibody. According to the invention, the anti-pro/latent myostatin antibody is apitegromab.

In some embodiments, apitegromab used in conjunction with an SMN therapy provides additive clinical benefits. In some embodiments, apitegromab used in conjunction with an SMN therapy provides synergistic clinical benefits. "Synergistic" means that when the two agents are used in conjunction with each other, either as add-on therapy or combination therapy, they together achieve efficacy that is greater than a sum of effects obtained by monotherapy of each.

Apitegromab may be administered to SMA patients who are either responders, poor responders, or non-responders of an SMN upregulator therapy. For poor responders or non-responders, concurrent inhibition of myostatin signaling may improve neuromuscular signaling due in part to enhanced muscle function, thereby rendering the innervating motor neurons of non-responders more responsive to the SMN upregulator. Without wishing to be bound by particular theory, it is contemplated that enhancement of the muscle component may affect the neuronal component by positive feedback, and vice versa, due to the bidirectional nature of neuromuscular signaling.

Although SMN upregulator poor responders and/or non-responders may nevertheless benefit from myostatin inhibition, an exemplary patient population includes those who are responders of an SMN upregulator. It is contemplated that motor function in these individuals may be further improved by a myostatin inhibition therapy such as apitegromab used in conjunction with a motor neuron-directed therapy, such as an SMN upregulator therapy.

The phrase "in conjunction with," in the context of treatment regimens for SMA that comprise two or more therapeutic agents (e.g., apitegromab used in conjunction with an SMN upregulator therapy), means that therapeutic effects of a first therapy overlaps temporally and/or spatially with therapeutic effects of a second and/or additional therapy in the subject receiving the two or more therapies. The two or more therapies need not be administered as a single formulation, nor do they have to be administered concurrently, nor via the same route. The first, second, and/or additional compositions may be administered concurrently (e.g., simultaneously), separately, or sequentially. Thus, the two or more therapies may be formulated as a single formulation for concurrent administration, or as separate formulations, for sequential, concurrent, or simultaneous administration of the therapies. When a subject who has been treated with a first therapy to treat SMA (e.g., apitegromab) is administered with a second and additional therapies to treat the SMA (e.g., an SMN upregulator therapy), the second and additional therapies may be referred to as an "add-on" therapy or an "adjunct" or "adjunctive" therapy.

In some embodiments, the treatment regimens described herein that comprise two or more therapeutic agents (e.g., apitegromab used in conjunction with an SMN-targeted therapy such as an upregulator therapy) achieve improved clinical benefits in patients as compared to a monotherapy using each agent alone. Specifically, targeting affected muscles with a myostatin inhibition therapy such as apitegromab, in conjunction with a motor neuron-directed therapy such as an SMN upregulator therapy, may produce a beneficial clinical outcome relative to the myostatin inhibition therapy or motor neuron-directed therapy alone. Such effects may be additive or synergistic as compared to the respective monotherapies. In some embodiments, the effects of combining the two or more therapies are additive, i.e., the effects of the therapies used together are equal or about equal to the sum of the effects of the therapies when used independently. In some embodiments, the effects of combining the two or more therapies are synergistic, i.e., super-additive, i.e., the effects of the therapies used together are greater than the sum of the effects of the therapies when used independently.

In some embodiments, one or more beneficial therapeutic effects of using apitegromab in conjunction with an SMN upregulator therapy (e.g., an effect on at least one symptom or the risk/rate of disease progression) is/are additive. In some embodiments, one or more beneficial therapeutic effects of using apitegromab in conjunction with an SMN upregulator therapy (e.g., an effect on at least one symptom or the risk/rate of disease progression) is/are synergistic. In some embodiments, apitegromab used in conjunction with an SMN upregulator therapy has a combined effect that is additive, synergistic, and/or provides one or more additional combination benefits. In some embodiments, apitegromab used in conjunction with an SMN upregulator therapy provides additive or synergistic effects on at least one efficacy parameter for SMA (e.g., an HFMSE score or an RULM score). In some embodiments, apitegromab used in conjunction with an SMN upregulator therapy provides additive or synergistic effects on an HFMSE score in a SMA patient and/or an SMA patient population. In some embodiments, apitegromab used in conjunction with an SMN upregulator therapy provides further increases in the HFMSE score, as compared to treatment with an SMN upregulator therapy alone (see, e.g., Example 1 and Darras et.al. (2019) Neurology. 92(21) e2492-e2506). In some embodiments, the further improvements in HFMSE score are additive. In some embodiments, the further improvements in HFMSE score are synergistic. In some embodiments, apitegromab used in conjunction with an SMN therapy provides additive or synergistic effects on an RULM score in a SMA patient and/or an SMA patient population. In some embodiments, apitegromab used in conjunction with an SMN therapy provides further increases in an RULM score, as compared to treatment with an SMN therapy alone. In some embodiments, the further improvements in RULM score are additive. In some embodiments, the further improvements in RULM score are synergistic. In some embodiments, apitegromab used in conjunction with an SMN therapy provides additive or synergistic effects on an RULM score in an SMA patient over at least 24 months, wherein, optionally, the combination therapy improves the RULM score over 24 months or the combination therapy maintains an increase in RULM score over 24 months.

### Patient Selection

The disclosure includes identification or selection of suitable human subjects to be treated with a selective myostatin inhibitor, such as apitegromab. Suitable human subjects are patients who are likely to benefit from apitegromab therapy, either as monotherapy or in conjunction with (or in combination with) another therapy.

In some embodiments, a patient or patient population is an early- or infantile-onset SMA patient or patient population. In some embodiments, a patient or patient population is a later-onset SMA patient or patient population. In some embodiments, a patient or patient population is a type 2 SMA patient or patient population. In some embodiments, a patient or patient population is a non-ambulatory type 3 SMA patient or patient population. In some embodiments, a patient or patient population is an ambulatory type 3 SMA patient or patient population. In some embodiments, a patient or patient population is an ambulatory later-onset SMA patient or patient population. In some embodiments, a patient or patient population is a non-ambulatory patient or patient population. In some embodiments, a patient or patient population is a symptomatic patient or patient population. In some embodiments, a patient or patient population has two or more copies of the SMN2 gene, e.g., 2-3 copies, 2-4 copies, 3-4 copies, etc. In some embodiments, a patient or patient population has later-onset SMA and has two or more copies of the SMN2 gene, e.g., 2-3 copies, 2-4 copies, 3-4 copies, etc.

SMA patients who are likely to benefit from such therapy include those who meet one or more of the following criteria: has a documented diagnosis of 5q SMA and later-onset (e.g., type 2 or 3) SMA prior to receiving a therapy for SMA; a non-ambulatory subject who is able to sit independently per WHO motor milestones definition; an ambulatory subject who is able to independently ambulate without aids over 10 meters in 30 seconds or less; a subject having an RHS score of less than or equal to 63 and/or an HFMSE score of 10 or greater (e.g., having an HFMSE score ≥10 and ≤45 at screening); a subject who does not use tracheostomy with positive pressure or chronic daytime non-invasive ventilatory support for greater than 16 hours daily within two weeks prior to treatment; a subject who does not have any acute or comorbid condition interfering with the well-being of the subject within two weeks prior to treatment; a subject who does not have severe scoliosis or contractures; and/or subject who does not use systemic corticosteroids, valproic acid, or therapies with potential muscular or neuromuscular effects within 60 days except approved SMN up-regulator (also known as SMN corrector) therapy. Therapies with potential muscular or neuromuscular effects include androgens, insulin-like growth factor, growth hormone, systemic beta-agonist, botulinum toxin, muscle relaxants, muscle enhancing supplements or acetylcholinesterase inhibitors. In some embodiments, the patient has a documented diagnosis of 5q SMA and later-onset (e.g., type 2 or 3) SMA prior to receiving a therapy for SMA and meets one or more of the additional criteria listed above. In some embodiments, there is a positive correlation between age-normalized change in motor function and age-normalized fold change in latent myostatin levels, e.g., in patients with type 2 SMA. In various embodiments, the methods disclosed herein include the selection of such a patient or patient population(s) for treatment with apitegromab, e.g., according to a dosage or regimen disclosed herein.

SMA patients who may be responsive to apitegromab therapy, either as monotherapy or in conjunction with (or in combination with) another therapy, include those having any type of SMA up to the age of two years, as well as those who show a phenotype of SMA type 1 or have up to three SMN2 copies. In some embodiments, apitegromab may have enhanced therapeutic efficacy in younger patients, e.g., a population who is less than 21 years of age, because the patients have greater background anabolic activity. In some embodiments, younger patients, e.g., patients less than 21 years of age, or patients who are anabolically active, receive the apitegromab therapy. In some embodiments, the patient is aged two years or younger, e.g., newborn to 24 months old. In some embodiments, the patient is six weeks or younger, e.g., newborn to six weeks old. In some embodiments, the patient is 2-21 years old. In some embodiments, the patient is 13-21 years old. In some embodiments, the patient is aged 12 years or younger. In some embodiments, the patient is 2-12 years old. In some embodiments, the patient is 5-12 years old. In some embodiments, apitegromab may be particularly efficacious in patients before the start of puberty (e.g., before age 12). In some embodiments, apitegromab treatment may be efficacious in preventing drastic decline in motor function associated with the start of puberty in younger patients (e.g., patients younger than 12 years old). In some embodiments, the patient is about two years old. In some embodiments, the patient is younger than two years old. In some embodiments, the patient shows a phenotype of SMA type 1. In some embodiments, the patient has up to three SMN2 copies. In various embodiments, the methods disclosed herein include the selection of such a patient or patient population(s) for treatment with apitegromab, e.g., according to a dosage or regimen disclosed herein.

Patients who may be responsive to apitegromab therapy, either as monotherapy or in conjunction with (or in combination with) another therapy, include patients having type 2 SMA with baseline (i.e., prior to the start of apitegromab therapy) serum latent myostatin (LM) concentrations of at least 1 ng/mL (e.g., greater than 1.5 ng/ml, greater than 2 ng/ml, greater than 2.5 ng/ml, greater than 3 ng/ml, or greater). In some embodiments, an apitegromab therapy in conjunction with (or in combination with) another therapy, may be more effective in patients with higher baseline serum LM concentrations as compared to patients with lower baseline serum LM concentrations. In some embodiments, such therapy may be particularly effective in patients who are younger than 12 years of age. In some embodiments, such therapy may be particularly effective in patients who are 2-5 years of age. In some embodiments, such therapy may be particularly effective in patients with type 2 SMA who are 12-21 years of age. In some embodiments, such therapy may be particularly effective in patients with type 3 SMA who are 12-21 years of age. In some embodiments, such therapy may be particularly effective in nonambulatory patients who are 2-21 years of age. In some embodiments, such therapy may be particularly effective in nonambulatory patients who are 2-12 years of age. In some embodiments, such therapy may be particularly effective in type 2 and type 3 nonambulatory patients who are 2-21 years of age. In some embodiments, such therapy may be effective in patients who are younger than two years of age. In some embodiments, type 2 SMA patients who are younger than 12 years of age and have baseline serum LM concentrations of at least 1 ng/mL (e.g., greater than 1.5 ng/ml, greater than 2 ng/ml, greater than 2.5 ng/ml, greater than 3 ng/ml, or greater) may be responsive to apitegromab therapy. In some embodiments, type 2 SMA patients who are younger than two years of age and have baseline serum LM concentrations of at least 1 ng/mL (e.g., greater than 1.5 ng/ml, greater than 2 ng/ml, greater than 2.5 ng/ml, greater than 3 ng/ml, or greater) may be responsive to apitegromab therapy. In some embodiments, the apitegromab therapy comprises administering more than 2 mg/kg apitegromab, e.g., 10 mg/kg or 20 mg/kg.

In some embodiments, a method of treating SMA comprises administering an apitegromab therapy in conjunction with (or in combination with) another therapy, to a patient having baseline serum LM concentrations of at least 1 ng/mL (e.g., greater than 1.5 ng/ml, greater than 2 ng/ml, greater than 2.5 ng/ml, greater than 3 ng/ml, or greater). In some embodiments, a method of treating SMA comprises administering an apitegromab therapy in conjunction with (or in combination with) another therapy, to a patient who is younger than 12 years of age and who has baseline serum LM concentrations of at least 1 ng/mL (e.g., greater than 1.5 ng/ml, greater than 2 ng/ml, greater than 2.5 ng/ml, greater than 3 ng/ml, or greater). In some embodiments, a method of treating SMA comprises administering an apitegromab therapy in conjunction with (or in combination with) another therapy, to a patient who is younger than 2 years of age and who has baseline serum LM concentrations of at least 1 ng/mL (e.g., greater than 1.5 ng/ml, greater than 2 ng/ml, greater than 2.5 ng/ml, greater than 3 ng/ml, or greater). In some embodiments, the apitegromab therapy comprises administering more than 2 mg/kg apitegromab, e.g., 10 mg/kg or 20 mg/kg.

In some embodiments, apitegromab is used for treating SMA in conjunction with (or in combination with) another therapy, in a patient having baseline serum LM concentrations of at least 1 ng/mL (e.g., greater than 1.5 ng/ml, greater than 2 ng/ml, greater than 2.5 ng/ml, greater than 3 ng/ml, or greater). In some embodiments, apitegromab is used for treating SMA in conjunction with (or in combination with) another therapy, in a patient younger than 12 years of age having baseline serum LM concentrations of at least 1 ng/mL (e.g., greater than 1.5 ng/ml, greater than 2 ng/ml, greater than 2.5 ng/ml, greater than 3 ng/ml, or greater). In some embodiments, apitegromab is used for treating SMA in conjunction with (or in combination with) another therapy, in a patient younger than two years of age having baseline serum LM concentrations of at least 1 ng/mL (e.g., greater than 1.5 ng/ml, greater than 2 ng/ml, greater than 2.5 ng/ml, greater than 3 ng/ml, or greater). In some embodiments, the apitegromab is suitable for administering at more than 2 mg/kg apitegromab, e.g., 10 mg/kg or 20 mg/kg.

In various embodiments, an SMA patient (e.g., any of the exemplary SMA patients described above) is on (is receiving) or has been treated with an SMN-targeted therapy. In some embodiments, the SMN-targeted therapy comprises nusinersen, risdiplam, and/or onasemnogene abeparvovec-xioi. In some embodiments, the patient is on (is receiving) or has been treated with nusinersen. In some embodiments, the patient has been previously treated with nusinersen. In some embodiments, the patient is on (is receiving) or has been treated with risdiplam. In some embodiments, the patient has been previously treated with risdiplam. In some embodiments, the patient is on (is receiving) or has previously received onasemnogene abeparvovec-xioi.

In various embodiments, the present disclosure provides methods of treating a patient or patient population(s) with apitegromab in conjunction with (or in combination with) a SMN-targeted therapy, wherein the patient or patient population(s) meets one or more of the following criteria: has any type of SMA before or up to the age of two years, shows a phenotype of SMA type 1, and/or has up to three SMN2 copies. In some embodiments, treatment comprises apitegromab therapy in conjunction with (or in combination with) a SMN-targeted therapy, such nusinersen, risdiplam, or onasemnogene abeparvovec-xioi, e.g., according to a dosage or regimen disclosed herein. In some embodiments, the patient is on (is receiving) or has been treated with nusinersen. In some embodiments, the patient has been previously treated with nusinersen. In some embodiments, the patient is on (is receiving) or has been treated with risdiplam. In some embodiments, the patient has been previously treated with risdiplam. In some embodiments, the patient has previously received onasemnogene abeparvovec-xioi.

Patients may be symptomatic or pre-symptomatic.

Pre-symptomatic patients include those who have been genetically identified as carriers of one or more mutations in the SMN1 gene, alone or in combination with identification of one or more additional genetic modifications (e.g., a determination of SMN2 copy number). Genetic identification of SMA patients may be carried out as part of newborn or in utero screening. At the time of the identification (e.g., diagnosis), the patient (e.g., newborn) may not show obvious/apparent symptoms (asymptomatic) but based on the genetic characteristics and possibly other factors, the patient may be expected to develop the disease.

In severe cases, newborn babies may already show sign of the disease (i.e., symptomatic). Typically, such a patient is likely to have type 1 SMA based on the natural history of SMA and is not generally expected to gain the ability to sit independently without intervention.

In some embodiments, the patient is a newborn patient (age of zero to six months). In some embodiments, the patient is a pediatric patient, between the age of six months and 17 years. In some embodiments, the patient is younger than five years old. In some, the patient is five years old or younger, e.g., 2-5 years of age. In some embodiments, the patient is aged two years or younger, e.g., newborn to 24 months old. In some embodiments, the patient is aged six weeks or younger, e.g., newborn to six weeks old. In some embodiments, the patient is two years old or older. In some embodiments, the patient is 2-10 years of age. In some embodiments, the patient is 2-12 years of age. In some embodiments, the patient is 13-21 years of age. In some embodiments, the patient is five years old or older, e.g., 5-17 years of age. In some embodiments, the patient is between the ages of 5-21 years. In some embodiments, the patient is an adult.

In some embodiments, the patient has received or is treated with an SMN corrector therapy, such as an SMN2 upregulator therapy and/or an SMN1 gene therapy. In some embodiments, the patient initiates or initiated with the SMN corrector therapy before the age of five. In some embodiments, the patient initiates or initiated the SMN corrector therapy at or after the age of five.

In some embodiments, the patient is showing disease progression such that motor function is declining, indicated by a one point or greater decrease in a motor function assessment test scores (e.g., HFMSE, RHS, and/or RULM scores) over a period of 12 months or 24 months, prior to treatment with apitegromab.

In some embodiments, a patient treated herein is in a period of stable disease decline such that the rate of decline in motor function (e.g., as measured by HFMSE and RHS scores) has not significantly changed over a period of at least six months prior to starting treatment with apitegromab. In some embodiments, the patient has stable disease such that the patient's motor function (e.g., as measured by HFMSE and RHS scores) has not significantly changed over a period of at least six months prior to starting treatment with apitegromab. In some embodiments, the patient has non-ambulatory SMA. In some embodiments, the patient has type 2 or type 3 non-ambulatory SMA. In some embodiments, the patient has ambulatory SMA. In some embodiments, the patient has type 3 ambulatory SMA. In some embodiments, the patient initiated SMN upregulator/corrector therapy before the age of five. In some embodiments, the patient initiated SMN upregulator/corrector therapy after the age of five. In some embodiments, the patient is aged two years or younger, e.g., newborn to 24 months old. In some embodiments, the patient is 2-12 years old. In some embodiments, the patient is aged six weeks or younger, e.g., newborn to six weeks old. In some embodiments, the patient is 2-12 years old and initiated SMN upregulator/corrector therapy (e.g., nusinersen, risdiplam, and/or onasemnogene abeparvovec-xioi) before the age of 5. In some embodiments, the patient is 2-12 years old and initiated SMN upregulator/corrector therapy (e.g., nusinersen, risdiplam, and/or onasemnogene abeparvovec-xioi) after the age of five. In some embodiments, the patient is 5-12 years old and initiated SMN upregulator/corrector therapy (e.g., nusinersen, risdiplam, and/or onasemnogene abeparvovec-xioi) after the age of five.

In some embodiments, the patient is a patient with 5q spinal muscular atrophy (SMA) with a bi-allelic mutation in the SMN1 gene and a clinical diagnosis of SMA type 1, or the patient is a patient with 5q SMA with a bi-allelic mutation in the SMN1 gene and up to three copies of the SMN2 gene. Such a patient may be treated with a selective myostatin inhibitor in conjunction with a gene therapy (e.g., SMN1 gene therapy). In some embodiments, the patient weighs between 2.6 kg and 21.0 kg at the time of receiving the gene therapy. In some embodiments, the patient's motor milestones may be monitored, such as head control, rolling from back to sides, sitting without support for 30 seconds, sitting without support for at least 10 seconds, etc.

In some embodiments, the patient is a type 2 SMA patient who is able to sit but has never walked, wherein the patient is between 2 and 18 years of age. In some embodiments, an SMN1 gene therapy is used in the treatment of type 2 SMA in a patient who is between 2-18 years of age and who is able to sit but has never gained the ability to walk, and wherein the patient is treated with a myostatin inhibitor, wherein the myostatin inhibitor is the myostatin-selective inhibitor, apitegromab. In some embodiments, a myostatin inhibitor is used in the treatment of type 2 SMA in a patient who is between 2-18 years of age and who is able to sit but has never gained the ability to walk, and wherein the patient is treated with a SMN1 gene therapy, wherein optionally the SMN1 gene therapy comprises onasemnogene abeparvovec-xioi. According to the invention, the myostatin inhibitor is the myostatin-selective inhibitor, apitegromab. In some embodiments, an SMN1 gene therapy and a myostatin inhibitor are used as a combination therapy in the treatment of SMA in a patient who is between 2-18 years of age and who is able to sit but has never gained the ability to walk, and wherein the patient is treated with a myostatin inhibitor, wherein the myostatin inhibitor is the myostatin-selective inhibitor, apitegromab, and wherein optionally, the SMN1 gene therapy comprises onasemnogene abeparvovec-xioi. An antibody variant may have nucleic acid and/or amino acid sequences of significant homology (e.g., >90% sequence identity) and retains one or more physical and/or functional properties as compared to a known antibody.

In some embodiments, the patient has a stable disease as assessed by motor function, such that the motor function assessment test score (e.g., HFMSE and RHS scores) has not significantly changed over a period of at least six months prior to treatment with apitegromab. In some embodiments, the patient has received or is treated with an SMN corrector therapy.

In some embodiments, the patient has undergone spinal fusion, e.g., primary posterior spinal fusion.

According to the present disclosure, patient selection or categorization may be based on the highest motor milestone achieved, relative to the number of the SMN2 gene copies. Thus, the present disclosure includes therapeutic use of a myostatin-selective inhibitor (apitegromab) in the treatment of SMA in a patient, wherein the treatment comprises administration of a composition comprising the myostatin-selective inhibitor. According to the invention, the myostatin-selective inhibitor is apitegromab, which may be intravenously administered at a therapeutic dose, wherein the therapeutic dose is greater than 2 mg/kg and up to 20 mg/kg (such as 10 mg/kg). In some embodiments, the patient has 1, 2, 3 or 4 copies of the SMN2 gene and achieves one or more of the gross motor milestones of: 1) raises/supports head (i.e., neck holding, e.g., while laying on their stomach able to raise or hold head); 2) rolls over; 3) sits in tripod position (e.g., uses hands to support self while sitting) or sit with support; 4) sits without support; 5) stands with support/assistance; 6) creeps/crawls; 7) pulls to standing position; 8) walks with assistance (e.g., cruises along furniture); 9) stands without support; 10) takes several independent steps but falls; 11) walks alone (e.g., walks independently, walks without support); 12) squats to pick up an object (e.g., a toy); 13) walks/creeps up and down the stairs; 14) runs; 15) jumps; 16) alternates feet going upstairs; 17) hops on one foot; 18) alternates feet going downstairs.

In some embodiments, the patient has one copy of the SMN2 gene, and the highest motor milestone achieved is to raise or hold head while lying on stomach. In some embodiments, the patient has one copy of the SMN2 gene, and the highest motor milestone achieved is to roll over. In some embodiments, the patient has one copy of the SMN2 gene, and the highest motor milestone achieved is to sit with support. In some embodiments, the patient has one copy of the SMN2 gene, and the highest motor milestone achieved is to sit without support. In some embodiments, the patient has one copy of the SMN2 gene, and the highest motor milestone achieved is to stand with support. In some embodiments, the patient has one copy of the SMN2 gene, and the highest motor milestone achieved is to crawl. In some embodiments, the patient has one copy of the SMN2 gene, and the highest motor milestone achieved is to pull to standing position. In some embodiments, the patient has one copy of the SMN2 gene, and the highest motor milestone achieved is to walk with assistance. In some embodiments, the patient has one copy of the SMN2 gene, and the highest motor milestone achieved is to stand without support. In some embodiments, the patient has one copy of the SMN2 gene, and the highest motor milestone achieved is to walk independently (without support). In some embodiments, the patient has one copy of the SMN2 gene, and the highest motor milestone achieved is to run. In some embodiments, the patient has one copy of the SMN2 gene, and the highest motor milestone achieved is to jump. In some embodiments, the patient has one copy of the SMN2 gene, and the highest motor milestone achieved is to alternate feet going upstairs. In some embodiments, the patient has one copy of the SMN2 gene, and the highest motor milestone achieved is to hop on one foot. In some embodiments, the patient has one copy of the SMN2 gene, and the highest motor milestone achieved is to alternate feet going downstairs.

In some embodiments, the patient has two copies of the SMN2 gene, and the highest motor milestone achieved is to raise or hold head while lying on stomach. In some embodiments, the patient has two copies of the SMN2 gene, and the highest motor milestone achieved is to roll over. In some embodiments, the patient has two copies of the SMN2 gene, and the highest motor milestone achieved is to sit with support. In some embodiments, the patient has two copies of the SMN2 gene, and the highest motor milestone achieved is to sit without support. In some embodiments, the patient has two copies of the SMN2 gene, and the highest motor milestone achieved is to stand with support. In some embodiments, the patient has two copies of the SMN2 gene, and the highest motor milestone achieved is to crawl. In some embodiments, the patient has two copies of the SMN2 gene, and the highest motor milestone achieved is to pull to standing position. In some embodiments, the patient has two copies of the SMN2 gene, and the highest motor milestone achieved is to walk with assistance. In some embodiments, the patient has two copies of the SMN2 gene, and the highest motor milestone achieved is to stand without support. In some embodiments, the patient has two copies of the SMN2 gene, and the highest motor milestone achieved is to walk independently (without support). In some embodiments, the patient has two copies of the SMN2 gene, and the highest motor milestone achieved is to run. In some embodiments, the patient has two copies of the SMN2 gene, and the highest motor milestone achieved is to jump. In some embodiments, the patient has two copies of the SMN2 gene, and the highest motor milestone achieved is to alternate feet going upstairs. In some embodiments, the patient has two copies of the SMN2 gene, and the highest motor milestone achieved is to hop on one foot. In some embodiments, the patient has two copies of the SMN2 gene, and the highest motor milestone achieved is to alternate feet going downstairs.

In some embodiments, the patient has three copies of the SMN2 gene, and the highest motor milestone achieved is to raise or hold head while lying on stomach. In some embodiments, the patient has three copies of the SMN2 gene, and the highest motor milestone achieved is to roll over. In some embodiments, the patient has three copies of the SMN2 gene, and the highest motor milestone achieved is to sit with support. In some embodiments, the patient has three copies of the SMN2 gene, and the highest motor milestone achieved is to sit without support. In some embodiments, the patient has three copies of the SMN2 gene, and the highest motor milestone achieved is to stand with support. In some embodiments, the patient has three copies of the SMN2 gene, and the highest motor milestone achieved is to crawl. In some embodiments, the patient has three copies of the SMN2 gene, and the highest motor milestone achieved is to pull to standing position. In some embodiments, the patient has three copies of the SMN2 gene, and the highest motor milestone achieved is to walk with assistance. In some embodiments, the patient has three copies of the SMN2 gene, and the highest motor milestone achieved is to stand without support. In some embodiments, the patient has three copies of the SMN2 gene, and the highest motor milestone achieved is to walk independently (without support). In some embodiments, the patient has three copies of the SMN2 gene, and the highest motor milestone achieved is to run. In some embodiments, the patient has three copies of the SMN2 gene, and the highest motor milestone achieved is to jump. In some embodiments, the patient has three copies of the SMN2 gene, and the highest motor milestone achieved is to alternate feet going upstairs. In some embodiments, the patient has three copies of the SMN2 gene, and the highest motor milestone achieved is to hop on one foot. In some embodiments, the patient has three copies of the SMN2 gene, and the highest motor milestone achieved is to alternate feet going downstairs.

In some embodiments, the patient has four copies of the SMN2 gene, and the highest motor milestone achieved is to sit with support. In some embodiments, the patient has four copies of the SMN2 gene, and the highest motor milestone achieved is to sit without support. In some embodiments, the patient has four copies of the SMN2 gene, and the highest motor milestone achieved is to stand with support. In some embodiments, the patient has four copies of the SMN2 gene, and the highest motor milestone achieved is to crawl. In some embodiments, the patient has four copies of the SMN2 gene, and the highest motor milestone achieved is to pull to standing position. In some embodiments, the patient has four copies of the SMN2 gene, and the highest motor milestone achieved is to walk with assistance. In some embodiments, the patient has four copies of the SMN2 gene, and the highest motor milestone achieved is to stand without support. In some embodiments, the patient has four copies of the SMN2 gene, and the highest motor milestone achieved is to walk independently (without support). In some embodiments, the patient has four copies of the SMN2 gene, and the highest motor milestone achieved is to run. In some embodiments, the patient has four copies of the SMN2 gene, and the highest motor milestone achieved is to jump. In some embodiments, the patient has four copies of the SMN2 gene, and the highest motor milestone achieved is to alternate feet going upstairs. In some embodiments, the patient has four copies of the SMN2 gene, and the highest motor milestone achieved is to hop on one foot. In some embodiments, the patient has four copies of the SMN2 gene, and the highest motor milestone achieved is to alternate feet going downstairs.

In some embodiments, the patient has more than four copies of the SMN2 gene, and the highest motor milestone achieved is to sit with support. In some embodiments, the patient has more than four copies of the SMN2 gene, and the highest motor milestone achieved is to sit without support. In some embodiments, the patient has more than four copies of the SMN2 gene, and the highest motor milestone achieved is to stand with support. In some embodiments, the patient has more than four copies of the SMN2 gene, and the highest motor milestone achieved is to crawl. In some embodiments, the patient has more than four copies of the SMN2 gene, and the highest motor milestone achieved is to pull to standing position. In some embodiments, the patient has more than four copies of the SMN2 gene, and the highest motor milestone achieved is to walk with assistance. In some embodiments, the patient has more than four copies of the SMN2 gene, and the highest motor milestone achieved is to stand without support. In some embodiments, the patient has more than four copies of the SMN2 gene, and the highest motor milestone achieved is to walk independently (without support). In some embodiments, the patient has more than four copies of the SMN2 gene, and the highest motor milestone achieved is to run. In some embodiments, the patient has more than four copies of the SMN2 gene, and the highest motor milestone achieved is to jump. In some embodiments, the patient has more than four copies of the SMN2 gene, and the highest motor milestone achieved is to alternate feet going upstairs. In some embodiments, the patient has more than four copies of the SMN2 gene, and the highest motor milestone achieved is to hop on one foot. In some embodiments, the patient has more than four copies of the SMN2 gene, and the highest motor milestone achieved is to alternate feet going downstairs.

In some embodiments, the patient has one copy of the SMN2 gene and has achieved the WHO Motor Developmental Milestone of walking independently. In some embodiments, the patient has one copy of the SMN2 gene and has achieved the WHO Motor Developmental Milestone of standing independently. In some embodiments, the patient has one copy of the SMN2 gene and has achieved the WHO Motor Developmental Milestone of standing with assistance. In some embodiments, the patient has one copy of the SMN2 gene and has achieved the WHO Motor Developmental Milestone of hands and knees crawling. In some embodiments, the patient has one copy of the SMN2 gene and has achieved the WHO Motor Developmental Milestone of walking with assistance.

In some embodiments, the patient has two copies of the SMN2 gene and has achieved the WHO Motor Developmental Milestone of walking independently. In some embodiments, the patient has two copies of the SMN2 gene and has achieved the WHO Motor Developmental Milestone of standing independently. In some embodiments, the patient has two copies of the SMN2 gene and has achieved the WHO Motor Developmental Milestone of standing with assistance. In some embodiments, the patient has two copies of the SMN2 gene and has achieved the WHO Motor Developmental Milestone of hands and knees crawling. In some embodiments, the patient has two copies of the SMN2 gene and has achieved the WHO Motor Developmental Milestone of walking with assistance.

In some embodiments, the patient has three copies of the SMN2 gene and has achieved the WHO Motor Developmental Milestone of walking independently. In some embodiments, the patient has three copies of the SMN2 gene and has achieved the WHO Motor Developmental Milestone of standing independently. In some embodiments, the patient has three copies of the SMN2 gene and has achieved the WHO Motor Developmental Milestone of standing with assistance. In some embodiments, the patient has three copies of the SMN2 gene and has achieved the WHO Motor Developmental Milestone of hands and knees crawling. In some embodiments, the patient has three copies of the SMN2 gene and has achieved the WHO Motor Developmental Milestone of walking with assistance.

In some embodiments, the patient has four copies of the SMN2 gene and has achieved the WHO Motor Developmental Milestone of walking independently. In some embodiments, the patient has four copies of the SMN2 gene and has achieved the WHO Motor Developmental Milestone of standing independently. In some embodiments, the patient has four copies of the SMN2 gene and has achieved the WHO Motor Developmental Milestone of standing with assistance. In some embodiments, the patient has four copies of the SMN2 gene and has achieved the WHO Motor Developmental Milestone of hands and knees crawling. In some embodiments, the patient has four copies of the SMN2 gene and has achieved the WHO Motor Developmental Milestone of walking with assistance.

In some embodiments, the patient has more than four copies of the SMN2 gene and has achieved the WHO Motor Developmental Milestone of walking independently. In some embodiments, the patient has more than four copies of the SMN2 gene and has achieved the WHO Motor Developmental Milestone of standing independently. In some embodiments, the patient has more than four copies of the SMN2 gene and has achieved the WHO Motor Developmental Milestone of standing with assistance. In some embodiments, the patient has more than four copies of the SMN2 gene and has achieved the WHO Motor Developmental Milestone of hands and knees crawling. In some embodiments, the patient has more than four copies of the SMN2 gene and has achieved the WHO Motor Developmental Milestone of walking with assistance.

In some embodiments, the subject is able to raise or support head before the age of three months, six months, nine months, or 12 months. In some embodiments, the subject is able to roll over before the age of six months, nine months or 12 months. In some embodiments, the subject is able to sit with support or sit in tripod position before the age of six months, nine months, or 12 months. In some embodiments, the subject is able to sit without support before the age of six months, nine months, or 12 months. In some embodiments, the subject is able to stand with support before the age of nine months or 12 months. In some embodiments, the subject is able to stand without support before the age of 12 months. In some embodiments, the subject is able to creep or crawl before the age of six months, nine months, or 12 months. In some embodiments, the subject is able to pull to standing position before the age of nine months or 12 months.

In some embodiments, subject is able to walk with assistance before the age of 12 months or 15 months. In some embodiments, the subject is able to walk without assistance before the age of 12 months, or 15 months. In some embodiments, the subject is able to run before the age of 15 months, 18 months or 24 months. In some embodiments, the subject is able to jump before the age of 15 months, 18 months or 24 months. In some embodiments, the subject is able to squat to pick up an object before the age of 15 months, 18 months or 24 months. In some embodiments, the subject is able to walk or creep up and down the stairs before the age of 15 months, 18 months or 24 months. In some embodiments, the subject is able to alternate feet going upstairs before the age of 30 months or 36 months(3 years). In some embodiments, the subject is able to alternate feet going downstairs before the age of 36 months (3 years). In some embodiments, the subject is able to hop on one foot before the age of four years or five years.

The present disclosure provides therapeutic use of apitegromab in the treatment of SMA in a patient, wherein the treatment comprises intravenous administration of a composition comprising apitegromab at a therapeutic dose, wherein the therapeutic dose is greater than 2 mg/kg and up to 20 mg/kg (such as 10 mg/kg or 20 mg/kg), and wherein optionally the patient may be selected from any of the following category or categories, or the patient is characterized in that:

The patient is aged two years or younger, e.g., newborn to 24 months old, wherein optionally the patient has non-ambulatory type 2 or type 3 SMA, wherein further optionally the patient is receiving an SMN therapy (e.g., nusinersen, risdiplam, and/or onasemnogene abeparvovec-xioi).

The patient is aged two years or younger, e.g., newborn to 24 months old, wherein optionally the patient has ambulatory SMA, wherein further optionally the patient is receiving an SMN therapy (e.g., nusinersen, risdiplam, and/or onasemnogene abeparvovec-xioi).

The patient is aged six weeks or younger, e.g., newborn to six weeks old.

The patient is aged two years or older, wherein optionally the patient has non-ambulatory type 2 or type 3 SMA, wherein further optionally the patient is receiving an SMN therapy (e.g., nusinersen, risdiplam, and/or onasemnogene abeparvovec-xioi).

The patient is aged two years or older, wherein optionally the patient has ambulatory SMA, wherein further optionally the patient is receiving an SMN therapy (e.g., nusinersen, risdiplam, and/or onasemnogene abeparvovec-xioi).

The patient is 2-21 years old, wherein optionally the patient has non-ambulatory type 2 or type 3 SMA, wherein further optionally the patient is receiving an SMN therapy (e.g., nusinersen, risdiplam, and/or onasemnogene abeparvovec-xioi).

The patient is 2-21 years old, wherein optionally the patient has ambulatory SMA, wherein further optionally the patient is receiving an SMN therapy (e.g., nusinersen, risdiplam, and/or onasemnogene abeparvovec-xioi).

The patient is 2-12 years old, 2-10 years old, 2-5 years old, or 5-12 years old.

The patient is 13-21 years old, wherein optionally the patient has non-ambulatory type 2 or type 3 SMA, wherein further optionally the patient is receiving an SMN therapy (e.g., nusinersen, risdiplam, and/or onasemnogene abeparvovec-xioi).

The patient is 13-21 years old, wherein optionally the patient has ambulatory SMA, wherein further optionally the patient is receiving an SMN therapy (e.g., nusinersen, risdiplam, and/or onasemnogene abeparvovec-xioi).

The patient has later-onset SMA, wherein optionally the patient is receiving an SMN therapy (e.g., nusinersen, risdiplam, and/or onasemnogene abeparvovec-xioi).

The patient has ambulatory SMA, wherein optionally the patient is receiving an SMN therapy (e.g., nusinersen, risdiplam, and/or onasemnogene abeparvovec-xioi).

The patient has non-ambulatory SMA, wherein optionally the patient is receiving an SMN therapy (e.g., nusinersen, risdiplam, and/or onasemnogene abeparvovec-xioi).

The patient has type 2 SMA.

The patient has type 3 SMA, wherein optionally the patient has non-ambulatory type 3 SMA.

The patient has type 3 SMA, wherein optionally the patient has ambulatory type 3 SMA.

The patient has at least two copies of the SMN2 gene, e.g., two, three, four, five, or six copies of the SMN2 gene; or, wherein optionally the patient has 2-4 copies of the SMN2 gene.

The patient has at least two copies of the SMN2 gene, e.g., two, three, or four, etc. copies of the SMN2 gene; or, wherein optionally the patient has 2-4 copies of the SMN2 gene, wherein optionally the patient has type 2 SMA or non-ambulatory type 3 SMA.

The patient has at least three copies of the SMN2 gene, e.g., three, four, five, or six copies of the SMN2 gene; or, wherein optionally the patient has 3-5 copies of the SMN2 gene, wherein optionally the patient has ambulatory SMA, wherein further optionally the ambulatory SMA is type 4 SMA.

The patient has SMA (e.g., any type of SMA) and is up to two years of age, wherein optionally the patient has been treated with an SMN therapy, wherein further optionally the SMN therapy comprises an SMN2 upregulator (such as nusinersen, risdiplam, and/or onasemnogene abeparvovec-xioi) or an SMN1 gene therapy.

The patient has type 1 SMA with up to three copies of the SMN2 gene, wherein optionally the patient has been treated with an SMN therapy, wherein further optionally the SMN therapy comprises an SMN2 upregulator (such as nusinersen, risdiplam, and/or onasemnogene abeparvovec-xioi) or an SMN1 gene therapy.

The patient is symptomatic of SMA.

The patient has not been treated with SMN-targeted therapy.

The patient is on (is receiving) or has been treated with an SMN-targeted therapy, wherein optionally the SMN-targeted therapy is a gene therapy, splice modifier, or combination thereof, wherein further optionally the splice modifier is a nucleic acid-based agent or low molecular weight compound that modifies SMN2 splicing.

The patient is on (is receiving) or has been treated with an SMN-targeted therapy, wherein optionally the SMN-targeted therapy is a gene therapy, splice modifier, or combination thereof, wherein further optionally the splice modifier is a nucleic acid-based agent or low molecular weight compound that modifies SMN2 splicing; and wherein the patient commenced the SMN-targeted therapy before the age of five.

The patient is on (is receiving) or has been treated with an SMN-targeted therapy, wherein optionally the SMN-targeted therapy is a gene therapy, splice modifier, or combination thereof, wherein further optionally the splice modifier is a nucleic acid-based agent or low molecular weight compound that modifies SMN2 splicing, and wherein the patient commenced the SMN-targeted therapy at age five or older.

The patient is on an SMN-targeted therapy for at least 12 months at the time of starting apitegromab therapy (e.g., prior to receiving a first dose of apitegromab).

The patient is on an SMN-targeted therapy for at least 15 months at the time of starting apitegromab therapy (e.g., prior to receiving a first dose of apitegromab).

The patient is on an SMN-targeted therapy for at least 24 months at the time of starting apitegromab therapy (e.g., prior to receiving a first dose of apitegromab).

The patient is five years old or younger at the time of starting apitegromab therapy.

The patient is two years old or younger at the time of starting apitegromab therapy.

The patient is six weeks old or younger at the time of starting apitegromab therapy.

The patient is before the age of five at the time of receiving a first dose of apitegromab.

The patient has a baseline HFMSE score of at least 10, e.g., at least 13, e.g., a score of 13-39, e.g., no greater than 39.

In some embodiments, the present disclosure provides therapeutic use of apitegromab in the treatment of SMA in a patient who is 2-12 years old and has received at least six months of an SMN upregulator/corrector therapy (e.g., nusinersen, risdiplam, and/or onasemnogene abeparvovec-xioi) before starting apitegromab treatment, wherein the apitegromab treatment comprises intravenous administration of a composition comprising apitegromab at 10 mg/kg or 20 mg/kg, e.g., once every four weeks or once monthly. In some embodiments, the patient initiated the SMN upregulator/corrector therapy before the age of five. In some embodiments, the patient initiated the SMN upregulator/corrector therapy after the age of five. In some embodiments, present disclosure provides therapeutic use of apitegromab in the treatment of SMA in a patient who is 2-12 years old and has received at least six months of risdiplam before starting apitegromab treatment, wherein the apitegromab treatment comprises intravenous administration of a composition comprising apitegromab at 10 mg/kg or 20 mg/kg once every four weeks or once monthly. In some embodiments, present disclosure provides therapeutic use of apitegromab in the treatment of SMA in a patient who is 2-12 years old and has received at least 10 months of nusinersen, risdiplam, and/or onasemnogene abeparvovec-xioi before starting apitegromab treatment, wherein the apitegromab treatment comprises intravenous administration of a composition comprising apitegromab at 10 mg/kg or 20 mg/kg once every four weeks or once monthly.

In some embodiments, the present disclosure provides therapeutic use of apitegromab in the treatment of SMA in a patient who is 5-12 years old and has received at least six months of an SMN upregulator/corrector therapy (e.g., nusinersen, risdiplam, and/or onasemnogene abeparvovec-xioi) before starting apitegromab treatment, wherein the apitegromab treatment comprises intravenous administration of a composition comprising apitegromab at 10 mg/kg or 20 mg/kg, e.g., once every four weeks or once monthly. In some embodiments, the patient initiated the SMN upregulator/corrector therapy before the age of five. In some embodiments, the patient initiated the SMN upregulator/corrector therapy after the age of five. In some embodiments, present disclosure provides therapeutic use of apitegromab in the treatment of SMA in a patient who is 5-12 years old and has received at least six months of risdiplam before starting apitegromab treatment, wherein the apitegromab treatment comprises intravenous administration of a composition comprising apitegromab at 10 mg/kg or 20 mg/kg once every four weeks or once monthly. In some embodiments, present disclosure provides therapeutic use of apitegromab in the treatment of SMA in a patient who is 5-12 years old and has received at least 10 months of nusinersen, risdiplam, and/or onasemnogene abeparvovec-xioi before starting apitegromab treatment, wherein the apitegromab treatment comprises intravenous administration of a composition comprising apitegromab at 10 mg/kg or 20 mg/kg once every four weeks or once monthly (e.g., for at least 52 weeks).

In some embodiments, the present disclosure provides therapeutic use of apitegromab in the treatment of a patient having later-onset (e.g., type 2 or type 3) SMA who has received at least six months of an SMN-targeted therapy (e.g., an SMN upregulator/corrector therapy, e.g., nusinersen, risdiplam, and/or onasemnogene abeparvovec-xioi) before starting apitegromab treatment, wherein the apitegromab treatment comprises intravenous administration of a composition comprising 10 mg/kg or 20 mg/kg apitegromab once every four weeks or once monthly for at least 52 weeks. In some embodiments, the patient has received at least six months of risdiplam prior to starting the apitegromab treatment. In some embodiments, the patient has received at least 10 months of nusinersen, risdiplam, and/or onasemnogene abeparvovec-xioi prior to starting the apitegromab treatment. In some embodiments, the subject is aged 2-21 years. In some embodiments, the patient initiated the SMN-targeted therapy before the age of five. In some embodiments, the patient initiated the SMN-targeted therapy after the age of five. In some embodiments, the subject has not received onasemnogene abeparvovec-xioi or apitegromab previously.

In some embodiments, the present disclosure provides therapeutic use of apitegromab in the treatment of SMA in a patient who is 13-21 years old and has received at least six months of risdiplam before starting apitegromab treatment, and who has not received onasemnogene abeparvovec-xioi or apitegromab previously, wherein the apitegromab treatment comprises intravenous administration of a composition comprising 10 mg/kg or 20 mg/kg apitegromab once every four weeks or once monthly, e.g., for at least 52 weeks. In some embodiments, the patient initiated risdiplam treatment before the age of five. In some embodiments, the patient initiated risdiplam treatment after the age of five.

In some embodiments, the present disclosure provides therapeutic use of apitegromab in the treatment of SMA in a patient who is 13-21 years old and has received at least 10 months of nusinersen, risdiplam, and/or onasemnogene abeparvovec-xioi before starting apitegromab treatment, and who has not received onasemnogene abeparvovec-xioi or apitegromab previously, wherein the apitegromab treatment comprises intravenous administration of a composition comprising 10 mg/kg or 20 mg/kg apitegromab once every four weeks or once monthly, e.g., for at least 52 weeks. In some embodiments, the patient initiated nusinersen, risdiplam, and/or onasemnogene abeparvovec-xioi treatment before the age of five. In some embodiments, the patient initiated nusinersen, risdiplam, and/or onasemnogene abeparvovec-xioi treatment after the age of five.

In some embodiments, the present disclosure provides therapeutic use of apitegromab in the treatment of SMA in a patient who is aged five years or younger, wherein the patient has not received an SMN-targeted therapy (e.g., an SMN upregulator/corrector therapy, e.g., nusinersen, risdiplam, or onasemnogene abeparvovec-xioi) before starting apitegromab treatment, wherein the treatment comprises concurrent (e.g., simultaneous, separate, or sequential) administration of a composition comprising apitegromab and a composition comprising an SMN-targeted therapy (e.g., an SMN upregulator/corrector therapy, e.g., nusinersen, risdiplam, or onasemnogene abeparvovec-xioi). In some embodiments, the composition comprising apitegromab is administered intravenously. In some embodiments, the composition comprising apitegromab is administered at a frequency of once every four weeks or once monthly.

In some embodiments, the present disclosure provides therapeutic use of apitegromab in the treatment of SMA in a patient who is aged 2 years or younger, e.g., newborn to 24 months old, wherein the patient has not received an SMN upregulator/corrector therapy before starting apitegromab treatment, wherein the treatment comprises concurrent (e.g., simultaneous, separate, or sequential) administration of a composition comprising apitegromab and a composition comprising an SMN-targeted therapy (e.g., an SMN upregulator/corrector therapy, e.g., nusinersen, risdiplam, or onasemnogene abeparvovec-xioi).

In some embodiments, the present disclosure provides therapeutic use of apitegromab in the treatment of SMA in a patient who is aged six weeks or younger, e.g., newborn to six weeks old, wherein the patient has not received an SMN-targeted therapy (e.g., an SMN upregulator/corrector therapy) before starting apitegromab treatment, wherein the treatment comprises concurrent (e.g., simultaneous, separate, or sequential) administration of a composition comprising apitegromab and a composition comprising an SMN-targeted therapy (e.g., an SMN upregulator/corrector therapy, e.g., nusinersen, risdiplam, or onasemnogene abeparvovec-xioi). In some embodiments, the patient has two copies of the SMN2 gene. In some embodiments, the composition comprising apitegromab is administered intravenously. In some embodiments, the composition comprising apitegromab is administered at a frequency of once every four weeks or once monthly.

In some embodiments, the present disclosure provides therapeutic use of a myostatin-selective inhibitor, apitegromab, in the treatment of SMA in a patient who is aged five years or younger. In some embodiments, the patient has not previously received an SMN upregulator/corrector therapy (e.g., an SMN1 gene therapy) before starting the myostatin-selective inhibitor treatment. In some embodiments, the patient has previously received or is receiving an SMN upregulator/corrector therapy (e.g., an SMN1 gene therapy). In some embodiments, the patient is administered the SMN upregulator/corrector therapy (e.g., SMN1 gene therapy) at age five years or younger. In some embodiments, the treatment of SMA comprises concurrent (e.g., simultaneous, separate, or sequential) administration of a composition comprising a myostatin-selective inhibitor, apitegromab, and a composition comprising an SMN upregulator/corrector therapy, e.g., an SMN1 gene therapy. In some embodiments, the composition comprising a myostatin-selective inhibitor, apitegromab, is administered intravenously. In some embodiments, the patient has two copies of the SMN2 gene. In some embodiments, the composition comprising a myostatin-selective inhibitor, apitegromab, is administered at a frequency of once every four weeks or once monthly. According to the invention, the myostatin-selective inhibitor is apitegromab.

In some embodiments, the present disclosure provides therapeutic use of a myostatin-selective inhibitor, apitegromab, in the treatment of SMA in a patient who is aged two years or younger, e.g., newborn to 24 months old. In some embodiments, the patient has not previously received an SMN upregulator/corrector therapy (e.g., an SMN1 gene therapy) before starting the myostatin-selective inhibitor treatment. In some embodiments, the patient has previously received or is receiving an SMN upregulator/corrector therapy (e.g., an SMN1 gene therapy). In some embodiments, the patient is administered the SMN upregulator/corrector therapy (e.g., SMN1 gene therapy) at age two years or younger (e.g., newborn to 24 months). In some embodiments, the treatment comprises concurrent (e.g., simultaneous, separate, or sequential) administration of a composition comprising a myostatin-selective inhibitor, apitegromab, and a composition comprising an SMN upregulator/corrector therapy (e.g., an SMN1 gene therapy). In some embodiments, the patient has two copies of the SMN2 gene. In some embodiments, the composition comprising a myostatin-selective inhibitor, apitegromab, is administered intravenously. In some embodiments, the composition comprising a myostatin-selective inhibitor, apitegromab, is administered at a frequency of once every four weeks or once monthly. According to the invention, the myostatin-selective inhibitor is apitegromab.

In some embodiments, the present disclosure provides therapeutic use of a myostatin-selective inhibitor, apitegromab, in the treatment of SMA in a patient who is aged six weeks or younger, e.g., newborn to six weeks old, wherein the treatment comprises concurrent (e.g., simultaneous, separate, or sequential) administration of a composition comprising a myostatin-selective inhibitor, apitegromab, and a composition comprising an SMN upregulator/corrector therapy, (e.g., an SMN1 gene therapy). In some embodiments, the patient has not previously received an SMN upregulator/corrector therapy (e.g., SMN1 gene therapy) before starting the myostatin-selective inhibitor treatment. In some embodiments, the patient has previously received or is receiving an SMN upregulator/corrector therapy (e.g., an SMN1 gene therapy). In some embodiments, the patient has two copies of the SMN2 gene. In some embodiments, the composition comprising a myostatin-selective inhibitor, apitegromab, is administered intravenously. In some embodiments, the composition comprising a myostatin-selective inhibitor, apitegromab, is administered at a frequency of once every four weeks or once monthly. According to the invention, the myostatin-selective inhibitor is apitegromab.

In some embodiments, apitegromab is used in the treatment of SMA in a patient who is two months or older, who is treated with an SMN therapy, such as nusinersen, risdiplam, or onasemnogene abeparvovec-xioi. In some embodiments, the apitegromab is administered as an adjunct to an SMN upregulator therapy comprising nusinersen, risdiplam, or onasemnogene abeparvovec-xioi.

The data disclosed herein further support the notion that myostatin inhibitor is helpful to improve SMA treatment in patients who receive SMN therapies. In particular, the data suggest that subpopulations of patients who experience or suffer from certain types of disease-related symptoms may particularly benefit from myostatin inhibitor therapy, pointing to patient selection considerations. Certain SMA patient subpopulations who are particularly likely to benefit from a myostatin inhibitor include but are not limited to the following cohorts of patients who manifest the indicated disease-related symptoms: patients who suffer from muscle weakness and/or tightness; patients who suffer from severe fatigue (for example, as assessed by art-recognized measures, such as PROMIS); patients who suffer from difficulty with or impaired bulbar function (e.g., difficulty coughing, swallowing/ feeding, difficulty chewing or smiling); patients who are prone to respiratory infections; and/or patients who suffer from difficulty with or impaired emptying (e.g., urgency and frequency of urination, and/or bowel movement). Accordingly, in some embodiments, a myostatin inhibitor is used in the treatment of SMA in a patient, wherein the treatment comprises administration of a myostatin inhibitor to treat SMA, wherein the patient has impaired bulbar function, wherein optionally, the patient has difficulty coughing, difficulty swallowing or feeding, and/or, difficulty chewing or smiling. In some embodiments, a myostatin inhibitor is used in the treatment of SMA in a patient, wherein the treatment comprises administration of a myostatin inhibitor to treat SMA, wherein the patient is prone to respiratory infections. In some embodiments, a myostatin inhibitor is used in the treatment of SMA in a patient, wherein the treatment comprises administration of a myostatin inhibitor to the patient who suffers from severe fatigue, wherein optionally the severity of fatigue is assessed by PROMIS. In some embodiments, a myostatin inhibitor is used in the treatment of SMA in a patient, wherein the treatment comprises administration of a myostatin inhibitor to the patient who has difficulty with or impaired emptying (e.g., urgency and frequency of urination, and/or bowel movement).

Accordingly, the present disclosure provides patient selection considerations aimed to help identify SMA patients who are more likely to benefit from muscle-enhancing therapies, such as myostatin inhibitors. According to the present disclosure, a myostatin inhibitor is used in the treatment of SMA in a patient who receives an SMN therapy, wherein the patient suffers from muscle weakness/tightness, fatigue, impaired bulbar function (optionally, coughing, and/or difficulty swallowing or feeding), and/or, impaired emptying (optionally, urgency and frequency of urination, and/or bowel movement). Myostatin inhibitors may include agents that inhibit myostatin, GDF11 and Activin A. Myostatin inhibitors may include agents that inhibit myostatin and GDF11 but not Activin A. Myostatin inhibitors may be agents that selectively inhibit myostatin (i.e., myostatin-selective inhibitor) which do not bind or inhibit GDF11 or Activin A. Myostatin-selective inhibitors include neutralizing antibodies or antigen-binding fragments thereof that selectively bind myostatin but not GDF11 or Activin A, and antibodies or antigen-binding fragments thereof that bind pro/latent myostatin thereby inhibiting activation of mature myostatin. The latter includes, for example, apitegromab (SRK-015), GYM329 (RO7204239), variants thereof, or antibodies that cross-block or cross-compete for antigen binding with apitegromab or GYM329. According to the invention, the myostatin-selective inhibitor is apitegromab.

### Additional Indications

Data presented herein indicate that the disclosed treatment regimens, e.g., including dose selections, for apitegromab may provide clinical benefit for human patients beyond those with SMA. Apitegromab may provide clinical benefit for human patients diagnosed with other indications that share certain attributes of SMA. Such attributes include one or more of the criteria such as: disease with relatively young patient population, disease in which muscles are structurally intact or function is retained, disease that impacts fast-twitch fiber, and the availability of an established endpoint that relies on fast-twitch fibers.

Beyond SMA, additional indications that may benefit from treatment with the antibodies disclosed herein (e.g., apitegromab) include but are not limited to dystrophies such as Becker's muscular dystrophy, Becker's muscular dystrophy with micro-dystrophin gene therapy, Duchenne's muscular dystrophy, Duchenne's muscular dystrophy with micro-dystrophin gene therapy, facioscapulohumeral muscular dystrophy (FSHD), and other muscular dystrophies, as well as Pompe Disease, Pompe disease with enzyme replacement therapy (including but not limited to Lumizyme and Nexviazyme), late-onset Pompe disease, post-cancer muscle recovery, and glucocorticoid-induced myopathy (e.g., in a subset of patients unable to discontinue steroid therapy). Becker's muscular dystrophy may be particularly suitable for treatment with the antibodies disclosed herein (e.g., apitegromab). Becker's muscular dystrophy patients typically have higher levels of circulating myostatin as compared to Duchenne's muscular dystrophy patients (see, e.g., Burch et al. (2017) J Neurol. 264(3):541-553; Mariot et al. (2017) Nat Commun. 8: 1859). The indication treated may be amyotrophic lateral sclerosis (ALS). The indication treated may be post-cancer muscle recovery, e.g., post-cancer muscle recovery in pediatric patients (as some children may develop severe muscle wasting from chemotherapy). The antibodies disclosed herein (e.g., apitegromab) may be used as monotherapy or as add-on therapy to provide muscle- targeted approaches to enhance other stabilizing treatments (e.g., treatments such as gene therapy in Duchenne's muscular dystrophy or enzyme replacement therapy in lysosomal storage disorders).

### Dosage Selection and Administration

In various embodiments of the methods, uses, and compositions disclosed herein, an effective amount of an anti-pro/latent myostatin antibody (e.g., apitegromab) is administered to a human subject in need of the treatment via intravenous administration, e.g., by continuous infusion over a period of time.

In some embodiments, apitegromab is administered to a patient as a weight-based dose, i.e., a dose dependent on the patient's bodyweight. In some embodiments, suitable dosages of apitegromab include greater than 2 and up to 20 mg/kg, optionally about 5, 10, 15, or 20 mg/kg. In some embodiments, the therapeutic dose of apitegromab is 10 mg/kg. In some embodiments, the therapeutic dose of apitegromab is 20 mg/kg. In some embodiments, a dose greater than 20 mg/kg may be used while retaining a similar safety profile, although there may be diminished need for higher dosage given the surprisingly good therapeutic and PK profile of e.g., 20 mg/kg and 2 mg/kg, respectively.

PK analyses of apitegromab show a correlation between drug clearance and age. These data indicate that younger patients (with lighter body weight) show slower clearance of apitegromab than older patients (with heavier body weight). This finding may point to dose selection for suitable intermediate doses to be, for example, 5 mg/kg, 7.5 mg/kg, 10 mg/kg, 12 mg/kg, 15 mg/kg, etc. Apitegromab demonstrated linear pharmacodynamics in both healthy volunteers and subjects treated with apitegromab with a mean terminal half-life of 23-40 days.

Target engagement analyses from 6-month and 12-month studies indicated that apitegromab at 20 mg/kg achieved target saturation, whilst 2 mg/kg showed partial target engagement. In some embodiments, due to this target saturation at 20 mg/kg, a lower dose is used, e.g., a dose of 10 mg/kg. Target engagement analysis after 24-months was consistent with the earlier studies and further demonstrated that apitegromab target engagement correlated with motor function improvements. Both the amount of latent myostatin and the fold change in latent myostatin, when normalized to the age at which the subjects started treatment provide pharmacodynamic markers of improvements in motor function.

In some embodiments, a suitable dosage of apitegromab is 20 mg/kg. In some embodiments, a suitable dosage of apitegromab is 10 mg/kg. In some embodiments, the apitegromab is intravenously administered to a patient every four weeks or monthly at 5 mg/kg, 7.5 mg.kg, 10 mg/kg or 12 mg/kg.

In some embodiments, apitegromab is administered to a patient at a dose of 20 mg/kg. In some embodiments, due to its favorable safety profile when administered at a dose of 20 mg/kg, apitegromab may be administered to a patient at a dose of more than 20 mg/kg, e.g., wherein the dose is up to 30 mg/kg, e.g., 25 mg/kg. In some embodiments, apitegromab is administered to a patient at a dose of more than 20 mg/kg (e.g., about 25 mg/kg, about 30 mg/kg). In some embodiments, apitegromab is intravenously administered to a patient at a dose of 10-20 mg/kg every four weeks or monthly.

In some embodiments, apitegromab is initially administered to a patient at 2 mg/kg, and the dose is increased to a dose of at least 10 mg/kg (e.g., 10 mg/kg, 15 mg/kg, 20 mg/kg) at a later time if additional motor improvement is needed. In some embodiments, the dose is increased to at least 10 mg/kg after the patient has been treated with 2 mg/kg apitegromab for at least four months. In some embodiments, the dose is increased to at least 10 mg/kg after the patient has been treated with 2 mg/kg apitegromab for six months. In some embodiments, the dose is increased to at least 10 mg/kg after the patient has been treated with 2 mg/kg apitegromab for 12 months. In some embodiments, the dose is increased to at least 10 mg/kg after the patient has been treated with 2 mg/kg apitegromab for 24 months. In some embodiments, increasing the apitegromab dose from 2 mg/kg to at least 10 mg/kg (e.g., 10 mg/kg, 15 mg/kg, 20 mg/kg) produces a dose response, e.g., as determined by the subject's latent myostatin concentrations. In some embodiments, motor improvement is measured by RULM. In some embodiments, motor improvement is measured by RHS. In some embodiments, motor improvement is measured by HFMSE.

In some embodiments, apitegromab is administered to a patient about once every four weeks, once a month, etc. Such antibody may be administered via intravenous injection/infusion, e.g., by intravenous infusion, or another suitable route of administration (e.g., subcutaneously (e.g., under the skin) or intrathecally (e.g., intra spinally). Similarly, an SMN upregulator, e.g., splice modifier, may be administered orally, e.g., by mouth, or another suitable route of administration.

In some embodiments, the subject has received an SMN upregulator prior to the administration of the apitegromab. In some embodiments, the subject is concurrently receiving an SMN upregulator at the same time as the administration of the apitegromab. In some embodiments, the subject will receive an SMN upregulator after administration of the apitegromab.

In some embodiments, the subject receiving an SMN upregulator is administered apitegromab at least 24 hours (e.g., at least 36 hours, at least 48 hours, or more) prior to a dose (e.g., a maintenance dose) of the SMN upregulator (e.g., nusinersen, risdiplam, and/or onasemnogene abeparvovec-xioi). In some embodiments, the subject receiving an SMN upregulator is administered apitegromab at least 14 days (e.g., least 21 days, or more) after a dose (e.g., a maintenance dose) of the SMN upregulator (e.g., nusinersen, risdiplam, and/or onasemnogene abeparvovec-xioi).

In some embodiments, the subject has received an SMN upregulator within six months of administration of the apitegromab. In some embodiments, the subject has received an SMN upregulator within three months of administration of the apitegromab. In some embodiments, the subject has received an SMN upregulator within six months, five months, four months, three months, two months, or one month of administration of the apitegromab. In some embodiments, the subject has received an SMN upregulator within four weeks, three weeks, two weeks, or one week of administration of the apitegromab. In some embodiments, the subject has received an SMN upregulator on the same day as administration of the apitegromab.

In some embodiments, the subject is expected to receive an SMN upregulator within six months of administration of the apitegromab. In some embodiments, the subject is expected to receive an SMN upregulator within three months of administration of the apitegromab. In some embodiments, the subject is expected to receive an SMN upregulator within six months, five months, four months, three months, two months, or one month of administration of the apitegromab. In some embodiments, the subject is expected to receive an SMN upregulator within four weeks, three weeks, two weeks, or one week of administration of the apitegromab.

In some embodiments, the SMN upregulator is an antisense nucleotide and is administered to the central nervous system of the subject via intrathecal injection. In some embodiments, the antisense nucleotide is administered to the subject every few months, e.g., monthly, every two months, every three months, every four months, every five months, every six months or every 12 months. In other embodiments, an initial treatment may involve more frequent dosing, followed by a less frequent maintenance dose thereafter.

In some embodiments, the SMN upregulator is a small molecule and is orally administered to the subject. In some embodiments, the small molecule is administered to the subject daily. In other embodiments, the small molecule is administered to the subject weekly, biweekly, or monthly.

In some embodiments, the SMN upregulator is a gene therapy and is administered via intravenous injection. In some embodiments, the SMN upregulator is a gene therapy and is administered via intrathecal injection. In some embodiments, an initial treatment may include more frequent dosing, followed by a less frequent maintenance dose thereafter. Less frequent maintenance doses may be preferable in order to avoid improper immune responses to the gene therapy.

In some embodiments, the apitegromab is administered to the subject via intravenous administration, e.g., by intravenous infusion. In some embodiments, the apitegromab is administered once every four weeks or monthly, to the subject. In some embodiments, an initial treatment may involve more frequent dosing, followed by a less frequent maintenance dose thereafter. In some embodiments, apitegromab may be initially dosed at a higher dose (e.g., a loading dose) followed by one or more subsequent lower doses (e.g., one or more maintenance doses). In some embodiments, an initial dose or doses of apitegromab may be administered at 20 mg/kg, followed by one or more lower dose (e.g., 15 mg/kg. 10 mg/kg, 5 mg/kg, 2 mg/kg, or 1 mg/kg), e.g., four weeks or one month after administration of the initial 20 mg/kg dose, e.g., wherein the one or more lower doses are administered once every four weeks or once monthly thereafter, or at longer intervals than was used for the loading dose.

In some embodiments, the SMA therapy comprises intravenous administration of greater than 2 mg/kg and up to 20 mg/kg of apitegromab every four weeks or monthly. In some embodiments, the SMA therapy comprises intravenous administration of about 20 mg/kg of apitegromab every four weeks or monthly. In some embodiments, an SMA therapy disclosed herein (e.g., an SMA therapy comprising apitegromab) may achieve disease stabilization such that a patient's motor function is maintained over time, or in other words, prevented from declining, contrary to the natural history of the disease, which predicts progression over time. In some embodiments, a net zero change in motor function scores over an appropriate baseline reflects disease stabilization.

In some embodiments, the SMA therapy comprises intravenous administration of greater than 2 mg/kg and up to 20 mg/kg of apitegromab every four weeks or monthly. In some embodiments, the SMA therapy comprises intravenous administration of about 20 mg/kg of apitegromab every four weeks or monthly. In some embodiments, the SMA therapy comprises administering a therapeutically effective amount of apitegromab such that the administration is sufficient to increase the motor function of the subject by at least one milestone according to the WHO Motor Developmental Milestones. In some embodiments, the SMA therapy comprises administering a therapeutically effective amount of apitegromab such that the administration is sufficient to increase the motor function of the subject by one, two, or three milestones according to the WHO Motor Developmental Milestones. In some embodiments, the WHO Motor Developmental Milestones include one or more of ability to walk independently, ability to stand independently, standing with assistance, hands and knees crawling, and/or walking with assistance.

In some embodiments, an SMA therapy disclosed herein (e.g., an SMA therapy comprising apitegromab) may help maintain the disease status in a patient population that receives apitegromab, as compared to control group that does not. Maintenance of the disease status refers to preventing further deterioration of affected muscle, for example as assessed by changes in motor function over time, in these patients. In some embodiments, treatment may slow the disease progression, e.g., as assessed by a slower rate of change in disease function as compared to a suitable baseline (e.g., an untreated patient). Therefore, even where no improvement in motor function test scores is shown, the apitegromab may provide clinical benefits by countering disease progression. Thus, such clinical benefits may present as a longer period of time observed where the patient population treated with the apitegromab maintains prior test scores or shows a slower rate of decrease in scores over time, as compared to a control group. In some embodiments, a patient or patient population receiving or in need of treatment with apitegromab may be compared to a control patient or patient population. In some embodiments, the control patient or patient population is a patient or patient population that does not receive apitegromab.

In some embodiments, an SMA therapy disclosed herein (e.g., an SMA therapy comprising apitegromab) may slow the progression of the disease, e.g., by delaying a decrease in motor function as measured by a decreased score by HFMSE, RHS, MFM, MyoGrip or MyoPinch and/or by delaying a transition from ambulatory to non-ambulatory, delaying the need for respiratory aid or intervention, etc.

Based on the surprising degree of clinical benefit achieved by selective inhibition of myostatin in human patients as demonstrated herein, it is contemplated that different myostatin-selective inhibitors (other than apitegromab) may be used in the treatment of SMA or other muscle disorders. Accordingly, the present disclosure includes a myostatin-selective inhibitor for use in the treatment of a muscle disorder, such as SMA, in a human subject, wherein optionally, the subject is further treated with a motor neuron-directed therapy, wherein further optionally the motor neuron-directed therapy comprises an SMN upregulator therapy, such as SMN2 upregulator therapies and SMN1 gene therapies.

### Biological Effects of Treatment

An SMA therapy disclosed herein (e.g., an SMA therapy comprising apitegromab) may be suitable for the treatment of any form of SMA, particularly later-onset forms of SMA, in a human subject.

Patient populations who may benefit from the therapies described herein include those with non-ambulatory SMA and those with ambulatory SMA. In some embodiments, an SMA therapy disclosed herein is considered for non-ambulatory forms of SMA, such as type 2 and non-ambulatory type 3 SMA. In other embodiments, an SMA therapy disclosed herein is considered for ambulatory forms of SMA, such as ambulatory type 3 SMA.

Genotype-based diagnosis allows for the identification of patients carrying an SMA genetic mutation, including those who may be pre-symptomatic (not manifesting obvious disease phenotypes). In some embodiments, a subject suitable for treatment with the SMA therapies disclosed herein has a direct diagnosis of a mutation in SMN1 (e.g., a chromosomal direct diagnosis of 5q SMA). In some embodiments, the subject has a diagnosis of 5q SMA, in addition to a diagnosis based on motor phenotype. In some embodiments, a subject suitable for treatment with the SMA therapies disclosed herein does not have a mutation in SMN1 (e.g., a chromosomal direct diagnosis of 5q SMA). In some embodiments, the subject does not have a diagnosis of 5q SMA. In some embodiments, the subject has a diagnosis of non-5q SMA.

In some embodiments, the subject having SMA is two years of age or older. In some embodiments, the subject having SMA is 2 to 12, 5 to 21, 13 to 21, or 2 to 21 years old. In some embodiments, the subject having SMA is under the age of two years. Clinical effects of an anti-pro/latent myostatin antibody (e.g., apitegromab), administered alone or in conjunction with an SMN upregulator, can be monitored and/or evaluated for effectiveness by various means. Exemplary such biologically beneficial effects are provided herein. Beneficial biological effects in a subject can be achieved by administration of an anti-pro/latent myostatin antibody (e.g., apitegromab) as a monotherapy or in conjunction with an SMN upregulator. In some embodiments, apitegromab in conjunction with an SMN upregulator is administered in an amount effective to cause one or more of the biological effects described below.

The ability to assess functional scales that can be reliably measured in SMA patients may be used in tracking patients' disease progression as well as effects of therapy over time. Whilst muscle function may be assessed by physiological measurements, such as muscle strength and force generation, motor functional scales monitor disease progression in ways that relate to patients' functionality in everyday life and carries more meaning and relevance than a measure that quantitates strength *per se.* In some embodiments, a patient in need of treatment or being treated for SMA is evaluated using a functional scale, such as any one or more of the motor functional assessment tests or functional outcome measures described herein (e.g., one or more of HFMSE, RHS, 6MWT, WHO Motor Milestones, RULM, 30-second Sit-to-Stand, Motor Function Measure (MFM), CHOP-INTEND, Endurance Shuttle Nine Hole Peg Test [ESNHPT], and/or Endurance Shuttle Box and Block Test [ESBBT]).

In some embodiments, a patient with SMA (e.g., later-onset SMA) is evaluated using the RHS. In some embodiments, administration of the RHS includes the Timed Rise from Floor and 10-meter Walk/Run tests. In some embodiments, the RHS is a 36-item clinical assessment for physical abilities of patients with type 2 SMA, and ambulatory and non-ambulatory patients with type 3 SMA. The RHS includes 33 items that are graded on a scale of 0, 1, 2, where 0 denotes the lowest level of ability/function and 2 denotes the highest level of ability (Ramsey et al. (2017) PLoS One. 12(2):e0172346). The remaining three items are scored 0, 1, where 0 denotes an inability and 1 denotes an ability to achieve. The maximum achievable score is 69.

In some embodiments, a patient to be treated with apitegromab (e.g., a patient with ambulatory SMA) has a baseline RHS score of 26 or greater prior to treatment. In some embodiments, the patient has a baseline RHS score of 63 or below. In some embodiments, the patient has a baseline RHS score which ranges between 26-63. In some embodiments, the patient has ambulatory SMA, e.g., ambulatory type 3 SMA. In some embodiments, treatment with apitegromab improves a patient's RHS score. In some embodiments, apitegromab continually improves a patient's RHS score over 24 months of treatment. In some embodiments, apitegromab maintains an increased RHS score over 24 months of treatment.

In some embodiments, a patient with SMA (e.g., later-onset SMA) is evaluated using a 10-meter Walk/Run test. In some embodiments, the 10-meter Walk/Run test is an enhanced function of the RHS used for ambulatory patients with type 3 SMA. The 10-meter Walk/Run test is a measure of the time taken to walk/run 10 meters. In some embodiments, treatment with apitegromab improves a patient's 10-meter Walk/Run test score.

In some embodiments, a patient with SMA (e.g., later-onset SMA) is evaluated using a Timed Rise from Floor test. In some embodiments, the Timed Rise from Floor test is an enhanced function of the RHS used for ambulatory patients with type 3 SMA. The Timed Rise from Floor test is a measure of the time taken to rise to standing from the floor. In some embodiments, treatment with apitegromab improves a patient's Timed Rise from Floor test score.

In some embodiments, a patient with SMA (e.g., later-onset SMA) is evaluated using a 6-minute Walk Test (6MWT). The 6MWT is an assessment of exercise capacity and fatigue that has been used in clinical studies of ambulatory later-onset SMA patients (Young et al. (2016). Muscle Nerve. 54(5):836-842). Patients are directed to walk along a 25-meter course as fast as possible over six minutes. The minute distances and total distance walked over six minutes are measured. In some embodiments, treatment with apitegromab improves a patient's 6MWT score.

In some embodiments, a patient with SMA (e.g., later-onset SMA) is evaluated using a 30-second Sit-to-Stand Test. The 30-second Sit-to-Stand Test is used by researchers and clinicians as an assessment of functional lower limb strength (Jones et al. (1999) Res Q Exerc Sport. 70:113-119). The test was modified for the ambulatory SMA population based on research of the modified 30-second Sit-to-Stand being a reliable, feasible tool for use in a general geriatric population with a lower level of function (McAllister and Palombaro (2019). J Geriatr Phys Ther. 0(0):1-6) and was related to fall risk in institutionalized veterans (Applebaum et al. (2017). PLoS One. 12(5):e0176946; Le Berre et al. (2016) Percept Mot Skills. 123:138-152). The test measures the maximal number of times the patient can transition from sitting to standing in 30 seconds. In some embodiments, treatment with apitegromab improves a patient's 30-second Sit to Stand score.

In some embodiments, a patient to be treated with apitegromab (e.g., a patient with a non-ambulatory form of SMA, such as type 2 or non-ambulatory type 3 SMA) has a baseline HFMSE score of 12 or greater prior to treatment. In some embodiments, the patient has a baseline HFMSE score of 44 or below. In some embodiments, the patient has a baseline HFMSE score which ranges between 12-44. In some embodiments, the patient has a non-ambulatory form of SMA. In some embodiments, the patient has type 2 SMA. In some embodiments, the patient has non-ambulatory type 3 SMA. In some embodiments, treatment with apitegromab improves a patient's HFMSE score.

In some embodiments, a patient with SMA (e.g., later-onset SMA) is evaluated using the Revised Upper Limb Module (RULM). The RULM is a 20-item assessment of upper limb function in non-ambulatory SMA patients (young children as well as adults) (Mazzone et al. (2017) Muscle Nerve. 55(6):869-874). The 19 scored items test functions that relate to everyday life, such as placing hands from lap, pressing a button, and picking up a token. The items are scored 0, 1, 2, where 0 denotes unable, 1 denotes able with modification, and 2 denotes able with no difficulty. The maximum score achievable is 37. In some embodiments, the RULM is used to evaluate non-ambulatory SMA patients. In some embodiments, the RULM is completed by patients who are 30 months of age or older, e.g., at the time of the baseline assessment. In some embodiments, treatment with apitegromab improves a patient's RULM score.

In some embodiments, a patient with SMA (e.g., later-onset SMA) is evaluated using World Health Organization (WHO) Motor Development Milestones. The WHO Multicentre Growth Reference Study (MGRS) generated growth curves for assessing the growth and development of infants and young children around the world (de Onis et al. (2004) Food Nutr Bull. 25 Suppl:S1-89). The MGRS had as its primary objective the construction of curves and related tools to assess growth and development in children from birth to five years of age. Another feature of the MGRS is that it included the collection of ages of achievement of motor milestones, including gross motor development milestones such as sitting without support, hands-and-knees crawling, standing with assistance, walking with assistance, standing alone, and walking alone (Wijnhoven et al. (2004) Food Nutr Bull. 25(1 Suppl):S37-45; WHO Multicentre Growth Reference Study Group (2006) Acta Paediatr Suppl;450:86-95). In some embodiments, WHO MGRS performance criteria for gross motor development are utilized to assess motor development milestones, e.g., in patients with type 2 and non-ambulatory type 3 SMA. In some embodiments, treatment with apitegromab improves a patient's WHO MGRS score.

In some embodiments, a patient with SMA (e.g., later-onset SMA) is evaluated using the Motor Function Measure (MFM). The MFM assesses motor function across three domains, (1) standing position, ambulation and transfer; (2) axial and proximal motor function; and (3) distal motor function (Annoussamy et al. (2020) Annals of Clinical and Translational Neurology 8:359-373). The MFM was initially designed for children seven years of age or older and has 32 items in the three functional domains. MFM-20 is a modified version of the MFM scale with some of the difficult items removed, making it suitable for younger children. It comprises 20 items and is suitable for children two years of age and older (Pierzchiewicz et al., Child Neurology Open (2021) 8:1-9). In some embodiments, a patient with SMA (e.g., later-onset SMA) is evaluated using the MFM20. In some embodiments, treatment with apitegromab improves a patient's MFM20 score.

In some embodiments, a patient with SMA (e.g., early onset or type 1 SMA) is evaluated using the CHOP-INTEND test (Glanzman et al. (2010) Neuromus. Disord. 20(3):155-161). CHOP-INTEND measures five parameters of observational and elicited motor activity: (1) spontaneous movement of the left and right upper extremities; (2) spontaneous movement of the left and right lower extremities; (3) hand grip; (4) head in midline with visual stimulation; and (5) hip adduction. Scoring for each item is on a scale of 0-4 where 0 denotes no response, 1 minimal response, 2 partial, 4 nearly full and 4 complete response. The maximum score is 64. CHOP-INTEND was designed for and is validated for use in infants with SMA (Alfano et al. (2021) Pediatric Neurology 122:21-26).

The CHOP-INTEND maximum score of 64 is typically obtained by three to six months in healthy infants. Most patients on nusinersen (Duong et al (2021) Neuromuscul Dis, 8(1), 63-77; Pechmann et al. (2018) J Neuromuscul Dis, 5(2), 135-143; Aragon-Gawinska et al. (2018) Neurology, 91:14), on risdiplam (Baranello et al. (2021) N Engl J Med, 384(10), 915-923; Darras et al. (2021) N Engl J Med, 385(5), 427-435), and onasemnogene abeparvovec-xioi (Mendell et al. (2021) JAMA Neurol 78(7) 834-841; EMA/200482/2020 (2020) Zolgensma EPAR) reach a plateau with a CHOP-INTEND score less than 50 within the first three years of life.

In some embodiments, the patient evaluated with CHOP-INTEND is under two years of age. In some embodiments, the patient evaluated with CHOP-INTEND has early onset or type 1 SMA. In some embodiments, the patient evaluated with CHOP-INTEND has a baseline score of 40 or below.

In some embodiments, a patient with SMA (e.g., later-onset SMA) is evaluated using the Endurance Shuttle Nine Hole Peg Test (ESNHPT). The ESNHPT is an endurance test for patients severely affected with SMA. Patients are instructed to repeatedly perform the original 9 Hole Peg Test at 75% of their individual maximum speed. The shuttles refer to the set speed marked by auditory cues, with the test ending when the patient missed two consecutive beeps. Primary outcome parameter is "time to limitation (Tlim)," i.e., the time a task can be maintained at the pre-set intensity. Maximum test duration is 20 minutes (Stam et al. (2018) BMJ Open. 8(7):e019932). In some embodiments, the ESNHPT is used to evaluate non-ambulatory SMA patients. In some embodiments, the ESNHPT is completed by patients who are eight years of age or older. In some embodiments, treatment with apitegromab improves a patient's ESNHPT score.

In some embodiments, a patient with SMA (e.g., later-onset SMA) is evaluated using the Endurance Shuttle Box and Block Test (ESBBT). The ESBBT is a muscle endurance measurement tool that measures how fast a subject's proximal arm muscle fatigues. Patients may be instructed to repeatedly perform the original Box and Block Test at 75% of their individual maximum speed. The shuttles refer to the set speed marked by auditory cues, with the test ending when the patient missed two consecutive beeps. Primary outcome parameter is "time to limitation (Tlim)," i.e., the time a task can be maintained at the pre-set intensity. Maximal test duration is 20 minutes (Stam et al. (2018) BMJ Open. 8(7):e019932). The ESBBT has been validated in SMA (Bartels et al. (2020) Orphanet J Rare Dis 15:75). The ESSBT is the first validated and sensitive fatiguability test for proximal arm muscle function in SMA and, by adding the dimension of endurance, the results are complementary to those obtained by other measures of arm motor functions, e.g., RULM.

In some embodiments, the ESBBT is used to evaluate non-ambulatory SMA patients. In some embodiments, the ESBBT is completed by patients who are eight years of age or older. In some embodiments, treatment with apitegromab improves a patient's ESBBT score.

In some embodiments, treatment with apitegromab for 12 months is sufficient to increase the subject's ESBBT score by at least 109.1 points (e.g., increase the subject's ESBBT score by about 110 points to about 239 points). In some embodiments, treatment with apitegromab for 24 months is sufficient to increase the subject's ESBBT score by at least 147.9 points (e.g., increase the subject's ESBBT score by about 150 points to about 300 points). In some embodiments, the subject has nonambulatory type 2 or type 3 SMA and is aged 5-21 years has previously received an SMN upregulator/corrector therapy, e.g., nusinersen, risdiplam, and/or onasemnogene abeparvovec-xioi treatment, wherein the subject was first treated with the SMN upregulator/corrector therapy, e.g., nusinersen, risdiplam, and/or onasemnogene abeparvovec-xioi after five years of age.

Clinical benefits of SMA therapy may include 1) motor function improvement, 2) motor function maintenance (disease stabilization), 3) delay in disease progression, or (4) improvement in quality of life.

Motor function may be assessed by suitable means, such as HFMSE and/or RHS, the latter of which is often used to assess the motor function of ambulatory patients. In some embodiments, HFMSE is more frequently used to assess the motor function of non-ambulatory patients. An increase in the respective score over an appropriate baseline indicates motor function improvement. In some embodiments, the increase is at least one point, at least two points, at least three points, at least four points, or at least five points. In some embodiments, the increase is one point or greater, two points or greater, three points or greater, four points or greater, or five points or greater. In some embodiments, SMA therapy comprising apitegromab enhances motor function, such that an HFMSE score or an RHS score (e.g., an HFMSE score) measured at six months or 12 months or 24 months after initiation of treatment is at least 1, 3, 5, 7, or 10 points over a baseline, and wherein the baseline is obtained at or prior to initiation of the treatment. In some embodiments, SMA therapy comprising apitegromab produces at least a five-point increase in an HFMSE score relative to a baseline score after at least 12 months (e.g., after at least 24 months) of treatment. In some embodiments, SMA therapy comprising apitegromab produces a 6-20 point or greater increase in an HFMSE score relative to a baseline score after at least 12 months (e.g., after at least 24 months) of treatment. In some embodiments, SMA therapy comprising apitegromab produces at least a seven-point increase in an HFMSE score relative to a baseline score after at least 12 months (e.g., after at least 24 months or at least 36 months) of treatment.

In some embodiments, SMA therapy comprising apitegromab enhances motor function, such that an HFMSE score or an RHS score (e.g., an HFMSE score) measured at 24 months after initiation of treatment is at least 1, 3, 5, 7, or 10 points over a baseline, and wherein the baseline is obtained at or prior to initiation of the treatment. In some embodiments, SMA therapy comprising apitegromab produces at least a five-point increase in an HFMSE score relative to a baseline score after at least 24 months of treatment. In some embodiments, SMA therapy comprising apitegromab produces a 6-20 point or greater increase in an HFMSE score relative to a baseline score after at least 24 months of treatment. In some embodiments, SMA therapy comprising apitegromab produces at least a seven-point increase in an HFMSE score relative to a baseline score after at least 24 months of treatment. In some embodiments, SMA therapy comprising apitegromab produces an at least 2-point (e.g., at least 3-point, at least 4-point, or at least-5 point, at least 6-point) increase in an HFMSE score relative to a baseline after 12 months of apitegromab treatment, wherein the increase in HFMSE score is maintained at 24 months after initiating apitegromab treatment, optionally wherein the increase in HFMSE score is maintained at 36 months after initiating apitegromab treatment. In some embodiments, the subject's improved HFMSE score over baseline as measured at 12 months after initiating apitegromab treatment is maintained or further increased at 24 months. In some embodiments, the subject's improved HFMSE score over baseline as measured at 12 months after initiating apitegromab treatment is maintained or further increased at 36 months.

In some embodiments, an improvement in a motor function score (e.g., an HFMSE or RHS score) may be positively correlated with SMA severity and/or the length of treatment time with an SMN corrector therapy (e.g., nusinersen, risdiplam, and/or onasemnogene abeparvovec-xioi). In some embodiments, an improvement in a motor function score (e.g., an HFMSE or RHS score) may be inversely correlated with age and/or characteristics of advanced disease. In some embodiments, a characteristic of advanced disease includes scoliosis and/or joint contractures. In some embodiments, joint contracture can occur due to shortening of muscles, tendons, ligaments, causing deformity and symptoms include pain and loss of movement in the joint. In some embodiments, a method of treating SMA comprises administering apitegromab therapy in conjunction with (or in combination with) another therapy, to a patient lacking scoliosis and/or joint contracture. In some embodiments, apitegromab is used for treating SMA in conjunction with (or in combination with) another therapy, in a patient lacking scoliosis and/or joint contracture.

In some embodiments, SMA therapy comprising apitegromab stabilizes disease progression, such that an HFMSE score or an RHS score measured at six months or 12 months after initiation of the treatment is no less than a baseline or no more than a 0.1, 0.2, 0.3, 0.4, or 0.5 point decline, and wherein the baseline is obtained at or prior to initiation of the treatment, and optionally, wherein the stabilization is durable through at least 24 months of treatment. In some embodiments, SMA therapy comprising apitegromab stabilizes disease progression, such that an RHS score comprises less than a one point, two point, or three point decline in the RHS score relative to a baseline score after at least 12 months of treatment, and optionally, wherein the stabilization is durable through at least 24 months of treatment.

In some embodiments, SMA therapy comprising apitegromab stabilizes disease progression, such that an HFMSE score or an RHS score measured at 24 months after initiation of the treatment is no less than a baseline or no more than a 0.1, 0.2, 0.3, 0.4, or 0.5 point decline, and wherein the baseline is obtained at or prior to initiation of the treatment. In some embodiments, SMA therapy comprising apitegromab stabilizes disease progression, such that an RHS score comprises less than a one point, two point, or three point decline in the RHS score relative to a baseline score after at least 24 months of treatment.

In some embodiments, SMA therapy comprising apitegromab produces an at least 0.5-point (e.g., at least 0.75, 1, 1.25, 1.5, 2., 2.25, or 2.5-point) increase in a RULM score relative to a baseline 12 months after initiating apitegromab treatment. In some embodiments, the increase in the RULM score compared to baseline is maintained at 24 months after initiating apitegromab treatment. In some embodiments, the RULM score relative to baseline is further increased at 24 months after initiating apitegromab treatment compared to the RULM score at 12 months after initiating apitegromab treatment. In some embodiments, the subject's improved RULM score over baseline as measured at 12 months after initiating apitegromab treatment is maintained or further increased at 24 months. In some embodiments, the subject's improved RULM score over baseline as measured at 12 months after initiating apitegromab treatment is maintained or further increased at 36 months.

In some embodiments, SMA therapy comprising apitegromab improves muscle function, such that the patient's HFMSE score is increased by at least 2 points (e.g., by at least 3, 4, 5, or 6 points) at 24 months of treatment compared to baseline. In some embodiments, the patient's RULM score is increased by at least 0.5 points (e.g., at least 0.75, 1, 1.25, 1.5, 2., 2.25, or 2.5 points) at 24 months of treatment compared to baseline.

### Quality of Life

While a few previous studies reported on the effect of SMA treatment, e.g., nusinersen, on global quality of life, those results have so far demonstrated mixed results with no conclusive evidence of a significant improvement in quality of life. For instance, Montes et al. measured the six-minute walk test (6MWT) and fatigue in 14 ambulatory children 2-15 years old with type II (1) or III (13) SMA after receiving their first dose of nusinersen. Fatigue was considered as the subtractive difference in meters walked in the first minute compared to the meters walked in the sixth minute. They observed only a modest decrease or stabilization of 6MWT fatigue after nusinersen treatment and concluded that this modest effect may represent a nusinersen treatment effect but qualified the finding by noting that further understanding of the underlying mechanisms of fatigue may help understand the merely modest effect (Montes et al. (2019) Muscle Nerve 60(4):409-414).

Yao et al. assessed quality of life in children aged 17 or younger who were diagnosed with SMA and treated with nusinersen. As compared to children who did not receive nusinersen treatment, children who received nusinersen treatment had higher average scores in the Neuromuscular Diseases and Family Resources domains of the Pediatric Quality of Life Inventory 3.0 Neuromuscular Module (PedsQL NMM). However, the authors of the study cautioned that the results may not be conclusive due to the small sample size of children receiving nusinersen treatment (n=6). (Yao et al. (2021) Orphanet Journal of Rare Diseases 16:7.)

Mix et al., which evaluated global quality of life, health-related quality of life, and depressive symptoms in adolescent and adult patients with SMA under treatment with nusinersen, reported improvement in the patients' subject feeling of health over the first six months of treatment but not in the patients' subjective well-being. (Mix et al. (2021) Front Neurol 12:626787).

Other studies reported no change in quality of life in SMA patients as a result of the therapies provided in reported clinical trials. A literature review of the quality of life studies in patients with SMA (Landfeldt et al. (2019) Eur. J. Paediatric Neurology 23:347) found four relevant publications from reported clinical trials: a phase 1 trial of nusinersen, a phase 2 trial of olesoxime and a phase 2 trial of I-carnitine and valproic acid. None of the studies reported any significant change in quality of life.

Thimm et al. (2022) Frontiers in Neurology 12:8120 studied adult type 2 or type 3 SMA patients treated with nusinersen who were prospectively included in an assessment of health-related quality of life, measured by the short form of the Neuro-QoL for upper and lower extremity function prior to initiation of treatment and after 2, 6, 10 and 14 months of nusinersen treatment. The study did not show an improvement at any of the measured timepoints.

An earlier study reported on HRQoL in children aged 2-4 years who were diagnosed with type II or type III SMA, where a slight but not significant increase was observed after 85 days of treatment with nusinersen (Chiriboga et al. (2016) Neurology 86:890-7). A comprehensive literature review (Yang et al. (2022) Adv Ther 39:1915) found that, while the SMN-targeted therapies have improved the prognosis of survival for SMA patients, no treatments, other than those of Chiriboga, demonstrated a significant improvement in HRQoL.

In contrast, the present disclosure demonstrates that apitegromab is the first muscle- targeted treatment of SMA to provide improvements to the quality of life in patients with SMA. In some embodiments, these improvements are durable through at least 24 months of treatment.

In some embodiments, quality of life is assessed by conducting an interview with the subject using a semi-structured interview guide that includes questions about the subject's expectations about a meaningful improvement in their condition and their perception as to why they did or did not experience a meaningful improvement. The subject may be interviewed about their experiences with SMA and their treatment with respect to certain aspects of life, such as physical functioning, social impact, and emotional impact. Subjects may be asked to rate their quality of life by providing a score, e.g., on a scale of 0-10, where a score of zero indicates minimal impact on quality of life and a score of ten indicates extremely severe impact on quality of life.

In some embodiments, a patient with SMA (e.g., later-onset SMA) is evaluated using one of the family of instruments developed by the EUROQOL, such as the EQ-5d-3L. EQ-5D-5L, EQ-5D-Y and EQ-HWB. These multiutility instruments measure health-related quality of life. For example, the EQ-5D-5L is a descriptive system that comprises the dimensions of mobility, self-care, usual activities, pain/discomfort, and anxiety/depression.

In some embodiments, treatment with apitegromab improves a subject's Clinical Global Impression of Change (CGI-C). The CGI-C scores a clinician's impression of the subject's change in global health (Staunton et al. (2021) Health Qual Life Outcomes 19:184). In some embodiments, treatment with apitegromab improves a subject's Pediatric QoL Inventory (PedsQL). The PedsQL consists of generic core scales and disease-specific modules, e.g., the neuromuscular module, which can be used to evaluate health-related QoL in children 2-18 years old with neuromuscular diseases, including SMA (lannaccone et al. (2009) Neuromus Disord 19:805-812). In some embodiments, treatment with apitegromab improves a subject's Neuromuscular Gross Motor Outcome (GRO) score. The GRO is a gross motor outcome measure designed to assess whole body strength and function for all level of abilities across the lifespan of those with neuromuscular disease and has been validated in SMA types 1-3 (Alfano et al. (2021) Pediatric Neurology 122:21-26).

In some embodiments, treatment with apitegromab is sufficient to improve one or more measures of quality of life in an SMA subject, e.g., at 24 months after initiating the apitegromab treatment and/or at 36 months after initiating the apitegromab treatment.

In some embodiments, a patient with SMA (e.g., later-onset SMA) is evaluated, e.g., before and/or after treatment comprising apitegromab, using the Patient-reported Outcomes Measurement Information System (PROMIS). The PROMIS is a person-centered measure intended to be completed by the patient or parent proxy without help from anyone (Ader (2007) Med Care. 5(5):S1-S2). The fatigue profile domain measures a range of symptoms, from mild subjective feelings of tiredness to an overwhelming, debilitating, and sustained sense of exhaustion. The self-report measures are suitable for children 8-17 years old and the parent proxy report measures are suited for children 5-17 years old. Patients aged 18-21 may complete an adult form of PROMIS. In some embodiments, the PROMIS is completed by patients who are ≥ 2 years of age, ≥ 5 years of age or 5-21 years of age. In some embodiments, treatment with apitegromab improves a patient's PROMIS score.

The Pediatric Evaluation of Disability Inventory Computer Adaptive Test (PEDI-CAT) is a questionnaire completed by the caregiver that assesses the patient's ability to perform daily functions (Haley et al. (2005) Arch Phys Med Rehabil. 86(5):932-939). A domain directed to activities of daily living (ADL) comprises 68 items directed to areas that include getting dressed, keeping clean, home tasks, etc. A domain directed to mobility comprises 75 items directed to areas that include basic movement and transfers, standing and walking, steps and inclines, etc. The answers are scored on a four-point scale (unable to easy) and the test is suitable to assess function in newborns to 21-year-olds. Properties of the PEDI-CAT have been studied in and the test has been validated in the SMA population; a Rasch analysis revealed that the distribution of abilities for the Mobility and Daily Activities domains of the PEDI-CAT are best represented in the type 2 and type 3 populations (Pasternak et al. (2016) Muscle Nerve. 54(6):1097-1107).

In some embodiments, a patient with SMA (e.g., later-onset SMA) is evaluated using the PEDI-CAT. In some embodiments, a caregiver (who may or may not be a parent and/or legal guardian) completes the PEDI-CAT assessment. In some embodiments, the PEDI-CAT assessment is not administered to the patient or if a caregiver is not present. In some embodiments, the PEDI-CAT is filled out by the caregiver in a location where they are not watching the patient perform any functional assessment tests. In some embodiments, treatment with apitegromab improves a patient's PEDI-CAT score.

In some embodiments, treatment with apitegromab improves a subject's quality of life as evaluated on the PEDI-CAT scale, wherein, optionally, the subject's PEDI-CAT score may comprise a PEDI-CAT activities score and a PEDI-CAT mobility score. In some embodiments, treatment with apitegromab improves a subject's quality of life by evaluating the subject's level of fatigue, wherein, optionally, the subject's fatigue may be evaluated by a Patient Reported Outcomes Measurement Information System (PROMIS) score and/or an Endurance Shuttle Block and Box Test (ESBBT) score. In some embodiments, treatment with apitegromab for 12 months is sufficient to increase the subject's PEDI-CAT activities score by at least 1.9 points (e.g., increase the subject's PEDI-CAT activity score by about 2 points to about 4 points), increase the subject's PEDI-CAT mobility score by at least 0.4 points (e.g., increase the subject's PEDI-CAT mobility score by about 0.5 points to about 3 points), and/or decrease the subject's PROMIS score by at least 5.5 points (e.g., decrease the subject's PROMIS score by about 6 points to about 10 points), wherein, optionally, the subject has nonambulatory type 2 SMA and is at least two years of age. In some embodiments, the subject's PEDI-CAT activities score is further increased at 24 months as compared to 12 months after starting apitegromab treatment. In some embodiments, the subject has previously received an SMN upregulator/corrector therapy, e.g., nusinersen, risdiplam, and/or onasemnogene abeparvovec-xioi treatment, wherein the subject was first treated with the SMN upregulator/corrector therapy, e.g., nusinersen, risdiplam, and/or onasemnogene abeparvovec-xioi, before five years of age.

In some embodiments, treatment with apitegromab for 12 months is sufficient to increase the subject's PEDI-CAT activities score by at least 0.9 points (e.g., increase the subject's PEDI-CAT score by about 1 point to about 3 points) and/or decrease the subject's PROMIS score by at least 0.6 points (e.g., decrease the subject's PROMIS score by about 1 point to about 6 points, or by about 4 points to about 35 points), wherein, optionally, the subject has nonambulatory type 2 SMA and is aged 5-21 years. In some embodiments, the subject's PROMIS score is further decreased at 24 months as compared to 12 months after starting apitegromab treatment. In some embodiments, the subject has previously received an SMN upregulator/corrector therapy, e.g., nusinersen, risdiplam, and/or onasemnogene abeparvovec-xioi treatment, wherein the subject was first treated with the SMN upregulator/corrector therapy, e.g., nusinersen, risdiplam, and/or onasemnogene abeparvovec-xioi after five years of age.

In some embodiments, treatment with apitegromab for 24 months is sufficient to increase the subject's PEDI-CAT activities score by at least 1.8 points (e.g., increase the subject's PEDI-CAT activities score by about 2 to about 4 points), increase the subject's PEDI-CAT mobility score by at least 0.4 points (e.g., increase the subject's PEDI-CAT mobility score by about 0.4 to about 5 points), and/or decrease the subject's PROMIS score by at least 5.0 points (e.g., decrease the subject's PROMIS score by about 5 points to about 9 points), wherein, optionally, the subject has nonambulatory type 2 SMA and is at least two years of age. In some embodiments, the subject has previously received an SMN upregulator/corrector therapy, e.g., nusinersen, risdiplam, and/or onasemnogene abeparvovec-xioi treatment, wherein the subject was first treated with the SMN upregulator/corrector therapy, e.g., nusinersen, risdiplam, and/or onasemnogene abeparvovec-xioi before five years of age.

In some embodiments, treatment with apitegromab for 24 months is sufficient to increase the subject's PEDI-CAT activities score by at least 0.7 points (e.g., increase the subject's PEDI-CAT activities score by about 1 point to about 3 points) and/or decrease the subject's PROMIS score by at least 1.3 points (e.g., decrease the subject's PROMIS score by about 2 points to about 7 points or by about 4 points to about 10 points), wherein, optionally, the subject has nonambulatory type 2 SMA and is aged 5-21 years. In some embodiments, the subject has previously received an SMN upregulator/corrector therapy, e.g., nusinersen, risdiplam, and/or onasemnogene abeparvovec-xioi treatment, wherein the subject was first treated with the SMN upregulator/corrector therapy, e.g., nusinersen, risdiplam, and/or onasemnogene abeparvovec-xioi after five years of age.

In some embodiments, treatment with apitegromab is sufficient to increase the subject's PEDI-CAT activities of daily living score at 12 months compared to baseline, wherein, optionally, the subject's PEDI-CAT activities of daily living score is further increased at 24 months compared to 12 months. In some embodiments, treatment with apitegromab is sufficient to increase the subject's PEDI-CAT mobility score at 12 months compared to baseline, wherein, optionally, the subject's PEDI-CAT mobility score is further increased at 24 months compared to 12 months.

In some embodiments, treatment with apitegromab is sufficient to decrease the subject's PROMIS fatigue score at 12 months compared to baseline, wherein, optionally, the subject's PROMIS fatigue score is further decreased at 24 months compared to 12 months. In some embodiments, the subject's improvement in PROMIS fatigue score is maintained at 36 months.

In some embodiments, treatment with apitegromab is sufficient to increase the subject's ESBBT score at 12 months compared to baseline, wherein, optionally, the subject's ESBBT score is further increased at 24 months compared to 12 months.

The Columbia Suicide Severity Rating Scale (C-SSRS) measures suicidal ideation, the intensity of the ideation and suicidal behavior. It has been validated in various populations, including in children as young as five years old (Salvi (2019) Emerg Med Pract 21(5):CD3-CD4). In some embodiments, treatment with apitegromab will improve the subject's C-SSRS score.

In some embodiments, treatment with apitegromab improves a subject's quality of life as assessed by the severity of muscle function-related symptoms. In some embodiments, muscle function-related symptoms include muscle function severity, fatigue severity, bulbar function severity, and/or emptying severity. In some embodiments, muscle function-related symptoms are measured by the subject's rating of the severity of the symptom on a scale from 0-10, where zero indicates minimal severity and ten indicates high severity.

In some embodiments, treatment with apitegromab improves a subject's quality of life as assessed by the severity of fatigue-related symptoms. In some embodiments, fatigue-related symptoms are measured by the subject's rating of the severity of the symptom on a scale from 0-10, where zero indicates minimal severity and ten indicates high severity.

In some embodiments, treatment with apitegromab improves a subject's quality of life as assessed by symptoms related to bulbar function. Bulbar function refers to those functions that are controlled by the brainstem area; "bulb" is an archaic term for the medulla oblongata. Bulbar functions include swallowing, masticatory motor function and strength, coughing, breathing, snoring, and mandibular range of motion. Bulbar dysfunction can affect these activities. Impaired bulbar function persisted as a significant complication in most type 1 patients treated with nusinersen; only 4/24 patients remained orally fed after 24 months, as assessed by the p-FOIS (Westrate et al. (2022) Dev Med Child Neurol 64:907-914). Similarly, type 2 and type 3 patients treated with nusinersen showed no improvement in bulbar function after either 6 or 14 months of nusinersen treatment compared to that observed prior to treatment, while nusinersen was observed to improve their HFMSE scores (Brakemeier et al. (2021) Brain Sci 11:1244). In some embodiments, treatment with apitegromab improves a patient's bulbar function.

In some embodiments, bulbar function-related symptoms are measured by the subject's rating of the severity of the symptom on a scale from 0-10, where zero indicates minimal severity and ten indicates high severity.

In some embodiments, a patient with SMA is assessed for bulbar function using the Amyotrophic Lateral Sclerosis Functional Rating Scale Revised (AMLFRSR), which is a questionnaire-based scale that measures physical function in carrying out activities of daily living and has been shown to correlate with quality of life (Cedarbaum et al. (1999) J. Neurolog. Sci 169:13-21).

In some embodiments, a patient with SMA is assessed for bulbar function using the Sydney Swallow Questionnaire (SSQ), which is an instrument that assesses the severity of oral and pharyngeal dysphagia (Dwivedi et al. (2010) Oral Oncol. E10-e14).

In some embodiments, a patient with SMA is assessed using the MD Anderson Dysphagia Inventory (MDADI), which is a questionnaire that evaluates the relationship between swallowing and quality of life (Chen et al. (2001) Arch Otolaryngol Head Neck Surg 127(7):870-876).

In some embodiments, a patient with SMA is assessed using the SWAL-QoL, which is a 93-item quality of life outcome tool that measures dysphagia (McHorney et al. (2002) Dysphagia 17:97-114).

In some embodiments, a patient with SMA is assessed by conducting a qualitative, semi-structured interview with the patient or the patient's caregiver.

In some embodiments, a patient with SMA (e.g., early-onset SMA, including but not limited to type 1 SMA) is evaluated using the Pediatric Functional Oral Intake Scale (p-FOIS). The p-FOIS is a tool to assess change in functional eating abilities, via a 6-point, ordinal, clinician-rated outcome measurement scale for use in a pediatric feeding-disordered population.

In some embodiments, treatment with apitegromab improves a subject's quality of life as assessed by symptoms related to emptying. In some embodiments, symptoms related to emptying are measured by the subject's rating of the severity of the symptom on a scale from 0-10, where zero indicates minimal severity and ten indicates high severity.

In some embodiments, treatment with apitegromab improves a subject's quality of life as assessed by the severity of symptoms related to impacts to daily living. In some embodiments, impact to daily living may be assessed by physical functioning, balance, daily activities, mobility, sleep, transfers, and/or falls. In some embodiments, severity of symptoms are measured by the subject's rating of the severity of the symptom on a scale from 0-10, where zero indicates minimal severity and ten indicates high severity.

In some embodiments, an SMA subject selected for treatment with apitegromab has reported at least one of muscle weakness or tightness, fatigue, pain, impaired emptying function, or impaired bulbar function. In some embodiments, symptoms of impaired bulbar function include respiratory infection; use of assistive device; inability to cough, clear lung secretions; breathing difficulties; feeding/swallowing difficulties; difficulties with speech; and snoring. In some embodiments, symptoms of muscle weakness or tightness symptoms include problems with balance, tight muscles and tendons, decreased muscle tone, and difficulty with chewing or smiling. In some embodiments, symptoms of impaired emptying function include urinary difficulties and bowel difficulties. In some embodiments, treatment with apitegromab surprisingly results in improvement in quality of life in such patients, e.g., after 24 months of apitegromab treatment, wherein an improvement comprises an improvement in at least one of the symptoms of muscle weakness or tightness, fatigue, pain, impaired emptying function, or impaired bulbar function as reported by the subject or the subject's caregiver.

### Methods, Uses, and Compositions for Treating SMA

In various embodiments, apitegromab therapy is administered (alone or in combination with at least one additional therapy) in an amount to achieve an average serum concentration (C_{trough}) of between about 25 and 250 micrograms per milliliter. In some embodiments, apitegromab therapy is administered (alone or in combination with at least one additional therapy) in an amount to achieve an average serum concentration (Cₘₐₓ) of between about 25 and 700 micrograms per milliliter.

In some embodiments, the present disclosure provides a composition comprising apitegromab for use in treating SMA in a human subject, wherein the subject is administered apitegromab by intravenous infusion every four weeks or monthly for at least six months, 12 months, or 24 months in an amount sufficient to prevent or delay a reduction in an RHS score relative to a baseline score prior to treatment.

In some embodiments, the SMA is later-onset SMA. In some embodiments, the SMA is ambulatory type 3 SMA. In some embodiments, the subject is 2 to 12, 5 to 21, 13 to 21, or 2 to 21 years old. In some embodiments, the sufficient amount is a dose of greater than 2 mg/kg and up to 20 mg/kg, optionally about 5, 10, 15, or 20 mg/kg. In some embodiments, the sufficient amount is a dose of 10 mg/kg or 20 mg/kg apitegromab every four weeks or monthly.

In some embodiments, the apitegromab is administered in an amount to stabilize or produce a less than 0.4 point decrease in an RHS score, e.g., after at least 12 months of treatment. In some embodiments, the apitegromab is administered in an amount to produce an at least 0.2 point mean increase in an RHS score relative to a baseline score prior to treatment, optionally a 0.3 point, 0.5 point, at least 0.7 point, at least 1 point, at least 2 point, or at least 3 point mean increase in an RHS score relative to a baseline, e.g., a mean increase in a cohort of at least 11 subjects. In some embodiments, the apitegromab is administered in an amount to produce an at least 1 point mean increase in an RHS score relative to a baseline, e.g., a mean increase in a cohort of at least 11 subjects. In some embodiments, the apitegromab is administered in an amount to produce an at least 3 point mean increase in an RHS score relative to a baseline, e.g., a mean increase in a cohort of at least 11 subjects. In some embodiments, the apitegromab is administered in an amount to stabilize an RHS score, e.g., as evaluated after at least 12 months of treatment relative to baseline. In some embodiments, the apitegromab is administered in an amount to limit a reduction in an RHS score, e.g., as evaluated after at least 12 months of treatment relative to baseline, of about 0.5, 0.4, 0.3, 0.2 or 0.1.

In some embodiments, the subject is treated with an SMN upregulator therapy. In some embodiments, the SMN upregulator therapy is nusinersen or risdiplam. In some embodiments, the subject is treated with gene therapy. In some embodiments, the gene therapy is onasemnogene abeparvovec-xioi. In some embodiments, the SMN upregulator therapy is nusinersen. In some embodiments, the subject initiated the SMN upregulator therapy at or after the age of five. In some embodiments, the apitegromab is administered in an amount to stabilize an RHS score, e.g., as evaluated after at least 12 months of treatment relative to baseline. In some embodiments, the apitegromab is administered in an amount to limit a reduction in an RHS score, e.g., as evaluated after at least 12 months of treatment relative to baseline, of about 0.5, 0.4, 0.3, 0.2 or 0.1. In some embodiments, the apitegromab is administered in an amount to produce an at least 0.3 point mean increase in an RHS score relative to a baseline, e.g., a mean increase in a cohort of at least 11 subjects. In some embodiments, the apitegromab is administered in an amount to produce an at least 0.3 point mean increase in an RHS score relative to a baseline, e.g., a mean increase in a cohort of at least 11 subjects, after eight weeks. In some embodiments, the amount is a dose of 20 mg/kg apitegromab every four weeks or monthly.

In some embodiments, the apitegromab is administered in an amount to stabilize an RHS score, e.g., as evaluated after at least 12 months of treatment relative to baseline. In some embodiments, the apitegromab is administered in an amount to limit a reduction in an RHS score, e.g., as evaluated after at least 12 months of treatment relative to baseline, of about 0.5, 0.4, 0.3, 0.2 or 0.1. In some embodiments, the apitegromab is administered in an amount to produce an at least 0.7 point mean increase in an RHS score relative to a baseline, e.g., a mean increase in a cohort of at least 11 subjects. In some embodiments, the apitegromab is administered in an amount to produce an at least 0.7 point mean increase in an RHS score relative to a baseline, e.g., a mean increase in a cohort of at least 11 subjects, after eight weeks. In some embodiments, the amount is a dose of 20 mg/kg apitegromab every four weeks or monthly.

In some embodiments, the apitegromab is administered in an amount to achieve a serum concentration (C_{trough}) of at least about 100 micrograms per milliliter, e.g., about 100 to 500 micrograms per milliliter (e.g., about 100, 200, 300, 400, or 500 micrograms per milliliter), e.g., as determined in an average over at least a 12-month course of treatment. In some embodiments, the serum concentration (C_{trough}) is about 100-450 micrograms per milliliter. In some embodiments, the amount is a dose of 20 mg/kg apitegromab every four weeks or monthly.

In some embodiments, the apitegromab is administered in an amount to achieve a serum concentration (Cₘₐₓ) of at least about 300 micrograms per milliliter, e.g., as determined in an average over at least a 12-month course of treatment. In some embodiments, the serum concentration (Cₘₐₓ) is at least about 300-1100 micrograms per milliliter, e.g., 300-800. In some embodiments, the serum concentration (Cₘₐₓ) is at least 600 micrograms of apitegromab per milliliter (e.g., at least 600, 700, 800, 900, 1000 micrograms per milliliter or greater). In some embodiments, the serum concentration is between about 600-1000 micrograms per milliliter. In some embodiments, the serum concentration is a steady-state serum concentration. In some embodiments, the apitegromab is administered in an amount to achieve a steady-state serum concentration after about 112 days. In some embodiments, the amount is a dose of 20 mg/kg apitegromab every four weeks or monthly.

In some embodiments, the apitegromab is administered in an amount sufficient to achieve a target engagement as measured by a serum latent myostatin concentration of at least about 100 nanograms per milliliter, as determined at steady state over the course of at least 12 months of treatment. In some embodiments, the apitegromab is administered in an amount sufficient to achieve a target engagement as measured by a serum latent myostatin concentration of at least about 250 nanograms per milliliter, as determined at steady state over the course of at least 12 months of treatment. In some embodiments, the apitegromab is administered in an amount sufficient to achieve a target engagement as measured by a serum latent myostatin concentration of at least about 400 nanograms per milliliter. In some embodiments, the apitegromab is administered in an amount sufficient to achieve a target engagement as measured by a serum latent myostatin concentration of at least 500 nanograms per milliliter (e.g., at least 500, 550, 600, 650, 700, 750, 800, 850, 900, 950, 1000, 1050 or 1100 nanograms per milliliter). In some embodiments, the serum latent myostatin concentration is between about 550-2400 nanograms per milliliter. In some embodiments, the serum latent myostatin concentration is a steady-state concentration measured at trough. In some embodiments, the amount is a dose of 20 mg/kg apitegromab every four weeks or monthly.

In some embodiments, the apitegromab is administered in an amount to achieve one or more of the following in the subject: retaining motor function as compared to a decline in a control, delaying disease progression, delaying or preventing an ambulatory type 3 SMA patient from becoming non-ambulatory, delaying or preventing need for respiratory aid or intervention, reducing the rate of a decline in one or more motor function scores as compared to a control, and/or maintaining at least net zero change in one or more motor function scores as compared to a baseline. In some embodiments, the amount is a dose of 20 mg/kg apitegromab every four weeks or monthly. In an embodiment, a control refers to the natural history of a subject with untreated SMA.

In some embodiments, the present disclosure provides a composition comprising apitegromab for use in treating SMA in a human subject receiving an SMN upregulator, wherein the subject is administered the apitegromab by intravenous infusion every four weeks or monthly for at least 6 months, 12 months, or 24 months in an amount sufficient to produce an at least 0.5 or 1 point (e.g., at least 2, 3, 4, 5, or 6 points) mean increase in an HFMSE score relative to a baseline score prior to treatment, e.g., a mean increase in a cohort of at least 13 or 14 subjects.

In some embodiments, the SMA is later-onset SMA. In some embodiments, the SMA is type 2 SMA. In some embodiments, the SMA is non-ambulatory type 3 SMA. In some embodiments, the subject is 2 to 12, 5 to 21, 13 to 21, or 2 to 21 years old. In some embodiments, the SMN upregulator therapy is nusinersen or risdiplam. In some embodiments the gene therapy is onasemnogene abeparvovec-xioi. In some embodiments, the SMN upregulator therapy is nusinersen. In some embodiments, the sufficient amount is a dose of greater than 2 mg/kg and up to 20 mg/kg, optionally about 5, 10, 15, or 20 mg/kg. In some embodiments, the sufficient amount is a dose of 10 mg/kg or 20 mg/kg apitegromab every four weeks or monthly.

In some embodiments, the apitegromab is administered in an amount to produce an at least 0.5 point or 1 point increase in an HFMSE score relative to a baseline, e.g., a mean increase in a cohort of at least 13 or 14 subjects, after 16 weeks. In some embodiments, the apitegromab is administered in an amount to produce an at least 2 point, at least 3 point, at least 4 point, or at least 5 point increase in an HFMSE score relative to a baseline, e.g., a mean increase in a cohort of at least 13 or 14 subjects. In some embodiments, the apitegromab is administered in an amount to produce an at least 3 point increase in an HFMSE score relative to a baseline, e.g., a mean increase in a cohort of at least 13 or 14 subjects. In some embodiments, the apitegromab is administered in an amount to produce an at least 5 point increase in an HFMSE score relative to a baseline, e.g., a mean increase in a cohort of at least 13 or 14 subjects. In some embodiments, the amount is a dose of 20 mg/kg apitegromab every four weeks or monthly.

In some embodiments, the apitegromab is administered in an amount to achieve a serum concentration (Cₘₐₓ) of at least about 300 micrograms of apitegromab per milliliter, e.g., at least about 400, 500, 600, 700, 800, 900, 1000, or 1100. In some embodiments, the apitegromab is administered in an amount to achieve a serum concentration of at least 600 micrograms of apitegromab per milliliter (e.g., at least 600, 700, 800, 900, 1000 micrograms per milliliter or greater). In some embodiments, the serum concentration is between about 600-1000 micrograms per milliliter. In some embodiments, the serum concentration is a steady-state serum concentration. In some embodiments, the apitegromab is administered in an amount to achieve a steady-state serum concentration after about 112 days. In some embodiments, the amount is a dose of 20 mg/kg apitegromab every four weeks or monthly.

In some embodiments, the apitegromab is administered in an amount to achieve a serum concentration (C_{trough}) of at least about 100 micrograms per milliliter, e.g., at least about 200, 300, or 400 micrograms per milliliter. In some embodiments, the amount is a dose of 20 mg/kg apitegromab every four weeks or monthly.

In some embodiments, the apitegromab is administered in an amount sufficient to achieve a target engagement as measured by a serum latent myostatin concentration of at least about 250 nanograms per milliliter, e.g., at least about 400 or 500 nanograms per milliliter (e.g., at least 500, 550, 600, or 650 nanograms per milliliter). In some embodiments, the serum latent myostatin concentration is between about 550-1650 nanograms per milliliter. In some embodiments, the serum latent myostatin concentration is a steady-state concentration measured at trough. In some embodiments, the amount is a dose of 20 mg/kg apitegromab every four weeks or monthly.

In some embodiments, the apitegromab is administered in an amount to achieve one or more of the following in the subject: retaining motor function as compared to a decline in a control, delaying disease progression, delaying or preventing an ambulatory type 3 SMA patient from becoming non-ambulatory, delaying or preventing need for respiratory aid or intervention, reducing the rate of a decline in one or more motor function scores as compared to a control, and/or maintaining at least net zero change in one or more motor function scores as compared to a baseline. In some embodiments, the amount is a dose of 20 mg/kg apitegromab every four weeks or monthly.

In some embodiments, the present disclosure provides a composition comprising apitegromab for use in treating SMA in a human subject receiving an SMN upregulator, wherein the subject is administered apitegromab by intravenous infusion every four weeks or monthly for at least six months, 12 months, or 24 months in an amount sufficient to produce an at least two point mean increase in an HFMSE score relative to a baseline score prior to treatment, e.g., a mean increase in a cohort of at least eight or nine subjects, and wherein the subject initiated the SMN upregulator therapy before the age of five.

In some embodiments, the SMA is later-onset SMA. In some embodiments, the SMA is type 2 SMA. In some embodiments, the subject is 2 years of age or older. In some embodiments, the SMN upregulator therapy is nusinersen or risdiplam. In some embodiments, the gene therapy is onasemnogene abeparvovec. In some embodiments, the SMN upregulator therapy is nusinersen. In some embodiments, the sufficient amount is a dose of greater than 2 mg kg and up to 20 mg/kg, optionally about 5, 10, 15, or 20 mg/kg. In some embodiments, the sufficient amount is a dose of 10 mg/kg or 20 mg/kg apitegromab every four weeks or monthly.

In some embodiments, the apitegromab is administered in an amount to produce an at least two point mean increase in an HFMSE score relative to a baseline, e.g., a mean increase in a cohort of at least eight or nine subjects, after 12 months or 24 months. In some embodiments, the apitegromab is administered in an amount to produce an at least three point, at least four point, at least five point, at least six point, or at least seven point mean increase in an HFMSE score relative to a baseline, e.g., a mean increase in a cohort of at least eight or nine subjects, e.g., after at least 12 months of treatment, e.g., after at least 24 months of treatment. In some embodiments, the apitegromab is administered in an amount to produce an at least three point mean increase in an HFMSE score relative to a baseline, e.g., a mean increase in a cohort of at least eight or nine subjects. In some embodiments, the apitegromab is administered in an amount to produce an at least five point mean increase in an HFMSE score relative to a baseline, e.g., a mean increase in a cohort of at least eight or subjects. In some embodiments, the apitegromab is administered in an amount to produce an at least six point mean increase in an HFMSE score relative to a baseline, e.g., a mean increase in a cohort of at least eight or nine subjects. In some embodiments, the apitegromab is administered in an amount to produce an at least seven point mean increase in an HFMSE score relative to a baseline, e.g., a mean increase in a cohort of at least eight or nine subjects. In some embodiments, the amount is a dose of 20 mg/kg apitegromab every four weeks or monthly.

In some embodiments, the apitegromab is administered in an amount to achieve a serum concentration (Cₘₐₓ) of at least about 25-1100 micrograms of apitegromab per milliliter. In some embodiments, the apitegromab is administered at 2 mg/kg to obtain a serum concentration of about 25-55 micrograms per milliliter. In some embodiments, the apitegromab is administered at greater than 2 mg/kg and up to 20 mg/kg to obtain a serum concentration of about 250-1100 micrograms per milliliter. In some embodiments, the serum concentration is 600 micrograms of apitegromab per milliliter (e.g., at least 600, 700, 800, 900, 1000 micrograms per milliliter or greater). In some embodiments, the serum concentration is between about 600-1000 micrograms per milliliter. In some embodiments, the serum concentration is a steady-state serum concentration. In some embodiments, the apitegromab is administered in an amount to achieve a steady-state serum concentration after about 112 days or after about five half-lives. In some embodiments, the amount greater than 2 mg/kg and up to 20 mg/kg is a dose of 20 mg/kg apitegromab every four weeks or monthly. In some embodiments, increasing the dose of apitegromab yields a dose response in serum concentration (Cₘₐₓ), e.g., increasing the dose tenfold from 2 mg/kg and up to 20 mg/kg yields a tenfold increase in Cₘₐₓ.

In some embodiments, the apitegromab is administered in an amount to achieve a serum concentration (C_{trough}) of at least about 25 micrograms of apitegromab per milliliter, e.g., at least about 50, 100, 200, 300, or 400 micrograms of apitegromab per milliliter. In some embodiments, the apitegromab is administered at 2 mg/kg to obtain a serum concentration of about 25-55 micrograms per milliliter. In some embodiments, the apitegromab is administered at greater than 2 mg/kg and up to 20 mg/kg to obtain a serum concentration of about 100-400 micrograms per milliliter. In some embodiments, the amount greater than 2 mg/kg and up to 20 mg/kg is a dose of 20 mg/kg apitegromab every four weeks or monthly. In some embodiments, increasing the dose of apitegromab yields a dose response in serum concentration (C_{trough}), e.g., increasing the dose tenfold from 2 mg/kg and up to 20 mg/kg yields a tenfold increase in C_{trough}.

In some embodiments, the apitegromab is administered in an amount sufficient to achieve a target engagement as measured by a serum latent myostatin concentration of at least about 250 nanograms per milliliter, e.g., at least about 400 nanograms per milliliter or 500 nanograms per milliliter (e.g., at least 500, 550, 600, or 650 nanograms per milliliter). In some embodiments, the serum latent myostatin concentration is between about 550-1100 or 600-1000 nanograms per milliliter. In some embodiments, the serum latent myostatin concentration is a steady-state concentration measured at trough. In some embodiments, the amount is a dose of 20 mg/kg apitegromab every four weeks or monthly.

In some embodiments, the apitegromab is administered in an amount to achieve one or more of the following in the subject: retaining motor function as compared to a decline in a control, delaying disease progression, delaying or preventing an ambulatory type 3 SMA patient from becoming non-ambulatory, delaying or preventing need for respiratory aid or intervention, reducing the rate of a decline in one or more motor function scores as compared to a control, and/or maintaining at least net zero change in one or more motor function scores as compared to a baseline. In some embodiments, the amount is a dose of 20 mg/kg apitegromab every four weeks or monthly.

In some embodiments, the present disclosure provides a composition comprising apitegromab for use in treating SMA, wherein a patient population is administered apitegromab by intravenous infusion every four weeks or monthly for at least six months, 12 months, or 24 months in an amount sufficient to increase a clinical response rate (proportion of responders) in the patient population, and wherein the clinical response rate is measured by a 6-minute Walk Test and/or a 30-second Sit-to-Stand Test.

In some embodiments, the patient population is a later-onset SMA population. In some embodiments, the patient population is an ambulatory type 3 SMA population. In some embodiments, the patient population comprises human subjects 5 to 21 years old. In some embodiments, the patient population is treated with an SMN upregulator therapy. In some embodiments, the SMN upregulator therapy is nusinersen or risdiplam. In some embodiments, the gene therapy is onasemnogene abeparvovec. In some embodiments, the SMN regulator therapy is nusinersen. In some embodiments, the patient population initiated the SMN upregulator therapy after the age of five. In some embodiments, the sufficient amount is a dose of greater than 2 mg/kg and up to 20 mg/kg, optionally about 5, 10, 15, or 20 mg/kg. In some embodiments, the sufficient amount is a dose of 10 mg/kg or 20 mg/kg apitegromab every four weeks or monthly.

In some embodiments, the apitegromab is administered in an amount to achieve a serum concentration of at least 600 micrograms of apitegromab per milliliter (e.g., at least 600, 700, 800, 900, 1000 micrograms per milliliter or greater). In some embodiments, the serum concentration is between about 600-1000 micrograms per milliliter. In some embodiments, the serum concentration is a steady-state serum concentration. In some embodiments, the apitegromab is administered in an amount to achieve a steady-state serum concentration after about 112 days. In some embodiments, the amount is a dose of 10 mg/kg or 20 mg/kg apitegromab every four weeks or monthly.

In some embodiments, the apitegromab is administered in an amount sufficient to achieve a target engagement as measured by a serum latent myostatin concentration of at least 500 nanograms per milliliter (e.g., at least 500, 550, 600, or 650 nanograms per milliliter). In some embodiments, the serum latent myostatin concentration is between about 550-1100 or 600-1000 nanograms per milliliter. In some embodiments, the serum latent myostatin concentration is a steady-state concentration measured at trough. In some embodiments, the amount is a dose of 10 mg/kg or 20 mg/kg apitegromab every four weeks or monthly.

In some embodiments, the apitegromab is administered in an amount to achieve one or more of the following in the subject: retaining motor function as compared to a decline in a control, delaying disease progression, delaying or preventing an ambulatory type 3 SMA patient from becoming non-ambulatory, delaying or preventing need for respiratory aid or intervention, reducing the rate of a decline in one or more motor function scores as compared to a control, and/or maintaining at least net zero change in one or more motor function scores as compared to a baseline. In some embodiments, the amount is a dose of 10 mg/kg or 20 mg/kg apitegromab every four weeks or monthly.

In some embodiments, the present disclosure provides a composition comprising apitegromab for use in treating SMA, wherein a patient population is administered apitegromab by intravenous infusion every four weeks or monthly for at least six months, 12 months, or 24 months in an amount sufficient to increase a clinical response rate (proportion of responders) in the patient population, and wherein the clinical response rate is measured by RULM and/or WHO milestones

In some embodiments, the patient population is a later-onset SMA population. In some embodiments, the patient population is a type 2 SMA population. In some embodiments, the patient population is a non-ambulatory type 3 SMA population. In some embodiments, the patient population comprises human subjects ages two or older. In some embodiments, the patient population comprises human subjects 2 to 12, 5 to 21, 13 to 21, or 2 to 21 years old. In some embodiments, the patient population is treated with an SMN upregulator therapy. In some embodiments, the SMN upregulator therapy is nusinersen or risdiplam. In some embodiments, the gene therapy is onasemnogene abeparvovec. In some embodiments, the SMN regulator therapy is nusinersen. In some embodiments, the sufficient amount is a dose of greater than 2 mg/kg and up to 20 mg/kg, optionally about 5, 10, 15, or 20 mg/kg. In some embodiments, the sufficient amount is a dose of 10 mg/kg or 20 mg/kg apitegromab every four weeks or monthly.

In some embodiments, the apitegromab is administered in an amount to achieve a serum concentration of at least 600 micrograms of apitegromab per milliliter (e.g., at least 600, 700, 800, 900, 1000 micrograms per milliliter or greater). In some embodiments, the serum concentration is between about 600-1000 micrograms per milliliter. In some embodiments, the serum concentration is a steady-state serum concentration. In some embodiments, the apitegromab is administered in an amount to achieve a steady-state serum concentration after about 112 days. In some embodiments, the amount is a dose of 10 mg/kg or 20 mg/kg apitegromab every four weeks or monthly.

In some embodiments, the apitegromab is administered in an amount sufficient to achieve a target engagement as measured by a serum latent myostatin concentration of at least 500 nanograms per milliliter (e.g., at least 500, 550, 600, or 650 nanograms per milliliter). In some embodiments, the serum latent myostatin concentration is between about 550-1100 or 600-1000 nanograms per milliliter. In some embodiments, the serum latent myostatin concentration is a steady-state concentration measured at trough. In some embodiments, the amount is a dose of 10 mg/kg or 20 mg/kg apitegromab every four weeks or monthly.

In some embodiments, the apitegromab is administered in an amount to achieve one or more of the following in the subject: retaining motor function as compared to a decline in a control, delaying disease progression, delaying or preventing an ambulatory type 3 SMA patient from becoming non-ambulatory, delaying or preventing need for respiratory aid or intervention, reducing the rate of a decline in one or more motor function scores as compared to a control, and/or maintaining at least net zero change in one or more motor function scores as compared to a baseline. In some embodiments, the amount is a dose of 10 mg/kg or 20 mg/kg apitegromab every four weeks or monthly.

In some embodiments, the present disclosure provides apitegromab for use in treating later-onset SMA in a human subject who is two years of age or older, wherein a dose of greater than 2 mg/kg and up to 20 mg/kg of the apitegromab is administered by intravenous infusion monthly.

In some embodiments, the apitegromab is administered at a dose of 10 mg/kg or 20 mg/kg monthly. In some embodiments, the apitegromab is administered as an adjunct to an SMN upregulator therapy. In some embodiments, the apitegromab is administered as an adjunct to an SMN upregulator therapy comprising nusinersen. In some embodiments, the apitegromab is administered as an adjunct to an SMN upregulator therapy comprising risdiplam. In some embodiments, the apitegromab is administered as an adjunct to an SMN upregulator therapy comprising onasemnogene abeparvovec-xioi.

In some embodiments, the apitegromab is administered in an amount to achieve a serum concentration of at least 600 micrograms of apitegromab per milliliter (e.g., at least 600, 700, 800, 900, 1000 micrograms per milliliter or greater). In some embodiments, the serum concentration is between about 600-1000 micrograms per milliliter. In some embodiments, the serum concentration is a steady-state serum concentration. In some embodiments, the apitegromab is administered in an amount to achieve a steady-state serum concentration after about 112 days. In some embodiments, the amount is a dose of 10 mg/kg or 20 mg/kg apitegromab monthly.

In some embodiments, the apitegromab is administered in an amount sufficient to achieve a target engagement as measured by a serum latent myostatin concentration of at least 500 nanograms per milliliter (e.g., at least 500, 550, 600, or 650 nanograms per milliliter). In some embodiments, the serum latent myostatin concentration is between about 550-1100 or 600-1000 nanograms per milliliter. In some embodiments, the serum latent myostatin concentration is a steady-state concentration measured at trough. In some embodiments, the amount is a dose of 10 mg/kg or 20 mg/kg apitegromab monthly.

In some embodiments, the apitegromab is administered in an amount to achieve one or more of the following in the subject: retaining motor function as compared to a decline in a control, delaying disease progression, delaying or preventing an ambulatory type 3 SMA patient from becoming non-ambulatory, delaying or preventing need for respiratory aid or intervention, reducing the rate of a decline in one or more motor function scores as compared to a control, and/or maintaining at least net zero change in one or more motor function scores as compared to a baseline. In some embodiments, the amount is a dose of 10 mg/kg or 20 mg/kg apitegromab monthly.

In some embodiments, the present disclosure provides apitegromab for use in treating later-onset SMA in a human subject, wherein the treatment comprises administering to the subject more than 2 mg/kg and up to 20 mg/kg of apitegromab every four weeks or monthly, wherein the subject initiated a motor neuron-directed therapy for SMA before the age of 5 and wherein the motor neuron-directed therapy increases SMN1 or SMN2 expression.

In some embodiments, the treatment results in at least a two point mean increase in an HFMSE score relative to a baseline score prior to treatment, e.g., a mean increase in a cohort of at least eight or nine subjects, after six months, 12 months, or 24months. In some embodiments, the motor neuron-directed therapy is nusinersen or risdiplam. In some embodiments, the gene therapy is onasemnogene abeparvovec-xioi. In some embodiments, the motor neuron-directed therapy is nusinersen. In some embodiments, the apitegromab is administered by intravenous infusion. In some embodiments, the apitegromab is administered at a dose of greater than 2 mg/kg and up to 20 mg/kg every four weeks or monthly. In some embodiments, the apitegromab is administered at a dose of 10 mg/kg or 20 mg/kg every four weeks or monthly. In some embodiments, the later-onset SMA patient is also categorized as a type 2 SMA, type 3 SMA, or type 4 SMA patient. In some embodiments, the type 3 SMA is ambulatory or non-ambulatory.

In some embodiments, the apitegromab is administered in an amount to achieve a serum concentration of at least 600 micrograms of apitegromab per milliliter (e.g., at least 600, 700, 800, 900, 1000 micrograms per milliliter or greater). In some embodiments, the serum concentration is between about 600-1000 micrograms per milliliter. In some embodiments, the serum concentration is a steady-state serum concentration. In some embodiments, the apitegromab is administered in an amount to achieve a steady-state serum concentration after about 112 days. In some embodiments, the apitegromab is administered in an amount sufficient to achieve a target engagement as measured by a serum latent myostatin concentration of at least 500 nanograms per milliliter (e.g., at least 500, 550, 600, or 650 nanograms per milliliter). In some embodiments, the serum latent myostatin concentration is between about 550-1100 or 600-1000 nanograms per milliliter. In some embodiments, the serum latent myostatin concentration is a trough concentration. In some embodiments, the apitegromab is administered in an amount to achieve one or more of the following in the subject: retaining motor function as compared to a decline in a control, delaying disease progression, delaying or preventing an ambulatory type 3 SMA patient from becoming non-ambulatory, delaying or preventing need for respiratory aid or intervention, reducing the rate of a decline in one or more motor function scores as compared to a control, and/or maintaining at least net zero change in one or more motor function scores as compared to a baseline. In some embodiments, the amount is a dose of 10 mg/kg or 20 mg/kg apitegromab every four weeks or monthly.

In some embodiments, the present disclosure provides apitegromab for use in treating SMA in a human subject who did not receive an SMN upregulator therapy before the age of five, wherein the treatment comprises administering to the subject a dose of greater than 2 mg/kg and up to 20 mg/kg (e.g., 10 mg/kg or 20 mg/kg) of the apitegromab every four weeks or monthly, wherein the apitegromab stabilizes disease progression, such that an HFMSE score or an RULM score measured at six months, 12 months, or 24 months after initiation of the treatment is no less than a baseline, and wherein the baseline is obtained at or prior to initiation of the treatment.

In some embodiments, the apitegromab is administered by intravenous infusion. In some embodiments, the apitegromab is administered at a dose of 10 mg/kg every four weeks or monthly. In some embodiments, the apitegromab is administered at a dose of 20 mg/kg every four weeks or monthly.

In some embodiments, the apitegromab is administered in an amount to achieve a serum concentration of at least 600 micrograms of apitegromab per milliliter (e.g., at least 600, 700, 800, 900, 1000 micrograms per milliliter or greater). In some embodiments, the serum concentration is between about 600-1000 micrograms per milliliter. In some embodiments, the serum concentration is a steady-state serum concentration. In some embodiments, the apitegromab is administered in an amount to achieve a steady-state serum concentration after about 112 days. In some embodiments, the apitegromab is administered in an amount sufficient to achieve a target engagement as measured by a serum latent myostatin concentration of at least 500 nanograms per milliliter (e.g., at least 500, 550, 600, or 650 nanograms per milliliter). In some embodiments, the serum latent myostatin concentration is between about 550-1100 or 600-1000 nanograms per milliliter. In some embodiments, the serum latent myostatin concentration is a trough concentration. In some embodiments, the apitegromab is administered in an amount to achieve one or more of the following in the subject: retaining motor function as compared to a decline in a control, delaying disease progression, delaying or preventing an ambulatory type 3 SMA patient from becoming non-ambulatory, delaying or preventing need for respiratory aid or intervention, reducing the rate of a decline in one or more motor function scores as compared to a control, and/or maintaining at least net zero change in one or more motor function scores as compared to a baseline. In some embodiments, the amount is a dose of 10 mg/kg or 20 mg/kg apitegromab every four weeks or monthly.

In some embodiments, the present disclosure provides apitegromab for use in the treatment of later-onset SMA in a human subject, wherein the treatment comprises administering apitegromab in an amount to achieve a maximum serum concentration of at least 600 micrograms of apitegromab per milliliter (e.g., at least 600, 700, 800, 900, 1000 micrograms per milliliter or greater).

In some embodiments, the maximum serum concentration is at least 600 micrograms per milliliter. In some embodiments, the maximum serum concentration is between about 600-1000 micrograms per milliliter. In some embodiments, the serum concentration is a steady-state serum concentration. In some embodiments, the amount is a dose greater than 2 mg/kg apitegromab. In some embodiments, the amount is a dose of greater than 2 mg/kg and up to 20 mg/kg apitegromab, optionally about 5, 10, 15, or 20 mg/kg, administered by intravenous infusion every four weeks or monthly. In some embodiments, the amount is a dose of 10 mg/kg or 20 mg/kg apitegromab administered by intravenous infusion every four weeks or monthly. In some embodiments, the apitegromab is administered in an amount sufficient to produce an at least one point mean increase in an HFMSE score relative to a baseline score prior to treatment, e.g., a mean increase in a cohort of at least eight or nine subjects. In some embodiments, the apitegromab is administered in an amount sufficient to produce an at least two point mean increase in an HFMSE score relative to a baseline score prior to treatment, e.g., a mean increase in a cohort of at least nine subjects. In some embodiments, the later-onset SMA patient is a type 2 or type 3 SMA patient, and optionally wherein the subject is on an SMN upregulator therapy, and optionally wherein the subject initiated the SMN upregulator therapy before the age of five.

In some embodiments, the present disclosure provides apitegromab for use in the treatment of later-onset SMA in a human subject, wherein the treatment comprises administering apitegromab in an amount sufficient to achieve a target engagement as measured by a serum latent myostatin concentration of at least 500 nanograms per milliliter (e.g., at least 500, 550, 600, or 650 nanograms per milliliter).

In some embodiments, the serum latent myostatin concentration is between about 550-1100 or 600-1000 nanograms per milliliter. In some embodiments, the serum latent myostatin concentration is a steady-state concentration. In some embodiments, the amount is a dose greater than 2 mg/kg apitegromab. In some embodiments, the amount is a dose of greater than 2 mg/kg and up to 20 mg/kg apitegromab, optionally about 5, 10, 15, or 20 mg/kg, administered by intravenous infusion every four weeks or monthly. In some embodiments, the amount is a dose of 10 mg/kg or 20 mg/kg apitegromab administered by intravenous infusion every four weeks or monthly. In some embodiments, the apitegromab is administered in an amount sufficient to produce an at least one point mean increase in an HFMSE score relative to a baseline score prior to treatment, e.g., a mean increase in a cohort of at least eight or nine subjects. In some embodiments, the apitegromab is administered in an amount sufficient to produce an at least two point mean increase in an HFMSE score relative to a baseline score prior to treatment, e.g., a mean increase in a cohort of at least nine subjects. In some embodiments, the later-onset SMA is in a patient categorized as having type 2 or type 3 SMA, and optionally wherein the subject is on an SMN upregulator therapy, and optionally wherein the subject initiated the SMN upregulator therapy before the age of five.

In some embodiments, the present disclosure provides a composition comprising apitegromab for use in the treatment of SMA in a human subject, wherein the treatment comprises administration of apitegromab at a dose sufficient to achieve at least 600 micrograms per milliliter of serum exposure, which produces a clinical benefit characterized by: motor function improvement, disease stabilization, or delay in disease progression. In some embodiments, the treatment comprises intravenous administration of the composition at about 10 mg/kg or about 20 mg/kg apitegromab.

In some embodiments, the dose is about 10 mg/kg or about 20 mg/kg administered intravenously every four weeks or monthly. In some embodiments, the SMA is a later-onset SMA, wherein optionally the later-onset SMA is type 2 SMA, type 3 SMA, or type 4 SMA, wherein further optionally the type 3 SMA is ambulatory or non-ambulatory. In some embodiments, the SMA is a later-onset SMA, wherein optionally the later-onset SMA is phenotypically characterized by symptom onset of six months of age or later. In some embodiments, the SMA is a later-onset SMA, wherein optionally the later-onset SMA is genotypically characterized by having two or more copies of the SMN2 gene, wherein optionally the subject carries two, three, four, five, or six copies of the SMN2 gene.

In some embodiments, the human subject initiated an SMN upregulator therapy before the age of five, wherein optionally the subject has a baseline HFMSE score of between 12-44 or 14-42 prior to or at the time of initiation of the apitegromab treatment. In some embodiments, the human subject initiated an SMN upregulator therapy at or after the age of five, wherein optionally: a) the subject has a baseline HFMSE score of between 13-39, and/or b) the subject has a baseline RHS score of between 44-62, prior to or at the time of initiation of the apitegromab treatment.

In some embodiments, the motor function improvement comprises an at least one point increase over a baseline in a motor function assessment score after six months of treatment with the apitegromab. In some embodiments, the motor function improvement comprises an at least two point increase over a baseline in a motor function assessment score after six months of treatment with the apitegromab. In some embodiments, the motor function improvement comprises an at least three point increase over a baseline in a motor function assessment score after six months of treatment with the apitegromab. In some embodiments, the motor function improvement comprises an at least four point increase over a baseline in a motor function assessment score after six months of treatment with the apitegromab. In some embodiments, the motor function improvement comprises an at least five point increase over a baseline in a motor function assessment score after six months of treatment with the apitegromab. In some embodiments, the disease stabilization comprises no net loss in a motor function assessment score after six months of treatment with the apitegromab, wherein optionally, the subject is classified in a patient population that statistically manifests disease progression characterized by a decline in a motor function assessment score over a 12-month or 24-month period. In some embodiments, an improved motor function score as determined after 12 months of treatment is maintained after 24 months of treatment. In some embodiments, the motor function score is further improved after 24 months of treatment as compared to the motor function score after 12 months of treatment. In some embodiments, the motor function assessment score is an HFMSE score, an RHS score, or an RULM score.

In some embodiments, the present disclosure provides a method of achieving a target saturation in a SMA patient using apitegromab, the method comprising administering to the SMA patient about 10 mg/kg or about 20 mg/kg of apitegromab by intravenous infusion every four weeks or monthly.

In some embodiments, the present disclosure provides a method of achieving a target saturation in an SMA patient using apitegromab, the method comprising administering to the SMA patient apitegromab at a dose sufficient to achieve at least 600 micrograms per milliliter of serum exposure, wherein optionally the apitegromab is administered by intravenous infusion every four weeks or monthly.

In some embodiments, the present disclosure provides a method of treating ambulatory SMA in a human subject, the method comprising administering to the subject a composition comprising apitegromab by intravenous infusion every four weeks or monthly for at least six months, wherein the subject has a baseline RHS score of at least 26 prior to or at initiation of the apitegromab treatment, and wherein the apitegromab treatment that achieves a serum exposure of at least 600 micrograms per milliliter of apitegromab is sufficient to improve or stabilize the disease. In some embodiments, the subject initiated an SMN upregulator therapy at or after the age of five. In some embodiments, the baseline RHS score is no greater than 63.

In some embodiments, the present disclosure provides a method of treating type 2 or non-ambulatory type 3 SMA in a human subject, the method comprising administering to the subject a composition comprising apitegromab by intravenous infusion every four weeks or monthly for at least six months, wherein the subject initiated an SMN upregulator therapy at or after the age of five, and wherein the subject has a baseline HFMSE score of at least 13, no greater than 39, or both (between 13-39) prior to or at initiation of the apitegromab treatment that achieves a serum exposure of at least 600 micrograms per milliliter. In some embodiments, the composition is sufficient to achieve at least one point increase in the HFMSE score over the baseline after six months.

In some embodiments, the present disclosure provides a method of treating type 2 or non-ambulatory SMA in a human subject, the method comprising administering to the subject a composition comprising apitegromab by intravenous infusion every four weeks or monthly for at least six months, wherein the subject initiated an SMN upregulator therapy prior to the age of five, and the subject has a baseline HFMSE score of at least 12, no greater than 44, or both (between 12-44) prior to or at initiation of the apitegromab treatment that achieves a serum exposure of at least 600 micrograms per milliliter. In some embodiments, the composition is sufficient to achieve an at least one point or three point increase in the HFMSE score over the baseline after six months. In some embodiments, the apitegromab is dosed at about 20 mg/kg.

In some embodiments, the present disclosure provides a selective myostatin inhibitor for use in the treatment of later-onset SMA in a human patient, wherein the human patient has a non-ambulatory form of SMA, wherein the patient has a baseline HFMSE score ranging 12-44, and wherein the patient is treated with an SMN upregulator therapy, wherein the selective myostatin inhibitor is an antibody that specifically binds a latent myostatin complex, thereby preventing release of mature myostatin.

In some embodiments, the present disclosure provides a selective myostatin inhibitor and an SMN upregulator for use in the treatment of later-onset SMA in a human patient, wherein the patient has a baseline HFMSE score of no more than 44, wherein the selective myostatin inhibitor is an antibody that specifically binds a latent myostatin complex, thereby preventing release of mature myostatin, wherein further optionally, the SMN upregulator is an SMN2 upregulator and/or an SMN1 gene therapy.

In some embodiments, the present disclosure provides a composition comprising apitegromab for use in the treatment of SMA in a human subject, wherein the treatment comprises intravenous administration of the composition to the subject at a dose of greater than 2 mg/kg and up to 20 mg/kg of apitegromab every four weeks or monthly, wherein the subject initiates or initiated an SMN upregulator therapy before the age of five. In some embodiments, the SMA is later-onset SMA. In some embodiments, the subject has two or more copies of the SMN2 gene. In some embodiments, the SMA is type 2 SMA, wherein optionally, the subject has 2-4 copies of the SMN2 gene. In some embodiments, the subject is non-ambulatory. In some embodiments, the subject is two years of age or older. In some embodiments, the subject is genetically identified as a carrier of a mutation in the SMN1 gene0. In some embodiments, the subject has a baseline HFMSE score of at least 12, prior to or at the time of receiving a first dose of apitegromab. In some embodiments, the composition is administered to the subject in an amount sufficient to achieve sustained (steady state) target engagement of at least about 250 ng/mL of serum latent myostatin. In some embodiments, the amount or dose of apitegromab is 10 mg/kg or 20 mg/kg. In some embodiments, the subject has received at least four doses of the SMN upregulator therapy at the time of receiving a first dose of apitegromab. In some embodiments, the subject has type 2 SMA. In some embodiments, the apitegromab is administered in a dose sufficient to produce at least a 5 point increase in an HFMSE score relative to a baseline score after at least 12 months of treatment, e.g., at least 24 months of treatment. In some embodiments, the apitegromab is administered in a dose sufficient to produce a 6-20 point or greater increase in an HFMSE score, and preferably at least a seven point increase, relative to a baseline score after at least 12 months of treatment, e.g., at least 24 months of treatment. In some embodiments, a composition comprising apitegromab is intravenously dosed at 20 mg/kg every four weeks or monthly. In some embodiments, the composition comprising apitegromab is intravenously dosed at 10 mg/kg every four weeks or monthly. In some embodiments, the composition is formulated at a concentration of about 50 mg/mL

In some embodiments, the present disclosure provides a method of obtaining an at least seven point increase in an HFMSE score in a human subject, relative to a baseline score, comprising administering to the subject a composition comprising about 10 mg/kg or 20 mg/kg of apitegromab by intravenous infusion every four weeks or monthly for at least 12 months or at least 24 months, wherein the subject has later-onset SMA and initiated treatment with In some embodiments, the apitegromab is administered as an adjunct to an SMN upregulator therapy comprising before the age of five.

In some embodiments, the present disclosure provides a method for monitoring treatment of SMA in a human subject, comprising administering to the subject a composition comprising apitegromab by intravenous infusion every four weeks or monthly in an amount greater than 2 mg/kg and up to 20 mg/kg; and detecting a serum latent myostatin concentration, wherein a serum latent myostatin concentration of at least about 250 ng/mL indicates treatment efficacy, wherein the subject has later-onset (optionally type 2) SMA and initiated treatment with In some embodiments, the apitegromab is administered as an adjunct to an SMN upregulator therapy comprising before the age of five. In some embodiments, the subject is non-ambulatory. In some embodiments, the subject is two years of age or older. In some embodiments, the apitegromab is administered at a dose of 10 mg/kg or 20 mg/kg every four weeks or monthly. In some embodiments, the method further comprises administering an additional dose of apitegromab to the subject having a serum latent myostatin concentration of at least about 250 ng/mL.

In some embodiments, the present disclosure provides a method of treating SMA in a human subject, comprising administering to the subject a composition comprising apitegromab, wherein the apitegromab is administered every four weeks or monthly in an amount of greater than 2 mg/kg and up to about 20 mg/kg (e.g., 10 mg/kg or 20 mg/kg), wherein up to three consecutive doses of the apitegromab may be skipped if the subject has a serum latent myostatin concentration of at least about 250 ng/mL, wherein the subject has late onset SMA and initiated an SMN upregulator therapy before the age of five. In some embodiments, the apitegromab is administered by intravenous infusion. In some embodiments, the subject is non-ambulatory and/or has type 2 SMA. In some embodiments, the subject is two years of age or older.

In some embodiments, the present disclosure provides a method of treating SMA in a human subject, comprising administering to the subject a composition comprising a selective myostatin inhibitor, wherein the selective myostatin inhibitor is an antibody that selectively binds to pro/latent myostatin, and wherein the selective myostatin inhibitor is administered in an amount to obtain a serum latent myostatin concentration of at least about 100 ng/mL or preferably at least about 250 ng/mL at steady state in the subject, wherein the subject has later-onset (optionally type 2) SMA and initiated an SMN upregulator therapy before the age of five. In some embodiments, the serum concentration is a steady state concentration. In some embodiments, the subject is non-ambulatory. In some embodiments, the subject is two years of age or older. According to the invention, the selective myostatin inhibitor is apitegromab. In some embodiments, the apitegromab is administered by intravenous infusion. In some embodiments, the apitegromab is administered at a dose of 20 mg/kg every four weeks or monthly.

In some embodiments, the present disclosure provides a composition comprising apitegromab for use in the treatment of SMA in a human subject, wherein the treatment comprises intravenous administration of the composition to the subject at a dose of greater than 2 mg/kg and up to 20 mg/kg of apitegromab every four weeks or monthly, wherein the subject is treated with an SMN upregulator therapy, wherein the subject initiates or initiated the SMN upregulator therapy at or after the age of five. In some embodiments, the SMA is type 2 SMA or type 3 SMA, wherein optionally, the subject has 2-4 copies of the SMN2 gene. In some embodiments, the subject has non-ambulatory type 3 SMA. In some embodiments, the subject is genetically identified as a carrier of a mutation in the SMN1 gene. In some embodiments, the subject has a baseline HFMSE score of at least 13, prior to or at the time of receiving a first dose of apitegromab. In some embodiments, the composition is administered to the subject in an amount sufficient to achieve sustained (steady state) target engagement of at least about 100 ng/mL or preferably at least about 250 ng/mL of serum latent myostatin. In some embodiments, the dose of apitegromab is 10 mg/kg or 20 mg/kg. In some embodiments, the subject is 5-21 years old. In some embodiments, the apitegromab is administered in a dose sufficient to achieve no or minimal reduction in an HFMSE score relative to a baseline score after at least 12 months or 24 months of treatment. In some embodiments, the apitegromab is administered in a dose sufficient to produce an at least one point increase in an HFMSE score relative to a baseline score, and preferably at least a three point increase, after 12 months or 24 months of treatment. In some embodiments, the dose of apitegromab is 20 mg/kg.

In some embodiments, the present disclosure provides a method of stabilizing disease progression in a human subject having non-ambulatory SMA, comprising administering to the subject a composition comprising apitegromab by intravenous infusion every four weeks or monthly for at least 12 months or 24 months, wherein the subject initiates or initiated the SMN upregulator therapy at or after the age of five. In some embodiments, the apitegromab is administered in a dose sufficient to achieve no or minimal reduction in an HFMSE score relative to a baseline score after at least 12 months of treatment, e.g., at least 24 months of treatment. In some embodiments, the apitegromab is administered in a dose of about 20 mg/kg.

In some embodiments, the present disclosure provides a composition comprising apitegromab for use in the treatment of SMA in a human subject, wherein the treatment comprises intravenous administration of the composition to the subject at a dose of greater than 2 mg/kg and up to 20 mg/kg of apitegromab every four weeks or monthly, wherein the subject has ambulatory SMA, and wherein optionally, the subject has not received an SMN upregulator therapy. In some embodiments, the subject has ambulatory type 3 SMA, wherein optionally, the subject has three or more copies of the SMN2 gene. In some embodiments, the subject is genetically identified as a carrier of a mutation in the SMN1 gene. In some embodiments, the composition is administered to the subject in an amount sufficient to achieve sustained (steady state) target engagement of at least about 100 ng/mL or preferably at least about 250 ng/mL of serum latent myostatin. In some embodiments, the dose of apitegromab is 10 mg/kg or 20 mg/kg. In some embodiments, the subject has a baseline RHS score of at least 26, prior to or at the time of receiving a first dose of apitegromab. In some embodiments, the apitegromab is administered in a dose sufficient to stabilize an RHS score relative to a baseline score after at least 12 months of treatment. In some embodiments, stabilizing the RHS score comprises less than a one point, two point, or three point decline in the RHS score relative to a baseline score. In some embodiments, the apitegromab is administered in a dose sufficient to stabilize an RULM score relative to a baseline score after at least 24 months of treatment, wherein stabilizing the RULM score comprises no more than one point decline from baseline. In some embodiments, the dose of apitegromab is 20 mg/kg. In some embodiments, the subject initiated SMN upregulator therapy at or after the age of five.

In some embodiments, the present disclosure provides a method of preventing or delaying loss of ambulation in a human subject having SMA, comprising administering a composition comprising apitegromab to the subject at a dose of greater than 2 mg/kg and up to 20 mg/kg of apitegromab every four weeks or monthly for at least 12 months or 24 months, wherein the subject has ambulatory SMA, and wherein optionally, the subject has not received an SMN upregulator therapy. In some embodiments, the patient has ambulatory type 3 SMA. In some embodiments, the progression is assessed by a delay in ambulatory-to-non-ambulatory transition following 12 months or 24 months of apitegromab treatment, relative to the natural history of an ambulatory SMA patient population. In some embodiments, the progression is assessed by a slower rate of or lesser degree of decline in a motor function score over a baseline following 12 months or 24 months of apitegromab treatment, relative to the natural history of an ambulatory SMA patient population. In some embodiments, the composition is formulated at a concentration of about 50 mg/mL apitegromab. In some embodiments, the SMN upregulator therapy comprises nusinersen, onasemnogene abeparvovec-xioi, and/or risdiplam. In some embodiments, the SMN upregulator therapy comprises nusinersen. In some embodiments, the SMN upregulator therapy comprises intrathecal administration.

In some embodiments, the present disclosure provides an SMN upregulator therapy and a muscle- directed therapy for use in the treatment of SMA in a human subject, wherein the treatment comprises initiation of the SMN upregulator therapy prior to the age of five, and wherein the muscle-directed therapy comprises intravenous administration of a composition comprising apitegromab every four weeks or monthly at a dose of greater than 2 mg/kg and up to 20 mg/kg, wherein optionally the dose of apitegromab is about 10 mg/kg. In some embodiments, the composition comprising apitegromab is formulated at a concentration of about 50 mg/mL. In some embodiments, the SMN upregulator therapy comprises nusinersen, onasemnogene abeparvovec-xioi, and/or risdiplam.

In some embodiments, the present disclosure provides a method for treating later-onset SMA in a human subject, the method comprising intravenously administering to the subject a composition comprising apitegromab at a therapeutic dose of greater than 2 and up to 20 mg/kg every four weeks or monthly, wherein the composition is formulated at a concentration of about 50 mg/mL. In some embodiments, the therapeutic dose is a dose sufficient to achieve a steady-state serum concentration of at least about 100 ng/mL or preferably at least about 250 ng/mL of latent myostatin. In some embodiments, the subject has 2-4 copies of the SMN2 gene. In some embodiments, the subject has type 2 SMA. In some embodiments, the subject has or had the ability to sit up at age 12 months and lacks or lacked ability to walk at age 18 months. In some embodiments, the subject has non-ambulatory type 3 SMA. In some embodiments, the subject lost ability to walk by 18 months of age. In some embodiments, the subject has 3-6 copies of the SMN2 gene. In some embodiments, the subject has ambulatory type 3 SMA. In some embodiments, the subject has retained ability to walk at age 18 months. In some embodiments, the subject undergoes an ambulatory-to-non-ambulatory transition after 18 months of age. In some embodiments, the subject is treated with an SMN-targeted therapy In some embodiments, the subject initiates or initiated the SMN-targeted therapy prior to age five. In some embodiments, the subject initiates or initiated the SMN-targeted therapy at age five or older. In some embodiments, the SMN-targeted therapy is an SMN1-directed therapy and/or an SMN2-directed therapy. In some embodiments, the SMN1-directed therapy is a gene therapy. In some embodiments, the gene therapy comprises a viral vector comprising an SMN1 gene replacement. In some embodiments, the SMN2-directed therapy is or comprises a splice-modifying agent. In some embodiments, the splice-modifying agent comprises a nucleic acid that binds an SMN2 exon, thereby modifying splicing of the exon. In some embodiments, the splice-modifying agent comprises a low molecular weight compound that binds an SMN2 exon, thereby modifying splicing of the exon. In some embodiments, the therapeutic dose is sufficient to cause enhanced motor function in the subject after 12 months or 24 months of the apitegromab therapy, wherein optionally the motor function is assessed by HFMSE, RHS, or RULM. In some embodiments, the enhanced motor function comprises an increase in a motor function score over a baseline, wherein optionally the increase is at least 1 point, 3 points, 5 points, or 10 points. In some embodiments, the increase in a motor function score is maintained over 24 months of apitegromab treatment. In some embodiments, the baseline comprises a motor function score assessed following an SMN-targeted therapy. In some embodiments, the baseline comprises a motor function score assessed in a subject who has not received an SMN-targeted therapy. In some embodiments, the therapeutic dose is sufficient to cause a delay in disease progression, relative to the natural history of a SMA patient population to which the subject is categorized.

In some embodiments, the present disclosure provides a composition comprising apitegromab for use in the treatment of later-onset SMA in a human subject, wherein the treatment comprises intravenous administration of the composition to the subject at a therapeutic dose of greater than 2 and up to 20 mg/kg every 4 weeks or monthly, wherein optionally the composition is formulated at a concentration of about 50 mg/mL, wherein further optionally, the therapeutic dose is sufficient to achieve a steady-state serum concentration of at least about 100 ng/mL or preferably at least 250 ng/mL of latent myostatin. In some embodiments, the subject has 2-4 copies of the SMN2 gene. In some embodiments, the subject has type 2 SMA. In some embodiments, the subject has or had the ability to sit up at age 12 months and lacks or lacked ability to walk at age 18 months. In some embodiments, the subject has non-ambulatory type 3 SMA. In some embodiments, the subject lost ability to walk by 18 months of age. In some embodiments, the subject has 3-6 copies of the SMN2 gene. In some embodiments, the subject has ambulatory type 3 SMA. In some embodiments, the subject has retained ability to walk at age 18 months. In some embodiments, the subject undergoes an ambulatory-to-non-ambulatory transition after 18 months of age. In some embodiments, the subject is treated with an SMN-targeted therapy. In some embodiments, the subject initiates or initiated the SMN-targeted therapy prior to age 5. In some embodiments, the subject initiates or initiated the SMN-targeted therapy at age 5 or older. In some embodiments, the SMN-targeted therapy is an SMN1-directed therapy and/or an SMN2-directed therapy. In some embodiments, the SMN1-directed therapy is a gene therapy. In some embodiments, the gene therapy comprises a viral vector comprising an SMN1 gene replacement. In some embodiments, the SMN2-directed therapy is or comprises a splice-modifying agent. In some embodiments, the splice-modifying agent comprises a nucleic acid that binds an SMN2 exon, thereby modifying splicing of the exon. In some embodiments, the splice-modifying agent comprises a low molecular weight compound that binds an SMN2 exon, thereby modifying splicing of the exon. In some embodiments, the therapeutic dose is sufficient to cause enhanced motor function in the subject after 12 months or 24 months of the apitegromab therapy, wherein optionally the motor function is assessed by HFMSE, RHS, or RULM. In some embodiments, the enhanced motor function comprises an increase in a motor function score over a baseline, wherein optionally the increase is at least 1 point, 3 points, 5 points, or 10 points. In some embodiments, the increase in a motor function score is maintained over 24 months of apitegromab treatment. In some embodiments, the baseline comprises a motor function score assessed following an SMN-targeted therapy. In some embodiments, the baseline comprises a motor function score assessed in a subject who has not received an SMN-targeted therapy. In some embodiments, the therapeutic dose is sufficient to cause a delay in disease progression, relative to the natural history of a SMA patient population to which the subject is categorized.

In some embodiments, the present disclosure provides a method for treating non-ambulatory or later-onset SMA in a human subject in need thereof, the method comprising intravenously administering to the subject apitegromab at a Q4W (i.e., every four weeks) or monthly dosage of greater than 2 mg/kg and up to 20 mg/kg. In some embodiments, the monthly dosage is 10 mg/kg administered via intravenous infusion. In some embodiments, the monthly dosage is 20 mg/kg administered via intravenous infusion.

In some embodiments, the present disclosure provides a method for treating later-onset SMA in a human subject in need thereof, the method comprising intravenously administering to the subject apitegromab in an amount sufficient to achieve a steady-state serum latent myostatin concentration of at least about 10, 20, 30, 40, 60, 80, 100, 200, 300 or 400 ng/mL

In some embodiments, the present disclosure provides a method for treating non-ambulatory or later-onset SMA in a human subject in need thereof, the method comprising intravenously administering to the subject apitegromab in an amount sufficient to achieve a steady-state serum latent myostatin concentration of at least about 250 ng/mL.

In some embodiments, the present disclosure provides a method for slowing progression of SMA in a human subject with ambulatory SMA, the method comprising intravenously administering to the subject apitegromab at a Q4W (i.e., every four weeks) or monthly dosage of greater than 2 mg/kg and up to 20 mg/kg. In some embodiments, the subject has ambulatory type 3 SMA. In some embodiments, the progression is assessed by a delay in ambulatory-to-non-ambulatory transition following 12 months or 24 months of apitegromab treatment, relative to the natural history of an ambulatory SMA patient population. In some embodiments, the progression is assessed by a slower rate of or lesser degree of decline in a motor function score over a baseline following 12 months or 24 months of apitegromab treatment, relative to the natural history of an ambulatory SMA patient population.

In some embodiments the present disclosure provides a method for increasing HFMSE scores and maintaining the increase for at least 24 months in a human subject with SMA, comprising treating the subject with apitegromab once every four weeks or once monthly for at least 24 months with a dosage of greater than 2 mg/kg and up to 20 mg/kg.

In some embodiments the present disclosure provides a method for increasing the RULM scores in a subject having SMA, wherein the RULM scores are increased as compared to the RULM scores observed at 12 months or 24 months of treatment with apitegromab by at least one point, at least two points, or at least three points, wherein the subject is treated with apitegromab at a dosage of greater than 2 mg/kg and up to 20 mg/kg for at least 24 months by at least one point, at least two points or at least three points.

In some embodiments the present disclosure provides a method for improving motor skills of a human subject having SMA, comprising treating the subject with apitegromab at a dosage of greater than 2 mg/kg and up to 20 mg/kg for at least 24 months to increase both HFMSE and RULM scores in the human subject. In some embodiments, the HFMSE and/or RULM scores are further increased at 24 months as compared to at 12 months following the start of apitegromab treatment. In some embodiments, the increase in HFMSE and/or RULM scores at 12 months are maintained at 24 months. In some embodiments, the HFMSE and RULM scores are correlated at 24 months following the start of apitegromab treatment.

In some embodiments, the present disclosure provides a method of predicting therapeutic efficacy in a subject having SMA, the method comprising a) administering to the subject apitegromab at a dose greater than 2 mg/kg and up to 20 mg/kg once every four weeks or once monthly, and b) determining a change in HFMSE score at 12 months after starting apitegromab treatment as compared to a baseline HFMSE score, wherein an increase in HFMSE score as compared to baseline is predictive of therapeutic efficacy at 24 months following the start of treatment. In some embodiments, the present disclosure provides use of a subject's HFMSE score at 12 months after starting apitegromab treatment for predicting therapeutic efficacy in a subject having SMA, wherein the use comprises a) administering to the subject apitegromab at a dose greater than 2 mg/kg and up to 20 mg/kg once every four weeks or once monthly, and b) determining a change in HFMSE score at 12 months after starting apitegromab treatment as compared to a baseline HFMSE score, wherein an increase in HFMSE score as compared to baseline is predictive of therapeutic efficacy at 24 months following the start of treatment. In some embodiments, the increase in HFMSE score at 12 months as compared to baseline is predictive of an increase in RULM score at 24 months as compared to baseline. In some embodiments, the subject has non-ambulatory SMA.

In some embodiments, the present disclosure provides a method of determining therapeutic efficacy in a subject having SMA, the method comprising a) administering to the subject apitegromab at a dose greater than 2 mg/kg and up to 20 mg/kg once every four weeks or once monthly; b) determining a change in HFMSE score at 24 months after starting apitegromab treatment as compared to a baseline HFMSE score; and c) determining a change in RULM score at 24 months after starting apitegromab treatment as compared to a baseline RULM score; wherein an increase in HFMSE score from baseline and an increase in RULM score from baseline is indicative of therapeutic efficacy. In some embodiments, the present disclosure provides use of a subject's HFMSE and RULM scores at 24 months after starting apitegromab treatment for determining therapeutic efficacy in a subject having SMA, wherein the use comprises a) administering to the subject apitegromab at a dose greater than 2 mg/kg and up to 20 mg/kg once every four weeks or once monthly; b) determining a change in HFMSE score at 24 months after starting apitegromab treatment as compared to a baseline HFMSE score; and c) determining a change in RULM score at 24 months after starting apitegromab treatment as compared to a baseline RULM score; wherein an increase in HFMSE score from baseline and an increase in RULM score from baseline is indicative of therapeutic efficacy. In some embodiments, the subject has non-ambulatory SMA.

In some embodiments, the present disclosure provides a method of increasing an RULM score at 24 months following start of apitegromab treatment in a subject having SMA, wherein the subject is treated with an effective amount of apitegromab once every four weeks or once monthly, wherein the effective amount is sufficient to increase an HFMSE score at 12 months or 24 months after starting apitegromab treatment as compared to a baseline HFMSE score. In some embodiments, the present disclosure provides use of apitegromab for increasing an RULM score at 24 months following start of apitegromab treatment in a subject having SMA, wherein the subject is treated with an effective amount of apitegromab once every four weeks or once monthly, wherein the effective amount is sufficient to increase an HFMSE score at 12 months or 24 months after starting apitegromab treatment as compared to a baseline HFMSE score. In some embodiments, the effective amount of apitegromab is greater than 2 mg/kg and up to 20 mg/kg, e.g., 2 mg/kg, 5 mg/kg, 10 mg/kg, or 15 mg/kg. In some embodiments, the effective amount of apitegromab is an amount sufficient to increase HFMSE score by at least 2 points at 12 months or 24 months after starting apitegromab treatment as compared to a baseline HFMSE score. In some embodiments, the subject has non-ambulatory SMA.

In some embodiments, the present disclosure provides a method of predicting an increase in RULM score at 24 months after starting apitegromab treatment in a subject having SMA, the method comprising measuring the subject's change in HFMSE score at 12 months after starting apitegromab treatment as compared to a baseline HFMSE score, wherein an increase of at least two points in the subject's HFMSE score at 12 months predicts an increase in the subject's RULM score at 24 months as compared to a RULM baseline score. In some embodiments, the present disclosure provides use of a subject's HFMSE score at 12 months after starting apitegromab treatment for predicting an increase in RULM score at 24 months after starting apitegromab treatment, wherein the use comprises measuring the subject's change in HFMSE score at 12 months after starting apitegromab treatment as compared to a baseline HFMSE score, wherein an increase of at least two points in the subject's HFMSE score at 12 months predicts an increase in the subject's RULM score at 24 months as compared to a RULM baseline score, wherein the subject has SMA. In some embodiments, the subject is administered apitegromab at a dose of greater than 2 mg/kg and up to 20 mg/kg, e.g., 2 mg/kg, 5 mg/kg, 10 mg/kg, or 15 mg/kg, and at a frequency of once every month or once every four weeks. In some embodiments, the subject has non-ambulatory SMA.

In some embodiments, the present disclosure provides a method for maintaining motor skills in a subject having SMA, the method comprising administering to the subject apitegromab once every four weeks or once monthly for at least 24 months, wherein the apitegromab is administered at a dose greater than 2 mg/kg and up to 20 mg/kg, and wherein the motor function of the subject is not decreased at 24 months as compared to at 12 months following start of administration. In some embodiments, the subject's motor skills are assessed by HFMSE or RULM scores. In some embodiments, the subject's RULM score is increased by at least one point, at least two points, or at least three points at 12 months following the start of apitegromab treatment. In some embodiments, the subject's RULM score is further increased at 24 months as compared to 12 months following the start of apitegromab treatment.

Having now described the invention in detail, the same will be more clearly understood by reference to the following examples, which are included for purposes of illustration only and are not intended to be limiting.

Previous publications have documented preclinical studies of apitegromab in animals (WO 2022/093724) and a phase 1 clinical trial in healthy adult subjects (Barrett et al. (2021) Adv. Ther. 38:3203).

### EXAMPLES

### Example 1. Twelve-Month Treatment Study of Apitegromab in Subjects with Later-onset SMA

### Summary

The primary objectives of this Phase 2 trial known as TOPAZ (ClinicalTrials.gov identifier NCT03921528) were to (1) assess the safety and tolerability of apitegromab in subjects with later-onset type 2 and type 3 spinal muscular atrophy (SMA) and (2) to assess the efficacy of apitegromab by measuring changes in motor function. The results obtained in the first year of the study are described in WO 2022/093724.

Subjects received intravenous (IV) apitegromab infusions every four weeks for 12 months in one of three groups. Group 1 assessed low (2 mg/kg) and high (20 mg/kg) dose apitegromab administered in a blinded manner to younger, earlier-treated non-ambulatory participants two years of age or older (≥2 years old) who were receiving nusinersen treatment initiated prior to age five. Group 2 assessed apitegromab at a dose of 20 mg/kg in non-ambulatory subjects 5-21 years old who were receiving nusinersen treatment initiated at five years of age or older. Group 3 assessed apitegromab at a dose of 20 mg/kg in ambulatory subjects 5-21 years of age who were receiving nusinersen treatment initiated at five years of age or older, or who had not received an SMN-upregulator treatment for at least six months prior to screening.

All three groups received intravenous infusions of apitegromab at monthly intervals. Apitegromab solution was diluted in normal saline and infused over two hours (±10-minute) via peripheral IV line. Subjects receiving nusinersen were required to receive their maintenance dose ≥24 hours after receiving apitegromab or ≥14 days prior to a scheduled apitegromab dose. Apitegromab was administered every 28 days (±7 days after Day 14) until Day 364. Subjects completing the 12-month treatment period were given an opportunity to enroll in an extension period of the trial (described in Example 2) or, alternatively, to conclude participation in the trial with a 12-week safety follow-up after their last dose. At the time of the start of the study, only nusinersen was licensed for treatment of SMA.

The primary efficacy measure was the mean change from baseline using two SMA-specific motor scales. The Hammersmith Functional Motor Scale Expanded (HFMSE) was used for the non-ambulatory groups and the Revised Hammersmith Scale (RHS) was used for the ambulatory group

Apitegromab was found to be safe and well tolerated by the trial subjects. Also, apitegromab administered to SMA subjects either as a monotherapy, or administered to subjects on an SMN-targeted therapy, proved efficacious in improving motor function.

### Trial Groups and Demographics

Overall study inclusion and exclusion criteria, and those specific to the three treatment groups, are shown in **Table 11.**

**Table 11. Study inclusion and exclusion criteria**

| | **Group 1** | **Group 2** | **Group 3** |
|---|---|---|---|
| | **Non-ambulatory Subjects ≥2 Years Old** | **Non-ambulatory Subjects 5-21 Years Old** | **Ambulatory Subjects 5-21 Years Old** |
| Design | • Open label, but blinded randomization, 1:1, to 2 mg/kg or 20 mg/kg apitegromab every 4 weeks | • Open-label, single-arm | • Open-label, single-arm |
| | | • IV 20 mg/kg apitegromab every 4 weeks | • Equal arms, apitegromab alone or with nusinersen |
| | | • 12-month treatment period | • IV 20 mg/kg apitegromab every 4 Weeks |
| | • 12-month treatment period | | • 12-month treatment period |
| Subjects | • Type 2 SMA | • Type 2 or non-ambulatory Type 3 SMA | • Ambulatory Type 3 SMA |
| | • Concomitant nusinersen initiated prior to age 5 Years | | • 5 to 21 years old (n=23) |
| | | • Concomitant nusinersen started ≥5 years old | • RHS scores ≤63 |
| | • ≥2 years old (n=20) | • 5 to 21 years old (n=15) | |
| | • HFMSE scores ≥10 | • HFMSE scores ≥10 | |
| Primary Objectives | • Safety | • Safety | • Safety |
| | • Mean change from baseline in HFMSE | • Mean change from baseline in HFMSE | • Mean change in baseline RHS scores |

*Group 1*: Younger, earlier treated nonambulatory (Type 2) SMA subjects on nusinersen, i.e., who received the same nusinersen therapy for at least six months prior to screening and were anticipated to remain on that therapy throughout the duration of the study, were randomized 1:1 into a low apitegromab dose arm (2 mg/kg/dose) or a high apitegromab dose arm (20 mg/kg/dose). Age eligibility was defined by the combination of age greater than or equal to two years and the initiation of nusinersen before five years of age. Enrollment further required a minimum score of ≥10 out of a possible total of 66 on the non-ambulatory HFMSE scale.

*Group 2:* Older, later-treated non-ambulatory type 2 and type 3 SMA subjects ages 5-21 years on nusinersen all received the higher 20 mg/kg/dose regimen in an unblinded fashion. Eligibility was defined by having started nusinersen after the age of five years. As with Group 1, enrollment further required a minimum score of ≥10 out of a possible total of 66 on the non-ambulatory HFMSE scale.

*Group 3:* Ambulatory subjects ages 5 to 21 years old were recruited into two equal arms; (1) subjects not on nusinersen; and (2) subjects who initiated nusinersen treatment after five years of age. All subjects received apitegromab 20 mg/kg. The primary efficacy outcome measure for this group was the change in Revised Hammersmith Scale (RHS) score. Eligibility was restricted to subjects with an initial score of ≤63 out of a total possible score of 69.

A total of 58 participants were enrolled in Groups 1-3 and their demographics and baseline characteristics are shown in **Table 12.** As this study was conducted during the COVID-19 era of access restrictions, one subject in Group 2 and three subjects in Group 3 each missed three doses of apitegromab during the 12-month treatment period and were thus excluded from the primary analysis per prespecified protocol criteria. The remaining 54 participants comprise the "intent to treat" ("ITT") population. One subject among the older (5-21 years) non-ambulatory subjects received concomitant treatment with an acetylcholinesterase inhibitor before and during the study and was excluded from the per-protocol analysis due to this protocol violation.

**Table 12. Demographics and baseline characteristics of participants**

| | | **Group 1** | | **Group 2** | **Group 3** | |
|---|---|---|---|---|---|---|
| | | **Non-ambulatory Subjects >2 Years Old, HFMSE scores ≥10** | | **Non-ambulatory Subjects 5-21 Years Old, HFMSE** Scores ≥10 | **Ambulatory Subjects 5-21 Years Old, RHS Scores ≤63** | |
| | | Apitegromab 2 mg/kg + Nusinersen | Apitegromab 20 mg/kg + Nusinersen | Apitegromab 20 mg/kg + Nusinersen | Apitegromab 20 mg/kg Monotherapy | Apitegromab 20 mg/kg + Nusinersen |
| **n (Baseline Population)** | | 10 | 10 | 15 | 11 | 12 |
| **Mean Age, years (min, max)** | | 4.1 (2, 6) | 3.8 (2, 6) | 11.7 (8, 19) | 12.1 (7, 19) | 13.1 (7, 21) |
| **Mean Age at Diagnosis, years (min, max)** | | 1.2(1, 2) | 1.2 (1,3) | 3.1 (1, 16) | 5.9 (2, 15) | 4.5 (2, 15) |
| **Mean Age at Symptom Onset, years (min, max)** | | 0.9 (0.5, 1.2) | 1.0 (0.5, 3.5) | 1.4 (0.5, 2) | 3.7 (0.8, 11) | 3.0 (0.7, 14) |
| **Female (%)** | | 30 | 50 | 53 | 73 | 58 |
| **SMN2 Gene Copies, n (%)** | | | | | | |
| | **2** | 1 (10) | 1 (10) | 0 | 1 (9) | 0 |
| | **3** | 8 (80) | 8 (80) | 11 (73) | 4 (36) | 9 (75) |
| | **4** | 1 (10) | 0 | 2 (13) | 4 (36) | 1 (8) |
| **Mean Nusinersen Maintenance Doses at Baseline, n (min, max)** | | 5.5 (2, 9) | 5.4 (3, 8) | 5.1 (2, 9) | N/A | 5.6 (2, 8) |
| **Discontinued** | | 0 | 0 | 0 | 0 | 1 |
| **Mean Baseline RHS Score (min, max)** | | N/A | N/A | N/A | 47.6 (26, 63) | 51.3 (43, 62) |
| **Mean Baseline HFMSE Score (min, max)** | | 26.1 (12, 44) | 23.5 (14, 42) | 22.7 (13, 39) | N/A | N/A |
| **Mean Baseline RULM Score (min, max)** | | 25.0 (18, 34) | 22.6 (16, 32) | 26.6 (19, 34) | -- | -- |

Subjects receiving nusinersen had received a mean of nine doses of nusinersen prior to enrollment, i.e., a two-month loading sequence of four doses, followed by five doses at the recommended four-month interval, for a mean duration of 25.9 months at the time of trial enrollment. Prior to enrollment, the mean duration of nusinersen treatment in the younger, earlier treated non-ambulatory Group 1, the older, later treated non-ambulatory Group 2, and those in the ambulatory Group 3 who were on nusinersen, was 26.0, 25.2, and 26.6 months, respectively, as estimated from the time of the dose prior to the trial screening.

### Safety

Safety reviews were prepared approximately every 12 weeks by an independent contract research organization for review by a safety surveillance team that did not review efficacy data. A physical examination was performed every 28 days prior to each drug infusion. A 12-lead electrocardiogram (ECG) was obtained after the first 28 days and every 56 days thereafter. Comprehensive clinical laboratory assessments were made every two weeks for six weeks and every 28 days thereafter. These included clinical hematology, serum chemistry and urinalysis. All safety laboratory analyses were performed at a central laboratory. Vital signs were assessed every 15 minutes (±5 minutes) during the infusion and one and two hours (±15 minutes) after the infusion.

Subjects or their parent or guardian were queried at each clinic visit in an open-ended manner that encouraged reporting all possible between-visit adverse events (AEs). Reported AEs were monitored until resolution or stabilization of the event. Clinical and laboratory AEs were graded using the National Cancer Institute Common Terminology Criteria for Adverse Events, Version 5.0. Safety assessments continued for 12 weeks after the last dose of apitegromab. The results are shown in **Table 13.**

The serious treatment emergent adverse events (TEAEs), i.e., adverse events that were temporally related to the trial treatment but not necessarily caused by the trial treatment, were determined to be unrelated to treatment. "Preferred Term" refers to the terminology of the Medical Dictionary for Regulatory Activities. Therefore, apitegromab displayed an acceptable safety profile after 12 months of therapy at doses up to at least 20 mg/kg. The incidence and severity of AEs were consistent with the underlying patient population and nusinersen therapy. The five most frequently reported treatment-emergent AEs were headache (24%), pyrexia (22%), upper respiratory tract infection (22%), cough (22%), and nasopharyngitis (21%). Most reported AEs were compatible with the underlying disorder and nusinersen therapy, and all serious events were unrelated to apitegromab.

**Table 13. Safety assessment results**

| **Treatment-Emergent Adverse Events, n (%)** | **Apitegromab 2 mg/kg (n=10)** | | | **Apitegromab 20 mg/kg (n=48)** | | | **Total (*N*=58)** | | |
|---|---|---|---|---|---|---|---|---|---|
| Any TEAE | 9 (90.0) | | | 44 (91.7) | | | 53 (91.4) | | |
| Serious TEAEs | 1 (10.0) | | | 4 (8.3) | | | 5 (8.6) | | |
| TEAEs Leading to Discontinuation | 0 | | | 1 (2.1) | | | 1 (1.7) | | |
| ≥Grade 3 TEAEs | 0 | | | 3 (6.2) | | | 3 (5.2) | | |

| Preferred Term, n (%) | **Grade 1** | **Grade 2** | **Total** | **Grade 1** | **Grade 2** | **Total** | **Grade 1** | **Grade 2** | **Total** |
|---|---|---|---|---|---|---|---|---|---|
| Headache | 2 (20.0) | 0 | 2 (20.0) | 11 (22.9) | 1 (2.1) | 12 (25.0) | 13 (22.4) | 1 (1.7) | 14 (24.1) |
| Upper Respiratory Tract Infection | 2 (20.0) | 1 (10.0) | 3 (30.0) | 7 (14.6) | 3 (6.3) | 10 (20.8) | 9 (15.5) | 4 (6.9) | 13 (22.4) |
| Pyrexia | 1 (10.0) | 2 (20.0) | 3 (30.0) | 6 (12.5) | 4 (8.3) | 10 (20.8) | 7 (12.1) | 6 (10.3) | 13 (22.4) |
| Cough | 0 | 3 (30.0) | 3 (30.0) | 8 (16.7) | 2 (4.2) | 10 (20.8) | 8 (13.8) | 5 (8.6) | 13 (22.4) |
| Nasopharyngitis | 1 (10.0) | 2 (20.0) | 3 (30.0) | 8 (16.7) | 1 (2.1) | 9 (18.8) | 9 (15.5) | 3 (5.2) | 12 (20.7) |

### Pharmacokinetics, Pharmacodynamics, and Immunogenicity

For pharmacokinetic (PK), pharmacodynamic (PD), and immunogenicity assessments, blood samples for determining apitegromab concentrations, serum latent myostatin concentrations and anti-apitegromab antibodies, respectively, were obtained every 28 days prior to each drug infusion *via* peripheral venipuncture.

When data from the current study were combined with data from the Phase 1 single ascending dose study (Barrett et al. (2021) Adv. Ther. 38:3203), there was no difference in the pharmacokinetic profiles between SMA subjects and healthy volunteers, after accounting for difference in age and weight. Stability of mean apitegromab levels in all three groups, independent of dose, confirms appropriateness of the four-week dosing interval. In these trials, apitegromab demonstrated a dose proportional PK profile with a linear PK profile over the dose range of 2-30 mg/kg. The 2 mg/kg and the 20 mg/kg doses demonstrated dose-dependent and dose-proportional increases in apitegromab exposure with the 20 mg/kg dose achieving approximately ten-fold increases in serum concentration compared to the 2 mg/kg dose. Using a two-compartment model, apitegromab demonstrated linear elimination from the central compartment. The volume of distribution of apitegromab was 4.30 liters and its clearance was 0.118 liters per day. The mean terminal half-life was 23-40 days.

Both the 2 mg/kg and the 20 mg/kg doses showed high target engagement, as measured by latent myostatin; levels after treatment showed a greater than 100-fold increase from baseline levels and the 20 mg/kg dose had a higher level of target engagement than the 2 mg/kg dose. The difference in response to high- and low-dose apitegromab in Group 1 was mirrored by a graded elevation of latent serum myostatin level, which is a surrogate pharmacodynamic measure of the antibody engaging with its target antigen. Subjects on both the 2 mg/kg and 20 mg/kg doses quickly reached a dose-proportional high steady level of measured myostatin. The elevated latent myostatin levels of the higher dose were similar across all three groups of different ages and functional abilities, indicating linearity of dose to weight and age.

Biochemical measures of latent myostatin as a surrogate for target engagement demonstrated rapid achievement of steady state at both concentrations, but the difference in the levels suggests that the low dose had not fully saturated the target, thus did not achieve an optimal treatment effect. As shown below, however, the magnitude of efficacy increased with the magnitude of target engagement as the dose of apitegromab increased (Day et al. (2022) BMC Pediatrics 22:632).

The robust and consistent results of the PK and PD assessments and the favorable safety profile of apitegromab suggest minimal immunogenicity or hypersensitivity reactions, which is in agreement with the results of the Phase 1 apitegromab trial (Barrett et al. (2021) Adv. Ther. 38:3203).

### Efficacy

Motor function outcomes were assessed at screening, baseline (Day 1) and study days 56, 112, 168, 224, 280 and 364. The efficacy endpoints for each group are summarized in **Table 14.** Each item on the HFMSE permits a score of 0, 1, or 2, thus an increase in the total HFMSE scale score of three points represents measurable improvements in two or three scored motor item skills, and a six-point increase in total HFMSE represents improvements in three to six motor skills.

**Table 14. Motor function assessment at 12-months.**

| | | | | | | | |
|---|---|---|---|---|---|---|---|
| | | | | | | | |

| | **Group 1** | | | **Group 2** | **Group 3** | | |
|---|---|---|---|---|---|---|---|
| | **Non-ambulatory Subjects >2 Years Old (HFMSE)** | | | **Non-ambulatory Subjects 5-21 Years Old (HFMSE)** | **Ambulatory Subjects 5-21 Years Old (RHS)** | | |
| | Apitegromab 2 mg/kg + Nusinersen | Apitegromab 20 mg/kg + Nusinersen | Pooled | Apitegromab 20 mg/kg + Nusinersen | Apitegromab 20 mg/kg Monotherapy | Apitegromab 20 mg/kg + Nusinersen | Pooled |
| n (Intent-to-Treat Population) | 8 | 9^{a} | 17 | 14 | 11 | 12 | 23 |
| Primary Efficacy Endpoint | Mean change in baseline HFMSE scores | | | Mean change in baseline HFMSE scores | Mean change in baseline RHS scores | | |
| Mean Change in Baseline Score (95% Cl) | 5.3 (-1.5, 12.2) | 7.1 (1.8, 12.5) | 6.2 (2.2, 10.1) | 0.6 (-1.4, 2.7) | -0.4 (-3.9, 3.1) | -0.3 (-2.0, 1.4) | -0.3 (-2.1, 1.4) |
| Subjects Achieving ≥1-Point Increase, n (%) | 7 (77.8) | 7 (87.5) | 14 (82) | 9 (64.3) | 4 (36.0) | 5 (42) | 9 (39) |
| Subjects Achieving ≥3-Point Increase, n (%) | 5 (55.6) | 5 (62.5) | 10 (59) | 4 (28.6) | 3 (27) | 2 (17) | 5 (22) |
| Subjects Achieving ≥5-Point Increase, n (%) | 5 (55.6) | 5 (62.5) | 10 (59) | 2 (14.3) | 1 (9) | 0 | 1 (4.4) |

Group 1 subjects receiving apitegromab at 20 mg/kg achieved a mean 7.1-point increase in HFMSE score; 62.5% demonstrated an increase of ≥5 points. Three subjects (35%) achieved an increase of >10 points. Group 1 subjects receiving apitegromab 2 mg/kg achieved a mean 5.3-point increase in HFMSE score; 56% demonstrated an increase of ≥5 points. When the high- and low-dose arms were combined, the RULM increased; 31% (5/16) of the subjects achieved a ≥2-point increase over baseline. Subjects in this younger, earlier treated Group 1 also displayed gains in WHO motor milestones.

This difference in clinical response to high and low dose apitegromab was mirrored in the pharmacodynamic response, i.e., the graded elevation of latent serum myostatin levels. Subjects on both the 2 mg/kg and the 20 mg/kg doses quickly reached a dose-proportional high steady level of latent myostatin.

The increase in HFMSE scores in the Group 1 subjects increased over the 12-month treatment period and was dose dependent. Both the 2 mg/kg and the 20 mg/kg doses resulted in early improvements, i.e., as early as two months.

The older, non-ambulatory Group 2, who initiated SMN-targeted therapy later, showed more modest changes, with mean increase in HFMSE scores of 0.6-points (n=14) **(Table 14).** The majority of subjects either stabilized or increased their baseline HFMSE scores during the 12 months of treatment with apitegromab. The expected clinical course in this study population treated with nusinersen alone would be one of either motor function decline or stability (Chiriboga et al. (2020) Neurology 94:166; Darras et al. (2019) Neurology 92(21):e2492-e2506; Mercuri et al. (2019) Neurology 93:e1241-e1247). However, in the present study, the number of subjects achieving three- or five- point increases in HFMSE scores were four (28.6%) and two (14.3%), respectively. Increases on the RULM scale were similar to that observed in the younger, earlier treated group, with 36% (n=5) of subjects achieving a clinically significant ≥2-point improvement from baseline (Coratti et al. (2020) Neuromuscul Disord 30:756).

In a *post hoc* analysis, subjects in this non-ambulatory Group 2 were considered as either pre-or post-pubertal, with a presumption of puberty around 12 years of age. Those 5-11 years old had a greater mean HFMSE increase from baseline of 1.6 points compared to subjects 12-21 years old. A higher percentage of this pre-pubertal subpopulation also achieved improved HFMSE scores; 50% experienced a >3-point increase in HFMSE. One subject within this group also gained two new WHO motor milestones.

Group 3 subjects receiving apitegromab manifested improvement in a subset of subjects, as ≥3-point (27%) and ≥5-point (9%) improvements in RHS scores. Overall, 17% of the subjects who were or were not receiving chronic nusinersen in Group 3 achieved ≥3-point improvements in RHS scale.

The ambulatory Group 3 was recruited into two equal arms, those receiving nusinersen and those not receiving any SMN- targeted therapy. All received apitegromab 20 mg/kg (n=23). The observed mean change in RHS score was -0.3 points from baseline. The majority of these subjects showed either stabilization or improvement of greater than one point in RHS score as a result of treatment. This change in or stabilization of motor function compares favorably with the declines in motor function over time expected for this study population (Coratti et al (2020) Neuromuscul Disord 30:756). A sizeable percentage of subjects receiving apitegromab as monotherapy (n=11) demonstrated positive responses; 27% achieved at least a three-point increase and 9% achieved at least a five-point increase in the RHS score. Among subjects receiving apitegromab and nusinersen, two (17%), subjects achieved a three-point increase in RHS.

When both non-ambulatory groups were combined, no correlation was observed between the duration of prior nusinersen therapy and the 12-month HFMSE change from baseline. This is further evidence that the improvements in motor function demonstrated in this trial are at least in part due to apitegromab and not merely due to the SMN-targeted therapy. These subjects were receiving an SMN-targeted therapy for which the observed benefit is known to be most robust in the youngest and least impaired, with age the most important factor in the initial rate of improvement and overall treatment response (Kong et al. (2021) Sci Transl Med 13:eabb6871). Subjects on SMN-targeted therapy experience that the rate of HFMSE improvement slows as a function of treatment duration as the children develop more normally because further neurodegeneration has been blocked or slowed. Subjects in the current trial had received nusinersen for a mean of two or more years, which is well into the time period in which only modest gains are associated with nusinersen (Mercuri et al. (2018) N Engl J Med 378: 625; Finkel et al. (2017) N Engl J Med 377:1723). Against this background of SMN-targeted therapy, the mean 7.1-point improvement in HFMSE scores seen in the younger, earlier treated non-ambulatory subjects receiving high dose apitegromab is superior to that seen in longer-term nusinersen follow-up studies.

Nonambulatory subjects 5 to 21 years old (n=14) achieved improvements in baseline HFMSE scores by ≥3 points (n=4, 29%). Ambulatory subjects 5-21 years old (n=23) achieved improvements in baseline RHS scores by ≥3 points (n=5, 22%). As stated above, any increase or stabilization of an HFMSE score is an improvement over comparable natural history cohorts, who have demonstrated progressive decreases. Slowing of disease progression and stabilization of disease course are considered clinically meaningful by patients with type 2 and type 3 SMA and their caregivers (Mercuri et al. (2016) Neuromuscul Disord 26:126-131; Kaufmann et al. (2012) Neurology 79:1889-1897). A 3-point change in HFMSE score represents a clinically meaningful change involving two or three skills. A 6-point improvement reflects improvements in three to six skills. Improvements in motor skills include, e.g., trunk control while rolling or sitting and transitioning from lying to sitting or improved consolidation of functions allowing for better maneuverability, and transitioning and integration of proximal and distal functions that enable more advanced use of their current motor skills, e.g., sitting without support or crawling (Roualt et al. (2017) Neuromusul Disord 27:428-438).

As stated above, there was no relationship between the duration of nusinersen treatment and the response to apitegromab treatment. Additionally, changes in motor function scores were comparable for subjects in the ambulatory group whether on apitegromab monotherapy or apitegromab with nusinersen, providing even further evidence of the efficacy of apitegromab treatment.

Apitegromab treatment in combination with nusinersen demonstrated the benefits of apitegromab beyond the effects of nusinersen. No correlation was observed between the duration of prior nusinersen therapy and the improvements in HFMSE scores over 12 months. Also, both dosage groups, 2 mg/kg and 20 mg/kg, manifested an early benefit in motor scores. Additionally, the greater latency of the low dose cohort further supports apitegromab attributable effects.

### Statistical Analysis

Safety and efficacy endpoints were analyzed at 12 months by groups, dose arms, group-dose combinations, and whether SMN-targeted therapy was included in the treatment regimen. Vital signs and laboratory outcomes were evaluated using paired t-tests within treatment groups, unpaired t-tests between Group 1 dose arms and 2-sided 95% confidence intervals (Cls) were computed for paired and unpaired differences.

The incidence of TEAEs was assessed using an exact binomial test for single groups and Fisher's Exact test to compare the Group 1 low and high dose subjects. The primary efficacy endpoints were analyzed using paired t-tests within groups. Continuous endpoints were analyzed using paired t-tests within groups and the corresponding two-sided 95% confidence intervals (Cis) were computed. Unpaired t-tests for between-group analysis in Group 1 were performed and two-sided 95% CI of difference were computed. The proportion and the 95% CI based on Wilson's method were calculated for binary endpoints. The continuous endpoints were further explored by longitudinal models addressing population and by subject variation with time, dose, and dose-time interaction as covariates with age, SMN-targeted treatment duration, and screening HFMSE scores added as potential covariates for Group 1 outcomes.

Prespecified interim analyses to evaluate the initial drug exposure (PK) and target engagement (PD) were conducted. For Groups 2 and 3, the analysis was conducted after approximately four subjects in each group had received at least two doses of apitegromab. For Group 1, the analysis was conducted after approximately six subjects had received at least two doses of apitegromab. Based on the interim analysis results and available safety data, group-specific dose levels could be changed but the four-week dosing frequency remained constant.

### Example 2. Twenty-Four-Month Treatment Study of Apitegromab in Subjects with Later-onset SMA

### Summary

Non-ambulatory types 2 and 3 SMA subjects between the ages of 2 and 21 who were treated with apitegromab and an approved SMN-targeted therapy for 24 months demonstrated sustained and durable improvements in motor function as measured by mean change from baseline in HFMSE and RULM scores. No new safety risks were identified after 24 months of treatment. The pharmacokinetic and pharmacodynamic data were consistent with a clinically observed dose response through 24 months and confirmed apitegromab target engagement. The 20 mg/kg (high) dose outperformed the 2 mg/kg (low) dose, however, when the subjects receiving the low dose were switched to the high dose, the gap in motor function performance narrowed. Measurements of activities of daily living, fatigue and endurance in this study provided the first evidence of sustained improvement in the quality of life of SMA patients.

### Open Label Extension Demographics

Fifty seven of the 58 subjects from the Phase 2 trial described in Example 1 elected to continue treatment in an open label extension period that lengthened the 12-month treatment period to 24-months. 55 subjects completed the 24-month extension period, all of whom received apitegromab at a dose of 20 mg/kg every four weeks in an open-label manner. The demographics and baseline characteristics of these subjects is shown in **Table 15.**

**Table 15. Demographics and baseline characteristics of extension study subjects**

| | **Non-ambulatory** | | | **Ambulatory** | |
|---|---|---|---|---|---|
| | **Age 5-21** | **Age ≥2** | | **Age 5-21** | |
| | **Type 2/3** | **Type 2** | | **Type 3** | |
| | **20 mg/kg + nusinersen** | **2 mg/kg + nusinersen** | **20 mg/kg + nusinersen** | **20 mg/kg monotherapy** | **20 mg/kg + nusinersen** |
| **N (dosed)** | 15 | 10 | 10 | 11 | 12 |
| **Mean age at screening (min, max)** | 11.7 (8, 19) | 4.1 (2, 6) | 3.8 (2, 6) | 12.1 (7, 19) | 13.1 (7, 21) |
| **Nusinersen doses at baseline** | 5.1 (2, 9) | 5.5 (2, 9) | 5.4 (3, 8) | NA | 5.6 (2, 8) |
| **Discontinuations** | 1 | 0 | 0 | 0 | 2 |
| **Scoliosis (n, %)** | 11, 73.3 | 4, 40 | 3, 30 | 7, 63.6 | 4, 33.3 |
| **Contractures (n, %)** | 13, 86.7 | 8, 80 | 4, 40 | 6, 54.5 | 7, 58.3 |
| **Mean RHS score (min, max)** | NA | NA | NA | 47.6 (26, 63) | 51.3 (43, 62) |
| **Mean HFMSE score (min, max)** | 22.7 (13, 39) | 26.1 (12,44) | 23.5 (14, 42) | NA | NA |

Non-ambulatory subjects were considered in two cohorts: (1) type 2/3, age 5-21 who started on nusinersen therapy on or after age 5; and (2) type 2, age ≥2 who started on nusinersen therapy before age 5. The ambulatory cohort comprised type 3 subjects 5-21 years of age, some of whom were treated with apitegromab as monotherapy and some of whom were treated with apitegromab and nusinersen, as shown in **Table 15.**

### Safety

No new safety risks were identified over the 24 months of treatment, as shown in **Table 16.** The incidence and types of treatment emergent adverse events (TEAEs) were consistent with the underlying disease and SMN-targeted therapies. No deaths or unexpected serious adverse reactions were reported. A total of 14 serious TEAEs were reported over the 24-month treatment period; all were assessed by the respective trial investigators as unrelated to apitegromab. The five most common TEAEs were headache, pyrexia, upper respiratory tract infection, cough and nasopharyngitis; they were mostly mild to moderate in severity, consistent with that of the earlier 12-month trial period.

**Table 16. TEAEs observed over 24 months of apitegromab treatment**

| **Treatment Emergent Adverse Events (TEAE)** | **Apitegromab 2 mg/kg (n=10)** | **Apitegromab 20 mg/kg (n=48)** | **Total (n=58)** |
|---|---|---|---|
| | **n (%)** | **n (%)** | **n (%)** |
| **Any TEAE** | 10 (100) | 45 (993.8 | 55 (94.8) |
| **Any Serious TEAE** | 3 (30) | 11 (22.9) | 14 (24.1) |
| **Any TEAE Leading to Drug Discontinuation** | 0 (0.0) | 1 (2.1) | 1 (1.7) |
| **Any Grade 3 or Higher TEAE** | 2 (20) | 9 (18.8) | 11 (19) |

### Efficacy

The non-ambulatory subjects continued to improve in motor function over 24 months of apitegromab treatment. A post hoc analysis pooled together subjects from both non-ambulatory cohorts, i.e., non-ambulatory patients from the cohort that had initiated nusinersen treatment before five years of age and the cohort who initiated nusinersen therapy at the age of five or older. These subjects were 2-12 years old and had been treated with either 2 mg/kg or 20 mg/kg apitegromab. This pooled cohort consisted of 16 subjects, with 50% having initiated nusinersen before five years of age and 50% initiated nusinersen therapy at the age of five or older. A sensitivity analysis excluded any HFSME data following scoliosis surgery performed during the study. It did not exclude subjects who missed apitegromab doses due to COVID site access restrictions (Crawford et al (2022) TOPAZ extension: 24 month efficacy and safety of apitegromab in patients with later-onset Spinal Muscular Atrophy (type 2 and type 3 SMA), presented at Cure SMA, June 2022).

**FIG. 2A** shows that the mean change over baseline in HFSME (95% CI) at 12 months was an increase of 3.6 points and at 24 months was an increase of 4.4 points.

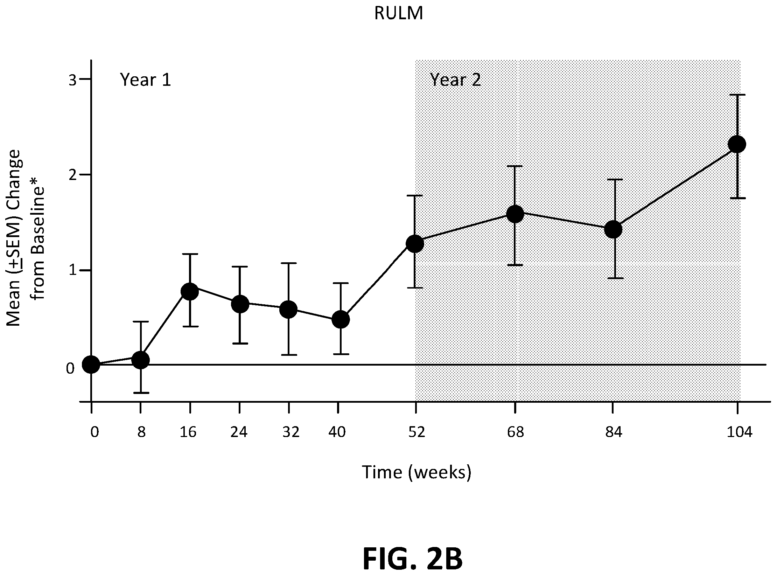

**FIG. 2B** shows that the mean change in RULM (95% CI) at 12 months was an increase of 1.3 points and at 24 months was an increase of 2.3 points. A greater than or equal to a one-point increase in RULM was observed in 48% of these subjects after 12 months and in 67% after 24 months. A greater than or equal to a two-point increase in RULM was observed in 40% of these subjects after 12 months and in 50% after 24 months.

Complications of SMA weakness, such as contracture and scoliosis, accumulate over disease progression and may constrain improvements in functional abilities. Scoliosis surgery has been shown to negatively impact motor abilities in SMA patients. Dunaway Young et al. (2020) J Neuromusc Dis 7:183 observed that 14/17 SMA patients lost more than three points on the HFMSE scale as assessed at least three months post-surgery; the remaining three patients had minimal changes. Those who lost more than three points had a mean loss of 12.1 points (SD=8.9).

**Table 17** shows these improvements in non-ambulatory type 2/3 subjects aged 2-21 years treated with apitegromab and nusinersen.

**Table 17. HFMSE and RULM change in type 2/3 subjects aged 2-21 years.**

| | **12 Months** | **24 Months** |
|---|---|---|
| **Mean Change in HFSME (95%Cl)** | Increase of 3.6 (1.2, 6.0) | Increase of 4.4 (2.0, 6.9) |
| **≥1 point change in HFMSE** | Achieved in 72% (23,32) | Achieved in 64% (18,28) |
| **≥3 point change in HFMSE** | Achieved in 42% (13,32) | Achieved in 46% (13/28) |
| **Mean Change in RULM (95%CI)** | Increase of 1.3 (0.2, 2.3) | Increase of 2.3 (1.2, 3.4) |
| **≥1 point change in RULM** | Achieved in 48% (15/31) | Achieved in 67% (20/30) |
| **≥2 point change in RULM** | Achieved in 40% (12/31) | Achieved in 50% (15,30) |

*Post hoc* observation showed an inverse relationship between two downstream complications of SMA severity (contractures and scoliosis) and change from baseline on the respective primary functional motor scales. At the time of enrollment, any existing scoliosis or contractures had to have been stable over the previous six months and, based upon clinical judgement, anticipated to be stable for the duration of the study. These restrictions were intended to limit confounders of a measurable apitegromab effect during the study. In all Groups 1-3 combined, 66% of subjects had contractures, and 50% had scoliosis. Subjects in the non-ambulatory SMA Groups 1 and 2 without the presence of scoliosis and contractures at the outset of the study (65% and 40% baseline pooled absence, respectively) approached mean changes of motor function improvements of six points compared to two points among those subjects with scoliosis and contractures at time of enrollment. Subjects in the ambulatory type 3 SMA Group (pooled subjects measured with the RHS) without scoliosis and contractures (52% and 50% absence at baseline, respectively) approached increases of one point in RHS, while those with scoliosis or contractures at time of enrollment had a decrease of minus one point.

The ambulatory subjects who received apitegromab monotherapy stabilized their motor function over the 24-month treatment period. These subjects are typically expected to decline if untreated or treated with nusinersen alone (Yang et al. (2022) Adv Ther 39:1915). Their mean change in RHS (95% CI) at 12 months was a decrease of 0.3 points and at 24 months was a decrease of 1.8 points. At 12 months 22% achieved a greater than or equal to a three-point increase from baseline in RHS and at 24 months 24% achieved a greater than or equal to a three-point increase from baseline.

The ambulatory subjects who received both apitegromab and nusinersen, and were also typically expected to decline, also stabilized their motor function over 24 months. Their mean change in RHS (95% CI) at 12 months was a decrease of 0.3 points and at 24 months was a decrease of 0.7 points. At 12 months 17% achieved a greater than or equal to a three-point increase from baseline in RHS and at 24 months 20% achieved a greater than or equal to a three-point increase from baseline.

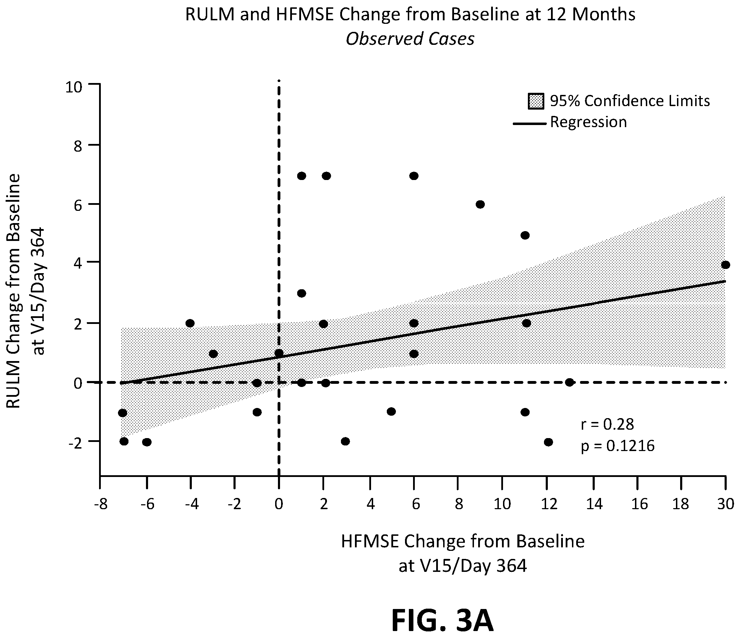

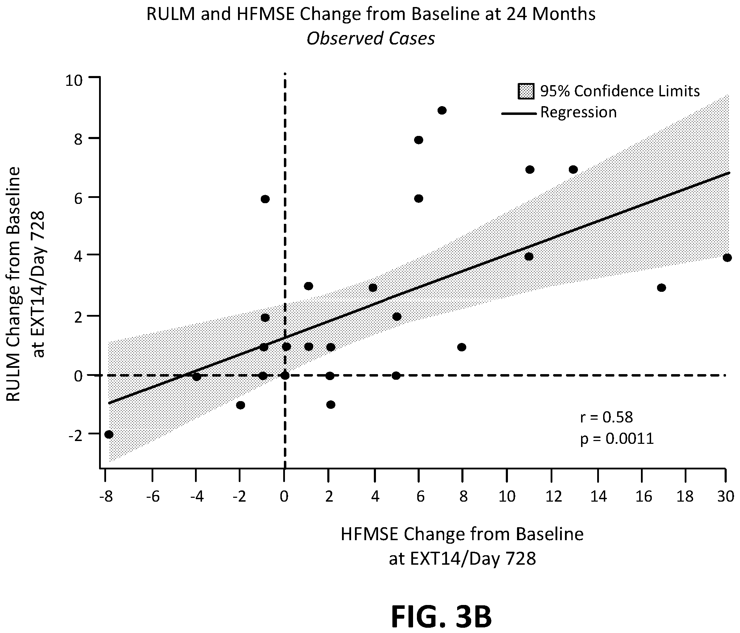

Further evidence of the durability of the therapeutic effect of apitegromab is the correlative relationship between the two measures of motor function, HFSME and RULM, and the increase in that correlation over time. FIG. 3 shows that, over two years, most of the non-ambulatory subjects improved both their HFMSE and RULM scores at both 12 months (FIG. 3A) and 24 months (FIG. 3B). The correlation between the two measures at 12 months was r=0.28, p=0.1216; this increased to r=0.58, p=0.0011 at 24 months. This increase shows that apitegromab treatment provides additional gains in motor function in the second year of treatment, above those attained in the first year.

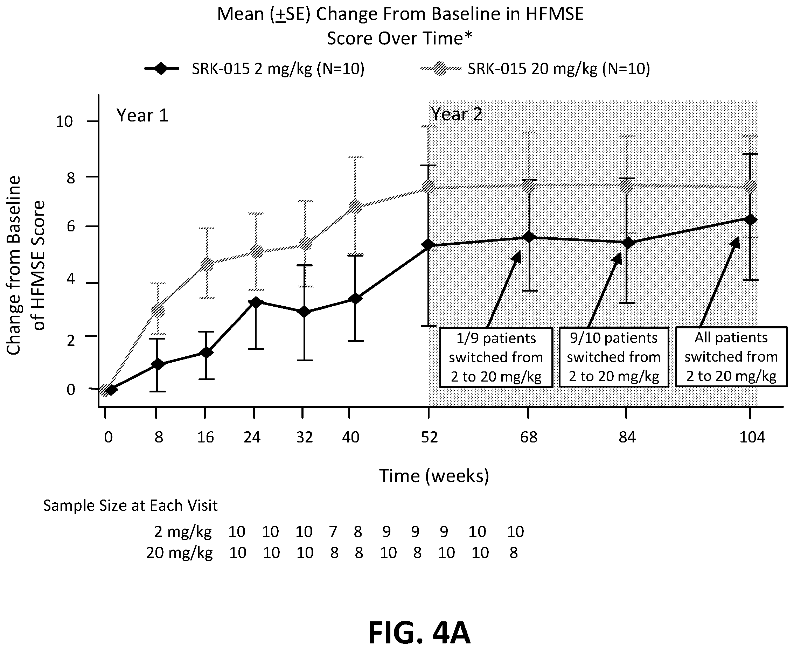

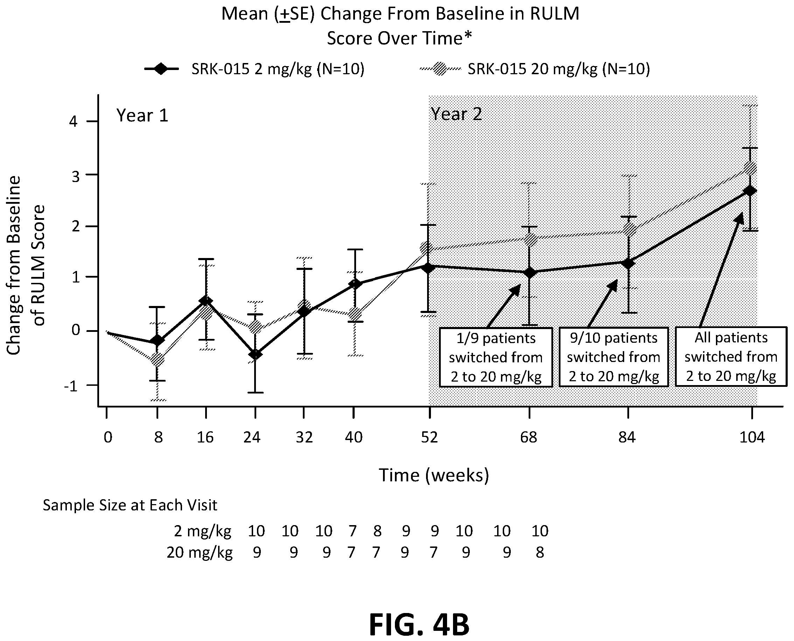

The younger, non-ambulatory subjects in the blinded high- vs. low-dose arm (Type 2, age ≥2) provided additional evidence of a specific apitegromab treatment benefit. Those in the high dose arm had higher HFSME and RULM scores than those in the low dose arm. However, when switched to the high dose, the gap in performance closed (FIG. 4). As more and more subjects were switched to the higher dose, the gap in both HFMSE and RULM continued to narrow, and by week 104, when all subjects who had been dosed at 2 mg/kg had been switched to 20 mg/kg, the outcomes were similar.

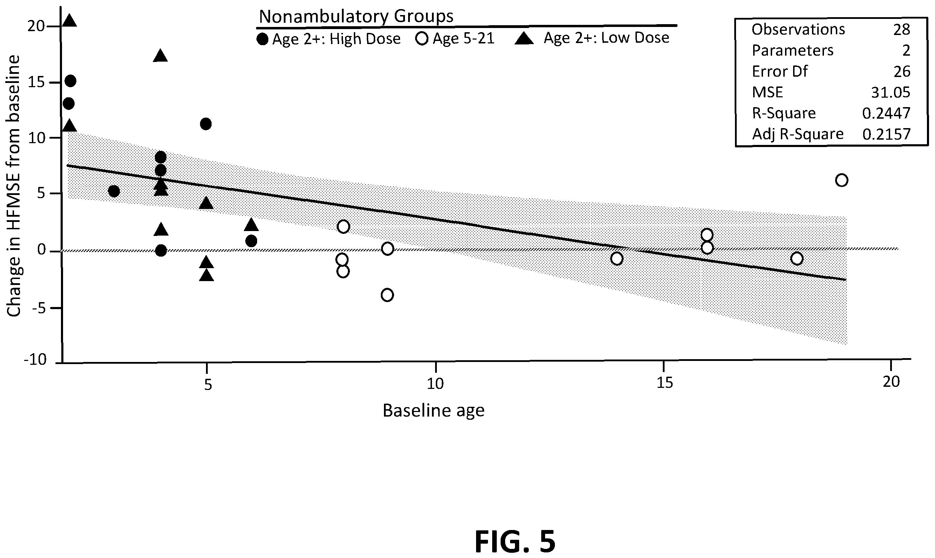

Over two years of apitegromab treatment, the improvement in HFSME correlated with age in type 2 and nonambulatory type 3 subjects, with the younger subjects showing a greater increase in HFMSE scores over baseline than the older subjects **(****FIG. 5****).** After adjusting for age, the baseline weight of the subject was not correlated with a change in HFMSE.

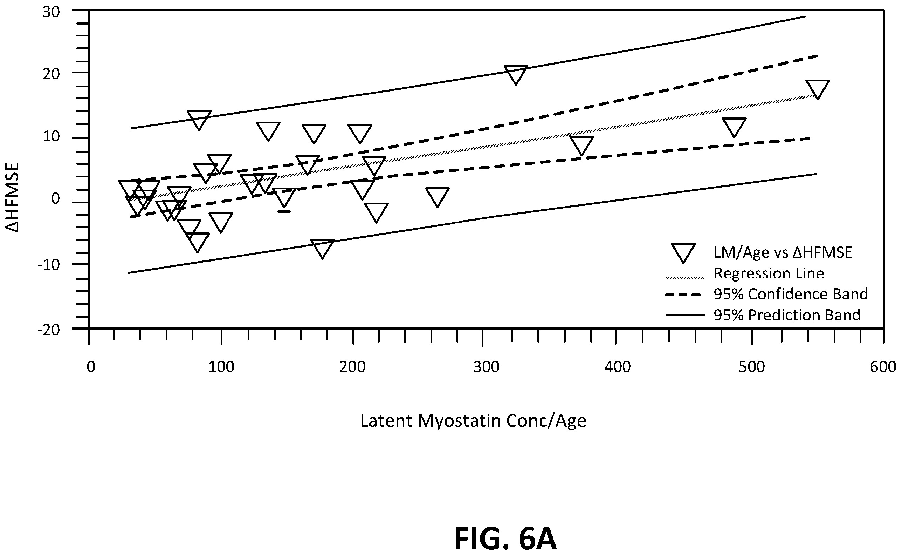

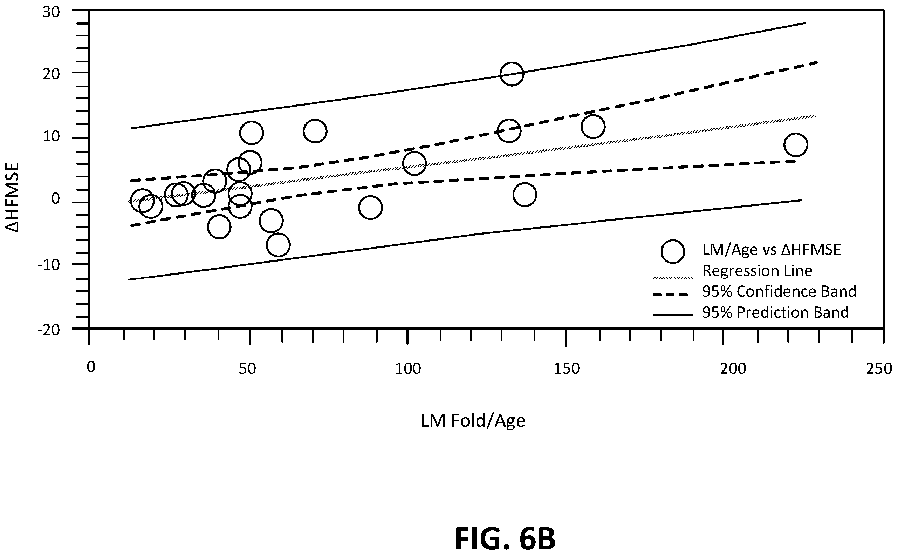

In the second year of apitegromab treatment, target engagement continued to correlate with improvements in motor function, i.e., continued to provide a pharmacodynamic marker for efficacy. Both the amount of latent myostatin and the fold change in latent myostatin, when normalized to the age at which the subject started treatment, provided pharmacodynamic markers of improvements in motor function, as shown in **FIG. 6A****.** A linear regression plot of the change from baseline in HFSME score as a function of the latent myostatin concentration showed that the correlation was highly significant (p=0.0003, n=30). As shown in **FIG. 6B****,** a linear regression plot of the change from baseline in HFSME score as a function of the fold change in latent myostatin showed that the correlation was also highly significant (p=0.0070, n=22).

### Quality of Life

The following results demonstrate that apitegromab is the first muscle-targeted approach to SMA that provides improvements to the quality of life in patients with SMA. These improvements are in addition to any improvements which may be observed with background SMN upregulator treatment (SMN therapy).

Quality of life was assessed by measures of activities of daily living (ADL) and fatigue. Activities of daily living were measured by the Pediatric Evaluation of Disability Inventory Computer Adaptive Test (PEDI-CAT) scale. Fatigue was measured by both the Patient Reported Outcomes Measurement Information System (PROMIS) and the Endurance Shuttle Block and Box Test (ESBBT).

The PEDI-CAT measured pediatric functioning in daily activities and mobility. It is scored on a scale of 1 to 4, with 1 meaning the subject is unable to perform and 4 meaning the subject is easily able to perform the task. Nonambulatory type 2 subjects ≥2 years of age who began nusinersen treatment prior to five years of age improved their activities of daily living scores an average of 1.9 points above baseline after 12 months and 3.0 points above baseline after 24 months. These subjects improved their mobility scores an average of 0.4 points above baseline after 12 months and an average of 2.6 points above baseline after 24 months. Nonambulatory types 2 and 3 subjects 5-21 years of age who began nusinersen treatment after five years of age improved their activities of daily living scores an average of 0.9 points above baseline after 12 months and 0.7 points above baseline after 24 months. Ambulatory type 3 subjects 5-21 years of age improved their activities of daily living scores an average of 2.8 points above baseline after 12 months and 1.8 points above baseline after 24 months.

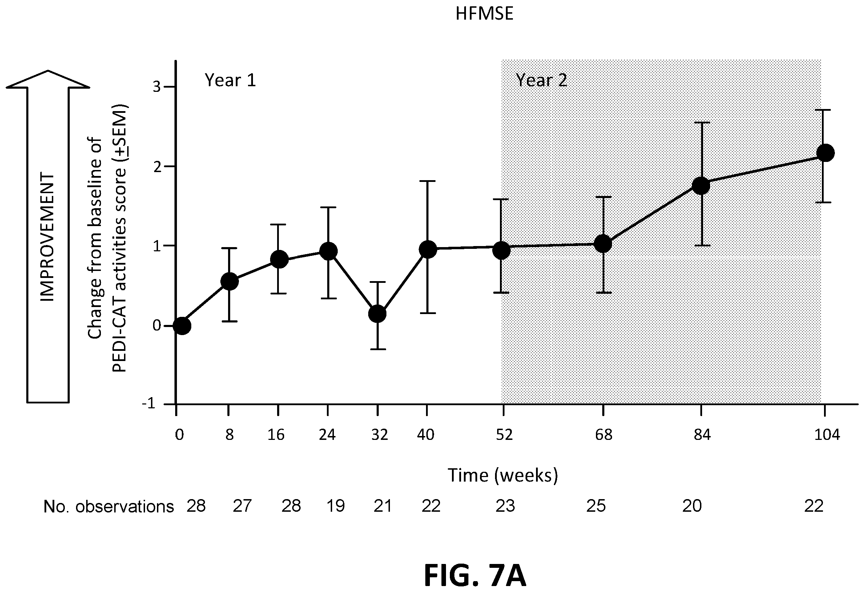

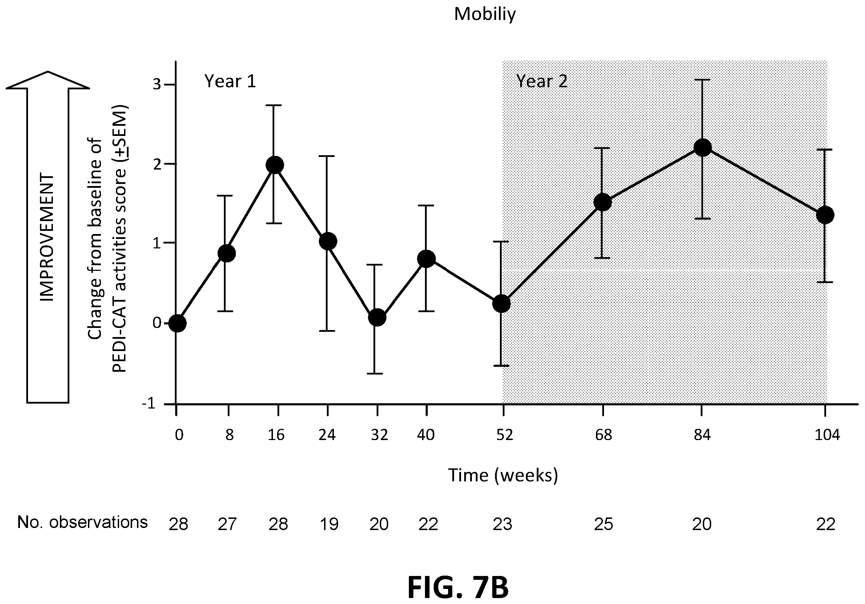

The effect of apitegromab on the activities of daily living and the mobility domains of the PEDI-CAT for the pooled cohort of type 2 and nonambulatory type 3 subjects, as described above for the efficacy studies, is shown in **FIG. 7****.** The change from baseline of both the ADL and mobility continued to increase in the second year of treatment.

PROMIS is a patient reported outcome measure of fatigue. The scale ranges from mild subjective feelings of tiredness to debilitating and sustained feelings of exhaustion. Lower scores reflect less fatigue. Parents provide the scores for subjects under the age of five, subjects provide the scores if they are 18 or older and either may provide the scores of subjects 5-17 years old.

Nonambulatory type 2 subjects ≥2 years of age who began nusinersen treatment prior to five years of age lowered their PROMIS scores by an average of 5.5 points from baseline after 12 months and an average of 5.0 points from baseline after 24 months. Nonambulatory type 2 and 3 subjects 5-21 years of age who began nusinersen treatment after five years of age lowered their scores an average of 0.6 points from baseline after 12 months and an average of 1.3 points from baseline after 24 months. Ambulatory type 3 subjects 5-21 years of age lowered their scores an average of 3.2 points from baseline after 12 months and an average of 4.5 points from baseline after 24 months.

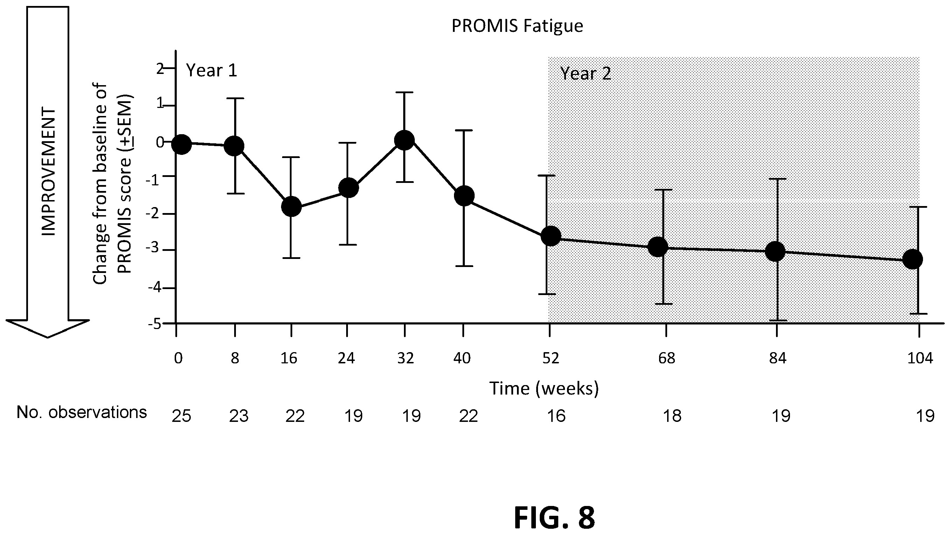

The effect of apitegromab on fatigue, as measured by PROMIS, for the pooled cohort of type 2 and nonambulatory type 3 subjects, as described above for the efficacy studies, is shown in **FIG. 8****.** The change from baseline of the fatigue score continued to decrease in the second year of treatment, reflecting a decrease in the subjects' fatigue.

The PEDI-CAT and the PROMIS results are summarized in Table**18**. The results include the 95% confidence intervals and the number of subjects per group.

**Table 18. Improvements in activities of daily living, mobility, and fatigue**

| **Subject Characteristics** | **12-Month Endpoints** | | | **24-Month Endpoints** | | |
|---|---|---|---|---|---|---|
| | **PEDI-CAT activities** | **PEDI-CAT mobility** | **PROMIS** | **PEDI-CAT activities** | **PEDI-CAT mobility** | **PROMIS** |
| **Nonambulatory Type 2 ≥2 yrs; nusinersen prior to five yrs** | Increase of 1.9 (-0.3, 4.1) (n=11) | Increase of 0.4 (-2.4, 3.2) (n=11) | Decrease of -5.5 (-10.1, 0.9) (n=4) | Increase of 3.0 (1.8, 4.1) (n=14) | Increase of 2.6 (0.4, 4.7) (n=14) | Decrease of -5.0 (-8.9, 1.1) (n=10) |
| **Nonambulatory Type 2/3 5-21 yrs; nusinersen after five yrs** | Increase of 0.9 (-1.4, 3.2) (n=12) | ND | Parent | Increase of 0.7 (-1.8, 3.2) (n=8) | ND | Parent |
| | | | Decrease of -0.6 (-5.9, 4.7) (n=10) | | | Decrease of -1.3 (-6.7, 4.0) (n=9) |
| | | | Adult | | | Adult |
| | | | Decrease of -3,5 (-35.3, 28.3) (n=2) | | | Decrease of -3.5 (-9.9, 2.9) (n=2) |

The ESBBT is a muscle endurance measurement tool that provides a measure of how fast a subject fatigues. It is the first validated and sensitive fatiguability test for proximal arm function in SMA and, by adding the dimension of endurance, is complementary to other outcome measures that focus on arm motor functions such as the RULM (Mazzone et al. (2017) Muscle Nerve 55:869-874). Improvements in proximal arm function indicates an improvement in the quality of life related to self-sufficiency and endurance. The subject was asked to move blocks individually from one box to another for one minute; moving a greater number of blocks indicates greater muscular endurance. Nonambulatory type 2 and type 3 subjects 5-21 years of age who received nusinersen after the age of five showed an increased ESBBT score of 280.8 points from baseline (95% Cl -22.7-584.2; n=8) after 12 months of apitegromab treatment and an increase of 147.9 points from baseline (95% Cl 0.6-295.2; n=8) after 24 months of apitegromab treatment. Subjects who missed three consecutive doses of apitegromab were excluded. The increase over the 12 and 24 month treatment periods is consistent with the increased arm motor function measured by RULM.

Quality of life was also assessed by conducting qualitative interviews with twelve non-ambulatory type 2 and type 3 SMA subjects while enrolled in the 24-month extension, or their caregivers. Trained interviewers conducted in-depth concept elicitation interviews with subjects aged 12-21 years or the caregivers of subjects aged 4-13 years. A semi-structured interview guide was directed to the subjects' perceptions and experiences with SMA and with their treatment with apitegromab. Participants were asked about changes they may have experienced or observed during the study and their experience with any meaningful treatment benefits. Specifically, participants were probed on physical functioning, social impact, and emotional impact. During the interview, subjects were asked about specific symptoms and asked whether the symptom was observed prior to entering the TOPAZ trial, if it started during the trial, and the symptom frequency and severity, as well as changes resulting from the trial.

Subjects or their caretakers reported signs and symptoms that they experienced prior to entering the Phase 2 study. The incidence of bulbar function symptoms were respiratory infections (10/12); use of assistive devices (9/12); inability to cough or clear lung secretions (7/12); breathing difficulties (5/12); feeding/swallowing difficulties (3/12); difficulties with speech (2/12); and snoring (1/12). The incidence of muscle and balance symptoms were muscle weakness (12/12) problems with balance 11/12; tight muscles and tendons (7/12); muscle tone (5/12); and difficulty chewing or smiling (3/12). The incidence of emptying function symptoms were urinary difficulties (1/12); and bowel difficulties (7/12). Subjects also reported other impacts of SMA that they experienced prior to the study. The incidence of the impacts of SMA were fatigue (12/12) and pain (4/12).

In addition, subjects or their caretakers reported impacts of SMA that they experienced prior to entering the study. The incidence of impacts were fatigue (12/12); physical functioning (11/12); dependency (11/12) daily activities (9/12); social activities (9/12); psychosocial an emotional (7/12) relationships (5/12); and pain (4/12).

Where symptoms were reported as problematic before entering the trial, the subject or caretaker was asked whether any changes had occurred during the study. The incidence of improvement in bulbar symptoms were respiratory infections (5/9); use of assistive devices (9/9); inability to cough, clear lung secretions (7/7); breathing difficulties (2/6); feeding/swallowing difficulties (3/3); difficulties with speech (2/2); and snoring (1/1).

The incidence of improvement in muscle and balance symptoms were muscle weakness (12/12) problems with balance (7/11); tight muscles and tendons (3/7); muscle tone ( 5/5); and difficulty chewing or smiling (3/3). The incidence of improvement in emptying function symptoms were urinary difficulties (1/1); and bowel difficulties (5/7). The incidence of improvement in fatigue was (10/12) and in pain was (2/4).

The incidence of improvement in the impacts of SMA were fatigue (12/12); physical functioning (7/11); dependency (7/11) daily activities (8/9); social activities (5/9); psychosocial an emotional (4/7) relationships (3/5); and pain (4/12).

For each symptom and each impact in which a change was reported, the participant was asked to rate the symptom on a scale from 0-10, with zero being not severe and ten being extremely severe, both before entry into the trial and at the time of the interview.

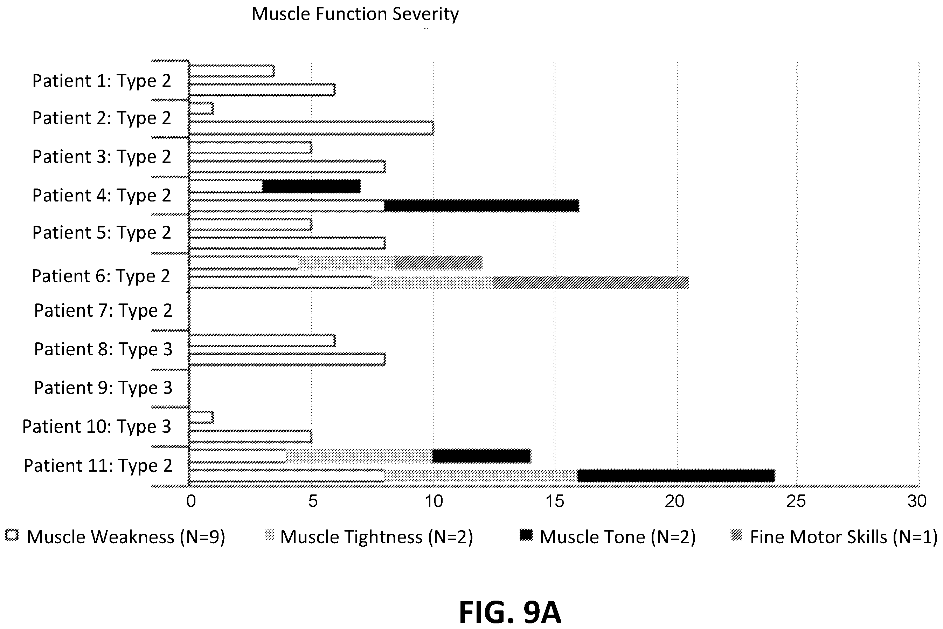

The results of the symptom analysis are shown in **FIGs. 9 A-D.** The symptoms were grouped by the inventors into six categories, (1) muscle function severity; (2) fatigue severity; (3) bulbar function severity; (4) emptying severity; (5) impacts to daily living severity; and (6) social impact severity. The components of each group are shown as a pair of horizontal lines comparing the severity of the symptoms before entering the study (pre) and 24 months post treatment initiation (post).

**FIG. 9A** shows the results of the assessment of muscle functioning. The inventors grouped the measurements of muscle weakness, muscle tightness, muscle tone and fine muscle skills into the category of muscle function severity. Nine of the eleven participants reported results for one or more of the components of this category and rated each on a scale of 0-10. All nine of the participants that reported symptoms related to muscle function reported that they were less severe post treatment.

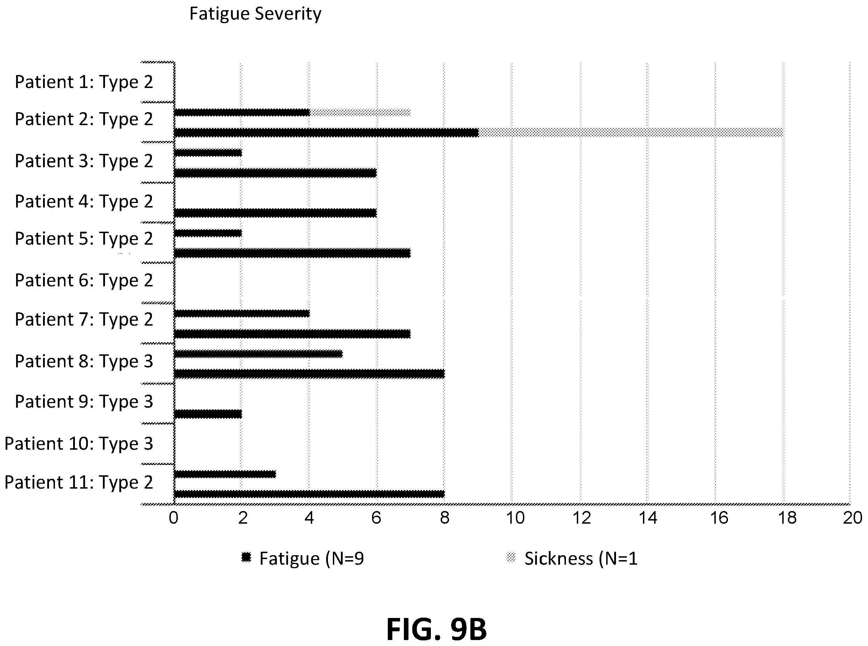

**FIG. 9B** shows the results of assessment of fatigue. The inventors grouped the measurements of fatigue and sickness into the category of fatigue severity. Eight of the eleven participants reported results for one or more of the components of this category and rated each on a scale of 0-10. All eight of the participants that reported symptoms related to fatigue reported that they were less severe post treatment.

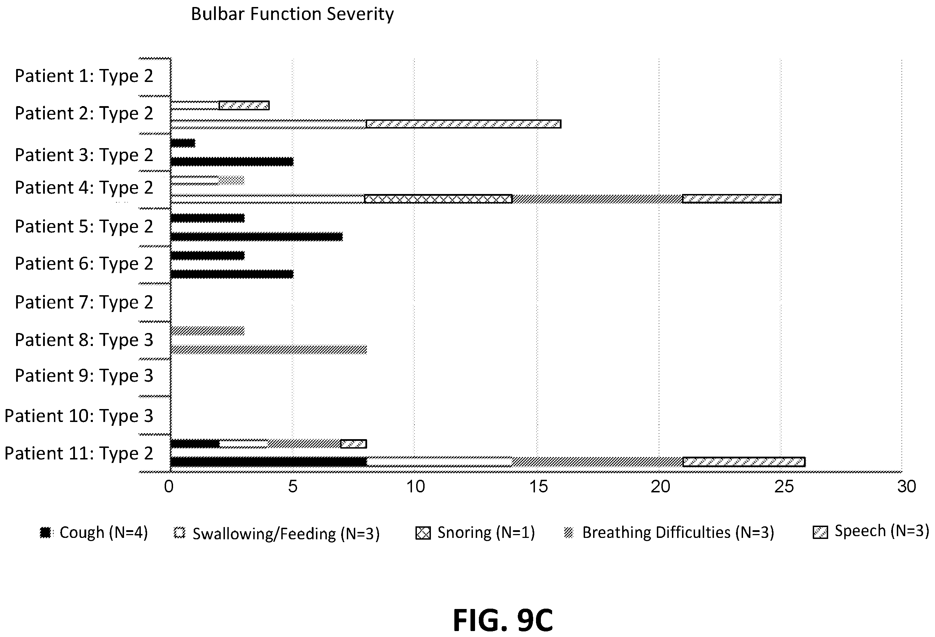

**FIG. 9C** shows the results of the assessment of bulbar function. The inventors grouped the measurements of cough, swallowing or feeding, snoring, breathing and speech into the category of bulbar function severity. Seven of the eleven participants reported results for one or more of the components of this category and rated each on a scale of 0-10. All seven of the participants that reported symptoms related to bulbar function reported that they were less severe post treatment.

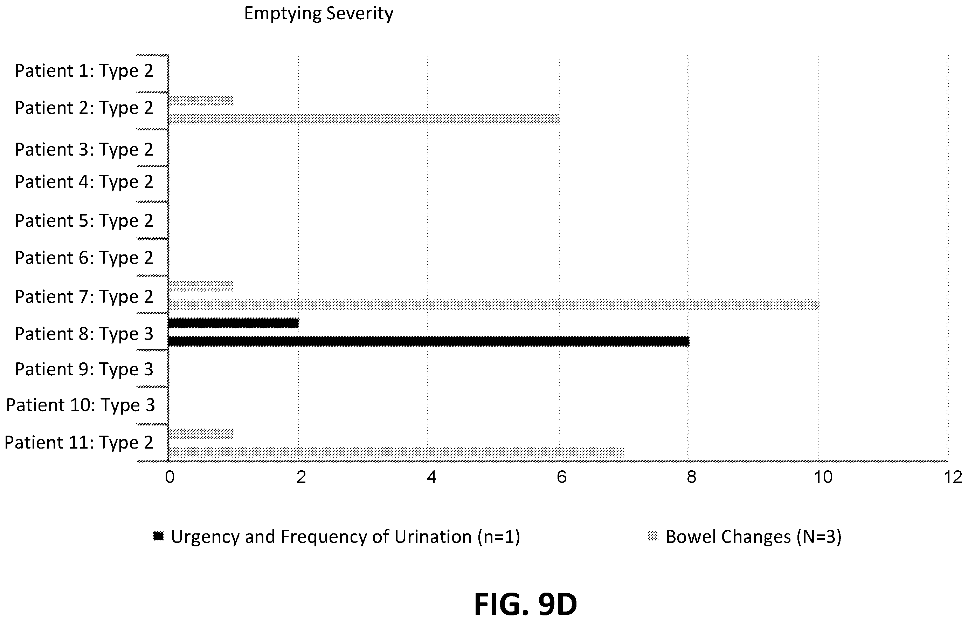

**FIG. 9D** shows the results of the assessment of emptying. The inventors grouped the measurements urgency and frequency of urination and bowel changes into the category of emptying severity. Four of the eleven participants reported results for one or more of the components of this category and rated each on a scale of 0-10. All four of the participants that reported symptoms related to emptying reported that they were less severe post treatment.

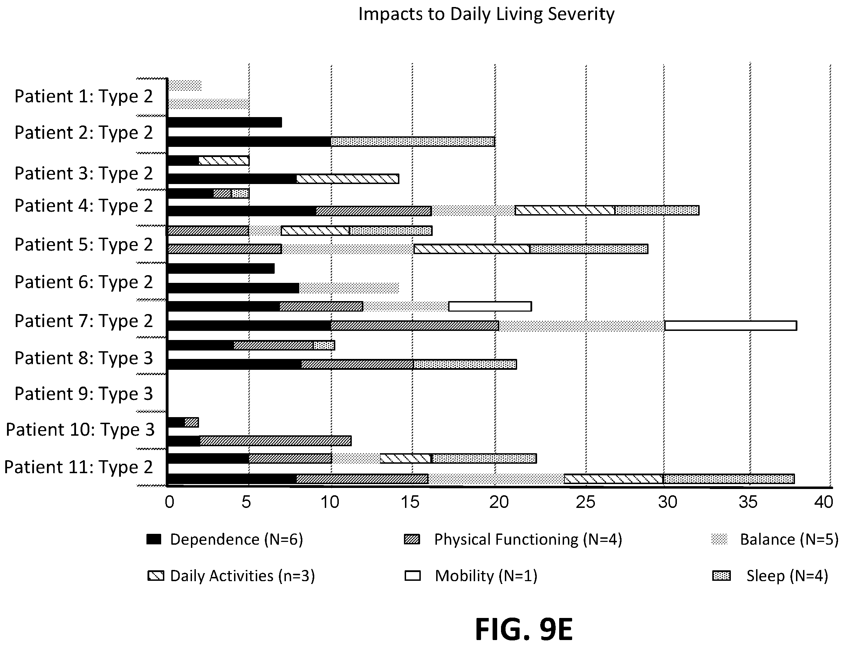

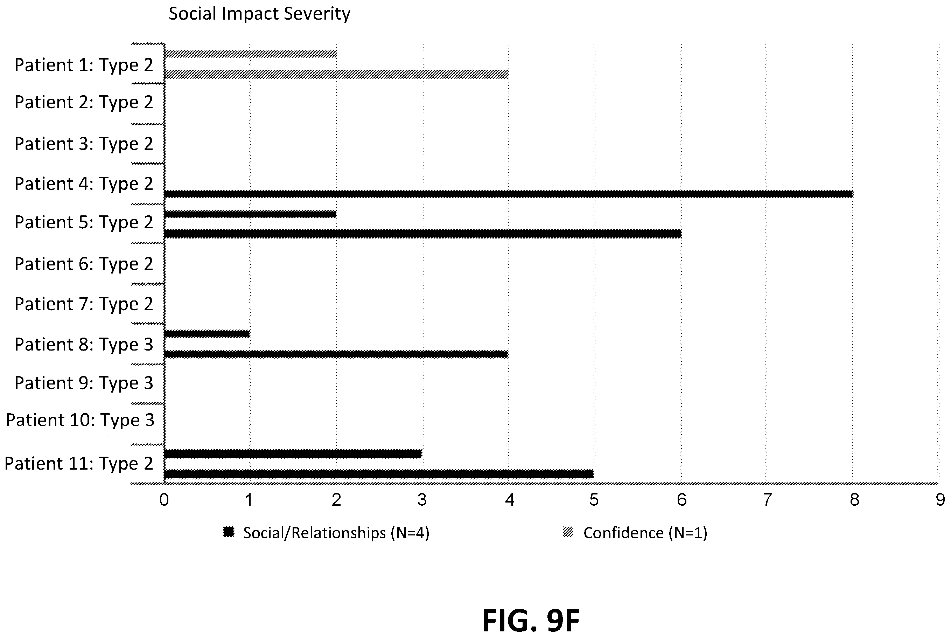

The results of an assessment of impacts to daily living are shown in **FIGs. 9E** and **9F****.** **FIG. 9E** shows the results of the measurements of dependence, physical functioning, balance, daily activities, mobility, sleep, transfers and falls. Ten of the eleven participants reported results for one or more of the components of this category and rated each on a scale of 0-10. All ten of the participants that reported symptoms related to these impacts to daily living reported that they were less severe post treatment.

**FIG.** 9F shows the results of the assessment of social impacts. The inventors grouped the measurements of social relationships and confidence into the category of social impact severity. Five of the eleven participants reported results for one or more of the components of this category and rated each on a scale of 0-10. All five of the participants that reported symptoms related to impacts to daily living reported that they were less severe post treatment.

Treatment with apitegromab in subjects treated with nusinersen was associated with sustained improvements in patient/caregiver reported outcomes of function and perceived fatigue as measured by PROMIS over 36 months in subjects with type 2 and nonambulatory type 3 SMA. Motor function as assessed by HFMSE and RULM showed sustained improvements in subjects with type 2 and nonambulatory type 3 SMA throughout 36 months.

### Example 3. Phase 3 Study of Apitegromab and SMN-targeted Therapy in Subjects with Later-Onset Spinal Muscular Atrophy

SAPPHIRE (ClinicalTrials.gov Identifier: NCT05156320) is a randomized, double-blind, placebo controlled, phase 3 clinical trial. The purpose is to assess the safety and efficacy of apitegromab for improving motor function in subjects with later-onset spinal muscular atrophy (SMA) who are receiving background therapy with nusinersen or risdiplam. Survivor motor neuron (SMN) upregulator therapies have been shown to significantly improve clinical outcomes by preventing or reducing the decline in motor function, however, subjects continue to suffer from substantial motor functional impairment because targeted SMN upregulator therapies focus on SMN-dependent pathways and do not directly impact skeletal muscle to reverse the atrophy that has already taken place. Consequently, there remains an unmet medical need for a complementary therapeutic muscle-directed therapy that may address muscle atrophy and thereby improve motor function in subjects with SMA. Through its novel mechanism of action as a selective inhibitor of myostatin activation, apitegromab has the potential to produce a clinically meaningful effect on motor function in a broad population of subjects with SMA who are being treated with background SMN upregulator therapies.

This trial will be conducted in subjects 2-21 years of age at screening, who have been diagnosed with later-onset SMA (i.e., type 2 and type 3 SMA) and are receiving an approved SMN upregulator therapy to confirm the efficacy and safety of apitegromab as an adjunctive therapy to nusinersen and evaluate the efficacy and safety of apitegromab as an adjunctive therapy to risdiplam. Since both nusinersen and the SMN upregulator risdiplam treat the SMN protein deficiency in SMA by increasing the production of functional SMN protein, previously reported positive results observed with nusinersen support investigating apitegromab as an adjunctive therapy to both nusinersen and risdiplam.

### Patient population

Subjects will meet prespecified inclusion criteria that include that they are age 2-21 years at screening and have a life expectancy of at least two years from screening. They must have a documented diagnosis of 5q SMA and have been diagnosed with later onset, i.e., type 2 or type 3 SMA before receiving either nusinersen or risdiplam. Subjects must be nonambulatory at screening and able to sit independently according to WHO motor milestones at screening. Subjects must have completed either at least ten months of dosing with nusinersen or at least six months of dosing with risdiplam prior to screening. Subjects must have an HFMSE score ≥10 and ≤45 at screening and have no physical limitations that would prevent them from undergoing motor function outcome measures. Subjects must be able to receive study drug infusions and provide blood samples intravenously and female subjects of reproductive age must agree to adhere to contraception.

Exclusion criteria include having received onasemnogene abeparvovec-xioi at any time; previous treatment with apitegromab; a prior history of severe hypersensitivity reaction or intolerance to SMN therapies; and a prior history of a hypersensitivity reaction to a monoclonal antibody or recombinant protein bearing an Fc domain, apitegromab, or excipients of apitegromab. Exclusion criteria also include a requirement for invasive ventilation or tracheostomy; nutritional status that was not stable over the past six months and is not anticipated to be stable throughout the trial; medical necessity for a gastric/nasogastric feeding tube, where the majority of feeds are given by this route; and major orthopedic or other interventional procedure, including spine or hip surgery having the potential to substantially limit the ability of the subjects to be evaluated on any motor function outcome measures within six months before screening or anticipated during the trial. Exclusion criteria further include treatment with other investigational drugs in a clinical trial within three months or five half- lives, whichever is longer, prior to screening; use of valproic acid or hydroxyurea within 90 days before screening; use of therapies with potentially significant muscle effects or potentially significant neuromuscular effects other than approved SMN therapy within 60 days before screening; use of systemic corticosteroids within 60 days before screening (inhaled or topical steroids are permitted); any acute or comorbid condition interfering with the well-being of the subjects within seven days before screening, including active systemic infection, the need for acute treatment, or subject observation due to any reason; severe contractures or scoliosis at screening; use of chronic daytime noninvasive ventilatory support for more than 16 hours daily in the two weeks before dosing, or are anticipated to regularly receive such daytime ventilator support chronically throughout the trial; pregnant or breastfeeding; and any other condition or clinically significant laboratory result or ECG value that, in the opinion of the Investigator, may compromise safety or compliance, would preclude the subject from successful completion of the trial, or interfere with the interpretation of the results.

### Trial Design

The trial will include Screening, Treatment, and Safety Follow-up Periods. Subjects will be randomized to receive 10 mg/kg apitegromab, 20 mg/kg apitegromab, or placebo by intravenous (IV) infusion. Approximately 204 male and female subjects with later-onset SMA will be randomized into either the Main Efficacy Population (2-12 years old at screening) or the Exploratory Subpopulation (13-21 years old at screening). Subjects in the Main Efficacy Population will be randomized separately from the subjects in the Exploratory Subpopulation.

For the Main Efficacy Population, approximately 156 subjects who are 2-12 years old at screening will be randomized 1:1:1 double-blind to receive apitegromab 10 mg/kg, apitegromab 20 mg/kg, or placebo every four weeks during a 52-week Treatment Period. Randomization for the Main Efficacy Population will be stratified by type of background therapy (i.e., nusinersen or risdiplam) and age at initiation of SMN upregulator therapy (≥5 and <5).

For the Exploratory Subpopulation, a maximum of 48 subjects who are 13-21 years old at screening will be randomized 2:1 double-blind to receive apitegromab 20 mg/kg or placebo every four weeks during the 52-week Treatment Period. Randomization for the Exploratory Subpopulation will be stratified by the type of background therapy (i.e., nusinersen or risdiplam).

During the Screening Period (day -28 through day -1) screening and eligibility determinations will be conducted after informed consent has been provided. Screening motor function outcome measures will be conducted a minimum of seven days before the first dose.

The Treatment Period commences on day 1. After dosing, subjects will be monitored for hypersensitivity reactions and after each dose subjects will be contacted by the site by telephone within seven days for a safety check-in. Subject site visits will occur every four weeks through the end of the Treatment Period. Activity assessments in the Treatment Period will include the Pediatric Evaluation of Disability Inventory-Computer Adapted Test (PEDI-CAT), the Patient Reported Outcomes Measurement Information System Fatigue Short Form (PROMIS), the Assessment of Caregiver Experience with Neuromuscular Disease (ACEND) Questionnaire and the Columbia-Suicide Severity Rating Scale (C-SSRS), which measures suicidal ideation and behavior, and treatment-emergent suicidal ideation and behavior.

Two dose levels (10 mg/kg and 20 mg/kg) will be evaluated for the Main Efficacy Population as compared to placebo. The Exploratory Subpopulation will be given a dose of 20 mg/kg or placebo every four weeks or monthly. All administrations of apitegromab will be via intravenous infusion.

All subjects, including those randomized to the placebo group, will continue to receive an approved SMA treatment (i.e., nusinersen or risdiplam). Subjects are expected to have remained on the same background SMA therapy from at least ten months before screening for nusinersen or at least six months before screening for risdiplam and throughout the duration of the trial. Subjects who receive nusinersen must receive their maintenance doses at least 24 hours after receiving study drug, or at least 14 days prior to any scheduled study drug administration visit. Any change in the timing of the subject's nusinersen treatment that would fall within 14 days prior to the scheduled administration of study drug should be discussed with the Medical Monitor in advance.

Subjects who complete the Treatment Period (12-month assessments at Visit 14) may be offered the option to enter an extension trial at that time. Subjects who choose not to enroll in the extension trial will be followed for a 20-week Safety Follow-up Period after Visit 14. Activity assessments in the Safety Follow-up Period will also include PEDI-CAT, PROMIS, ACEND Questionnaire and C-SSRS.

Total trial participation for an individual subject will consist of approximately four weeks for screening, 52 weeks of trial visits and twenty weeks of safety follow up.

An interim analysis may be conducted when at least 50% of the subjects in the Main Efficacy Population complete the Visit 14 assessments. The trial may claim early efficacy at the interim analysis if the boundary based on the prespecified alpha spending function is crossed. If early efficacy is claimed, the trial will be stopped, treatment assignment will be unblinded for both the Main Efficacy Population and the Exploratory Subpopulation, and all subjects will be offered the option to enter an extension trial. If early efficacy is not claimed, the trial will continue as double-blind randomized until all subjects in the Main Efficacy Population complete the 12-month assessments to further characterize the efficacy and safety profile.

The primary analysis of this trial will be performed using data from a "Main Efficacy Population," i.e., subjects who are 2 through 12 years old at screening. Focusing on the 2- through 12-year age range as the Main Efficacy Population facilitates a reliably powered trial, while investigation of apitegromab's effect on a broader age range can be achieved through an Exploratory Subpopulation analysis in a 13- through 21-year age range.

### Efficacy and Safety Assessments

The motor function outcome measures to be tested are the HFSME, the WHO Motor Development Milestones and the RULM. All subjects will perform the HFSME, which consists of 33 items graded on a scale of 0, 1 and 2, where 0 denotes unable, 1 denotes performed with modification or adaption and 2 denotes performance without modification or adaption. All subjects will also perform the WHO Motor Development Milestones assessment, which are a set of six gross motor milestones that are universal and fundamental to acquiring the ability to walk independently. Subjects who are at least 30 months of age at screening will perform the RULM, a 19-item assessment of upper limb function in nonambulatory subjects which tests functions that relate to everyday life.

Safety assessments will include demographics and medical history, vital signs, weight and height, physical examination, electrocardiogram, and the collection and documentation of concomitant medications. Clinical laboratory testing will include serum chemistry, hematology, urinalysis, and coagulation, PK and PD sample draw, pregnancy, and ADA testing; they will be performed using established methods. Because risdiplam distributes and increases SMN protein in the central nervous system, interventional suicidal ideation and behavior risk monitoring will be performed. The C-SSRS will be used for subjects six years of age and older and the Very Young Child/Cognitively Impaired Version of the C-SSRS will be used for subjects who are four to five years old.

The efficacy of apitegromab will be assessed by the change in the HFMSE total score from baseline to 12 months compared with placebo. The primary outcome is the difference in HFMSE mean change from baseline to 12 months in the intended to treat (ITT) population in the Main Efficacy Population treated with apitegromab vs. placebo. The ITT population is defined as all randomized subjects in the Main Efficiency Population, even if the subjects does not receive the correct treatment or otherwise does not follow the protocol. The primary hypothesis of interest is whether apitegromab 20 mg/kg has superior efficacy compared with placebo in subjects from 2 through 12 years old treated with background SMN upregulator therapies. If superiority is demonstrated with the 20 mg/kg group, the hypothesis of whether apitegromab 10 mg/kg has superior efficacy compared with placebo in subjects from 2 through 12 years old treated with background SMA upregulator therapies will be tested to determine the biologically optimal dose.

A secondary endpoint, the proportion of subjects with a greater than or equal to three-point change from baseline in the HFMSE total score at 12 months, will be compared between the apitegromab 20 mg/kg (and/or 10 mg/kg) group vs. placebo using a logistic model, adjusting for baseline HFMSE total score and the stratification factors at randomization.

Upon completion of the 12-month treatment period, all subjects will be offered the option of enrolling in an open label extension study in which the safety and tolerability of apitegromab will be further evaluated, along with an exploratory characterization of longer-term efficacy.

### Pharmacokinetics, Pharmacodynamics, and Immunogenicity

Whole blood samples will be collected to evaluate the pharmacokinetics (PK) of apitegromab, including measuring the serum concentration, on Treatment Period days 1, 29, 113, 169, 225, 281, 337 and 365 and at the end of the Follow-up Period. Once emerging PK data have been collected and analyzed, the PK sample schedule may be adjusted to reflect a decrease in the number of samples to be collected.

Whole blood will be collected for pharmacodynamic studies (PD) and immunogenicity assessments at the same time that PK samples are collected. Subject confidentiality will be maintained and apitegromab concentration information that would unblind the treatment assignment will not be reported to investigative sites or to blinded personnel until the treatment assignment has been unblinded. The PD of apitegromab will be evaluated by measuring serum circulating latent myostatin concentration. Once emerging PD data have been collected and analyzed, the PD sample schedule may be adjusted to reflect a decrease in the number of samples to be collected.

Serum samples will be screened for antibodies that bind to apitegromab and the titer of confirmed positive samples will be reported. Other analyses may be performed to verify the stability of any detected antibodies to apitegromab and/or to further characterize the immunogenicity of apitegromab. The detection and characterization of antibodies to apitegromab will be performed using a validated assay method. All samples collected for detection of antibodies to apitegromab will also be evaluated for apitegromab serum concentration to enable interpretation of the antibody data. Antibodies may be further characterized and/or evaluated for their ability to neutralize the activity of apitegromab. Samples may be stored for a maximum of fifteen years or according to local regulations following the last subject's last visit to enable further analysis of immune responses to apitegromab.

### Objectives and Endpoints

### Main efficacy population

An objective is to assess the efficacy of apitegromab compared with placebo using the HFMSE in subjects 2-12 years old. The endpoint is a change in baseline in HFMSE total score at 12 months

An objective is to assess the efficacy of apitegromab compared with placebo based on the number of subjects with clinical improvement in subjects 2-12 years old. The endpoint is the proportion of subjects with ≥3-point change from baseline in the HFMSE total score at 12 months.

An objective is to assess the efficacy of apitegromab compared with placebo by measuring changes in upper limb function using the Revised Upper Limb Module (RULM) in subjects 2-12 years old. The endpoint is the change from baseline in RULM total score at 12 months.

An objective is to assess the efficacy of apitegromab compared with placebo by measuring changes in number of World Health Organization (WHO) motor development milestones in subjects 2-12 years old. The endpoint is the change from baseline in the number of WHO motor development milestones attained at 12 months.

An objective is to further assess the efficacy of apitegromab compared with placebo by evaluating changes in additional motor function outcome measures and changes in HFMSE at other prespecified time points in subjects 2-12 years old. The endpoints are (i) the proportion of subjects achieving various magnitudes of change in HFMSE score from baseline at 12 months; (ii) the proportion of subjects achieving various magnitudes of change in RULM score from baseline at 12 months; (iii) the proportion of subjects who attain a new WHO motor development milestone relative to baseline at 12 months; (iv) the change from baseline in HFMSE total score at other prespecified time points; (v) the change from baseline in RULM total score at other prespecified time points; and (vi) the change from baseline in the number of WHO motor development milestones attained at other prespecified time points.

An objective is to assess the efficacy of apitegromab compared with placebo by measuring changes from baseline in motor function across the treatment period using the HFMSE in subjects 2-12 years old. The endpoint is change from baseline in HFMSE total score across time during the 12-month treatment period.

An objective is to assess the time to therapeutic effect of apitegromab compared with placebo using the HFMSE in subjects 2-12 years old. The endpoint is time to therapeutic effect (≥3-point change from baseline in HFMSE total score) compared between apitegromab and placebo.

An objective is to evaluate the effects of apitegromab on subject/caregiver-reported disability, fatigability, and suicidal ideation and behavior in subjects 2-12 years old. The endpoints are (i) change from baseline in PEDI-CAT; (ii) change from baseline in the PROMIS fatigue questionnaire; (iii) change from baseline in ACEND; and (iv) change from baseline in C-SSRS.

### Main efficacy population/exploratory subpopulation

An objective is to assess the safety and tolerability of apitegromab in all randomized subjects with later-onset SMA who receive at least one dose of apitegromab. The endpoint is the incidence of treatment-emergent adverse events (TEAEs) and serious adverse events (SAEs) by severity.

An objective is to characterize the pharmacokinetics (PK) of apitegromab in all randomized subjects with later-onset SMA who receive at least one dose of apitegromab. The endpoint is the apitegromab concentration in serum from blood samples.

An objective is to evaluate the pharmacodynamic (PD) effects of apitegromab in all randomized subjects with later-onset SMA who receive at least one dose of apitegromab. The endpoint is the level of circulating latent myostatin concentrations in blood samples.

An objective is to evaluate the immunogenicity of apitegromab in all randomized subjects with later-onset SMA who receive at least one dose of apitegromab. The endpoint is the presence or absence of antidrug antibody (ADA) against apitegromab in serum from blood samples.

An objective is to assess the efficacy of apitegromab compared with placebo using the HFMSE in subjects 13-21 years old and in subjects 2-21 years old. The endpoint is the change from baseline in HFMSE total score at 12 months.

An objective is to assess the efficacy of apitegromab compared with placebo based on the number of subjects with clinical improvement in subjects 2-21 years old. The endpoint is the proportion of subjects with ≥3-point change from baseline in the HFMSE total score at 12 months.

An objective is to assess the efficacy of apitegromab compared with placebo by measuring changes in upper limb function between baseline and the end of the treatment period using the RULM in subjects 13-21 years old and in subjects 2-21 years old. The endpoint is the change in baseline in RULM total score at 12 months.

An objective is to assess the efficacy of apitegromab compared with placebo by measuring changes in the number of WHO motor development milestones in subjects 13-21 years old and in subjects 2-21 years old. The endpoint is the change from baseline in the number of WHO motor development milestones attained at 12 months.

An objective is to assess the efficacy of apitegromab compared with placebo by evaluating changes in additional motor function outcome measures and changes in HFMSE at other prespecified time points in subjects 13-21 years old and in subjects 2-21 years old. The endpoints are (i) the proportion of subjects achieving various magnitudes of change in HFMSE score from baseline at 12 months; (ii) the proportion of subjects achieving various magnitudes of change in RULM score from baseline at 12 months; (iii) the proportion of subjects who attain a new WHO motor development milestone relative to baseline at 12 months; (iv) the change from baseline in HFMSE total score at other prespecified time points; (v) the change from baseline in RULM total score at other prespecified time points; and (vi) the change from baseline in the number of WHO motor development milestones attained at other prespecified time points.

An objective is to assess the efficacy of apitegromab compared with placebo by measuring changes from baseline in motor function across the treatment period using the HFMSE in subjects 13-21 years old and in subjects 2-21 years old. The endpoint is the change from baseline in HFMSE total score across time during the 12-month treatment period.

An objective is to assess the time to stabilization of effect of apitegromab compared with placebo using the HFMSE in subjects 13-21 years old. The endpoint is the time to decline (at least a - 3-point change from baseline in HFMSE total score) compared between apitegromab and placebo.

An objective is to assess the time to therapeutic effect of apitegromab compared with placebo using the HFMSE in subjects 2-21 years old. The endpoint is the time to therapeutic effect (≥1-point change from baseline in HFMSE total score) compared between apitegromab and placebo.

An objective is to evaluate the effects of apitegromab on fatigability, caregiver-reported disability, and suicidal ideation and behavior in subjects 13-21 years old and in subjects 2-21 years old. The endpoints are (i) the change from baseline in PEDI-CAT; (ii) the change from baseline in PROMIS fatigue questionnaire; (iii) the change from baseline in ACEND; and (iv) the change from baseline in C-SSRS.

### Dose

The unit dose strength is 50±5 mg/ml. Doses will be diluted in normal saline and administered by IV infusion over a one-to-two-hour period, ±10-minute window. After dosing, subjects will be monitored for hypersensitivity reactions at the trial site for less than or equal to two hours, but no less than one hour after the completion of the infusion. After each of the first four doses, subjects will be contacted by the trial site by telephone within seven days for a safety check-in (with subsequent safety check-ins limited to subjects reporting an adverse event at the previous check-in).

Weight-based dosing, which was used in the feeder trials, will continue to be used because weight is a strong covariate on clearance of apitegromab. The dose regimen of 20 mg/kg Q4W, which is currently being used in the TOPAZ extension periods, was selected based on data from the TOPAZ feeder trial. The dose may be changed for all subjects at a future point based on further assessment of data from the feeder trials.

If an acute infusion reaction occurs, further dosing will be suspended and the investigator, in consultation with the medical monitor if needed, will evaluate the risk represented by the acute reaction. Such an evaluation will incorporate consideration of the nature of the event, relatedness to the drug, and seriousness and severity of the event. Intervention for subjects who have an acute infusion reaction should be performed in accordance with standard procedures and may include restarting the infusion at a slower rate, terminating the infusion, administration of medications, or other medically supportive measures, as necessary.

An overdose is defined as and will be dealt with as in section 5.2.1.3 of Example 2. Overdoses are recorded and are not considered adverse events.

### Concomitant Therapy

Concomitant therapies or interventional procedures that are medically indicated for any AEs the subject has during the trial or that are provided as part of standard supportive care for the subjects, are permitted at the discretion of the Investigator and supersede any of the restrictions outlined in this protocol.

All concomitant medications will be collected from baseline through 20 weeks after the final dose. Concomitant medications and a detailed history of medications will be documented for each subject. All concomitant medications including dietary supplements, over-the-counter medications, and oral herbal preparations, as well as changes in medication, will be recorded.

Investigational therapies are not permitted throughout the duration of the trial.

The concomitant use of the following drugs or products has the potential to interfere with the assessment of treatment effect in this trial. Accordingly, the use of the following is prohibited from baseline through the final visit: vaccinations within 14 days of any trial visit where motor function outcome measures are conducted; systemic corticosteroids (inhaled and topical steroids are allowed); valproic acid; hydroxyurea; any therapy with potentially significant muscle effects, e.g., androgens, insulin-like growth factor, growth hormone, systemic beta-agonist, botulinum toxin, muscle relaxants or muscle-enhancing supplements or agents with potentially significant neuromuscular effects, e.g., acetylcholinesterase inhibitors, other than approved SMN upregulator therapy.

Any medication or vaccine (including over the counter or prescription medicines, recreational drugs, vitamins, and/or herbal supplements) that the subjects is receiving at the time of completion of the informed consent process or receives during the trial must be recorded.

### Discontinuation of Study Drug and Subject Withdrawal

Dosing for all subjects in the trial may be suspended at any time for an emergent safety concern by a medical monitor, and/or sponsor until the concern can be evaluated and an appropriate course of action recommended. Additional data, input and deliberation may contribute to a decision to restart dosing and continue the trial with no changes, continue with protocol changes, or terminate the trial.

Dosing for any individual subjects may be temporarily discontinued of the subject has an SAE related to the study drug or a clinically significant nonserious AE related to the study drug, that warrants an interruption from dosing for that subject's well-being. If study drug dosing is interrupted, the subject may resume dosing only after the AE has resolved. Dosing may also be temporarily discontinued if there are other safety concerns.

Dosing for any individual subject may be permanently discontinued due to an AE or other safety concerns if (1) the subject has an SAE related to study drug or a clinically significant nonserious AE related to study drug, that warrants permanent discontinuation from further dosing for that subject's well-being; or (2) if there are safety concerns other than those described above that threaten the subject's safety. If study drug dosing is permanently discontinued due to an AE or other safety concerns and the subject does not want to continue with their monthly visit schedule, the subject will complete the assessments for the end of treatment visit and continue to be followed for 20 weeks after their final dose or until the resolution of any ongoing clinically significant AE, whichever occurs later.

Subjects who develop either an SAE or other toxicity meeting the individual permanent discontinuation criteria listed above will be carefully monitored and may be required to have additional assessments, including (1) additional clinical laboratory tests; (2) additional visits or extended duration of follow-up; or (3) consultation with a specialist.

Dosing for any individual subject may be permanently discontinued for reasons not related to safety if the subject or parent/guardian withdraws consent or the investigator recommends discontinuation for reasons unrelated to safety, including but not limited to noncompliance or worsening symptoms. The subject will complete the assessments for the end of treatment visit and continue to be followed for 20 weeks after their final dose. If the subject does not want to do this, they will complete an early termination visit and be discontinued from the trial.

Subjects who permanently discontinue study drug are encouraged to remain in the trial until they complete their monthly treatment period visits and the safety follow-up visits. At the time of discontinuation of study drug, the reason for discontinuation of treatment will be documented and the subject will resume their monthly visit schedule.

A subject and/or parent or guardian may withdraw consent to participate in the trial at any time at their own request. If the subject or parent/guardian withdraws consent for disclosure of future information, the sponsor may retain and continue to use any data collected before consent was withdrawn. A subject may be permanently discontinued from the trial at any time at the discretion of the investigator because of a protocol violation, a serious or intolerable AE, a clinically significant change in laboratory parameter, request by the sponsor or at the request of the parent or guardian. A subject must be permanently discontinued from the trial if they become pregnant.

At the time of withdrawal or discontinuation, if possible, an early termination visit should be conducted and any SAE followed to resolution. A subject will be considered lost to follow-up if they fail to return for scheduled visits and is unable to be contacted by the trial site.

### Statistical Analysis

Primary efficacy endpoint analysis of apitegromab will be assessed by the change in the HFMSE total score from Baseline to 12 months compared with placebo. The primary outcome will be the difference in HFMSE mean change from Baseline to 12 months in the ITT population between patients in the Main Efficacy Population treated with apitegromab vs. placebo. The primary analysis model will be a restricted maximum likelihood model (REML)-based mixed effects model with repeated measurement (MMRM). Change from Baseline in HFMSE will be analyzed using an MMRM analysis with unstructured covariance modeling of time, the treatment-by-time interaction, and within-patient errors. The model will have fixed effects of treatment, categorical time, the treatment-by-time interaction, Baseline HFMSE total score, the interaction between Baseline HFMSE total score and time, and the stratification factors at randomization as covariates. The Kenward-Roger method will be used to estimate the degrees of freedom. If the model with the unstructured covariance structure fails to converge, the Heterogeneous First-Order Autoregression covariance structure will be used to model the within-patient variability. Type III sums of squares for the least squares means will be used for the statistical comparison; 95% CI will also be reported (Kenward and Roger 1997). Sensitivity analyses will be performed to evaluate the impact of missing data on the primary efficacy analysis by deviations from the Missing at Random assumption.

Secondary efficacy endpoints analyses of the proportion of patients with ≥3-point change from Baseline in the HFMSE total score at 12 months, will be compared between the apitegromab 20 mg/kg (or 10 mg/kg) group vs. placebo using a logistic model, adjusting for Baseline HFMSE total score and the stratification factors at randomization. The key secondary endpoints, change from Baseline in RULM at 12 months and change from Baseline in WHO motor milestones attained at 12 months, will be analyzed the same way as the primary endpoint. The same strategy applied to the primary endpoint will be used to handle intercurrent events for the key secondary endpoints. Other continuous secondary endpoints will be analyzed using the same method as the change from Baseline HFMSE at 12 months, and discrete endpoints will be analyzed using a similar method to the proportion of patients with ≥3-point change from Baseline in the HFMSE total score at 12 months. 9.4.4. Multiplicity To control type I error caused by multiple comparisons, a hierarchical testing procedure will be applied: 1. The key secondary endpoints for the apitegromab 20 mg/kg group will only be tested against the placebo group if the analysis of HFMSE at 12 months is statistically significant. 2. The apitegromab 10 mg/kg group will only be tested against the placebo group if all the hypotheses of the key secondary endpoints are rejected for the apitegromab 20 mg/kg group vs. the placebo group. Within each dose group, key secondary endpoints will be tested by the following order: 1. Proportion of patients with ≥3-point change from Baseline in the HFMSE total score at 12 months 2. Change from Baseline in RULM at 12 months 3. Change from Baseline in WHO motor milestones attained at 12 months.

All safety data will be descriptively summarized by treatment group. TEAEs are defined as AEs that started or worsened in severity after the first dose of study drug. The number of TEAEs as well as the number and percentage of patients who have at least one TEAE will be summarized using the Medical Dictionary for Regulatory Activities for each system organ class (or a body system) and each preferred term by treatment group. SAEs and AEs that lead to discontinuation of investigational product will also be summarized by treatment group. All clinical laboratory results will be descriptively summarized by treatment group. Individual results that are outside of normal reference ranges will be flagged in data listings. Quantitative clinical hematology, chemistry, and urinalysis variables obtained at the Baseline to postbaseline visits will be summarized as changes from Baseline by treatment group and analyzed using analysis of covariance (ANCOVA) with treatment and Baseline value in the model. Change from Baseline to postbaseline in vital signs and body weight will be analyzed using ANCOVA with treatment and Baseline value,

Pharmacokinetic and pharmacodynamic endpoints analyses will include apitegromab concentrations and parameters and circulating latent myostatin concentrations will be determined using blood samples collected before and after dosing through the final follow-up visit.

### Example 4. Sustained Safety and Efficacy of Apitegromab

A Phase 3 extension trial will be conducted to assess safety and efficacy of apitegromab in ambulatory and non-ambulatory patients with later onset, e.g., type 2 and type 3 SMA who completed an investigational trial of apitegromab as a monotherapy or used with an approved SMN therapy. The two feeder trials will be NCT03921528 (Examples 1 and 2) and NCT05156320 (Example 3). The subject's last visit from the feeder trial will serve as the baseline visit. Approximately 260 male and female subjects ≥2 years of age will receive 20 mg/kg apitegromab every four weeks by IV infusion over a 104 week treatment period.

Safety assessments will include vital signs, weight and height, physical examination, electrocardiograms, documentation of concomitant medications, and clinical laboratory testing. Efficacy will be evaluated by motor function outcome measures including HFMSE, WHO motor development milestones, RULM, RHS, 10-meter walk/run, times rise from floor, 6-minute walk test, and 30-second sit to stand.

Pharmacokinetic analysis will be conducted by collecting whole blood samples and measuring the serum concentration of apitegromab. Pharmacodynamic analysis will be determined by collecting whole blood samples and measuring the serum concentration of circulating latent myostatin. The presence of anti-drug antibodies will be determined by collecting whole blood and screening the serum for the presence and titer of antibodies that bind to apitegromab using known assay methods.

Efficacy will be assessed by measuring changes in motor function outcomes at prespecified tidme points. The Hammersmith Functional Motor Scale Expanded (HFMSE), the Revised Upper Limb Module (RULM), and the WHO motor development milestone total scores will be measured in subjects, excluding the TOPAZ Cohort 1 subjects. Efficacy will be assessed in the TOPAZ Cohort 1 subjects my measuring changes in motor function as assessed by the Revised Hammersmith Scale (RHS), the 6-minute walk test, the 30-second sit to stand test, the 10-meter walk/run test, and the timed rise from the floor test.

Quality of life assessments will be measured as patient or caregiver-reported outcomes, including PEDI-CAT, PROMIS, ACEND, and C-SSRS.

An objective of the trial is to evaluate the long-term safety and tolerability of apitegromab in patients with type 2 and type 3 SMA. This objective will be evaluated by the incidence of TEAEs and SAEs, according to severity.

An objective of this trial is to evaluate the long-term efficacy of apitegromab by assessing changes in motor function outcome measures a prespecified time points. This objective will be evaluated by (1) measuring HFMSE score at prespecified time points (excluding TOPAZ cohort 1 subjects); (2) measuring RULM score at prespecified time points; (3) measuring the number of WHO motor development milestones attained at prespecified time points (excluding TOPAZ cohort 1 patients; and (4) measuring RHS score, results from the 6-minute walk test, the 30 second sit-to-stand test, at prespecified time points (TOPAZ cohort 1 subjects).

An objective of this trial is to further evaluate the immunogenicity of apitegromab. This objective will be evaluated by determining the presence or absence of anti-drug antibodies against apitegromab in serum from blood samples.

An objective of this trial is to further evaluate the pharmacodynamic effects of apitegromab. This objective will be evaluated by determining the concentration of circulating latent myostatin in blood samples at prespecified time points.

An objective of this trial is to further evaluate the effect of apitegromab on patient and/or caregiver-reported disability, fatiguability, and suicidal ideation and behavior. This objective will be evaluated by administering the PEDI-CAT at prespecified time points, administering the PROMIS fatigue questionnaire at prespecified time points, administering ACEND at prespecified time points, and administering the C-SSRS at prespecified time points.

### Example 5: Safety and Efficacy of Apitegromab in Patents with Severe SMA

Patients under the age or two years with the most severe SMA disease, e,g, one, two, or three SMN2 gene copy numbers (most likely type 1 or severe type 2) can be studied with a combination of pharmacokinetic, pharmacodynamic (including partial extrapolation using serum latent myostatin), safety, and efficacy measures. With the introduction of SMN therapies, earlier diagnosis that includes newborn and prenatal screening and earlier treatment, the natural course of SMA disease progression continues to evolve.

A double-blind, multiple dose phase 2 trial will be conducted in patients with type 1 SMA, who have either received or are receiving standard of care and remain symptomatic to evaluate the safety, efficacy, pharmacokinetics and pharmacodynamics of apitegromab as an adjunctive therapy. In an embodiment, children ≤2 years of age with type 1 SMA, defined as having a documented diagnosis of 5q SMA and/or a diagnosis of type 1 SMA based on gene mutation analysis with a biallelic mutation (deletion or point) in the SMA1 gene and one copy of the SMN2 gene will be eligible. In an embodiment, children ≤2 years of age with type 1 SMA, defined as having a documented diagnosis of 5q SMA and/or a diagnosis of type 1 SMA based on gene mutation analysis with a biallelic mutation (deletion or point) in the SMA1 gene and two copies of the SMN2 gene will be eligible. In an embodiment, children ≤2 years of age with type 1 SMA, defined as having a documented diagnosis of 5q SMA and/or a diagnosis of type 1 SMA based on gene mutation analysis with a biallelic mutation (deletion or point) in the SMA1 gene and three copies of the SMN2 gene will be eligible. In an embodiment, the subject is symptomatic, defined as having a greater than 1.5 point standard deviation from the norm on the BSID-IV Motor Composite Score or as having a CHOP-INTEND score of ≤40. Standard of care includes providing at least one dose of onasemnogene abeparvovec-xioi or treating with nusinersen or risdiplam.

Three dose levels will be evaluated and subjects will be randomized 1:1:1 to receive either the low, medium, or high dose intravenously every four weeks during a 52-week treatment period, following a screening period of up to four weeks. The trial will include a screening period, a treatment period and a 140-day safety follow-up period. Subjects who complete the treatment period will be offered the option to enroll in an open-label extension trial. Patients who choose not to enter the open label extension trial will be followed for a 20-week safety follow-up period.

An objective of the trial is to determine pharmacokinetic and pharmacodynamic parameters in type 1 SMA subjects. This objective will be evaluated by measuring PK parameters, including Cₘᵢₙ, Cₘₐₓ, Tₘₐₓ, AUC, t_{1/2}, clearance, and volume of distribution; and by measuring PD parameters, including latent myostatin serum concentration. Measurements of apitegromab concentrations and latent myostatin serum concentrations will inform population PK and PK/PD modeling in type 1 SMA, including extrapolation from type 2 SMA to type 1 SMA.

Rather than using conventional allometric scaling approaches, i.e., the model frequently applied to extrapolate adult PK data to a pediatric population (Malik et al. (2021) J Clin Pharmacol 61 Suppl 1:S193-S206), the model will be modified to account for the effect of the subject's maturation on the clearance previously observed for monoclonal antibodies (Robbie et al. (2012) Antimicrob Agents Chemother 56(9):4927-36).

An objective of the trial is to evaluate the relationship between serum exposure of apitegromab at high, medium, and low doses with an efficacy readout, e.g., the Hammersmith Infant Neurological Exam motor milestones (HINE)

An objective of the trial is to assess the safety and tolerability of apitegromab in symptomatic subjects with type 1 SMA. This objective will be evaluated by measuring the incidence of treatment-emergent adverse events (TEAE) and serious adverse events (SAE). Subjects will be monitored throughout the trial for safety.

An objective of the trial is to assess the efficacy of apitegromab at high dose compared to low dose. This objective will be evaluated by measuring the change from baseline in the HINE. In an embodiment, change from baseline in the HINE, HINE-1, or HINE-2 score will be compared between the high dose score and the low dose score at designated time points, including but not limited to 52 weeks. In an embodiment, change from baseline in the CHOP-INTEND score will be compared between the high dose score and the low dose score at designated time points, including but not limited to 52 weeks. In an embodiment, change from baseline in the BSID-IV score will be compared between the high dose score and the low dose score at designated time points, including but not limited to 52 weeks.

In an embodiment, the efficacy of apitegromab at high dose compared to low dose will be evaluated by comparing the proportion of subjects achieving at least a 4-point improvement from baseline in the CHOP-INTEND score at designated time points, including but not limited to 52 weeks. In an embodiment, the efficacy of apitegromab at high dose compared to low dose will be evaluated by comparing the proportion of subjects achieving a score of at least 70 in the BSID-IV Motor Subscale at designated time points, including but not limited to 52 weeks. In an embodiment, the efficacy of apitegromab at high dose compared to low dose will be evaluated by comparing the proportion of subjects who can sit without support for 30 seconds at designated time points, including but not limited to 52 weeks.

An objective of this trial is to evaluate the motor and QoL endpoints set forth above with respect to the response achieved with the concomitant SMN therapy, e.g., nusinersen, risdiplam, or onasemnogene abeparvovec-xioi.

An objective of this trial is to further evaluate the immunogenicity of apitegromab. This objective will be evaluated by measuring the presence or absence of neutralizing antidrug antibody against apitegromab in serum from subject's blood samples.

An objective of this trial is to evaluate the effect of low dose, medium dose, and high dose apitegromab on patient/caregiver-reported disability, fatiguability, and survival. This objective will be evaluated by administering the Patient/Caregiver Global Impression of Change and determining the proportion of patients with event-free survival. In an embodiment, patients with event-free survival are alive and do not require at least 16 hours of ventilation per day continuously for more than 21 days in the absence of an acute reversible event or tracheostomy.

An objective of this trial is to evaluate effect of the high-dose apitegromab and low-dose apitegromab on gross motor functions. This objective will be evaluated by administering the Gross Motor Outcome (GMO) test and comparing the GMO score in subjects receiving the high dose to subjects receiving the low dose at designated time points, including but not limited to 52 weeks.

In an embodiment, subjects will have been treated or are being treated with an SMN therapy and may have variable baseline functioning and variable response to SMN therapy.

In an embodiment subjects who have been or are being treated with an SMN therapy have stabilized with respect to one or more functional outcomes, further allowing for the evaluation of the effect of apitegromab.

In an embodiment, endpoints in this severely affected population will include responder rates in the ability to sit, decreased need for ventilation, or survival.

In an embodiment, subjects have been treated or are being treated with an SMN therapy and demonstrate impaired bulbar function. In an embodiment, the oral intake of the subject will be evaluated on the p-FIOS scale.

In an embodiment, subjects will have a CHOP-INTEND baseline score of ≤40 and have reached a plateau in response to an SMN therapy after an initial gain in function from the SMN therapy.

### Example 6. Effect of Apitegromab on Serum Creatinine

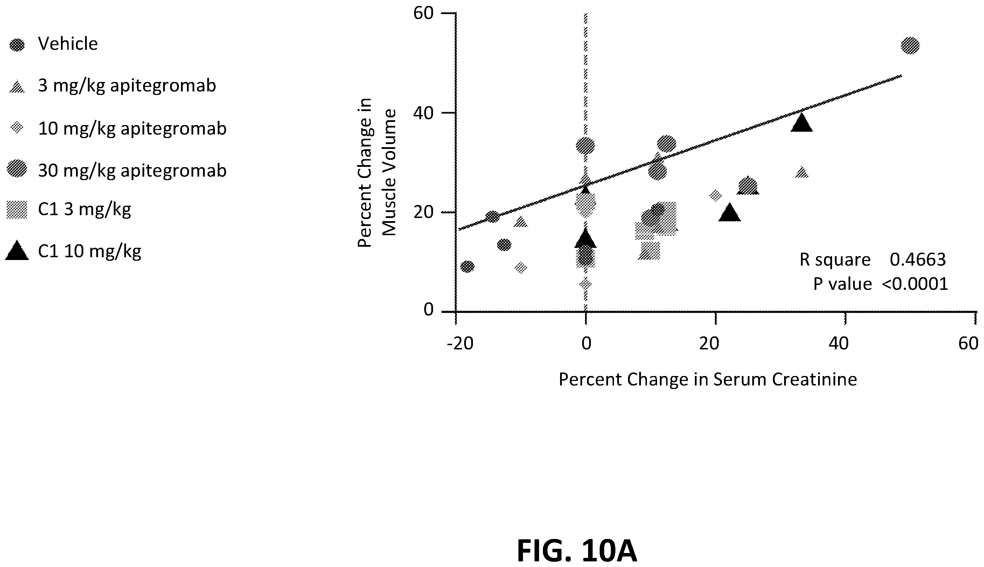

The effect of apitegromab on muscle volume was examined in non-human primates and the results are shown in **FIG. 10A****.** The animals were injected intravenously with apitegromab at 3 mg/kg, 10 mg/kg or 30 mg/kg or a control antibody (C1) at 3 mg/kg or 10 mg/kg once per week for eight weeks, followed by a four-week washout period. Muscle volume was measured by magnetic resonance imaging (MRI) of the vastus lateralis muscle and serum creatinine was measured by an enzymatic reaction performed in a conventional clinical laboratory analysis. The percent change in muscle volume linearly correlated with the percent change in serum creatinine (p<0.0001), as shown in **FIG. 10A****.**

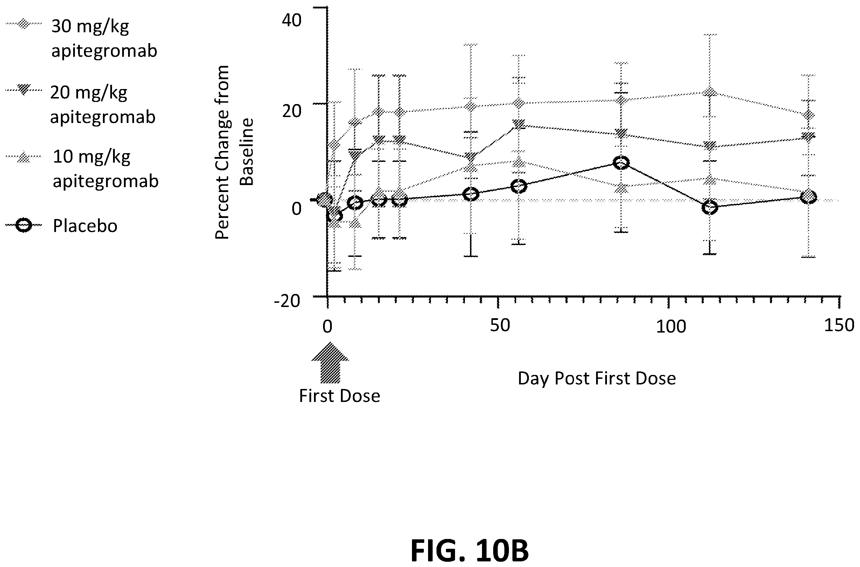

The effect of apitegromab on serum creatinine was also examined in healthy human volunteers. The subjects received apitegromab (n=6) or placebo (n=16) once per week for three weeks, followed by a period of drug washout. The change from baseline in serum creatinine was determined and the average percent change over time was determined. Serum creatinine levels increased in a dose dependent manner during the first two to three weeks of dosing, followed by a plateau, which was observed through the remainder of the study **(****FIG. 10B****).**

The effect of apitegromab on serum creatinine coincided with the pharmacokinetics observed with apitegromab, and it also correlated with the biomarker latent myostatin, which indicates the degree to which apitegromab engages with its target antigen (Barrett et al. Adv Ther. 221; 38:3203-3222).

## Claims

1. Apitegromab for use in the treatment of fatigue, impaired bulbar function and/or impaired emptying in a subject having spinal muscular atrophy (SMA) and who receives an SMN therapy, wherein the subject suffers from fatigue, impaired bulbar function and/or impaired emptying as measured by subject or caregiver reported outcomes.

2. Apitegromab for use according to claim 1, wherein the subject's level of fatigue is assessed using the Patient-reported Outcomes Measurement Information System (PROMIS).

3. Apitegromab for use according to any preceding claim wherein the impaired bulbar function is difficulty coughing, swallowing and/or feeding.

4. Apitegromab for use according to any preceding claim wherein the subject has symptoms of impaired bulbar function selected from respiratory infection, use of assistive device; inability to cough, clear lung secretions; breathing difficulties; feeding/swallowing difficulties; difficulties with speech; and snoring.

5. Apitegromab for use according to any preceding claim wherein the impaired bulbar function is assessed using the Amyotrophic Lateral Sclerosis Functional Rating Scale Revised (AMLFRSR), the Sydney Swallow Questionnaire (SSQ), the MD Anderson Dysphagia Inventory (MDADI), the SWAL-QoL score or the Pediatric Functional Oral Intake Scale (p-FOIS).

6. Apitegromab for use according to any preceding claim wherein the impaired emptying is urgency and frequency of urination and/or bowel movement.

7. Apitegromab for use according to any preceding claim wherein the subject is prone to respiratory infections.

8. Apitegromab for use according to any preceding claim, wherein the subject has type 2 SMA or type 3 SMA.

9. Apitegromab for use according to any preceding claim, wherein the subject has 2 copies, 3 copies, 4 copies or 5 copies of the smn2 gene.

10. Apitegromab for use according to any preceding claim, wherein the subject has ambulatory SMA or non-ambulatory SMA.

11. Apitegromab for use according to any preceding claim, wherein the subject is 2 years of age or older at the time of starting the administration of apitegromab.

12. Apitegromab for use according to claim 11, wherein the subject is 2-13 years of age.

13. Apitegromab for use according to claim 11, wherein the subject is 2-21 years of age.

14. Apitegromab for use according to any preceding claim, wherein the SMN therapy is a smn2 splice modifier or a smn1 gene therapy.

## Patentansprüche

1. Apitegromab zur Verwendung bei der Behandlung von Ermüdung, einer beeinträchtigten bulbären Funktion und/oder gestörten Entleerung bei einem Individuum mit spinaler Muskelatrophie (SMA) und das eine SMN-Therapie erhält, wobei das Individuum an Ermüdung, einer beeinträchtigten bulbären Funktion und/oder gestörten Entleerung leidet, die mittels vom Individuum oder Betreuer berichteten Ergebnissen gemessen werden.

2. Apitegromab zur Verwendung nach Anspruch 1, wobei der Ermüdungsgrad des Individuums unter Verwendung des Patient-reported Outcomes Measurement Information System (PROMIS) beurteilt wird.

3. Apitegromab zur Verwendung nach einem der vorhergehenden Ansprüche, wobei die beeinträchtigte bulbäre Funktion in Schwierigkeiten beim Husten, Schlucken und/oder bei der Nahrungsaufnahme besteht.

4. Apitegromab zur Verwendung nach einem der vorhergehenden Ansprüche, wobei das Individuum Symptome einer beeinträchtigten bulbären Funktion aufweist, die ausgewählt sind aus einer Atemwegsinfektion, Verwendung eines Hilfsmittels; einer Unfähigkeit zu Husten, zum Befreien der Lunge von Sekreten; Atembeschwerden; Schwierigkeiten bei der Nahrungsaufnahme/Schluckschwierigkeiten; Schwierigkeiten beim Sprechen und einem Schnarchen.

5. Apitegromab zur Verwendung nach einem der vorhergehenden Ansprüche, wobei die beeinträchtigte bulbäre Funktion unter Verwendung der Amyotrophic Lateral Sclerosis Functional Rating Scale Revised (AMLFRSR), des Sydney Swallow Questionnaire (SSQ), des MD Anderson Dysphagia Inventory (MDADI), des SWAL-QoL Score oder der Pediatric Functional Oral Intake Scale (p-FOIS) beurteilt wird.

6. Apitegromab zur Verwendung nach einem der vorhergehenden Ansprüche, wobei es sich bei der gestörten Entleerung um Harndrang und die Häufigkeit des Urinierens und/oder der Darmentleerung handelt.

7. Apitegromab zur Verwendung nach einem der vorhergehenden Ansprüche, wobei das Individuum für Atemwegsinfektionen anfällig ist.

8. Apitegromab zur Verwendung nach einem der vorhergehenden Ansprüche, wobei das Individuum eine SMA Typ 2 oder eine SMA Typ 3 aufweist.

9. Apitegromab zur Verwendung nach einem der vorhergehenden Ansprüche, wobei das Individuum 2 Kopien, 3 Kopien, 4 Kopien oder 5 Kopien des smn2-Gens aufweist.

10. Apitegromab zur Verwendung nach einem der vorhergehenden Ansprüche, wobei das Individuum eine ambulante SMA oder eine nicht-ambulante SMA aufweist.

11. Apitegromab zur Verwendung nach einem der vorhergehenden Ansprüche, wobei das Individuum zum Zeitpunkt des Beginns der Verabreichung von Apitegromab 2 Jahre oder älter ist.

12. Apitegromab zur Verwendung nach Anspruch 11, wobei das Individuum 2-13 Jahre alt ist.

13. Apitegromab zur Verwendung nach Anspruch 11, wobei das Individuum 2-21 Jahre alt ist.

14. Apitegromab zur Verwendung nach einem der vorhergehenden Ansprüche, wobei es sich bei der SMN-Therapie um einen smn2-Spleiß-Modifikator oder eine smn1-Gentherapie handelt.

## Revendications

1. Apitegromab destiné à être utilisé dans le traitement de la fatigue, de l'altération de la fonction bulbaire et/ou d'altération de la vidange chez un sujet atteint d'atrophie musculaire spinale (SMA) et qui reçoit une thérapie SMN, dans lequel le sujet souffre de fatigue, d'altération de la fonction bulbaire et/ou d'altération de la vidange telle que mesurée par des résultats rapportés par le sujet ou le soignant.

2. Apitegromab destiné à être utilisé selon la revendication 1, dans lequel le niveau de fatigue du sujet est évalué en utilisant le système d'information de mesure des résultats rapportés par le patient (PROMIS).

3. Apitegromab destiné à être utilisé selon l'une quelconque des revendications précédentes, dans lequel l'altération de la fonction bulbaire est la difficulté à tousser, à avaler et/ou à se nourrir.

4. Apitegromab destiné à être utilisé selon l'une quelconque des revendications précédentes, dans lequel le sujet a des symptômes d'une altération de la fonction bulbaire choisis parmi une infection respiratoire, l'utilisation de dispositif fonctionnel ; l'incapacité à tousser, effacer les sécrétions pulmonaires ; des difficultés respiratoires ; des difficultés à se nourrir/avaler ; des difficultés de parole ; et un ronflement.

5. Apitegromab destiné à être utilisé selon l'une quelconque des revendications précédentes, dans lequel l'altération de la fonction bulbaire est évaluée en utilisant l'échelle fonctionnelle d'évaluation de la sclérose latérale amyotrophique révisée (AMLFRSR), le Sydney Swallow Questionnaire (SSQ), l'inventaire de dysphagie MD Anderson (MDADI), le score SWAL-QoL ou l'échelle d'apport oral fonctionnel pédiatrique (p-FOIS) .

6. Apitegromab destiné à être utilisé selon l'une quelconque des revendications précédentes, dans lequel l'altération de la vidange est l'urgence et la fréquence de miction et/ou de mouvement intestinal.

7. Apitegromab destiné à être utilisé selon l'une quelconque des revendications précédentes, dans lequel le sujet est sujet à des infections respiratoires.

8. Apitegromab destiné à être utilisé selon l'une quelconque des revendications précédentes, dans lequel le sujet a une SMA de type 2 ou une SMA de type 3.

9. Apitegromab destiné à être utilisé selon l'une quelconque des revendications précédentes, dans lequel le sujet a 2 copies, 3 copies, 4 copies ou 5 copies du gène smn2.

10. Apitegromab destiné à être utilisé selon l'une quelconque des revendications précédentes, dans lequel le sujet a une SMA ambulatoire ou une SMA non ambulatoire.

11. Apitegromab destiné à être utilisé selon l'une quelconque des revendications précédentes, dans lequel le sujet est âgé de 2 ans ou plus au moment du début de l'administration d'apitegromab.

12. Apitegromab destiné à être utilisé selon la revendication 11, dans lequel le sujet est âgé de 2-13 ans.

13. Apitegromab destiné à être utilisé selon la revendication 11, dans lequel le sujet est âgé de 2-21 ans.

14. Apitegromab destiné à être utilisé selon l'une quelconque des revendications précédentes, dans lequel la thérapie SMN est un modificateur d'épissage smn2 ou une thérapie génique smn1.
